(19) Europäisches Patentamt
European Patent Office
Office européen des brevets

(11) EP 4 071 166 A1

(12) EUROPEAN PATENT APPLICATION
published in accordance with Art. 153(4) EPC

(43) Date of publication:
**12.10.2022 Bulletin 2022/41**

(21) Application number: **20900221.1**

(22) Date of filing: **10.12.2020**

(51) International Patent Classification (IPC):
***C07K 14/475** (2006.01)* ***A61K 38/18** (2006.01)*
***A61K 47/68** (2017.01)*

(52) Cooperative Patent Classification (CPC):
**A61K 38/18; A61K 47/68; C07K 14/475**

(86) International application number:
**PCT/KR2020/018053**

(87) International publication number:
**WO 2021/118256 (17.06.2021 Gazette 2021/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.12.2019 KR 20190165052**

(71) Applicant: **Lg Chem, Ltd.**
**Seoul 07336 (KR)**

(72) Inventors:
- **KIM, Yeonchul**
  **Daejeon 34122 (KR)**
- **SON, Young Dok**
  **Daejeon 34122 (KR)**
- **NA, Kyubong**
  **Daejeon 34122 (KR)**
- **HONG, Ji Ho**
  **Daejeon 34122 (KR)**
- **JUNG, Saem**
  **Daejeon 34122 (KR)**
- **JIN, Myung Won**
  **Daejeon 34122 (KR)**
- **PARK, Ji A**
  **Daejeon 34122 (KR)**
- **NOH, Soomin**
  **Daejeon 34122 (KR)**
- **PARK, Hyuntaek**
  **Daejeon 34122 (KR)**

(74) Representative: **Hoffmann Eitle**
**Patent- und Rechtsanwälte PartmbB**
**Arabellastraße 30**
**81925 München (DE)**

# (54) FUSION POLYPEPTIDE COMPRISING GDF15 AND POLYPEPTIDE REGION CAPABLE OF O-GLYCOSYLATION

(57) Disclosed are: a fusion polypeptide comprising growth differentiation factor 15 (GDF15) and a polypeptide region capable of O-glycosylation; a pharmaceutical composition comprising the fusion polypeptide; and a method for increasing the in vivo duration of GDF15, comprising the step of fusing a polypeptide region capable of O-glycosylation.

【FIG. 2b】

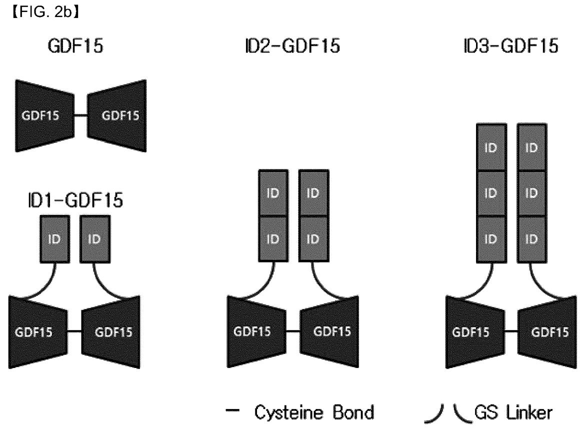


ID: ESPKAQASSVPTAQPQAEGSLAKATTAPATT RNT (SEQ ID NO: 1)
GS Linker: GGGGSGGGGS GGGGSGGGGS (SEQ ID NO: 17)
GDF15: SEQ ID NO: 3

EP 4 071 166 A1

<110> LG CHEM, LTD.

<120> FUSION POLYPEPTIDE COMPRISING O-GLYCOSYLATED POLYPEPTIDE REGION AND GDF15

<130> OPP20204754KR

<150> KR 10-2019-0165052
<151> 2019-12-11

<160> 113

<170> koPatentIn 3.0

<210> 1
<211> 34
<212> PRT
<213> Artificial Sequence

<220>
<223> Human IgD hinge region (ID1)

<400> 1
Glu Ser Pro Lys Ala Gln Ala Ser Ser Val Pro Thr Ala Gln Pro Gln
1 5 10 15

Ala Glu Gly Ser Leu Ala Lys Ala Thr Thr Ala Pro Ala Thr Thr Arg
20 25 30

Asn Thr

<210> 2
<211> 19
<212> PRT
<213> Artificial Sequence

<220>
<223> Human IgA1 hinge region

<400> 2
Val Pro Ser Thr Pro Pro Thr Pro Ser Pro Ser Thr Pro Pro Thr Pro
1 5 10 15

Ser Pro Ser

<210> 3
<211> 112
<212> PRT
<213> Artificial Sequence

<220>
<223> mature GDF15 (wild type)

<400> 3
Ala Arg Asn Gly Asp His Cys Pro Leu Gly Pro Gly Arg Cys Cys Arg
1 5 10 15

```
Leu His Thr Val Arg Ala Ser Leu Glu Asp Leu Gly Trp Ala Asp Trp
            20                  25                  30

Val Leu Ser Pro Arg Glu Val Gln Val Thr Met Cys Ile Gly Ala Cys
        35                  40                  45

Pro Ser Gln Phe Arg Ala Ala Asn Met His Ala Gln Ile Lys Thr Ser
    50                  55                  60

Leu His Arg Leu Lys Pro Asp Thr Val Pro Ala Pro Cys Cys Val Pro
65                  70                  75                  80

Ala Ser Tyr Asn Pro Met Val Leu Ile Gln Lys Thr Asp Thr Gly Val
                85                  90                  95

Ser Leu Gln Thr Tyr Asp Asp Leu Leu Ala Lys Asp Cys His Cys Ile
            100                 105                 110



<210>   4
<211>   98
<212>   PRT
<213>   Artificial Sequence

<220>
<223>   GDF15 (mutant)


<400>   4
Cys Arg Leu His Thr Val Arg Ala Ser Leu Glu Asp Leu Gly Trp Ala
1               5                   10                  15

Asp Trp Val Leu Ser Pro Arg Glu Val Gln Val Thr Met Cys Ile Gly
            20                  25                  30

Ala Cys Pro Ser Gln Phe Arg Ala Ala Asn Met His Ala Gln Ile Lys
        35                  40                  45

Thr Ser Leu His Arg Leu Lys Pro Asp Thr Val Pro Ala Pro Cys Cys
    50                  55                  60

Val Pro Ala Ser Tyr Asn Pro Met Val Leu Ile Gln Lys Thr Asp Thr
65                  70                  75                  80

Gly Val Ser Leu Gln Thr Tyr Asp Asp Leu Leu Ala Lys Asp Cys His
                85                  90                  95

Cys Ile



<210>   5
<211>   339
<212>   DNA
<213>   Artificial Sequence

<220>
<223>   Polynucleotide encoding Mature GDF15
```

<400> 5
gcccggaacg gcgaccactg ccccctgggg cccggacggt gctgccggct gcacaccgtg 60

cgggcctccc tggaggacct gggctgggcc gactgggtgc tgtccccaag ggaggtgcaa 120

gtgaccatgt gcatcggcgc ctgcccatct cagttccggg ccgccaacat gcacgctcag 180

atcaagacca gcctgcaccg gctgaagccc gacaccgtgc ccgccccctg ctgcgtgccc 240

gcctcctaca accccatggt gctgattcag aagaccgaca ccggcgtgag cctgcagacc 300

tacgacgacc tgctggccaa ggactgccac tgcatctaa 339

<210> 6
<211> 306
<212> DNA
<213> Artificial Sequence

<220>
<223> Polynucleotide encoding 3 human IgD Hinge regions (ID3)

<400> 6
gagagcccta aggctcaggc ctctagcgtg ccaacagctc agccacaagc tgaaggaagc 60

ctggccaagg ctacaaccgc ccctgccaca acacggaata cagagtcccc caaggcccag 120

gctagcagcg tgcctaccgc ccagcctcag gccgagggct ccctggctaa ggccacaacc 180

gctcccgcta caaccaggaa caccgagtct ccaaaggcac aggcctcctc cgtgcccact 240

gcacaacccc aagcagaggg cagcctcgcc aaggcaacca cagccccagc caccacccgg 300

aacaca 306

<210> 7
<211> 384
<212> PRT
<213> Artificial Sequence

<220>
<223> Human IgD heavy chain constant region (UniProtKB P01880)

<400> 7
Ala Pro Thr Lys Ala Pro Asp Val Phe Pro Ile Ile Ser Gly Cys Arg
1 5 10 15

His Pro Lys Asp Asn Ser Pro Val Val Leu Ala Cys Leu Ile Thr Gly
20 25 30

Tyr His Pro Thr Ser Val Thr Val Thr Trp Tyr Met Gly Thr Gln Ser
35 40 45

Gln Pro Gln Arg Thr Phe Pro Glu Ile Gln Arg Arg Asp Ser Tyr Tyr
50 55 60

Met Thr Ser Ser Gln Leu Ser Thr Pro Leu Gln Gln Trp Arg Gln Gly
65 70 75 80

Glu Tyr Lys Cys Val Val Gln His Thr Ala Ser Lys Ser Lys Lys Glu

85 90 95

Ile Phe Arg Trp Pro Glu Ser Pro Lys Ala Gln Ala Ser Ser Val Pro
100 105 110

Thr Ala Gln Pro Gln Ala Glu Gly Ser Leu Ala Lys Ala Thr Thr Ala
115 120 125

Pro Ala Thr Thr Arg Asn Thr Gly Arg Gly Gly Glu Glu Lys Lys Lys
130 135 140

Glu Lys Glu Lys Glu Glu Gln Glu Glu Arg Glu Thr Lys Thr Pro Glu
145 150 155 160

Cys Pro Ser His Thr Gln Pro Leu Gly Val Tyr Leu Leu Thr Pro Ala
165 170 175

Val Gln Asp Leu Trp Leu Arg Asp Lys Ala Thr Phe Thr Cys Phe Val
180 185 190

Val Gly Ser Asp Leu Lys Asp Ala His Leu Thr Trp Glu Val Ala Gly
195 200 205

Lys Val Pro Thr Gly Gly Val Glu Glu Gly Leu Leu Glu Arg His Ser
210 215 220

Asn Gly Ser Gln Ser Gln His Ser Arg Leu Thr Leu Pro Arg Ser Leu
225 230 235 240

Trp Asn Ala Gly Thr Ser Val Thr Cys Thr Leu Asn His Pro Ser Leu
245 250 255

Pro Pro Gln Arg Leu Met Ala Leu Arg Glu Pro Ala Ala Gln Ala Pro
260 265 270

Val Lys Leu Ser Leu Asn Leu Leu Ala Ser Ser Asp Pro Pro Glu Ala
275 280 285

Ala Ser Trp Leu Leu Cys Glu Val Ser Gly Phe Ser Pro Pro Asn Ile
290 295 300

Leu Leu Met Trp Leu Glu Asp Gln Arg Glu Val Asn Thr Ser Gly Phe
305 310 315 320

Ala Pro Ala Arg Pro Pro Pro Gln Pro Arg Ser Thr Thr Phe Trp Ala
325 330 335

Trp Ser Val Leu Arg Val Pro Ala Pro Pro Ser Pro Gln Pro Ala Thr
340 345 350

Tyr Thr Cys Val Val Ser His Glu Asp Ser Arg Thr Leu Leu Asn Ala
355 360 365

Ser Arg Ser Leu Glu Val Ser Tyr Val Thr Asp His Gly Pro Met Lys
370 375 380

<210> 8
<211> 353
<212> PRT
<213> Artificial Sequence

<220>
<223> Human IgA heavy chain constant region (UniProtKB P01876)

<400> 8
Ala Ser Pro Thr Ser Pro Lys Val Phe Pro Leu Ser Leu Cys Ser Thr
1 5 10 15

Gln Pro Asp Gly Asn Val Val Ile Ala Cys Leu Val Gln Gly Phe Phe
20 25 30

Pro Gln Glu Pro Leu Ser Val Thr Trp Ser Glu Ser Gly Gln Gly Val
35 40 45

Thr Ala Arg Asn Phe Pro Pro Ser Gln Asp Ala Ser Gly Asp Leu Tyr
50 55 60

Thr Thr Ser Ser Gln Leu Thr Leu Pro Ala Thr Gln Cys Leu Ala Gly
65 70 75 80

Lys Ser Val Thr Cys His Val Lys His Tyr Thr Asn Pro Ser Gln Asp
85 90 95

Val Thr Val Pro Cys Pro Val Pro Ser Thr Pro Pro Thr Pro Ser Pro
100 105 110

Ser Thr Pro Pro Thr Pro Ser Pro Ser Cys Cys His Pro Arg Leu Ser
115 120 125

Leu His Arg Pro Ala Leu Glu Asp Leu Leu Leu Gly Ser Glu Ala Asn
130 135 140

Leu Thr Cys Thr Leu Thr Gly Leu Arg Asp Ala Ser Gly Val Thr Phe
145 150 155 160

Thr Trp Thr Pro Ser Ser Gly Lys Ser Ala Val Gln Gly Pro Pro Glu
165 170 175

Arg Asp Leu Cys Gly Cys Tyr Ser Val Ser Ser Val Leu Pro Gly Cys
180 185 190

Ala Glu Pro Trp Asn His Gly Lys Thr Phe Thr Cys Thr Ala Ala Tyr
195 200 205

Pro Glu Ser Lys Thr Pro Leu Thr Ala Thr Leu Ser Lys Ser Gly Asn
210 215 220

Thr Phe Arg Pro Glu Val His Leu Leu Pro Pro Pro Ser Glu Glu Leu
225 230 235 240

Ala Leu Asn Glu Leu Val Thr Leu Thr Cys Leu Ala Arg Gly Phe Ser
245 250 255

Pro Lys Asp Val Leu Val Arg Trp Leu Gln Gly Ser Gln Glu Leu Pro
260 265 270

Arg Glu Lys Tyr Leu Thr Trp Ala Ser Arg Gln Glu Pro Ser Gln Gly
275 280 285

Thr Thr Thr Phe Ala Val Thr Ser Ile Leu Arg Val Ala Ala Glu Asp
290 295 300

```
Trp Lys Lys Gly Asp Thr Phe Ser Cys Met Val Gly His Glu Ala Leu
305                 310                 315                 320

Pro Leu Ala Phe Thr Gln Lys Thr Ile Asp Arg Leu Ala Gly Lys Pro
                325                 330                 335

Thr His Val Asn Val Ser Val Val Met Ala Glu Val Asp Gly Thr Cys
            340                 345                 350

Tyr



<210>   9
<211>   5
<212>   PRT
<213>   Artificial Sequence

<220>
<223>   a part of human IgD hinge region


<400>   9
Ser Ser Val Pro Thr
  1               5


<210>   10
<211>   7
<212>   PRT
<213>   Artificial Sequence

<220>
<223>   a part of human IgD hinge region


<400>   10
Thr Thr Ala Pro Ala Thr Thr
  1               5


<210>   11
<211>   58
<212>   PRT
<213>   Artificial Sequence

<220>
<223>   a extended part of human IgD hinge region


<400>   11
Glu Ser Pro Lys Ala Gln Ala Ser Ser Val Pro Thr Ala Gln Pro Gln
  1               5                   10                  15

Ala Glu Gly Ser Leu Ala Lys Ala Thr Thr Ala Pro Ala Thr Thr Arg
             20                  25                  30

Asn Thr Gly Arg Gly Gly Glu Glu Lys Lys Lys Glu Lys Glu Lys Glu
         35                  40                  45

Glu Gln Glu Glu Arg Glu Thr Lys Thr Pro
     50                  55
```

<210> 12
<211> 8
<212> PRT
<213> Artificial Sequence

<220>
<223> a part of human IgA1 hinge region

<400> 12
Ser Thr Pro Pro Thr Pro Ser Pro
1 5

<210> 13
<211> 5
<212> PRT
<213> Artificial Sequence

<220>
<223> peptide linker

<400> 13
Gly Gly Gly Gly Ser
1 5

<210> 14
<211> 22
<212> PRT
<213> Artificial Sequence

<220>
<223> Signal Peptide

<400> 14
Met His Arg Pro Glu Ala Met Leu Leu Leu Leu Thr Leu Ala Leu Leu
1 5 10 15

Gly Gly Pro Thr Trp Ala
20

<210> 15
<211> 8
<212> PRT
<213> Artificial Sequence

<220>
<223> His-Tag

<400> 15
His His His His His His His His
1 5

<210> 16
<211> 7
<212> PRT

<213> Artificial Sequence

<220>
<223> TEV Cleavage Site

<400> 16
Glu Asn Leu Tyr Phe Gln Gly
1 5

<210> 17
<211> 20
<212> PRT
<213> Artificial Sequence

<220>
<223> GS linker

<400> 17
Gly Gly Gly Gly Ser Gly Gly Gly Gly Ser Gly Gly Gly Gly Ser Gly
1 5 10 15

Gly Gly Gly Ser
20

<210> 18
<211> 68
<212> PRT
<213> Artificial Sequence

<220>
<223> 2 human IgD hinge regions (ID2)

<400> 18
Glu Ser Pro Lys Ala Gln Ala Ser Ser Val Pro Thr Ala Gln Pro Gln
1 5 10 15

Ala Glu Gly Ser Leu Ala Lys Ala Thr Thr Ala Pro Ala Thr Thr Arg
20 25 30

Asn Thr Glu Ser Pro Lys Ala Gln Ala Ser Ser Val Pro Thr Ala Gln
35 40 45

Pro Gln Ala Glu Gly Ser Leu Ala Lys Ala Thr Thr Ala Pro Ala Thr
50 55 60

Thr Arg Asn Thr
65

<210> 19
<211> 102
<212> PRT
<213> Artificial Sequence

<220>
<223> 3 human IgD hinge regions (ID3)

<400> 19
Glu Ser Pro Lys Ala Gln Ala Ser Ser Val Pro Thr Ala Gln Pro Gln
1 5 10 15

Ala Glu Gly Ser Leu Ala Lys Ala Thr Thr Ala Pro Ala Thr Thr Arg
20 25 30

Asn Thr Glu Ser Pro Lys Ala Gln Ala Ser Ser Val Pro Thr Ala Gln
35 40 45

Pro Gln Ala Glu Gly Ser Leu Ala Lys Ala Thr Thr Ala Pro Ala Thr
50 55 60

Thr Arg Asn Thr Glu Ser Pro Lys Ala Gln Ala Ser Ser Val Pro Thr
65 70 75 80

Ala Gln Pro Gln Ala Glu Gly Ser Leu Ala Lys Ala Thr Thr Ala Pro
85 90 95

Ala Thr Thr Arg Asn Thr
100

<210> 20
<211> 8
<212> PRT
<213> Artificial Sequence

<220>
<223> a part of human IgA hinge region

<400> 20
Ser Thr Pro Pro Thr Pro Ser Pro
1 5

<210> 21
<211> 102
<212> DNA
<213> Artificial Sequence

<220>
<223> Polynucleotide encoding human IgD hinge region (ID1)

<400> 21
gagagcccta aggctcaggc ctctagcgtg ccaacagctc agccacaagc tgaaggaagc 60

ctggccaagg ctacaaccgc ccctgccaca acacggaata ca 102

<210> 22
<211> 204
<212> DNA
<213> Artificial Sequence

<220>
<223> Polynucleotide encoding 2 human IgD hinge regions (ID2)

<400> 22
gagagcccta aggctcaggc ctctagcgtg ccaacagctc agccacaagc tgaaggaagc 60

ctggccaagg ctacaaccgc ccctgccaca acacggaata cagagtcccc caaggcccag 120

gctagcagcg tgcctaccgc ccagcctcag gccgagggct ccctggctaa ggccacaacc 180

gctcccgcta caaccaggaa cacc 204

<210> 23
<211> 90
<212> PRT
<213> Artificial Sequence

<220>
<223> MUCL1_HUMAN

<400> 23
Met Lys Phe Leu Ala Val Leu Val Leu Leu Gly Val Ser Ile Phe Leu
1 5 10 15

Val Ser Ala Gln Asn Pro Thr Thr Ala Ala Pro Ala Asp Thr Tyr Pro
20 25 30

Ala Thr Gly Pro Ala Asp Asp Glu Ala Pro Asp Ala Glu Thr Thr Ala
35 40 45

Ala Ala Thr Thr Ala Thr Thr Ala Ala Pro Thr Thr Ala Thr Thr Ala
50 55 60

Ala Ser Thr Thr Ala Arg Lys Asp Ile Pro Val Leu Pro Lys Trp Val
65 70 75 80

Gly Asp Leu Pro Asn Gly Arg Val Cys Pro
85 90

<210> 24
<211> 97
<212> PRT
<213> Artificial Sequence

<220>
<223> SMAGP_HUMAN

<400> 24
Met Thr Ser Leu Leu Thr Thr Pro Ser Pro Arg Glu Glu Leu Met Thr
1 5 10 15

Thr Pro Ile Leu Gln Pro Thr Glu Ala Leu Ser Pro Glu Asp Gly Ala
20 25 30

Ser Thr Ala Leu Ile Ala Val Val Ile Thr Val Val Phe Leu Thr Leu
35 40 45

Leu Ser Val Val Ile Leu Ile Phe Phe Tyr Leu Tyr Lys Asn Lys Gly
50 55 60

Ser Tyr Val Thr Tyr Glu Pro Thr Glu Gly Glu Pro Ser Ala Ile Val
65 70 75 80

Gln Met Glu Ser Asp Leu Ala Lys Gly Ser Glu Lys Glu Glu Tyr Phe

85 90 95

Ile

<210> 25
<211> 128
<212> PRT
<213> Artificial Sequence

<220>
<223> GLPC_HUMAN

<400> 25
Met Trp Ser Thr Arg Ser Pro Asn Ser Thr Ala Trp Pro Leu Ser Leu
1 5 10 15

Glu Pro Asp Pro Gly Met Ala Ser Ala Ser Thr Thr Met His Thr Thr
20 25 30

Thr Ile Ala Glu Pro Asp Pro Gly Met Ser Gly Trp Pro Asp Gly Arg
35 40 45

Met Glu Thr Ser Thr Pro Thr Ile Met Asp Ile Val Val Ile Ala Gly
50 55 60

Val Ile Ala Ala Val Ala Ile Val Leu Val Ser Leu Leu Phe Val Met
65 70 75 80

Leu Arg Tyr Met Tyr Arg His Lys Gly Thr Tyr His Thr Asn Glu Ala
85 90 95

Lys Gly Thr Glu Phe Ala Glu Ser Ala Asp Ala Ala Leu Gln Gly Asp
100 105 110

Pro Ala Leu Gln Asp Ala Gly Asp Ser Ser Arg Lys Glu Tyr Phe Ile
115 120 125

<210> 26
<211> 134
<212> PRT
<213> Artificial Sequence

<220>
<223> ANFB_HUMAN

<400> 26
Met Asp Pro Gln Thr Ala Pro Ser Arg Ala Leu Leu Leu Leu Leu Phe
1 5 10 15

Leu His Leu Ala Phe Leu Gly Gly Arg Ser His Pro Leu Gly Ser Pro
20 25 30

Gly Ser Ala Ser Asp Leu Glu Thr Ser Gly Leu Gln Glu Gln Arg Asn
35 40 45

His Leu Gln Gly Lys Leu Ser Glu Leu Gln Val Glu Gln Thr Ser Leu

50 55 60

Glu Pro Leu Gln Glu Ser Pro Arg Pro Thr Gly Val Trp Lys Ser Arg
65 70 75 80

Glu Val Ala Thr Glu Gly Ile Arg Gly His Arg Lys Met Val Leu Tyr
85 90 95

Thr Leu Arg Ala Pro Arg Ser Pro Lys Met Val Gln Gly Ser Gly Cys
100 105 110

Phe Gly Arg Lys Met Asp Arg Ile Ser Ser Ser Ser Gly Leu Gly Cys
115 120 125

Lys Val Leu Arg Arg His
130

<210> 27
<211> 144
<212> PRT
<213> Artificial Sequence

<220>
<223> CSF2_HUMAN

<400> 27
Met Trp Leu Gln Ser Leu Leu Leu Leu Gly Thr Val Ala Cys Ser Ile
1 5 10 15

Ser Ala Pro Ala Arg Ser Pro Ser Pro Ser Thr Gln Pro Trp Glu His
20 25 30

Val Asn Ala Ile Gln Glu Ala Arg Arg Leu Leu Asn Leu Ser Arg Asp
35 40 45

Thr Ala Ala Glu Met Asn Glu Thr Val Glu Val Ile Ser Glu Met Phe
50 55 60

Asp Leu Gln Glu Pro Thr Cys Leu Gln Thr Arg Leu Glu Leu Tyr Lys
65 70 75 80

Gln Gly Leu Arg Gly Ser Leu Thr Lys Leu Lys Gly Pro Leu Thr Met
85 90 95

Met Ala Ser His Tyr Lys Gln His Cys Pro Pro Thr Pro Glu Thr Ser
100 105 110

Cys Ala Thr Gln Ile Ile Thr Phe Glu Ser Phe Lys Glu Asn Leu Lys
115 120 125

Asp Phe Leu Leu Val Ile Pro Phe Asp Cys Trp Glu Pro Val Gln Glu
130 135 140

<210> 28
<211> 150
<212> PRT
<213> Artificial Sequence

<220>
<223> GLPA_HUMAN

<400> 28
Met Tyr Gly Lys Ile Ile Phe Val Leu Leu Leu Ser Glu Ile Val Ser
1 5 10 15

Ile Ser Ala Ser Ser Thr Thr Gly Val Ala Met His Thr Ser Thr Ser
20 25 30

Ser Ser Val Thr Lys Ser Tyr Ile Ser Ser Gln Thr Asn Asp Thr His
35 40 45

Lys Arg Asp Thr Tyr Ala Ala Thr Pro Arg Ala His Glu Val Ser Glu
50 55 60

Ile Ser Val Arg Thr Val Tyr Pro Pro Glu Glu Glu Thr Gly Glu Arg
65 70 75 80

Val Gln Leu Ala His His Phe Ser Glu Pro Glu Ile Thr Leu Ile Ile
85 90 95

Phe Gly Val Met Ala Gly Val Ile Gly Thr Ile Leu Leu Ile Ser Tyr
100 105 110

Gly Ile Arg Arg Leu Ile Lys Lys Ser Pro Ser Asp Val Lys Pro Leu
115 120 125

Pro Ser Pro Asp Thr Asp Val Pro Leu Ser Ser Val Glu Ile Glu Asn
130 135 140

Pro Glu Thr Ser Asp Gln
145 150

<210> 29
<211> 158
<212> PRT
<213> Artificial Sequence

<220>
<223> SRGN_HUMAN

<400> 29
Met Met Gln Lys Leu Leu Lys Cys Ser Arg Leu Val Leu Ala Leu Ala
1 5 10 15

Leu Ile Leu Val Leu Glu Ser Ser Val Gln Gly Tyr Pro Thr Arg Arg
20 25 30

Ala Arg Tyr Gln Trp Val Arg Cys Asn Pro Asp Ser Asn Ser Ala Asn
35 40 45

Cys Leu Glu Glu Lys Gly Pro Met Phe Glu Leu Leu Pro Gly Glu Ser
50 55 60

Asn Lys Ile Pro Arg Leu Arg Thr Asp Leu Phe Pro Lys Thr Arg Ile
65 70 75 80

Gln Asp Leu Asn Arg Ile Phe Pro Leu Ser Glu Asp Tyr Ser Gly Ser
85 90 95

Gly Phe Gly Ser Gly Ser Gly Ser Gly Ser Gly Ser Gly Ser Gly Phe
100 105 110

Leu Thr Glu Met Glu Gln Asp Tyr Gln Leu Val Asp Glu Ser Asp Ala
115 120 125

Phe His Asp Asn Leu Arg Ser Leu Asp Arg Asn Leu Pro Ser Asp Ser
130 135 140

Gln Asp Leu Gly Gln His Gly Leu Glu Glu Asp Phe Met Leu
145 150 155

<210> 30
<211> 162
<212> PRT
<213> Artificial Sequence

<220>
<223> PDPN_HUMAN

<400> 30
Met Trp Lys Val Ser Ala Leu Leu Phe Val Leu Gly Ser Ala Ser Leu
1 5 10 15

Trp Val Leu Ala Glu Gly Ala Ser Thr Gly Gln Pro Glu Asp Asp Thr
20 25 30

Glu Thr Thr Gly Leu Glu Gly Gly Val Ala Met Pro Gly Ala Glu Asp
35 40 45

Asp Val Val Thr Pro Gly Thr Ser Glu Asp Arg Tyr Lys Ser Gly Leu
50 55 60

Thr Thr Leu Val Ala Thr Ser Val Asn Ser Val Thr Gly Ile Arg Ile
65 70 75 80

Glu Asp Leu Pro Thr Ser Glu Ser Thr Val His Ala Gln Glu Gln Ser
85 90 95

Pro Ser Ala Thr Ala Ser Asn Val Ala Thr Ser His Ser Thr Glu Lys
100 105 110

Val Asp Gly Asp Thr Gln Thr Thr Val Glu Lys Asp Gly Leu Ser Thr
115 120 125

Val Thr Leu Val Gly Ile Ile Val Gly Val Leu Leu Ala Ile Gly Phe
130 135 140

Ile Gly Ala Ile Ile Val Val Val Met Arg Lys Met Ser Gly Arg Tyr
145 150 155 160

Ser Pro

<210> 31
<211> 165
<212> PRT
<213> Artificial Sequence

<220>
<223> CGB7_HUMAN

<400> 31
Met Glu Met Phe Gln Gly Leu Leu Leu Leu Leu Leu Leu Ser Met Gly
1 5 10 15

Gly Thr Trp Ala Ser Arg Glu Met Leu Arg Pro Arg Cys Arg Pro Ile
20 25 30

Asn Ala Thr Leu Ala Val Glu Lys Glu Gly Cys Pro Val Cys Ile Thr
35 40 45

Val Asn Thr Thr Ile Cys Ala Gly Tyr Cys Pro Thr Met Thr Arg Val
50 55 60

Leu Gln Gly Val Leu Pro Ala Leu Pro Gln Val Val Cys Asn Tyr Arg
65 70 75 80

Asp Val Arg Phe Glu Ser Ile Arg Leu Pro Gly Cys Pro Arg Gly Val
85 90 95

Asn Pro Val Val Ser Tyr Ala Val Ala Leu Ser Cys Gln Cys Ala Leu
100 105 110

Cys Arg Arg Ser Thr Thr Asp Cys Gly Gly Pro Lys Asp His Pro Leu
115 120 125

Thr Cys Asp Asp Pro Arg Phe Gln Ala Ser Ser Ser Ser Lys Ala Pro
130 135 140

Pro Pro Ser Leu Pro Ser Pro Ser Arg Leu Pro Gly Pro Ser Asp Thr
145 150 155 160

Pro Ile Leu Pro Gln
165

<210> 32
<211> 165
<212> PRT
<213> Artificial Sequence

<220>
<223> CGB3_HUMAN

<400> 32
Met Glu Met Phe Gln Gly Leu Leu Leu Leu Leu Leu Leu Ser Met Gly
1 5 10 15

Gly Thr Trp Ala Ser Lys Glu Pro Leu Arg Pro Arg Cys Arg Pro Ile
20 25 30

Asn Ala Thr Leu Ala Val Glu Lys Glu Gly Cys Pro Val Cys Ile Thr
35 40 45

Val Asn Thr Thr Ile Cys Ala Gly Tyr Cys Pro Thr Met Thr Arg Val
50 55 60

Leu Gln Gly Val Leu Pro Ala Leu Pro Gln Val Val Cys Asn Tyr Arg
65 70 75 80

Asp Val Arg Phe Glu Ser Ile Arg Leu Pro Gly Cys Pro Arg Gly Val
85 90 95

Asn Pro Val Val Ser Tyr Ala Val Ala Leu Ser Cys Gln Cys Ala Leu
100 105 110

Cys Arg Arg Ser Thr Thr Asp Cys Gly Gly Pro Lys Asp His Pro Leu
115 120 125

Thr Cys Asp Asp Pro Arg Phe Gln Asp Ser Ser Ser Ser Lys Ala Pro
130 135 140

Pro Pro Ser Leu Pro Ser Pro Ser Arg Leu Pro Gly Pro Ser Asp Thr
145 150 155 160

Pro Ile Leu Pro Gln
165

<210> 33
<211> 180
<212> PRT
<213> Artificial Sequence

<220>
<223> IGF2_HUMAN

<400> 33
Met Gly Ile Pro Met Gly Lys Ser Met Leu Val Leu Leu Thr Phe Leu
1 5 10 15

Ala Phe Ala Ser Cys Cys Ile Ala Ala Tyr Arg Pro Ser Glu Thr Leu
20 25 30

Cys Gly Gly Glu Leu Val Asp Thr Leu Gln Phe Val Cys Gly Asp Arg
35 40 45

Gly Phe Tyr Phe Ser Arg Pro Ala Ser Arg Val Ser Arg Arg Ser Arg
50 55 60

Gly Ile Val Glu Glu Cys Cys Phe Arg Ser Cys Asp Leu Ala Leu Leu
65 70 75 80

Glu Thr Tyr Cys Ala Thr Pro Ala Lys Ser Glu Arg Asp Val Ser Thr
85 90 95

Pro Pro Thr Val Leu Pro Asp Asn Phe Pro Arg Tyr Pro Val Gly Lys
100 105 110

Phe Phe Gln Tyr Asp Thr Trp Lys Gln Ser Thr Gln Arg Leu Arg Arg
115 120 125

Gly Leu Pro Ala Leu Leu Arg Ala Arg Arg Gly His Val Leu Ala Lys
130 135 140

Glu Leu Glu Ala Phe Arg Glu Ala Lys Arg His Arg Pro Leu Ile Ala
145 150 155 160

Leu Pro Thr Gln Asp Pro Ala His Gly Gly Ala Pro Pro Glu Met Ala
165 170 175

Ser Asn Arg Lys
180

<210> 34
<211> 182
<212> PRT
<213> Artificial Sequence

<220>
<223> CASK_HUMAN

<400> 34
Met Lys Ser Phe Leu Leu Val Val Asn Ala Leu Ala Leu Thr Leu Pro
1 5 10 15

Phe Leu Ala Val Glu Val Gln Asn Gln Lys Gln Pro Ala Cys His Glu
20 25 30

Asn Asp Glu Arg Pro Phe Tyr Gln Lys Thr Ala Pro Tyr Val Pro Met
35 40 45

Tyr Tyr Val Pro Asn Ser Tyr Pro Tyr Tyr Gly Thr Asn Leu Tyr Gln
50 55 60

Arg Arg Pro Ala Ile Ala Ile Asn Asn Pro Tyr Val Pro Arg Thr Tyr
65 70 75 80

Tyr Ala Asn Pro Ala Val Val Arg Pro His Ala Gln Ile Pro Gln Arg
85 90 95

Gln Tyr Leu Pro Asn Ser His Pro Pro Thr Val Val Arg Arg Pro Asn
100 105 110

Leu His Pro Ser Phe Ile Ala Ile Pro Pro Lys Lys Ile Gln Asp Lys
115 120 125

Ile Ile Ile Pro Thr Ile Asn Thr Ile Ala Thr Val Glu Pro Thr Pro
130 135 140

Ala Pro Ala Thr Glu Pro Thr Val Asp Ser Val Val Thr Pro Glu Ala
145 150 155 160

Phe Ser Glu Ser Ile Ile Thr Ser Thr Pro Glu Thr Thr Thr Val Ala
165 170 175

Val Thr Pro Pro Thr Ala
180

<210> 35
<211> 198
<212> PRT
<213> Artificial Sequence

<220>
<223> SDC4_HUMAN

<400> 35
Met Ala Pro Ala Arg Leu Phe Ala Leu Leu Leu Phe Phe Val Gly Gly
1 5 10 15

Val Ala Glu Ser Ile Arg Glu Thr Glu Val Ile Asp Pro Gln Asp Leu
            20                  25                  30

Leu Glu Gly Arg Tyr Phe Ser Gly Ala Leu Pro Asp Asp Glu Asp Val
        35                  40                  45

Val Gly Pro Gly Gln Glu Ser Asp Asp Phe Glu Leu Ser Gly Ser Gly
    50                  55                  60

Asp Leu Asp Asp Leu Glu Asp Ser Met Ile Gly Pro Glu Val Val His
65                  70                  75                  80

Pro Leu Val Pro Leu Asp Asn His Ile Pro Glu Arg Ala Gly Ser Gly
                85                  90                  95

Ser Gln Val Pro Thr Glu Pro Lys Lys Leu Glu Glu Asn Glu Val Ile
            100                 105                 110

Pro Lys Arg Ile Ser Pro Val Glu Glu Ser Glu Asp Val Ser Asn Lys
        115                 120                 125

Val Ser Met Ser Ser Thr Val Gln Gly Ser Asn Ile Phe Glu Arg Thr
    130                 135                 140

Glu Val Leu Ala Ala Leu Ile Val Gly Gly Ile Val Gly Ile Leu Phe
145                 150                 155                 160

Ala Val Phe Leu Ile Leu Leu Leu Met Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210> 36
<211> 201
<212> PRT
<213> Artificial Sequence

<220>
<223> SDC2_HUMAN

<400> 36
Met Arg Arg Ala Trp Ile Leu Leu Thr Leu Gly Leu Val Ala Cys Val
1               5                   10                  15

Ser Ala Glu Ser Arg Ala Glu Leu Thr Ser Asp Lys Asp Met Tyr Leu
            20                  25                  30

Asp Asn Ser Ser Ile Glu Glu Ala Ser Gly Val Tyr Pro Ile Asp Asp
        35                  40                  45

Asp Asp Tyr Ala Ser Ala Ser Gly Ser Gly Ala Asp Glu Asp Val Glu
    50                  55                  60

Ser Pro Glu Leu Thr Thr Ser Arg Pro Leu Pro Lys Ile Leu Leu Thr
65                  70                  75                  80

```
Ser Ala Ala Pro Lys Val Glu Thr Thr Thr Leu Asn Ile Gln Asn Lys
                85                  90                  95

Ile Pro Ala Gln Thr Lys Ser Pro Glu Glu Thr Asp Lys Glu Lys Val
            100                 105                 110

His Leu Ser Asp Ser Glu Arg Lys Met Asp Pro Ala Glu Glu Asp Thr
        115                 120                 125

Asn Val Tyr Thr Glu Lys His Ser Asp Ser Leu Phe Lys Arg Thr Glu
    130                 135                 140

Val Leu Ala Ala Val Ile Ala Gly Gly Val Ile Gly Phe Leu Phe Ala
145                 150                 155                 160

Ile Phe Leu Ile Leu Leu Leu Val Tyr Arg Met Arg Lys Lys Asp Glu
                165                 170                 175

Gly Ser Tyr Asp Leu Gly Glu Arg Lys Pro Ser Ser Ala Ala Tyr Gln
            180                 185                 190

Lys Ala Pro Thr Lys Glu Phe Tyr Ala
        195                 200


<210>   37
<211>   208
<212>   PRT
<213>   Artificial Sequence

<220>
<223>   HBEGF_HUMAN


<400>   37
Met Lys Leu Leu Pro Ser Val Val Leu Lys Leu Phe Leu Ala Ala Val
 1               5                  10                  15

Leu Ser Ala Leu Val Thr Gly Glu Ser Leu Glu Arg Leu Arg Arg Gly
            20                  25                  30

Leu Ala Ala Gly Thr Ser Asn Pro Asp Pro Pro Thr Val Ser Thr Asp
        35                  40                  45

Gln Leu Leu Pro Leu Gly Gly Gly Arg Asp Arg Lys Val Arg Asp Leu
    50                  55                  60

Gln Glu Ala Asp Leu Asp Leu Leu Arg Val Thr Leu Ser Ser Lys Pro
65                  70                  75                  80

Gln Ala Leu Ala Thr Pro Asn Lys Glu Glu His Gly Lys Arg Lys Lys
                85                  90                  95

Lys Gly Lys Gly Leu Gly Lys Lys Arg Asp Pro Cys Leu Arg Lys Tyr
            100                 105                 110

Lys Asp Phe Cys Ile His Gly Glu Cys Lys Tyr Val Lys Glu Leu Arg
        115                 120                 125

Ala Pro Ser Cys Ile Cys His Pro Gly Tyr His Gly Glu Arg Cys His
    130                 135                 140

Gly Leu Ser Leu Pro Val Glu Asn Arg Leu Tyr Thr Tyr Asp His Thr
```

145 150 155 160

Thr Ile Leu Ala Val Val Ala Val Val Leu Ser Ser Val Cys Leu Leu
165 170 175

Val Ile Val Gly Leu Leu Met Phe Arg Tyr His Arg Arg Gly Gly Tyr
180 185 190

Asp Val Glu Asn Glu Glu Lys Val Lys Leu Gly Met Thr Asn Ser His
195 200 205

<210> 38
<211> 222
<212> PRT
<213> Artificial Sequence

<220>
<223> PRG2_HUMAN

<400> 38
Met Lys Leu Pro Leu Leu Leu Ala Leu Leu Phe Gly Ala Val Ser Ala
1 5 10 15

Leu His Leu Arg Ser Glu Thr Ser Thr Phe Glu Thr Pro Leu Gly Ala
20 25 30

Lys Thr Leu Pro Glu Asp Glu Glu Thr Pro Glu Gln Glu Met Glu Glu
35 40 45

Thr Pro Cys Arg Glu Leu Glu Glu Glu Glu Glu Trp Gly Ser Gly Ser
50 55 60

Glu Asp Ala Ser Lys Lys Asp Gly Ala Val Glu Ser Ile Ser Val Pro
65 70 75 80

Asp Met Val Asp Lys Asn Leu Thr Cys Pro Glu Glu Glu Asp Thr Val
85 90 95

Lys Val Val Gly Ile Pro Gly Cys Gln Thr Cys Arg Tyr Leu Leu Val
100 105 110

Arg Ser Leu Gln Thr Phe Ser Gln Ala Trp Phe Thr Cys Arg Arg Cys
115 120 125

Tyr Arg Gly Asn Leu Val Ser Ile His Asn Phe Asn Ile Asn Tyr Arg
130 135 140

Ile Gln Cys Ser Val Ser Ala Leu Asn Gln Gly Gln Val Trp Ile Gly
145 150 155 160

Gly Arg Ile Thr Gly Ser Gly Arg Cys Arg Arg Phe Gln Trp Val Asp
165 170 175

Gly Ser Arg Trp Asn Phe Ala Tyr Trp Ala Ala His Gln Pro Trp Ser
180 185 190

Arg Gly Gly His Cys Val Ala Leu Cys Thr Arg Gly Gly His Trp Arg
195 200 205

Arg Ala His Cys Leu Arg Arg Leu Pro Phe Ile Cys Ser Tyr
210 215 220

<210> 39
<211> 240
<212> PRT
<213> Artificial Sequence

<220>
<223> IBP6_HUMAN

<400> 39
Met Thr Pro His Arg Leu Leu Pro Pro Leu Leu Leu Leu Leu Ala Leu
1 5 10 15

Leu Leu Ala Ala Ser Pro Gly Gly Ala Leu Ala Arg Cys Pro Gly Cys
20 25 30

Gly Gln Gly Val Gln Ala Gly Cys Pro Gly Gly Cys Val Glu Glu Glu
35 40 45

Asp Gly Gly Ser Pro Ala Glu Gly Cys Ala Glu Ala Glu Gly Cys Leu
50 55 60

Arg Arg Glu Gly Gln Glu Cys Gly Val Tyr Thr Pro Asn Cys Ala Pro
65 70 75 80

Gly Leu Gln Cys His Pro Pro Lys Asp Asp Glu Ala Pro Leu Arg Ala
85 90 95

Leu Leu Leu Gly Arg Gly Arg Cys Leu Pro Ala Arg Ala Pro Ala Val
100 105 110

Ala Glu Glu Asn Pro Lys Glu Ser Lys Pro Gln Ala Gly Thr Ala Arg
115 120 125

Pro Gln Asp Val Asn Arg Arg Asp Gln Gln Arg Asn Pro Gly Thr Ser
130 135 140

Thr Thr Pro Ser Gln Pro Asn Ser Ala Gly Val Gln Asp Thr Glu Met
145 150 155 160

Gly Pro Cys Arg Arg His Leu Asp Ser Val Leu Gln Gln Leu Gln Thr
165 170 175

Glu Val Tyr Arg Gly Ala Gln Thr Leu Tyr Val Pro Asn Cys Asp His
180 185 190

Arg Gly Phe Tyr Arg Lys Arg Gln Cys Arg Ser Ser Gln Gly Gln Arg
195 200 205

Arg Gly Pro Cys Trp Cys Val Asp Arg Met Gly Lys Ser Leu Pro Gly
210 215 220

Ser Pro Asp Gly Asn Gly Ser Ser Ser Cys Pro Thr Gly Ser Ser Gly
225 230 235 240

<210> 40

<211> 260
<212> PRT
<213> Artificial Sequence

<220>
<223> PCSK1_HUMAN

<400> 40
Met Ala Gly Ser Pro Leu Leu Trp Gly Pro Arg Ala Gly Gly Val Gly
1 5 10 15

Leu Leu Val Leu Leu Leu Leu Gly Leu Phe Arg Pro Pro Pro Ala Leu
20 25 30

Cys Ala Arg Pro Val Lys Glu Pro Arg Gly Leu Ser Ala Ala Ser Pro
35 40 45

Pro Leu Ala Glu Thr Gly Ala Pro Arg Arg Phe Arg Arg Ser Val Pro
50 55 60

Arg Gly Glu Ala Ala Gly Ala Val Gln Glu Leu Ala Arg Ala Leu Ala
65 70 75 80

His Leu Leu Glu Ala Glu Arg Gln Glu Arg Ala Arg Ala Glu Ala Gln
85 90 95

Glu Ala Glu Asp Gln Gln Ala Arg Val Leu Ala Gln Leu Leu Arg Val
100 105 110

Trp Gly Ala Pro Arg Asn Ser Asp Pro Ala Leu Gly Leu Asp Asp Asp
115 120 125

Pro Asp Ala Pro Ala Ala Gln Leu Ala Arg Ala Leu Leu Arg Ala Arg
130 135 140

Leu Asp Pro Ala Ala Leu Ala Ala Gln Leu Val Pro Ala Pro Val Pro
145 150 155 160

Ala Ala Ala Leu Arg Pro Arg Pro Pro Val Tyr Asp Asp Gly Pro Ala
165 170 175

Gly Pro Asp Ala Glu Glu Ala Gly Asp Glu Thr Pro Asp Val Asp Pro
180 185 190

Glu Leu Leu Arg Tyr Leu Leu Gly Arg Ile Leu Ala Gly Ser Ala Asp
195 200 205

Ser Glu Gly Val Ala Ala Pro Arg Arg Leu Arg Arg Ala Ala Asp His
210 215 220

Asp Val Gly Ser Glu Leu Pro Pro Glu Gly Val Leu Gly Ala Leu Leu
225 230 235 240

Arg Val Lys Arg Leu Glu Thr Pro Ala Pro Gln Val Pro Ala Arg Arg
245 250 255

Leu Leu Pro Pro
260

<210> 41
<211> 272

<212> PRT
<213> Artificial Sequence

<220>
<223> IL2RA_HUMAN

<400> 41

```
Met Asp Ser Tyr Leu Leu Met Trp Gly Leu Leu Thr Phe Ile Met Val
1               5                   10                  15

Pro Gly Cys Gln Ala Glu Leu Cys Asp Asp Asp Pro Pro Glu Ile Pro
            20                  25                  30

His Ala Thr Phe Lys Ala Met Ala Tyr Lys Glu Gly Thr Met Leu Asn
        35                  40                  45

Cys Glu Cys Lys Arg Gly Phe Arg Arg Ile Lys Ser Gly Ser Leu Tyr
    50                  55                  60

Met Leu Cys Thr Gly Asn Ser Ser His Ser Ser Trp Asp Asn Gln Cys
65                  70                  75                  80

Gln Cys Thr Ser Ser Ala Thr Arg Asn Thr Thr Lys Gln Val Thr Pro
                85                  90                  95

Gln Pro Glu Glu Gln Lys Glu Arg Lys Thr Thr Glu Met Gln Ser Pro
            100                 105                 110

Met Gln Pro Val Asp Gln Ala Ser Leu Pro Gly His Cys Arg Glu Pro
        115                 120                 125

Pro Pro Trp Glu Asn Glu Ala Thr Glu Arg Ile Tyr His Phe Val Val
    130                 135                 140

Gly Gln Met Val Tyr Tyr Gln Cys Val Gln Gly Tyr Arg Ala Leu His
145                 150                 155                 160

Arg Gly Pro Ala Glu Ser Val Cys Lys Met Thr His Gly Lys Thr Arg
                165                 170                 175

Trp Thr Gln Pro Gln Leu Ile Cys Thr Gly Glu Met Glu Thr Ser Gln
            180                 185                 190

Phe Pro Gly Glu Glu Lys Pro Gln Ala Ser Pro Glu Gly Arg Pro Glu
        195                 200                 205

Ser Glu Thr Ser Cys Leu Val Thr Thr Thr Asp Phe Gln Ile Gln Thr
    210                 215                 220

Glu Met Ala Ala Thr Met Glu Thr Ser Ile Phe Thr Thr Glu Tyr Gln
225                 230                 235                 240

Val Ala Val Ala Gly Cys Val Phe Leu Leu Ile Ser Val Leu Leu Leu
                245                 250                 255

Ser Gly Leu Thr Trp Gln Arg Arg Gln Arg Lys Ser Arg Arg Thr Ile
            260                 265                 270
```

<210> 42

<211> 273
<212> PRT
<213> Artificial Sequence

<220>
<223> SCF_HUMAN

<400> 42

```
Met Lys Lys Thr Gln Thr Trp Ile Leu Thr Cys Ile Tyr Leu Gln Leu
1               5                   10                  15

Leu Leu Phe Asn Pro Leu Val Lys Thr Glu Gly Ile Cys Arg Asn Arg
            20                  25                  30

Val Thr Asn Asn Val Lys Asp Val Thr Lys Leu Val Ala Asn Leu Pro
        35                  40                  45

Lys Asp Tyr Met Ile Thr Leu Lys Tyr Val Pro Gly Met Asp Val Leu
    50                  55                  60

Pro Ser His Cys Trp Ile Ser Glu Met Val Val Gln Leu Ser Asp Ser
65                  70                  75                  80

Leu Thr Asp Leu Leu Asp Lys Phe Ser Asn Ile Ser Glu Gly Leu Ser
                85                  90                  95

Asn Tyr Ser Ile Ile Asp Lys Leu Val Asn Ile Val Asp Asp Leu Val
            100                 105                 110

Glu Cys Val Lys Glu Asn Ser Ser Lys Asp Leu Lys Lys Ser Phe Lys
        115                 120                 125

Ser Pro Glu Pro Arg Leu Phe Thr Pro Glu Glu Phe Phe Arg Ile Phe
    130                 135                 140

Asn Arg Ser Ile Asp Ala Phe Lys Asp Phe Val Val Ala Ser Glu Thr
145                 150                 155                 160

Ser Asp Cys Val Val Ser Ser Thr Leu Ser Pro Glu Lys Asp Ser Arg
                165                 170                 175

Val Ser Val Thr Lys Pro Phe Met Leu Pro Pro Val Ala Ala Ser Ser
            180                 185                 190

Leu Arg Asn Asp Ser Ser Ser Ser Asn Arg Lys Ala Lys Asn Pro Pro
        195                 200                 205

Gly Asp Ser Ser Leu His Trp Ala Ala Met Ala Leu Pro Ala Leu Phe
    210                 215                 220

Ser Leu Ile Ile Gly Phe Ala Phe Gly Ala Leu Tyr Trp Lys Lys Arg
225                 230                 235                 240

Gln Pro Ser Leu Thr Arg Ala Val Glu Asn Ile Gln Ile Asn Glu Glu
                245                 250                 255

Asp Asn Glu Ile Ser Met Leu Gln Glu Lys Glu Arg Glu Phe Gln Glu
            260                 265                 270

Val
```

<210> 43
<211> 279
<212> PRT
<213> Artificial Sequence

<220>
<223> ODAM_HUMAN

<400> 43

```
Met Lys Ile Ile Ile Leu Leu Gly Phe Leu Gly Ala Thr Leu Ser Ala
1               5                   10                  15

Pro Leu Ile Pro Gln Arg Leu Met Ser Ala Ser Asn Ser Asn Glu Leu
            20                  25                  30

Leu Leu Asn Leu Asn Asn Gly Gln Leu Leu Pro Leu Gln Leu Gln Gly
        35                  40                  45

Pro Leu Asn Ser Trp Ile Pro Pro Phe Ser Gly Ile Leu Gln Gln Gln
    50                  55                  60

Gln Gln Ala Gln Ile Pro Gly Leu Ser Gln Phe Ser Leu Ser Ala Leu
65                  70                  75                  80

Asp Gln Phe Ala Gly Leu Leu Pro Asn Gln Ile Pro Leu Thr Gly Glu
                85                  90                  95

Ala Ser Phe Ala Gln Gly Ala Gln Ala Gly Gln Val Asp Pro Leu Gln
            100                 105                 110

Leu Gln Thr Pro Pro Gln Thr Gln Pro Gly Pro Ser His Val Met Pro
        115                 120                 125

Tyr Val Phe Ser Phe Lys Met Pro Gln Glu Gln Gly Gln Met Phe Gln
    130                 135                 140

Tyr Tyr Pro Val Tyr Met Val Leu Pro Trp Glu Gln Pro Gln Gln Thr
145                 150                 155                 160

Val Pro Arg Ser Pro Gln Gln Thr Arg Gln Gln Gln Tyr Glu Glu Gln
                165                 170                 175

Ile Pro Phe Tyr Ala Gln Phe Gly Tyr Ile Pro Gln Leu Ala Glu Pro
            180                 185                 190

Ala Ile Ser Gly Gly Gln Gln Gln Leu Ala Phe Asp Pro Gln Leu Gly
        195                 200                 205

Thr Ala Pro Glu Ile Ala Val Met Ser Thr Gly Glu Glu Ile Pro Tyr
    210                 215                 220

Leu Gln Lys Glu Ala Ile Asn Phe Arg His Asp Ser Ala Gly Val Phe
225                 230                 235                 240

Met Pro Ser Thr Ser Pro Lys Pro Ser Thr Thr Asn Val Phe Thr Ser
                245                 250                 255

Ala Val Asp Gln Thr Ile Thr Pro Glu Leu Pro Glu Glu Lys Asp Lys
            260                 265                 270

Thr Asp Ser Leu Arg Glu Pro
```

275

<210> 44
<211> 314
<212> PRT
<213> Artificial Sequence

<220>
<223> OSTP_HUMAN

<400> 44
Met Arg Ile Ala Val Ile Cys Phe Cys Leu Leu Gly Ile Thr Cys Ala
1 5 10 15

Ile Pro Val Lys Gln Ala Asp Ser Gly Ser Ser Glu Glu Lys Gln Leu
20 25 30

Tyr Asn Lys Tyr Pro Asp Ala Val Ala Thr Trp Leu Asn Pro Asp Pro
35 40 45

Ser Gln Lys Gln Asn Leu Leu Ala Pro Gln Asn Ala Val Ser Ser Glu
50 55 60

Glu Thr Asn Asp Phe Lys Gln Glu Thr Leu Pro Ser Lys Ser Asn Glu
65 70 75 80

Ser His Asp His Met Asp Asp Met Asp Asp Glu Asp Asp Asp Asp His
85 90 95

Val Asp Ser Gln Asp Ser Ile Asp Ser Asn Asp Ser Asp Asp Val Asp
100 105 110

Asp Thr Asp Asp Ser His Gln Ser Asp Glu Ser His His Ser Asp Glu
115 120 125

Ser Asp Glu Leu Val Thr Asp Phe Pro Thr Asp Leu Pro Ala Thr Glu
130 135 140

Val Phe Thr Pro Val Val Pro Thr Val Asp Thr Tyr Asp Gly Arg Gly
145 150 155 160

Asp Ser Val Val Tyr Gly Leu Arg Ser Lys Ser Lys Lys Phe Arg Arg
165 170 175

Pro Asp Ile Gln Tyr Pro Asp Ala Thr Asp Glu Asp Ile Thr Ser His
180 185 190

Met Glu Ser Glu Glu Leu Asn Gly Ala Tyr Lys Ala Ile Pro Val Ala
195 200 205

Gln Asp Leu Asn Ala Pro Ser Asp Trp Asp Ser Arg Gly Lys Asp Ser
210 215 220

Tyr Glu Thr Ser Gln Leu Asp Asp Gln Ser Ala Glu Thr His Ser His
225 230 235 240

Lys Gln Ser Arg Leu Tyr Lys Arg Lys Ala Asn Asp Glu Ser Asn Glu
245 250 255

His Ser Asp Val Ile Asp Ser Gln Glu Leu Ser Lys Val Ser Arg Glu
260 265 270

Phe His Ser His Glu Phe His Ser His Glu Asp Met Leu Val Val Asp
275 280 285

Pro Lys Ser Lys Glu Glu Asp Lys His Leu Lys Phe Arg Ile Ser His
290 295 300

Glu Leu Asp Ser Ala Ser Ser Glu Val Asn
305 310

<210> 45
<211> 317
<212> PRT
<213> Artificial Sequence

<220>
<223> SIAL_HUMAN

<400> 45
Met Lys Thr Ala Leu Ile Leu Leu Ser Ile Leu Gly Met Ala Cys Ala
1 5 10 15

Phe Ser Met Lys Asn Leu His Arg Arg Val Lys Ile Glu Asp Ser Glu
20 25 30

Glu Asn Gly Val Phe Lys Tyr Arg Pro Arg Tyr Tyr Leu Tyr Lys His
35 40 45

Ala Tyr Phe Tyr Pro His Leu Lys Arg Phe Pro Val Gln Gly Ser Ser
50 55 60

Asp Ser Ser Glu Glu Asn Gly Asp Asp Ser Ser Glu Glu Glu Glu Glu
65 70 75 80

Glu Glu Glu Thr Ser Asn Glu Gly Glu Asn Asn Glu Glu Ser Asn Glu
85 90 95

Asp Glu Asp Ser Glu Ala Glu Asn Thr Thr Leu Ser Ala Thr Thr Leu
100 105 110

Gly Tyr Gly Glu Asp Ala Thr Pro Gly Thr Gly Tyr Thr Gly Leu Ala
115 120 125

Ala Ile Gln Leu Pro Lys Lys Ala Gly Asp Ile Thr Asn Lys Ala Thr
130 135 140

Lys Glu Lys Glu Ser Asp Glu Glu Glu Glu Glu Glu Glu Glu Gly Asn
145 150 155 160

Glu Asn Glu Glu Ser Glu Ala Glu Val Asp Glu Asn Glu Gln Gly Ile
165 170 175

Asn Gly Thr Ser Thr Asn Ser Thr Glu Ala Glu Asn Gly Asn Gly Ser
180 185 190

Ser Gly Gly Asp Asn Gly Glu Glu Gly Glu Glu Glu Ser Val Thr Gly
195 200 205

Ala Asn Ala Glu Asp Thr Thr Glu Thr Gly Arg Gln Gly Lys Gly Thr
210 215 220

```
Ser Lys Thr Thr Thr Ser Pro Asn Gly Gly Phe Glu Pro Thr Thr Pro
225                 230                 235                 240

Pro Gln Val Tyr Arg Thr Thr Ser Pro Pro Phe Gly Lys Thr Thr Thr
                245                 250                 255

Val Glu Tyr Glu Gly Glu Tyr Glu Tyr Thr Gly Ala Asn Glu Tyr Asp
            260                 265                 270

Asn Gly Tyr Glu Ile Tyr Glu Ser Glu Asn Gly Glu Pro Arg Gly Asp
        275                 280                 285

Asn Tyr Arg Ala Tyr Glu Asp Glu Tyr Ser Tyr Phe Lys Gly Gln Gly
    290                 295                 300

Tyr Asp Gly Tyr Asp Gly Gln Asn Tyr Tyr His His Gln
305                 310                 315


<210>   46
<211>   317
<212>   PRT
<213>   Artificial Sequence

<220>
<223>   APOE_HUMAN


<400>   46
Met Lys Val Leu Trp Ala Ala Leu Leu Val Thr Phe Leu Ala Gly Cys
1               5                   10                  15

Gln Ala Lys Val Glu Gln Ala Val Glu Thr Glu Pro Glu Pro Glu Leu
            20                  25                  30

Arg Gln Gln Thr Glu Trp Gln Ser Gly Gln Arg Trp Glu Leu Ala Leu
        35                  40                  45

Gly Arg Phe Trp Asp Tyr Leu Arg Trp Val Gln Thr Leu Ser Glu Gln
    50                  55                  60

Val Gln Glu Glu Leu Leu Ser Ser Gln Val Thr Gln Glu Leu Arg Ala
65                  70                  75                  80

Leu Met Asp Glu Thr Met Lys Glu Leu Lys Ala Tyr Lys Ser Glu Leu
                85                  90                  95

Glu Glu Gln Leu Thr Pro Val Ala Glu Glu Thr Arg Ala Arg Leu Ser
            100                 105                 110

Lys Glu Leu Gln Ala Ala Gln Ala Arg Leu Gly Ala Asp Met Glu Asp
        115                 120                 125

Val Cys Gly Arg Leu Val Gln Tyr Arg Gly Glu Val Gln Ala Met Leu
    130                 135                 140

Gly Gln Ser Thr Glu Glu Leu Arg Val Arg Leu Ala Ser His Leu Arg
145                 150                 155                 160

Lys Leu Arg Lys Arg Leu Leu Arg Asp Ala Asp Asp Leu Gln Lys Arg
                165                 170                 175

Leu Ala Val Tyr Gln Ala Gly Ala Arg Glu Gly Ala Glu Arg Gly Leu
```

180 185 190

Ser Ala Ile Arg Glu Arg Leu Gly Pro Leu Val Glu Gln Gly Arg Val
195 200 205

Arg Ala Ala Thr Val Gly Ser Leu Ala Gly Gln Pro Leu Gln Glu Arg
210 215 220

Ala Gln Ala Trp Gly Glu Arg Leu Arg Ala Arg Met Glu Glu Met Gly
225 230 235 240

Ser Arg Thr Arg Asp Arg Leu Asp Glu Val Lys Glu Gln Val Ala Glu
245 250 255

Val Arg Ala Lys Leu Glu Glu Gln Ala Gln Gln Ile Arg Leu Gln Ala
260 265 270

Glu Ala Phe Gln Ala Arg Leu Lys Ser Trp Phe Glu Pro Leu Val Glu
275 280 285

Asp Met Gln Arg Gln Trp Ala Gly Leu Val Glu Lys Val Gln Ala Ala
290 295 300

Val Gly Thr Ser Ala Ala Pro Val Pro Ser Asp Asn His
305 310 315

<210> 47
<211> 322
<212> PRT
<213> Artificial Sequence

<220>
<223> EPYC_HUMAN

<400> 47
Met Lys Thr Leu Ala Gly Leu Val Leu Gly Leu Val Ile Phe Asp Ala
1 5 10 15

Ala Val Thr Ala Pro Thr Leu Glu Ser Ile Asn Tyr Asp Ser Glu Thr
20 25 30

Tyr Asp Ala Thr Leu Glu Asp Leu Asp Asn Leu Tyr Asn Tyr Glu Asn
35 40 45

Ile Pro Val Asp Lys Val Glu Ile Glu Ile Ala Thr Val Met Pro Ser
50 55 60

Gly Asn Arg Glu Leu Leu Thr Pro Pro Pro Gln Pro Glu Lys Ala Gln
65 70 75 80

Glu Glu Glu Glu Glu Glu Glu Ser Thr Pro Arg Leu Ile Asp Gly Ser
85 90 95

Ser Pro Gln Glu Pro Glu Phe Thr Gly Val Leu Gly Pro His Thr Asn
100 105 110

Glu Asp Phe Pro Thr Cys Leu Leu Cys Thr Cys Ile Ser Thr Thr Val
115 120 125

Tyr Cys Asp Asp His Glu Leu Asp Ala Ile Pro Pro Leu Pro Lys Asn
130 135 140

Thr Ala Tyr Phe Tyr Ser Arg Phe Asn Arg Ile Lys Lys Ile Asn Lys
145 150 155 160

Asn Asp Phe Ala Ser Leu Ser Asp Leu Lys Arg Ile Asp Leu Thr Ser
165 170 175

Asn Leu Ile Ser Glu Ile Asp Glu Asp Ala Phe Arg Lys Leu Pro Gln
180 185 190

Leu Arg Glu Leu Val Leu Arg Asp Asn Lys Ile Arg Gln Leu Pro Glu
195 200 205

Leu Pro Thr Thr Leu Thr Phe Ile Asp Ile Ser Asn Asn Arg Leu Gly
210 215 220

Arg Lys Gly Ile Lys Gln Glu Ala Phe Lys Asp Met Tyr Asp Leu His
225 230 235 240

His Leu Tyr Leu Thr Asp Asn Asn Leu Asp His Ile Pro Leu Pro Leu
245 250 255

Pro Glu Asn Leu Arg Ala Leu His Leu Gln Asn Asn Asn Ile Leu Glu
260 265 270

Met His Glu Asp Thr Phe Cys Asn Val Lys Asn Leu Thr Tyr Ile Arg
275 280 285

Lys Ala Leu Glu Asp Ile Arg Leu Asp Gly Asn Pro Ile Asn Leu Ser
290 295 300

Lys Thr Pro Gln Ala Tyr Met Cys Leu Pro Arg Leu Pro Val Gly Ser
305 310 315 320

Leu Val

<210> 48
<211> 332
<212> PRT
<213> Artificial Sequence

<220>
<223> CLM9_HUMAN

<400> 48
Met Arg Leu Leu Val Leu Leu Trp Gly Cys Leu Leu Leu Pro Gly Tyr
1 5 10 15

Glu Ala Leu Glu Gly Pro Glu Glu Ile Ser Gly Phe Glu Gly Asp Thr
20 25 30

Val Ser Leu Gln Cys Thr Tyr Arg Glu Glu Leu Arg Asp His Arg Lys
35 40 45

Tyr Trp Cys Arg Lys Gly Gly Ile Leu Phe Ser Arg Cys Ser Gly Thr
50 55 60

Ile Tyr Ala Glu Glu Glu Gly Gln Glu Thr Met Lys Gly Arg Val Ser
65 70 75 80

Ile Arg Asp Ser Arg Gln Glu Leu Ser Leu Ile Val Thr Leu Trp Asn
85 90 95

Leu Thr Leu Gln Asp Ala Gly Glu Tyr Trp Cys Gly Val Glu Lys Arg
100 105 110

Gly Pro Asp Glu Ser Leu Leu Ile Ser Leu Phe Val Phe Pro Gly Pro
115 120 125

Cys Cys Pro Pro Ser Pro Ser Pro Thr Phe Gln Pro Leu Ala Thr Thr
130 135 140

Arg Leu Gln Pro Lys Ala Lys Ala Gln Gln Thr Gln Pro Pro Gly Leu
145 150 155 160

Thr Ser Pro Gly Leu Tyr Pro Ala Ala Thr Thr Ala Lys Gln Gly Lys
165 170 175

Thr Gly Ala Glu Ala Pro Pro Leu Pro Gly Thr Ser Gln Tyr Gly His
180 185 190

Glu Arg Thr Ser Gln Tyr Thr Gly Thr Ser Pro His Pro Ala Thr Ser
195 200 205

Pro Pro Ala Gly Ser Ser Arg Pro Pro Met Gln Leu Asp Ser Thr Ser
210 215 220

Ala Glu Asp Thr Ser Pro Ala Leu Ser Ser Gly Ser Ser Lys Pro Arg
225 230 235 240

Val Ser Ile Pro Met Val Arg Ile Leu Ala Pro Val Leu Val Leu Leu
245 250 255

Ser Leu Leu Ser Ala Ala Gly Leu Ile Ala Phe Cys Ser His Leu Leu
260 265 270

Leu Trp Arg Lys Glu Ala Gln Gln Ala Thr Glu Thr Gln Arg Asn Glu
275 280 285

Lys Phe Cys Leu Ser Arg Leu Thr Ala Glu Glu Lys Glu Ala Pro Ser
290 295 300

Gln Ala Pro Glu Gly Asp Val Ile Ser Met Pro Pro Leu His Thr Ser
305 310 315 320

Glu Glu Glu Leu Gly Phe Ser Lys Phe Val Ser Ala
325 330

<210> 49
<211> 350
<212> PRT
<213> Artificial Sequence

<220>
<223> YIPF3_HUMAN

<400> 49
Met Ala Thr Thr Ala Ala Pro Ala Gly Gly Ala Arg Asn Gly Ala Gly
1 5 10 15

Pro Glu Trp Gly Gly Phe Glu Glu Asn Ile Gln Gly Gly Gly Ser Ala

20 25 30

Val Ile Asp Met Glu Asn Met Asp Asp Thr Ser Gly Ser Ser Phe Glu
35 40 45

Asp Met Gly Glu Leu His Gln Arg Leu Arg Glu Glu Glu Val Asp Ala
50 55 60

Asp Ala Ala Asp Ala Ala Ala Ala Glu Glu Glu Asp Gly Glu Phe Leu
65 70 75 80

Gly Met Lys Gly Phe Lys Gly Gln Leu Ser Arg Gln Val Ala Asp Gln
85 90 95

Met Trp Gln Ala Gly Lys Arg Gln Ala Ser Arg Ala Phe Ser Leu Tyr
100 105 110

Ala Asn Ile Asp Ile Leu Arg Pro Tyr Phe Asp Val Glu Pro Ala Gln
115 120 125

Val Arg Ser Arg Leu Leu Glu Ser Met Ile Pro Ile Lys Met Val Asn
130 135 140

Phe Pro Gln Lys Ile Ala Gly Glu Leu Tyr Gly Pro Leu Met Leu Val
145 150 155 160

Phe Thr Leu Val Ala Ile Leu Leu His Gly Met Lys Thr Ser Asp Thr
165 170 175

Ile Ile Arg Glu Gly Thr Leu Met Gly Thr Ala Ile Gly Thr Cys Phe
180 185 190

Gly Tyr Trp Leu Gly Val Ser Ser Phe Ile Tyr Phe Leu Ala Tyr Leu
195 200 205

Cys Asn Ala Gln Ile Thr Met Leu Gln Met Leu Ala Leu Leu Gly Tyr
210 215 220

Gly Leu Phe Gly His Cys Ile Val Leu Phe Ile Thr Tyr Asn Ile His
225 230 235 240

Leu His Ala Leu Phe Tyr Leu Phe Trp Leu Leu Val Gly Gly Leu Ser
245 250 255

Thr Leu Arg Met Val Ala Val Leu Val Ser Arg Thr Val Gly Pro Thr
260 265 270

Gln Arg Leu Leu Leu Cys Gly Thr Leu Ala Ala Leu His Met Leu Phe
275 280 285

Leu Leu Tyr Leu His Phe Ala Tyr His Lys Val Val Glu Gly Ile Leu
290 295 300

Asp Thr Leu Glu Gly Pro Asn Ile Pro Pro Ile Gln Arg Val Pro Arg
305 310 315 320

Asp Ile Pro Ala Met Leu Pro Ala Ala Arg Leu Pro Thr Thr Val Leu
325 330 335

Asn Ala Thr Ala Lys Ala Val Ala Val Thr Leu Gln Ser His
340 345 350

<210> 50
<211> 352
<212> PRT
<213> Artificial Sequence

<220>
<223> CCR5_HUMAN

<400> 50
```
Met Asp Tyr Gln Val Ser Ser Pro Ile Tyr Asp Ile Asn Tyr Tyr Thr
1               5                   10                  15

Ser Glu Pro Cys Gln Lys Ile Asn Val Lys Gln Ile Ala Ala Arg Leu
            20                  25                  30

Leu Pro Pro Leu Tyr Ser Leu Val Phe Ile Phe Gly Phe Val Gly Asn
        35                  40                  45

Met Leu Val Ile Leu Ile Leu Ile Asn Cys Lys Arg Leu Lys Ser Met
    50                  55                  60

Thr Asp Ile Tyr Leu Leu Asn Leu Ala Ile Ser Asp Leu Phe Phe Leu
65                  70                  75                  80

Leu Thr Val Pro Phe Trp Ala His Tyr Ala Ala Ala Gln Trp Asp Phe
                85                  90                  95

Gly Asn Thr Met Cys Gln Leu Leu Thr Gly Leu Tyr Phe Ile Gly Phe
            100                 105                 110

Phe Ser Gly Ile Phe Phe Ile Ile Leu Leu Thr Ile Asp Arg Tyr Leu
        115                 120                 125

Ala Val Val His Ala Val Phe Ala Leu Lys Ala Arg Thr Val Thr Phe
    130                 135                 140

Gly Val Val Thr Ser Val Ile Thr Trp Val Val Ala Val Phe Ala Ser
145                 150                 155                 160

Leu Pro Gly Ile Ile Phe Thr Arg Ser Gln Lys Glu Gly Leu His Tyr
                165                 170                 175

Thr Cys Ser Ser His Phe Pro Tyr Ser Gln Tyr Gln Phe Trp Lys Asn
            180                 185                 190

Phe Gln Thr Leu Lys Ile Val Ile Leu Gly Leu Val Leu Pro Leu Leu
        195                 200                 205

Val Met Val Ile Cys Tyr Ser Gly Ile Leu Lys Thr Leu Leu Arg Cys
    210                 215                 220

Arg Asn Glu Lys Lys Arg His Arg Ala Val Arg Leu Ile Phe Thr Ile
225                 230                 235                 240

Met Ile Val Tyr Phe Leu Phe Trp Ala Pro Tyr Asn Ile Val Leu Leu
                245                 250                 255

Leu Asn Thr Phe Gln Glu Phe Phe Gly Leu Asn Asn Cys Ser Ser Ser
            260                 265                 270

Asn Arg Leu Asp Gln Ala Met Gln Val Thr Glu Thr Leu Gly Met Thr
        275                 280                 285
```

```
His Cys Cys Ile Asn Pro Ile Ile Tyr Ala Phe Val Gly Glu Lys Phe
    290                 295                 300

Arg Asn Tyr Leu Leu Val Phe Phe Gln Lys His Ile Ala Lys Arg Phe
305                 310                 315                 320

Cys Lys Cys Cys Ser Ile Phe Gln Gln Glu Ala Pro Glu Arg Ala Ser
                325                 330                 335

Ser Val Tyr Thr Arg Ser Thr Gly Glu Gln Glu Ile Ser Val Gly Leu
            340                 345                 350
```

<210> 51
<211> 353
<212> PRT
<213> Artificial Sequence

<220>
<223> TPO_HUMAN

<400> 51

```
Met Glu Leu Thr Glu Leu Leu Leu Val Val Met Leu Leu Leu Thr Ala
1               5                   10                  15

Arg Leu Thr Leu Ser Ser Pro Ala Pro Pro Ala Cys Asp Leu Arg Val
            20                  25                  30

Leu Ser Lys Leu Leu Arg Asp Ser His Val Leu His Ser Arg Leu Ser
        35                  40                  45

Gln Cys Pro Glu Val His Pro Leu Pro Thr Pro Val Leu Leu Pro Ala
    50                  55                  60

Val Asp Phe Ser Leu Gly Glu Trp Lys Thr Gln Met Glu Glu Thr Lys
65                  70                  75                  80

Ala Gln Asp Ile Leu Gly Ala Val Thr Leu Leu Leu Glu Gly Val Met
                85                  90                  95

Ala Ala Arg Gly Gln Leu Gly Pro Thr Cys Leu Ser Ser Leu Leu Gly
            100                 105                 110

Gln Leu Ser Gly Gln Val Arg Leu Leu Leu Gly Ala Leu Gln Ser Leu
        115                 120                 125

Leu Gly Thr Gln Leu Pro Pro Gln Gly Arg Thr Thr Ala His Lys Asp
    130                 135                 140

Pro Asn Ala Ile Phe Leu Ser Phe Gln His Leu Leu Arg Gly Lys Val
145                 150                 155                 160

Arg Phe Leu Met Leu Val Gly Gly Ser Thr Leu Cys Val Arg Arg Ala
                165                 170                 175

Pro Pro Thr Thr Ala Val Pro Ser Arg Thr Ser Leu Val Leu Thr Leu
            180                 185                 190

Asn Glu Leu Pro Asn Arg Thr Ser Gly Leu Leu Glu Thr Asn Phe Thr
```

```
        195                 200                 205

Ala Ser Ala Arg Thr Thr Gly Ser Gly Leu Leu Lys Trp Gln Gln Gly
    210                 215                 220

Phe Arg Ala Lys Ile Pro Gly Leu Leu Asn Gln Thr Ser Arg Ser Leu
225                 230                 235                 240

Asp Gln Ile Pro Gly Tyr Leu Asn Arg Ile His Glu Leu Leu Asn Gly
                245                 250                 255

Thr Arg Gly Leu Phe Pro Gly Pro Ser Arg Arg Thr Leu Gly Ala Pro
            260                 265                 270

Asp Ile Ser Ser Gly Thr Ser Asp Thr Gly Ser Leu Pro Pro Asn Leu
        275                 280                 285

Gln Pro Gly Tyr Ser Pro Ser Pro Thr His Pro Pro Thr Gly Gln Tyr
    290                 295                 300

Thr Leu Phe Pro Leu Pro Pro Thr Leu Pro Thr Pro Val Val Gln Leu
305                 310                 315                 320

His Pro Leu Leu Pro Asp Pro Ser Ala Pro Thr Pro Thr Pro Thr Ser
                325                 330                 335

Pro Leu Leu Asn Thr Ser Tyr Thr His Ser Gln Asn Leu Ser Gln Glu
            340                 345                 350

Gly
```

<210> 52
<211> 367
<212> PRT
<213> Artificial Sequence

<220>
<223> FETUA_HUMAN

<400> 52

```
Met Lys Ser Leu Val Leu Leu Leu Cys Leu Ala Gln Leu Trp Gly Cys
1               5                   10                  15

His Ser Ala Pro His Gly Pro Gly Leu Ile Tyr Arg Gln Pro Asn Cys
            20                  25                  30

Asp Asp Pro Glu Thr Glu Glu Ala Ala Leu Val Ala Ile Asp Tyr Ile
        35                  40                  45

Asn Gln Asn Leu Pro Trp Gly Tyr Lys His Thr Leu Asn Gln Ile Asp
    50                  55                  60

Glu Val Lys Val Trp Pro Gln Gln Pro Ser Gly Glu Leu Phe Glu Ile
65                  70                  75                  80

Glu Ile Asp Thr Leu Glu Thr Thr Cys His Val Leu Asp Pro Thr Pro
                85                  90                  95

Val Ala Arg Cys Ser Val Arg Gln Leu Lys Glu His Ala Val Glu Gly
            100                 105                 110
```

Asp Cys Asp Phe Gln Leu Leu Lys Leu Asp Gly Lys Phe Ser Val Val
115 120 125

Tyr Ala Lys Cys Asp Ser Ser Pro Asp Ser Ala Glu Asp Val Arg Lys
130 135 140

Val Cys Gln Asp Cys Pro Leu Leu Ala Pro Leu Asn Asp Thr Arg Val
145 150 155 160

Val His Ala Ala Lys Ala Ala Leu Ala Ala Phe Asn Ala Gln Asn Asn
165 170 175

Gly Ser Asn Phe Gln Leu Glu Glu Ile Ser Arg Ala Gln Leu Val Pro
180 185 190

Leu Pro Pro Ser Thr Tyr Val Glu Phe Thr Val Ser Gly Thr Asp Cys
195 200 205

Val Ala Lys Glu Ala Thr Glu Ala Ala Lys Cys Asn Leu Leu Ala Glu
210 215 220

Lys Gln Tyr Gly Phe Cys Lys Ala Thr Leu Ser Glu Lys Leu Gly Gly
225 230 235 240

Ala Glu Val Ala Val Thr Cys Met Val Phe Gln Thr Gln Pro Val Ser
245 250 255

Ser Gln Pro Gln Pro Glu Gly Ala Asn Glu Ala Val Pro Thr Pro Val
260 265 270

Val Asp Pro Asp Ala Pro Pro Ser Pro Pro Leu Gly Ala Pro Gly Leu
275 280 285

Pro Pro Ala Gly Ser Pro Pro Asp Ser His Val Leu Leu Ala Ala Pro
290 295 300

Pro Gly His Gln Leu His Arg Ala His Tyr Asp Leu Arg His Thr Phe
305 310 315 320

Met Gly Val Val Ser Leu Gly Ser Pro Ser Gly Glu Val Ser His Pro
325 330 335

Arg Lys Thr Arg Thr Val Val Gln Pro Ser Val Gly Ala Ala Ala Gly
340 345 350

Pro Val Val Pro Pro Cys Pro Gly Arg Ile Arg His Phe Lys Val
355 360 365

<210> 53
<211> 368
<212> PRT
<213> Artificial Sequence

<220>
<223> PGS1_HUMAN

<400> 53
Met Trp Pro Leu Trp Arg Leu Val Ser Leu Leu Ala Leu Ser Gln Ala

```
1               5                   10                  15

Leu Pro Phe Glu Gln Arg Gly Phe Trp Asp Phe Thr Leu Asp Asp Gly
            20                  25                  30

Pro Phe Met Met Asn Asp Glu Glu Ala Ser Gly Ala Asp Thr Ser Gly
        35                  40                  45

Val Leu Asp Pro Asp Ser Val Thr Pro Thr Tyr Ser Ala Met Cys Pro
    50                  55                  60

Phe Gly Cys His Cys His Leu Arg Val Val Gln Cys Ser Asp Leu Gly
65                  70                  75                  80

Leu Lys Ser Val Pro Lys Glu Ile Ser Pro Asp Thr Thr Leu Leu Asp
                85                  90                  95

Leu Gln Asn Asn Asp Ile Ser Glu Leu Arg Lys Asp Asp Phe Lys Gly
            100                 105                 110

Leu Gln His Leu Tyr Ala Leu Val Leu Val Asn Asn Lys Ile Ser Lys
        115                 120                 125

Ile His Glu Lys Ala Phe Ser Pro Leu Arg Lys Leu Gln Lys Leu Tyr
    130                 135                 140

Ile Ser Lys Asn His Leu Val Glu Ile Pro Pro Asn Leu Pro Ser Ser
145                 150                 155                 160

Leu Val Glu Leu Arg Ile His Asp Asn Arg Ile Arg Lys Val Pro Lys
                165                 170                 175

Gly Val Phe Ser Gly Leu Arg Asn Met Asn Cys Ile Glu Met Gly Gly
            180                 185                 190

Asn Pro Leu Glu Asn Ser Gly Phe Glu Pro Gly Ala Phe Asp Gly Leu
        195                 200                 205

Lys Leu Asn Tyr Leu Arg Ile Ser Glu Ala Lys Leu Thr Gly Ile Pro
    210                 215                 220

Lys Asp Leu Pro Glu Thr Leu Asn Glu Leu His Leu Asp His Asn Lys
225                 230                 235                 240

Ile Gln Ala Ile Glu Leu Glu Asp Leu Leu Arg Tyr Ser Lys Leu Tyr
                245                 250                 255

Arg Leu Gly Leu Gly His Asn Gln Ile Arg Met Ile Glu Asn Gly Ser
            260                 265                 270

Leu Ser Phe Leu Pro Thr Leu Arg Glu Leu His Leu Asp Asn Asn Lys
        275                 280                 285

Leu Ala Arg Val Pro Ser Gly Leu Pro Asp Leu Lys Leu Leu Gln Val
    290                 295                 300

Val Tyr Leu His Ser Asn Asn Ile Thr Lys Val Gly Val Asn Asp Phe
305                 310                 315                 320

Cys Pro Met Gly Phe Gly Val Lys Arg Ala Tyr Tyr Asn Gly Ile Ser
                325                 330                 335

Leu Phe Asn Asn Pro Val Pro Tyr Trp Glu Val Gln Pro Ala Thr Phe
```

340 345 350

Arg Cys Val Thr Asp Arg Leu Ala Ile Gln Phe Gly Asn Tyr Lys Lys
355 360 365

<210> 54
<211> 377
<212> PRT
<213> Artificial Sequence

<220>
<223> IGHG3_HUMAN

<400> 54
Ala Ser Thr Lys Gly Pro Ser Val Phe Pro Leu Ala Pro Cys Ser Arg
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Thr Cys Asn Val Asn His Lys Pro Ser Asn Thr Lys Val Asp Lys
85 90 95

Arg Val Glu Leu Lys Thr Pro Leu Gly Asp Thr Thr His Thr Cys Pro
100 105 110

Arg Cys Pro Glu Pro Lys Ser Cys Asp Thr Pro Pro Pro Cys Pro Arg
115 120 125

Cys Pro Glu Pro Lys Ser Cys Asp Thr Pro Pro Pro Cys Pro Arg Cys
130 135 140

Pro Glu Pro Lys Ser Cys Asp Thr Pro Pro Pro Cys Pro Arg Cys Pro
145 150 155 160

Ala Pro Glu Leu Leu Gly Gly Pro Ser Val Phe Leu Phe Pro Pro Lys
165 170 175

Pro Lys Asp Thr Leu Met Ile Ser Arg Thr Pro Glu Val Thr Cys Val
180 185 190

Val Val Asp Val Ser His Glu Asp Pro Glu Val Gln Phe Lys Trp Tyr
195 200 205

Val Asp Gly Val Glu Val His Asn Ala Lys Thr Lys Pro Arg Glu Glu
210 215 220

Gln Tyr Asn Ser Thr Phe Arg Val Val Ser Val Leu Thr Val Leu His
225 230 235 240

```
Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375


<210>   55
<211>   383
<212>   PRT
<213>   Artificial Sequence

<220>
<223>   DLK1_HUMAN


<400>   55
Met Thr Ala Thr Glu Ala Leu Leu Arg Val Leu Leu Leu Leu Leu Ala
1               5                   10                  15

Phe Gly His Ser Thr Tyr Gly Ala Glu Cys Phe Pro Ala Cys Asn Pro
            20                  25                  30

Gln Asn Gly Phe Cys Glu Asp Asp Asn Val Cys Arg Cys Gln Pro Gly
        35                  40                  45

Trp Gln Gly Pro Leu Cys Asp Gln Cys Val Thr Ser Pro Gly Cys Leu
    50                  55                  60

His Gly Leu Cys Gly Glu Pro Gly Gln Cys Ile Cys Thr Asp Gly Trp
65                  70                  75                  80

Asp Gly Glu Leu Cys Asp Arg Asp Val Arg Ala Cys Ser Ser Ala Pro
                85                  90                  95

Cys Ala Asn Asn Arg Thr Cys Val Ser Leu Asp Asp Gly Leu Tyr Glu
            100                 105                 110

Cys Ser Cys Ala Pro Gly Tyr Ser Gly Lys Asp Cys Gln Lys Lys Asp
        115                 120                 125

Gly Pro Cys Val Ile Asn Gly Ser Pro Cys Gln His Gly Gly Thr Cys
```

130 135 140

Val Asp Asp Glu Gly Arg Ala Ser His Ala Ser Cys Leu Cys Pro Pro
145 150 155 160

Gly Phe Ser Gly Asn Phe Cys Glu Ile Val Ala Asn Ser Cys Thr Pro
165 170 175

Asn Pro Cys Glu Asn Asp Gly Val Cys Thr Asp Ile Gly Gly Asp Phe
180 185 190

Arg Cys Arg Cys Pro Ala Gly Phe Ile Asp Lys Thr Cys Ser Arg Pro
195 200 205

Val Thr Asn Cys Ala Ser Ser Pro Cys Gln Asn Gly Gly Thr Cys Leu
210 215 220

Gln His Thr Gln Val Ser Tyr Glu Cys Leu Cys Lys Pro Glu Phe Thr
225 230 235 240

Gly Leu Thr Cys Val Lys Lys Arg Ala Leu Ser Pro Gln Gln Val Thr
245 250 255

Arg Leu Pro Ser Gly Tyr Gly Leu Ala Tyr Arg Leu Thr Pro Gly Val
260 265 270

His Glu Leu Pro Val Gln Gln Pro Glu His Arg Ile Leu Lys Val Ser
275 280 285

Met Lys Glu Leu Asn Lys Lys Thr Pro Leu Leu Thr Glu Gly Gln Ala
290 295 300

Ile Cys Phe Thr Ile Leu Gly Val Leu Thr Ser Leu Val Val Leu Gly
305 310 315 320

Thr Val Gly Ile Val Phe Leu Asn Lys Cys Glu Thr Trp Val Ser Asn
325 330 335

Leu Arg Tyr Asn His Met Leu Arg Lys Lys Lys Asn Leu Leu Leu Gln
340 345 350

Tyr Asn Ser Gly Glu Asp Leu Ala Val Asn Ile Ile Phe Pro Glu Lys
355 360 365

Ile Asp Met Thr Thr Phe Ser Lys Glu Ala Gly Asp Glu Glu Ile
370 375 380

<210> 56
<211> 392
<212> PRT
<213> Artificial Sequence

<220>
<223> MCP_HUMAN

<400> 56
Met Glu Pro Pro Gly Arg Arg Glu Cys Pro Phe Pro Ser Trp Arg Phe
1 5 10 15

```
Pro Gly Leu Leu Leu Ala Ala Met Val Leu Leu Leu Tyr Ser Phe Ser
            20                  25                  30

Asp Ala Cys Glu Glu Pro Pro Thr Phe Glu Ala Met Glu Leu Ile Gly
        35                  40                  45

Lys Pro Lys Pro Tyr Tyr Glu Ile Gly Glu Arg Val Asp Tyr Lys Cys
    50                  55                  60

Lys Lys Gly Tyr Phe Tyr Ile Pro Pro Leu Ala Thr His Thr Ile Cys
65                  70                  75                  80

Asp Arg Asn His Thr Trp Leu Pro Val Ser Asp Asp Ala Cys Tyr Arg
                85                  90                  95

Glu Thr Cys Pro Tyr Ile Arg Asp Pro Leu Asn Gly Gln Ala Val Pro
            100                 105                 110

Ala Asn Gly Thr Tyr Glu Phe Gly Tyr Gln Met His Phe Ile Cys Asn
        115                 120                 125

Glu Gly Tyr Tyr Leu Ile Gly Glu Glu Ile Leu Tyr Cys Glu Leu Lys
    130                 135                 140

Gly Ser Val Ala Ile Trp Ser Gly Lys Pro Pro Ile Cys Glu Lys Val
145                 150                 155                 160

Leu Cys Thr Pro Pro Pro Lys Ile Lys Asn Gly Lys His Thr Phe Ser
                165                 170                 175

Glu Val Glu Val Phe Glu Tyr Leu Asp Ala Val Thr Tyr Ser Cys Asp
            180                 185                 190

Pro Ala Pro Gly Pro Asp Pro Phe Ser Leu Ile Gly Glu Ser Thr Ile
        195                 200                 205

Tyr Cys Gly Asp Asn Ser Val Trp Ser Arg Ala Ala Pro Glu Cys Lys
    210                 215                 220

Val Val Lys Cys Arg Phe Pro Val Val Glu Asn Gly Lys Gln Ile Ser
225                 230                 235                 240

Gly Phe Gly Lys Lys Phe Tyr Tyr Lys Ala Thr Val Met Phe Glu Cys
                245                 250                 255

Asp Lys Gly Phe Tyr Leu Asp Gly Ser Asp Thr Ile Val Cys Asp Ser
            260                 265                 270

Asn Ser Thr Trp Asp Pro Pro Val Pro Lys Cys Leu Lys Val Leu Pro
        275                 280                 285

Pro Ser Ser Thr Lys Pro Pro Ala Leu Ser His Ser Val Ser Thr Ser
    290                 295                 300

Ser Thr Thr Lys Ser Pro Ala Ser Ser Ala Ser Gly Pro Arg Pro Thr
305                 310                 315                 320

Tyr Lys Pro Pro Val Ser Asn Tyr Pro Gly Tyr Pro Lys Pro Glu Glu
                325                 330                 335

Gly Ile Leu Asp Ser Leu Asp Val Trp Val Ile Ala Val Ile Val Ile
            340                 345                 350
```

Ala Ile Val Val Gly Val Ala Val Ile Cys Val Val Pro Tyr Arg Tyr
355 360 365

Leu Gln Arg Arg Lys Lys Lys Gly Thr Tyr Leu Thr Asp Glu Thr His
370 375 380

Arg Glu Val Lys Phe Thr Ser Leu
385 390

<210> 57
<211> 392
<212> PRT
<213> Artificial Sequence

<220>
<223> PRP1_HUMAN

<400> 57
Met Leu Leu Ile Leu Leu Ser Val Ala Leu Leu Ala Leu Ser Ser Ala
1 5 10 15

Gln Asn Leu Asn Glu Asp Val Ser Gln Glu Glu Ser Pro Ser Leu Ile
20 25 30

Ala Gly Asn Pro Gln Gly Pro Ser Pro Gln Gly Gly Asn Lys Pro Gln
35 40 45

Gly Pro Pro Pro Pro Pro Gly Lys Pro Gln Gly Pro Pro Pro Gln Gly
50 55 60

Gly Asn Lys Pro Gln Gly Pro Pro Pro Pro Gly Lys Pro Gln Gly Pro
65 70 75 80

Pro Pro Gln Gly Asp Lys Ser Arg Ser Pro Arg Ser Pro Pro Gly Lys
85 90 95

Pro Gln Gly Pro Pro Pro Gln Gly Gly Asn Gln Pro Gln Gly Pro Pro
100 105 110

Pro Pro Pro Gly Lys Pro Gln Gly Pro Pro Pro Gln Gly Gly Asn Lys
115 120 125

Pro Gln Gly Pro Pro Pro Pro Gly Lys Pro Gln Gly Pro Pro Pro Gln
130 135 140

Gly Asp Lys Ser Gln Ser Pro Arg Ser Pro Pro Gly Lys Pro Gln Gly
145 150 155 160

Pro Pro Pro Gln Gly Gly Asn Gln Pro Gln Gly Pro Pro Pro Pro Pro
165 170 175

Gly Lys Pro Gln Gly Pro Pro Pro Gln Gly Gly Asn Lys Pro Gln Gly
180 185 190

Pro Pro Pro Pro Gly Lys Pro Gln Gly Pro Pro Pro Gln Gly Asp Lys
195 200 205

Ser Gln Ser Pro Arg Ser Pro Pro Gly Lys Pro Gln Gly Pro Pro Pro
210 215 220

Gln Gly Gly Asn Gln Pro Gln Gly Pro Pro Pro Pro Pro Gly Lys Pro

225 230 235 240

Gln Gly Pro Pro Gln Gln Gly Gly Asn Arg Pro Gln Gly Pro Pro Pro
245 250 255

Pro Gly Lys Pro Gln Gly Pro Pro Pro Gln Gly Asp Lys Ser Arg Ser
260 265 270

Pro Gln Ser Pro Pro Gly Lys Pro Gln Gly Pro Pro Pro Gln Gly Gly
275 280 285

Asn Gln Pro Gln Gly Pro Pro Pro Pro Pro Gly Lys Pro Gln Gly Pro
290 295 300

Pro Pro Gln Gly Gly Asn Lys Pro Gln Gly Pro Pro Pro Pro Gly Lys
305 310 315 320

Pro Gln Gly Pro Pro Ala Gln Gly Gly Ser Lys Ser Gln Ser Ala Arg
325 330 335

Ser Pro Pro Gly Lys Pro Gln Gly Pro Pro Gln Gln Glu Gly Asn Asn
340 345 350

Pro Gln Gly Pro Pro Pro Pro Ala Gly Gly Asn Pro Gln Gln Pro Gln
355 360 365

Ala Pro Pro Ala Gly Gln Pro Gln Gly Pro Pro Arg Pro Pro Gln Gly
370 375 380

Gly Arg Pro Ser Arg Pro Pro Gln
385 390

<210> 58
<211> 397
<212> PRT
<213> Artificial Sequence

<220>
<223> X3CL1_HUMAN

<400> 58
Met Ala Pro Ile Ser Leu Ser Trp Leu Leu Arg Leu Ala Thr Phe Cys
1 5 10 15

His Leu Thr Val Leu Leu Ala Gly Gln His His Gly Val Thr Lys Cys
20 25 30

Asn Ile Thr Cys Ser Lys Met Thr Ser Lys Ile Pro Val Ala Leu Leu
35 40 45

Ile His Tyr Gln Gln Asn Gln Ala Ser Cys Gly Lys Arg Ala Ile Ile
50 55 60

Leu Glu Thr Arg Gln His Arg Leu Phe Cys Ala Asp Pro Lys Glu Gln
65 70 75 80

Trp Val Lys Asp Ala Met Gln His Leu Asp Arg Gln Ala Ala Ala Leu
85 90 95

Thr Arg Asn Gly Gly Thr Phe Glu Lys Gln Ile Gly Glu Val Lys Pro
100 105 110

Arg Thr Thr Pro Ala Ala Gly Gly Met Asp Glu Ser Val Val Leu Glu
        115                 120                 125

Pro Glu Ala Thr Gly Glu Ser Ser Ser Leu Glu Pro Thr Pro Ser Ser
    130                 135                 140

Gln Glu Ala Gln Arg Ala Leu Gly Thr Ser Pro Glu Leu Pro Thr Gly
145                 150                 155                 160

Val Thr Gly Ser Ser Gly Thr Arg Leu Pro Pro Thr Pro Lys Ala Gln
                165                 170                 175

Asp Gly Gly Pro Val Gly Thr Glu Leu Phe Arg Val Pro Pro Val Ser
            180                 185                 190

Thr Ala Ala Thr Trp Gln Ser Ser Ala Pro His Gln Pro Gly Pro Ser
        195                 200                 205

Leu Trp Ala Glu Ala Lys Thr Ser Glu Ala Pro Ser Thr Gln Asp Pro
    210                 215                 220

Ser Thr Gln Ala Ser Thr Ala Ser Ser Pro Ala Pro Glu Glu Asn Ala
225                 230                 235                 240

Pro Ser Glu Gly Gln Arg Val Trp Gly Gln Gly Gln Ser Pro Arg Pro
                245                 250                 255

Glu Asn Ser Leu Glu Arg Glu Glu Met Gly Pro Val Pro Ala His Thr
            260                 265                 270

Asp Ala Phe Gln Asp Trp Gly Pro Gly Ser Met Ala His Val Ser Val
        275                 280                 285

Val Pro Val Ser Ser Glu Gly Thr Pro Ser Arg Glu Pro Val Ala Ser
    290                 295                 300

Gly Ser Trp Thr Pro Lys Ala Glu Glu Pro Ile His Ala Thr Met Asp
305                 310                 315                 320

Pro Gln Arg Leu Gly Val Leu Ile Thr Pro Val Pro Asp Ala Gln Ala
                325                 330                 335

Ala Thr Arg Arg Gln Ala Val Gly Leu Leu Ala Phe Leu Gly Leu Leu
            340                 345                 350

Phe Cys Leu Gly Val Ala Met Phe Thr Tyr Gln Ser Leu Gln Gly Cys
        355                 360                 365

Pro Arg Lys Met Ala Gly Glu Met Ala Glu Gly Leu Arg Tyr Ile Pro
    370                 375                 380

Arg Ser Cys Gly Ser Asn Ser Tyr Val Leu Val Pro Val
385                 390                 395

<210> 59
<211> 400
<212> PRT
<213> Artificial Sequence

<220>
<223> LEUK_HUMAN

<400> 59

Met Ala Thr Leu Leu Leu Leu Leu Gly Val Leu Val Val Ser Pro Asp
1 5 10 15

Ala Leu Gly Ser Thr Thr Ala Val Gln Thr Pro Thr Ser Gly Glu Pro
20 25 30

Leu Val Ser Thr Ser Glu Pro Leu Ser Ser Lys Met Tyr Thr Thr Ser
35 40 45

Ile Thr Ser Asp Pro Lys Ala Asp Ser Thr Gly Asp Gln Thr Ser Ala
50 55 60

Leu Pro Pro Ser Thr Ser Ile Asn Glu Gly Ser Pro Leu Trp Thr Ser
65 70 75 80

Ile Gly Ala Ser Thr Gly Ser Pro Leu Pro Glu Pro Thr Thr Tyr Gln
85 90 95

Glu Val Ser Ile Lys Met Ser Ser Val Pro Gln Glu Thr Pro His Ala
100 105 110

Thr Ser His Pro Ala Val Pro Ile Thr Ala Asn Ser Leu Gly Ser His
115 120 125

Thr Val Thr Gly Gly Thr Ile Thr Thr Asn Ser Pro Glu Thr Ser Ser
130 135 140

Arg Thr Ser Gly Ala Pro Val Thr Thr Ala Ala Ser Ser Leu Glu Thr
145 150 155 160

Ser Arg Gly Thr Ser Gly Pro Pro Leu Thr Met Ala Thr Val Ser Leu
165 170 175

Glu Thr Ser Lys Gly Thr Ser Gly Pro Pro Val Thr Met Ala Thr Asp
180 185 190

Ser Leu Glu Thr Ser Thr Gly Thr Thr Gly Pro Pro Val Thr Met Thr
195 200 205

Thr Gly Ser Leu Glu Pro Ser Ser Gly Ala Ser Gly Pro Gln Val Ser
210 215 220

Ser Val Lys Leu Ser Thr Met Met Ser Pro Thr Thr Ser Thr Asn Ala
225 230 235 240

Ser Thr Val Pro Phe Arg Asn Pro Asp Glu Asn Ser Arg Gly Met Leu
245 250 255

Pro Val Ala Val Leu Val Ala Leu Leu Ala Val Ile Val Leu Val Ala
260 265 270

Leu Leu Leu Leu Trp Arg Arg Arg Gln Lys Arg Arg Thr Gly Ala Leu
275 280 285

Val Leu Ser Arg Gly Gly Lys Arg Asn Gly Val Val Asp Ala Trp Ala
290 295 300

Gly Pro Ala Gln Val Pro Glu Glu Gly Ala Val Thr Val Thr Val Gly
305 310 315 320

Gly Ser Gly Gly Asp Lys Gly Ser Gly Phe Pro Asp Gly Glu Gly Ser
325 330 335

Ser Arg Arg Pro Thr Leu Thr Thr Phe Phe Gly Arg Arg Lys Ser Arg
340 345 350

Gln Gly Ser Leu Ala Met Glu Glu Leu Lys Ser Gly Ser Gly Pro Ser
355 360 365

Leu Lys Gly Glu Glu Glu Pro Leu Val Ala Ser Glu Asp Gly Ala Val
370 375 380

Asp Ala Pro Ala Pro Asp Glu Pro Glu Gly Gly Asp Gly Ala Ala Pro
385 390 395 400

<210> 60
<211> 410
<212> PRT
<213> Artificial Sequence

<220>
<223> LAMP2_HUMAN

<400> 60
Met Val Cys Phe Arg Leu Phe Pro Val Pro Gly Ser Gly Leu Val Leu
1 5 10 15

Val Cys Leu Val Leu Gly Ala Val Arg Ser Tyr Ala Leu Glu Leu Asn
20 25 30

Leu Thr Asp Ser Glu Asn Ala Thr Cys Leu Tyr Ala Lys Trp Gln Met
35 40 45

Asn Phe Thr Val Arg Tyr Glu Thr Thr Asn Lys Thr Tyr Lys Thr Val
50 55 60

Thr Ile Ser Asp His Gly Thr Val Thr Tyr Asn Gly Ser Ile Cys Gly
65 70 75 80

Asp Asp Gln Asn Gly Pro Lys Ile Ala Val Gln Phe Gly Pro Gly Phe
85 90 95

Ser Trp Ile Ala Asn Phe Thr Lys Ala Ala Ser Thr Tyr Ser Ile Asp
100 105 110

Ser Val Ser Phe Ser Tyr Asn Thr Gly Asp Asn Thr Thr Phe Pro Asp
115 120 125

Ala Glu Asp Lys Gly Ile Leu Thr Val Asp Glu Leu Leu Ala Ile Arg
130 135 140

Ile Pro Leu Asn Asp Leu Phe Arg Cys Asn Ser Leu Ser Thr Leu Glu
145 150 155 160

Lys Asn Asp Val Val Gln His Tyr Trp Asp Val Leu Val Gln Ala Phe
165 170 175

Val Gln Asn Gly Thr Val Ser Thr Asn Glu Phe Leu Cys Asp Lys Asp
180 185 190

Lys Thr Ser Thr Val Ala Pro Thr Ile His Thr Thr Val Pro Ser Pro

195 200 205

Thr Thr Thr Pro Thr Pro Lys Glu Lys Pro Glu Ala Gly Thr Tyr Ser
210 215 220

Val Asn Asn Gly Asn Asp Thr Cys Leu Leu Ala Thr Met Gly Leu Gln
225 230 235 240

Leu Asn Ile Thr Gln Asp Lys Val Ala Ser Val Ile Asn Ile Asn Pro
245 250 255

Asn Thr Thr His Ser Thr Gly Ser Cys Arg Ser His Thr Ala Leu Leu
260 265 270

Arg Leu Asn Ser Ser Thr Ile Lys Tyr Leu Asp Phe Val Phe Ala Val
275 280 285

Lys Asn Glu Asn Arg Phe Tyr Leu Lys Glu Val Asn Ile Ser Met Tyr
290 295 300

Leu Val Asn Gly Ser Val Phe Ser Ile Ala Asn Asn Asn Leu Ser Tyr
305 310 315 320

Trp Asp Ala Pro Leu Gly Ser Ser Tyr Met Cys Asn Lys Glu Gln Thr
325 330 335

Val Ser Val Ser Gly Ala Phe Gln Ile Asn Thr Phe Asp Leu Arg Val
340 345 350

Gln Pro Phe Asn Val Thr Gln Gly Lys Tyr Ser Thr Ala Gln Asp Cys
355 360 365

Ser Ala Asp Asp Asp Asn Phe Leu Val Pro Ile Ala Val Gly Ala Ala
370 375 380

Leu Ala Gly Val Leu Ile Leu Val Leu Leu Ala Tyr Phe Ile Gly Leu
385 390 395 400

Lys His His His Ala Gly Tyr Glu Gln Phe
405 410

<210> 61
<211> 417
<212> PRT
<213> Artificial Sequence

<220>
<223> LAMP1_HUMAN

<400> 61
Met Ala Ala Pro Gly Ser Ala Arg Arg Pro Leu Leu Leu Leu Leu Leu
1 5 10 15

Leu Leu Leu Leu Gly Leu Met His Cys Ala Ser Ala Ala Met Phe Met
20 25 30

Val Lys Asn Gly Asn Gly Thr Ala Cys Ile Met Ala Asn Phe Ser Ala
35 40 45

Ala Phe Ser Val Asn Tyr Asp Thr Lys Ser Gly Pro Lys Asn Met Thr
50 55 60

```
Phe Asp Leu Pro Ser Asp Ala Thr Val Val Leu Asn Arg Ser Ser Cys
 65                 70                  75                  80

Gly Lys Glu Asn Thr Ser Asp Pro Ser Leu Val Ile Ala Phe Gly Arg
                85                  90                  95

Gly His Thr Leu Thr Leu Asn Phe Thr Arg Asn Ala Thr Arg Tyr Ser
            100                 105                 110

Val Gln Leu Met Ser Phe Val Tyr Asn Leu Ser Asp Thr His Leu Phe
        115                 120                 125

Pro Asn Ala Ser Ser Lys Glu Ile Lys Thr Val Glu Ser Ile Thr Asp
    130                 135                 140

Ile Arg Ala Asp Ile Asp Lys Lys Tyr Arg Cys Val Ser Gly Thr Gln
145                 150                 155                 160

Val His Met Asn Asn Val Thr Val Thr Leu His Asp Ala Thr Ile Gln
                165                 170                 175

Ala Tyr Leu Ser Asn Ser Ser Phe Ser Arg Gly Glu Thr Arg Cys Glu
            180                 185                 190

Gln Asp Arg Pro Ser Pro Thr Thr Ala Pro Pro Ala Pro Pro Ser Pro
        195                 200                 205

Ser Pro Ser Pro Val Pro Lys Ser Pro Ser Val Asp Lys Tyr Asn Val
    210                 215                 220

Ser Gly Thr Asn Gly Thr Cys Leu Leu Ala Ser Met Gly Leu Gln Leu
225                 230                 235                 240

Asn Leu Thr Tyr Glu Arg Lys Asp Asn Thr Thr Val Thr Arg Leu Leu
                245                 250                 255

Asn Ile Asn Pro Asn Lys Thr Ser Ala Ser Gly Ser Cys Gly Ala His
            260                 265                 270

Leu Val Thr Leu Glu Leu His Ser Glu Gly Thr Thr Val Leu Leu Phe
        275                 280                 285

Gln Phe Gly Met Asn Ala Ser Ser Ser Arg Phe Phe Leu Gln Gly Ile
    290                 295                 300

Gln Leu Asn Thr Ile Leu Pro Asp Ala Arg Asp Pro Ala Phe Lys Ala
305                 310                 315                 320

Ala Asn Gly Ser Leu Arg Ala Leu Gln Ala Thr Val Gly Asn Ser Tyr
                325                 330                 335

Lys Cys Asn Ala Glu Glu His Val Arg Val Thr Lys Ala Phe Ser Val
            340                 345                 350

Asn Ile Phe Lys Val Trp Val Gln Ala Phe Lys Val Glu Gly Gly Gln
        355                 360                 365

Phe Gly Ser Val Glu Glu Cys Leu Leu Asp Glu Asn Ser Met Leu Ile
    370                 375                 380

Pro Ile Ala Val Gly Gly Ala Leu Ala Gly Leu Val Leu Ile Val Leu
385                 390                 395                 400
```

Ile Ala Tyr Leu Val Gly Arg Lys Arg Ser His Ala Gly Tyr Gln Thr
405 410 415

Ile

<210> 62
<211> 424
<212> PRT
<213> Artificial Sequence

<220>
<223> ZP3_HUMAN

<400> 62
Met Glu Leu Ser Tyr Arg Leu Phe Ile Cys Leu Leu Leu Trp Gly Ser
1 5 10 15

Thr Glu Leu Cys Tyr Pro Gln Pro Leu Trp Leu Leu Gln Gly Gly Ala
20 25 30

Ser His Pro Glu Thr Ser Val Gln Pro Val Leu Val Glu Cys Gln Glu
35 40 45

Ala Thr Leu Met Val Met Val Ser Lys Asp Leu Phe Gly Thr Gly Lys
50 55 60

Leu Ile Arg Ala Ala Asp Leu Thr Leu Gly Pro Glu Ala Cys Glu Pro
65 70 75 80

Leu Val Ser Met Asp Thr Glu Asp Val Val Arg Phe Glu Val Gly Leu
85 90 95

His Glu Cys Gly Asn Ser Met Gln Val Thr Asp Asp Ala Leu Val Tyr
100 105 110

Ser Thr Phe Leu Leu His Asp Pro Arg Pro Val Gly Asn Leu Ser Ile
115 120 125

Val Arg Thr Asn Arg Ala Glu Ile Pro Ile Glu Cys Arg Tyr Pro Arg
130 135 140

Gln Gly Asn Val Ser Ser Gln Ala Ile Leu Pro Thr Trp Leu Pro Phe
145 150 155 160

Arg Thr Thr Val Phe Ser Glu Glu Lys Leu Thr Phe Ser Leu Arg Leu
165 170 175

Met Glu Glu Asn Trp Asn Ala Glu Lys Arg Ser Pro Thr Phe His Leu
180 185 190

Gly Asp Ala Ala His Leu Gln Ala Glu Ile His Thr Gly Ser His Val
195 200 205

Pro Leu Arg Leu Phe Val Asp His Cys Val Ala Thr Pro Thr Pro Asp
210 215 220

Gln Asn Ala Ser Pro Tyr His Thr Ile Val Asp Phe His Gly Cys Leu
225 230 235 240

Val Asp Gly Leu Thr Asp Ala Ser Ser Ala Phe Lys Val Pro Arg Pro
245 250 255

Gly Pro Asp Thr Leu Gln Phe Thr Val Asp Val Phe His Phe Ala Asn
260 265 270

Asp Ser Arg Asn Met Ile Tyr Ile Thr Cys His Leu Lys Val Thr Leu
275 280 285

Ala Glu Gln Asp Pro Asp Glu Leu Asn Lys Ala Cys Ser Phe Ser Lys
290 295 300

Pro Ser Asn Ser Trp Phe Pro Val Glu Gly Ser Ala Asp Ile Cys Gln
305 310 315 320

Cys Cys Asn Lys Gly Asp Cys Gly Thr Pro Ser His Ser Arg Arg Gln
325 330 335

Pro His Val Met Ser Gln Trp Ser Arg Ser Ala Ser Arg Asn Arg Arg
340 345 350

His Val Thr Glu Glu Ala Asp Val Thr Val Gly Pro Leu Ile Phe Leu
355 360 365

Asp Arg Arg Gly Asp His Glu Val Glu Gln Trp Ala Leu Pro Ser Asp
370 375 380

Thr Ser Val Val Leu Leu Gly Val Gly Leu Ala Val Val Val Ser Leu
385 390 395 400

Thr Leu Thr Ala Val Ile Leu Val Leu Thr Arg Arg Cys Arg Thr Ala
405 410 415

Ser His Pro Val Ser Ala Ser Glu
420

<210> 63
<211> 430
<212> PRT
<213> Artificial Sequence

<220>
<223> K1C18_HUMAN

<400> 63
Met Ser Phe Thr Thr Arg Ser Thr Phe Ser Thr Asn Tyr Arg Ser Leu
1 5 10 15

Gly Ser Val Gln Ala Pro Ser Tyr Gly Ala Arg Pro Val Ser Ser Ala
20 25 30

Ala Ser Val Tyr Ala Gly Ala Gly Gly Ser Gly Ser Arg Ile Ser Val
35 40 45

Ser Arg Ser Thr Ser Phe Arg Gly Gly Met Gly Ser Gly Gly Leu Ala
50 55 60

Thr Gly Ile Ala Gly Gly Leu Ala Gly Met Gly Gly Ile Gln Asn Glu
65 70 75 80

Lys Glu Thr Met Gln Ser Leu Asn Asp Arg Leu Ala Ser Tyr Leu Asp

85 90 95

Arg Val Arg Ser Leu Glu Thr Glu Asn Arg Arg Leu Glu Ser Lys Ile
100 105 110

Arg Glu His Leu Glu Lys Lys Gly Pro Gln Val Arg Asp Trp Ser His
115 120 125

Tyr Phe Lys Ile Ile Glu Asp Leu Arg Ala Gln Ile Phe Ala Asn Thr
130 135 140

Val Asp Asn Ala Arg Ile Val Leu Gln Ile Asp Asn Ala Arg Leu Ala
145 150 155 160

Ala Asp Asp Phe Arg Val Lys Tyr Glu Thr Glu Leu Ala Met Arg Gln
165 170 175

Ser Val Glu Asn Asp Ile His Gly Leu Arg Lys Val Ile Asp Asp Thr
180 185 190

Asn Ile Thr Arg Leu Gln Leu Glu Thr Glu Ile Glu Ala Leu Lys Glu
195 200 205

Glu Leu Leu Phe Met Lys Lys Asn His Glu Glu Glu Val Lys Gly Leu
210 215 220

Gln Ala Gln Ile Ala Ser Ser Gly Leu Thr Val Glu Val Asp Ala Pro
225 230 235 240

Lys Ser Gln Asp Leu Ala Lys Ile Met Ala Asp Ile Arg Ala Gln Tyr
245 250 255

Asp Glu Leu Ala Arg Lys Asn Arg Glu Glu Leu Asp Lys Tyr Trp Ser
260 265 270

Gln Gln Ile Glu Glu Ser Thr Thr Val Val Thr Thr Gln Ser Ala Glu
275 280 285

Val Gly Ala Ala Glu Thr Thr Leu Thr Glu Leu Arg Arg Thr Val Gln
290 295 300

Ser Leu Glu Ile Asp Leu Asp Ser Met Arg Asn Leu Lys Ala Ser Leu
305 310 315 320

Glu Asn Ser Leu Arg Glu Val Glu Ala Arg Tyr Ala Leu Gln Met Glu
325 330 335

Gln Leu Asn Gly Ile Leu Leu His Leu Glu Ser Glu Leu Ala Gln Thr
340 345 350

Arg Ala Glu Gly Gln Arg Gln Ala Gln Glu Tyr Glu Ala Leu Leu Asn
355 360 365

Ile Lys Val Lys Leu Glu Ala Glu Ile Ala Thr Tyr Arg Arg Leu Leu
370 375 380

Glu Asp Gly Glu Asp Phe Asn Leu Gly Asp Ala Leu Asp Ser Ser Asn
385 390 395 400

Ser Met Gln Thr Ile Gln Lys Thr Thr Thr Arg Arg Ile Val Asp Gly
405 410 415

Lys Val Val Ser Glu Thr Asn Asp Thr Lys Val Leu Arg His

420 425 430

<210> 64
<211> 439
<212> PRT
<213> Artificial Sequence

<220>
<223> TICN1_HUMAN

<400> 64
Met Pro Ala Ile Ala Val Leu Ala Ala Ala Ala Ala Ala Trp Cys Phe
1 5 10 15

Leu Gln Val Glu Ser Arg His Leu Asp Ala Leu Ala Gly Gly Ala Gly
20 25 30

Pro Asn His Gly Asn Phe Leu Asp Asn Asp Gln Trp Leu Ser Thr Val
35 40 45

Ser Gln Tyr Asp Arg Asp Lys Tyr Trp Asn Arg Phe Arg Asp Asp Asp
50 55 60

Tyr Phe Arg Asn Trp Asn Pro Asn Lys Pro Phe Asp Gln Ala Leu Asp
65 70 75 80

Pro Ser Lys Asp Pro Cys Leu Lys Val Lys Cys Ser Pro His Lys Val
85 90 95

Cys Val Thr Gln Asp Tyr Gln Thr Ala Leu Cys Val Ser Arg Lys His
100 105 110

Leu Leu Pro Arg Gln Lys Lys Gly Asn Val Ala Gln Lys His Trp Val
115 120 125

Gly Pro Ser Asn Leu Val Lys Cys Lys Pro Cys Pro Val Ala Gln Ser
130 135 140

Ala Met Val Cys Gly Ser Asp Gly His Ser Tyr Thr Ser Lys Cys Lys
145 150 155 160

Leu Glu Phe His Ala Cys Ser Thr Gly Lys Ser Leu Ala Thr Leu Cys
165 170 175

Asp Gly Pro Cys Pro Cys Leu Pro Glu Pro Glu Pro Pro Lys His Lys
180 185 190

Ala Glu Arg Ser Ala Cys Thr Asp Lys Glu Leu Arg Asn Leu Ala Ser
195 200 205

Arg Leu Lys Asp Trp Phe Gly Ala Leu His Glu Asp Ala Asn Arg Val
210 215 220

Ile Lys Pro Thr Ser Ser Asn Thr Ala Gln Gly Arg Phe Asp Thr Ser
225 230 235 240

Ile Leu Pro Ile Cys Lys Asp Ser Leu Gly Trp Met Phe Asn Lys Leu
245 250 255

Asp Met Asn Tyr Asp Leu Leu Leu Asp Pro Ser Glu Ile Asn Ala Ile
260 265 270

Tyr Leu Asp Lys Tyr Glu Pro Cys Ile Lys Pro Leu Phe Asn Ser Cys
275 280 285

Asp Ser Phe Lys Asp Gly Lys Leu Ser Asn Asn Glu Trp Cys Tyr Cys
290 295 300

Phe Gln Lys Pro Gly Gly Leu Pro Cys Gln Asn Glu Met Asn Arg Ile
305 310 315 320

Gln Lys Leu Ser Lys Gly Lys Ser Leu Leu Gly Ala Phe Ile Pro Arg
325 330 335

Cys Asn Glu Glu Gly Tyr Tyr Lys Ala Thr Gln Cys His Gly Ser Thr
340 345 350

Gly Gln Cys Trp Cys Val Asp Lys Tyr Gly Asn Glu Leu Ala Gly Ser
355 360 365

Arg Lys Gln Gly Ala Val Ser Cys Glu Glu Glu Gln Glu Thr Ser Gly
370 375 380

Asp Phe Gly Ser Gly Gly Ser Val Val Leu Leu Asp Asp Leu Glu Tyr
385 390 395 400

Glu Arg Glu Leu Gly Pro Lys Asp Lys Glu Gly Lys Leu Arg Val His
405 410 415

Thr Arg Ala Val Thr Glu Asp Asp Glu Asp Glu Asp Asp Asp Lys Glu
420 425 430

Asp Glu Val Gly Tyr Ile Trp
435

<210> 65
<211> 442
<212> PRT
<213> Artificial Sequence

<220>
<223> SDC3_HUMAN

<400> 65
Met Lys Pro Gly Pro Pro His Arg Ala Gly Ala Ala His Gly Ala Gly
1 5 10 15

Ala Gly Ala Gly Ala Ala Ala Gly Pro Gly Ala Arg Gly Leu Leu Leu
20 25 30

Pro Pro Leu Leu Leu Leu Leu Leu Ala Gly Arg Ala Ala Gly Ala Gln
35 40 45

Arg Trp Arg Ser Glu Asn Phe Glu Arg Pro Val Asp Leu Glu Gly Ser
50 55 60

Gly Asp Asp Asp Ser Phe Pro Asp Asp Glu Leu Asp Asp Leu Tyr Ser
65 70 75 80

Gly Ser Gly Ser Gly Tyr Phe Glu Gln Glu Ser Gly Ile Glu Thr Ala
85 90 95

Met Arg Phe Ser Pro Asp Val Ala Leu Ala Val Ser Thr Thr Pro Ala
100 105 110

Val Leu Pro Thr Thr Asn Ile Gln Pro Val Gly Thr Pro Phe Glu Glu
115 120 125

Leu Pro Ser Glu Arg Pro Thr Leu Glu Pro Ala Thr Ser Pro Leu Val
130 135 140

Val Thr Glu Val Pro Glu Glu Pro Ser Gln Arg Ala Thr Thr Val Ser
145 150 155 160

Thr Thr Met Ala Thr Thr Ala Ala Thr Ser Thr Gly Asp Pro Thr Val
165 170 175

Ala Thr Val Pro Ala Thr Val Ala Thr Ala Thr Pro Ser Thr Pro Ala
180 185 190

Ala Pro Pro Phe Thr Ala Thr Thr Ala Val Ile Arg Thr Thr Gly Val
195 200 205

Arg Arg Leu Leu Pro Leu Pro Leu Thr Thr Val Ala Thr Ala Arg Ala
210 215 220

Thr Thr Pro Glu Ala Pro Ser Pro Pro Thr Thr Ala Ala Val Leu Asp
225 230 235 240

Thr Glu Ala Pro Thr Pro Arg Leu Val Ser Thr Ala Thr Ser Arg Pro
245 250 255

Arg Ala Leu Pro Arg Pro Ala Thr Thr Gln Glu Pro Asp Ile Pro Glu
260 265 270

Arg Ser Thr Leu Pro Leu Gly Thr Thr Ala Pro Gly Pro Thr Glu Val
275 280 285

Ala Gln Thr Pro Thr Pro Glu Thr Phe Leu Thr Thr Ile Arg Asp Glu
290 295 300

Pro Glu Val Pro Val Ser Gly Gly Pro Ser Gly Asp Phe Glu Leu Pro
305 310 315 320

Glu Glu Glu Thr Thr Gln Pro Asp Thr Ala Asn Glu Val Val Ala Val
325 330 335

Gly Gly Ala Ala Ala Lys Ala Ser Ser Pro Pro Gly Thr Leu Pro Lys
340 345 350

Gly Ala Arg Pro Gly Pro Gly Leu Leu Asp Asn Ala Ile Asp Ser Gly
355 360 365

Ser Ser Ala Ala Gln Leu Pro Gln Lys Ser Ile Leu Glu Arg Lys Glu
370 375 380

Val Leu Val Ala Val Ile Val Gly Gly Val Val Gly Ala Leu Phe Ala
385 390 395 400

Ala Phe Leu Val Thr Leu Leu Ile Tyr Arg Met Lys Lys Lys Asp Glu
405 410 415

Gly Ser Tyr Thr Leu Glu Glu Pro Lys Gln Ala Ser Val Thr Tyr Gln
420 425 430

Lys Pro Asp Lys Gln Glu Glu Phe Tyr Ala
        435                 440

<210> 66
<211> 457
<212> PRT
<213> Artificial Sequence

<220>
<223> CMGA_HUMAN

<400> 66
Met Arg Ser Ala Ala Val Leu Ala Leu Leu Leu Cys Ala Gly Gln Val
1               5                   10                  15

Thr Ala Leu Pro Val Asn Ser Pro Met Asn Lys Gly Asp Thr Glu Val
            20                  25                  30

Met Lys Cys Ile Val Glu Val Ile Ser Asp Thr Leu Ser Lys Pro Ser
        35                  40                  45

Pro Met Pro Val Ser Gln Glu Cys Phe Glu Thr Leu Arg Gly Asp Glu
    50                  55                  60

Arg Ile Leu Ser Ile Leu Arg His Gln Asn Leu Leu Lys Glu Leu Gln
65                  70                  75                  80

Asp Leu Ala Leu Gln Gly Ala Lys Glu Arg Ala His Gln Gln Lys Lys
                85                  90                  95

His Ser Gly Phe Glu Asp Glu Leu Ser Glu Val Leu Glu Asn Gln Ser
            100                 105                 110

Ser Gln Ala Glu Leu Lys Glu Ala Val Glu Glu Pro Ser Ser Lys Asp
        115                 120                 125

Val Met Glu Lys Arg Glu Asp Ser Lys Glu Ala Glu Lys Ser Gly Glu
    130                 135                 140

Ala Thr Asp Gly Ala Arg Pro Gln Ala Leu Pro Glu Pro Met Gln Glu
145                 150                 155                 160

Ser Lys Ala Glu Gly Asn Asn Gln Ala Pro Gly Glu Glu Glu Glu Glu
                165                 170                 175

Glu Glu Glu Ala Thr Asn Thr His Pro Pro Ala Ser Leu Pro Ser Gln
            180                 185                 190

Lys Tyr Pro Gly Pro Gln Ala Glu Gly Asp Ser Glu Gly Leu Ser Gln
        195                 200                 205

Gly Leu Val Asp Arg Glu Lys Gly Leu Ser Ala Glu Pro Gly Trp Gln
    210                 215                 220

Ala Lys Arg Glu Glu Glu Glu Glu Glu Glu Glu Glu Ala Glu Ala Gly
225                 230                 235                 240

Glu Glu Ala Val Pro Glu Glu Glu Gly Pro Thr Val Val Leu Asn Pro
                245                 250                 255

His Pro Ser Leu Gly Tyr Lys Glu Ile Arg Lys Gly Glu Ser Arg Ser

260 265 270

Glu Ala Leu Ala Val Asp Gly Ala Gly Lys Pro Gly Ala Glu Glu Ala
275 280 285

Gln Asp Pro Glu Gly Lys Gly Glu Gln Glu His Ser Gln Gln Lys Glu
290 295 300

Glu Glu Glu Glu Met Ala Val Val Pro Gln Gly Leu Phe Arg Gly Gly
305 310 315 320

Lys Ser Gly Glu Leu Glu Gln Glu Glu Glu Arg Leu Ser Lys Glu Trp
325 330 335

Glu Asp Ser Lys Arg Trp Ser Lys Met Asp Gln Leu Ala Lys Glu Leu
340 345 350

Thr Ala Glu Lys Arg Leu Glu Gly Gln Glu Glu Glu Glu Asp Asn Arg
355 360 365

Asp Ser Ser Met Lys Leu Ser Phe Arg Ala Arg Ala Tyr Gly Phe Arg
370 375 380

Gly Pro Gly Pro Gln Leu Arg Arg Gly Trp Arg Pro Ser Ser Arg Glu
385 390 395 400

Asp Ser Leu Glu Ala Gly Leu Pro Leu Gln Val Arg Gly Tyr Pro Glu
405 410 415

Glu Lys Lys Glu Glu Glu Gly Ser Ala Asn Arg Arg Pro Glu Asp Gln
420 425 430

Glu Leu Glu Ser Leu Ser Ala Ile Glu Ala Glu Leu Glu Lys Val Ala
435 440 445

His Gln Leu Gln Ala Leu Arg Arg Gly
450 455

<210> 67
<211> 458
<212> PRT
<213> Artificial Sequence

<220>
<223> CBPN_HUMAN

<400> 67
Met Ser Asp Leu Leu Ser Val Phe Leu His Leu Leu Leu Leu Phe Lys
1 5 10 15

Leu Val Ala Pro Val Thr Phe Arg His His Arg Tyr Asp Asp Leu Val
20 25 30

Arg Thr Leu Tyr Lys Val Gln Asn Glu Cys Pro Gly Ile Thr Arg Val
35 40 45

Tyr Ser Ile Gly Arg Ser Val Glu Gly Arg His Leu Tyr Val Leu Glu
50 55 60

Phe Ser Asp His Pro Gly Ile His Glu Pro Leu Glu Pro Glu Val Lys
65 70 75 80

Tyr Val Gly Asn Met His Gly Asn Glu Ala Leu Gly Arg Glu Leu Met
85 90 95

Leu Gln Leu Ser Glu Phe Leu Cys Glu Glu Phe Arg Asn Arg Asn Gln
100 105 110

Arg Ile Val Gln Leu Ile Gln Asp Thr Arg Ile His Ile Leu Pro Ser
115 120 125

Met Asn Pro Asp Gly Tyr Glu Val Ala Ala Ala Gln Gly Pro Asn Lys
130 135 140

Pro Gly Tyr Leu Val Gly Arg Asn Asn Ala Asn Gly Val Asp Leu Asn
145 150 155 160

Arg Asn Phe Pro Asp Leu Asn Thr Tyr Ile Tyr Tyr Asn Glu Lys Tyr
165 170 175

Gly Gly Pro Asn His His Leu Pro Leu Pro Asp Asn Trp Lys Ser Gln
180 185 190

Val Glu Pro Glu Thr Arg Ala Val Ile Arg Trp Met His Ser Phe Asn
195 200 205

Phe Val Leu Ser Ala Asn Leu His Gly Gly Ala Val Val Ala Asn Tyr
210 215 220

Pro Tyr Asp Lys Ser Phe Glu His Arg Val Arg Gly Val Arg Arg Thr
225 230 235 240

Ala Ser Thr Pro Thr Pro Asp Asp Lys Leu Phe Gln Lys Leu Ala Lys
245 250 255

Val Tyr Ser Tyr Ala His Gly Trp Met Phe Gln Gly Trp Asn Cys Gly
260 265 270

Asp Tyr Phe Pro Asp Gly Ile Thr Asn Gly Ala Ser Trp Tyr Ser Leu
275 280 285

Ser Lys Gly Met Gln Asp Phe Asn Tyr Leu His Thr Asn Cys Phe Glu
290 295 300

Ile Thr Leu Glu Leu Ser Cys Asp Lys Phe Pro Pro Glu Glu Glu Leu
305 310 315 320

Gln Arg Glu Trp Leu Gly Asn Arg Glu Ala Leu Ile Gln Phe Leu Glu
325 330 335

Gln Val His Gln Gly Ile Lys Gly Met Val Leu Asp Glu Asn Tyr Asn
340 345 350

Asn Leu Ala Asn Ala Val Ile Ser Val Ser Gly Ile Asn His Asp Val
355 360 365

Thr Ser Gly Asp His Gly Asp Tyr Phe Arg Leu Leu Leu Pro Gly Ile
370 375 380

Tyr Thr Val Ser Ala Thr Ala Pro Gly Tyr Asp Pro Glu Thr Val Thr
385 390 395 400

Val Thr Val Gly Pro Ala Glu Pro Thr Leu Val Asn Phe His Leu Lys
405 410 415

Arg Ser Ile Pro Gln Val Ser Pro Val Arg Arg Ala Pro Ser Arg Arg
420 425 430

His Gly Val Arg Ala Lys Val Gln Pro Gln Ala Arg Lys Lys Glu Met
435 440 445

Glu Met Arg Gln Leu Gln Arg Gly Pro Ala
450 455

<210> 68
<211> 461
<212> PRT
<213> Artificial Sequence

<220>
<223> FA9_HUMAN

<400> 68
Met Gln Arg Val Asn Met Ile Met Ala Glu Ser Pro Gly Leu Ile Thr
1 5 10 15

Ile Cys Leu Leu Gly Tyr Leu Leu Ser Ala Glu Cys Thr Val Phe Leu
20 25 30

Asp His Glu Asn Ala Asn Lys Ile Leu Asn Arg Pro Lys Arg Tyr Asn
35 40 45

Ser Gly Lys Leu Glu Glu Phe Val Gln Gly Asn Leu Glu Arg Glu Cys
50 55 60

Met Glu Glu Lys Cys Ser Phe Glu Glu Ala Arg Glu Val Phe Glu Asn
65 70 75 80

Thr Glu Arg Thr Thr Glu Phe Trp Lys Gln Tyr Val Asp Gly Asp Gln
85 90 95

Cys Glu Ser Asn Pro Cys Leu Asn Gly Gly Ser Cys Lys Asp Asp Ile
100 105 110

Asn Ser Tyr Glu Cys Trp Cys Pro Phe Gly Phe Glu Gly Lys Asn Cys
115 120 125

Glu Leu Asp Val Thr Cys Asn Ile Lys Asn Gly Arg Cys Glu Gln Phe
130 135 140

Cys Lys Asn Ser Ala Asp Asn Lys Val Val Cys Ser Cys Thr Glu Gly
145 150 155 160

Tyr Arg Leu Ala Glu Asn Gln Lys Ser Cys Glu Pro Ala Val Pro Phe
165 170 175

Pro Cys Gly Arg Val Ser Val Ser Gln Thr Ser Lys Leu Thr Arg Ala
180 185 190

Glu Thr Val Phe Pro Asp Val Asp Tyr Val Asn Ser Thr Glu Ala Glu
195 200 205

Thr Ile Leu Asp Asn Ile Thr Gln Ser Thr Gln Ser Phe Asn Asp Phe
210 215 220

Thr Arg Val Val Gly Gly Glu Asp Ala Lys Pro Gly Gln Phe Pro Trp
225 230 235 240

Gln Val Val Leu Asn Gly Lys Val Asp Ala Phe Cys Gly Gly Ser Ile
245 250 255

Val Asn Glu Lys Trp Ile Val Thr Ala Ala His Cys Val Glu Thr Gly
260 265 270

Val Lys Ile Thr Val Val Ala Gly Glu His Asn Ile Glu Glu Thr Glu
275 280 285

His Thr Glu Gln Lys Arg Asn Val Ile Arg Ile Ile Pro His His Asn
290 295 300

Tyr Asn Ala Ala Ile Asn Lys Tyr Asn His Asp Ile Ala Leu Leu Glu
305 310 315 320

Leu Asp Glu Pro Leu Val Leu Asn Ser Tyr Val Thr Pro Ile Cys Ile
325 330 335

Ala Asp Lys Glu Tyr Thr Asn Ile Phe Leu Lys Phe Gly Ser Gly Tyr
340 345 350

Val Ser Gly Trp Gly Arg Val Phe His Lys Gly Arg Ser Ala Leu Val
355 360 365

Leu Gln Tyr Leu Arg Val Pro Leu Val Asp Arg Ala Thr Cys Leu Arg
370 375 380

Ser Thr Lys Phe Thr Ile Tyr Asn Asn Met Phe Cys Ala Gly Phe His
385 390 395 400

Glu Gly Gly Arg Asp Ser Cys Gln Gly Asp Ser Gly Gly Pro His Val
405 410 415

Thr Glu Val Glu Gly Thr Ser Phe Leu Thr Gly Ile Ile Ser Trp Gly
420 425 430

Glu Glu Cys Ala Met Lys Gly Lys Tyr Gly Ile Tyr Thr Lys Val Ser
435 440 445

Arg Tyr Val Asn Trp Ile Lys Glu Lys Thr Lys Leu Thr
450 455 460

<210> 69
<211> 461
<212> PRT
<213> Artificial Sequence

<220>
<223> TNR1B_HUMAN

<400> 69
Met Ala Pro Val Ala Val Trp Ala Ala Leu Ala Val Gly Leu Glu Leu
1 5 10 15

Trp Ala Ala Ala His Ala Leu Pro Ala Gln Val Ala Phe Thr Pro Tyr
20 25 30

```
Ala Pro Glu Pro Gly Ser Thr Cys Arg Leu Arg Glu Tyr Tyr Asp Gln
        35                  40                  45

Thr Ala Gln Met Cys Cys Ser Lys Cys Ser Pro Gly Gln His Ala Lys
    50                  55                  60

Val Phe Cys Thr Lys Thr Ser Asp Thr Val Cys Asp Ser Cys Glu Asp
65                  70                  75                  80

Ser Thr Tyr Thr Gln Leu Trp Asn Trp Val Pro Glu Cys Leu Ser Cys
                85                  90                  95

Gly Ser Arg Cys Ser Ser Asp Gln Val Glu Thr Gln Ala Cys Thr Arg
            100                 105                 110

Glu Gln Asn Arg Ile Cys Thr Cys Arg Pro Gly Trp Tyr Cys Ala Leu
        115                 120                 125

Ser Lys Gln Glu Gly Cys Arg Leu Cys Ala Pro Leu Arg Lys Cys Arg
    130                 135                 140

Pro Gly Phe Gly Val Ala Arg Pro Gly Thr Glu Thr Ser Asp Val Val
145                 150                 155                 160

Cys Lys Pro Cys Ala Pro Gly Thr Phe Ser Asn Thr Thr Ser Ser Thr
                165                 170                 175

Asp Ile Cys Arg Pro His Gln Ile Cys Asn Val Val Ala Ile Pro Gly
            180                 185                 190

Asn Ala Ser Met Asp Ala Val Cys Thr Ser Thr Ser Pro Thr Arg Ser
        195                 200                 205

Met Ala Pro Gly Ala Val His Leu Pro Gln Pro Val Ser Thr Arg Ser
    210                 215                 220

Gln His Thr Gln Pro Thr Pro Glu Pro Ser Thr Ala Pro Ser Thr Ser
225                 230                 235                 240

Phe Leu Leu Pro Met Gly Pro Ser Pro Pro Ala Glu Gly Ser Thr Gly
                245                 250                 255

Asp Phe Ala Leu Pro Val Gly Leu Ile Val Gly Val Thr Ala Leu Gly
            260                 265                 270

Leu Leu Ile Ile Gly Val Val Asn Cys Val Ile Met Thr Gln Val Lys
        275                 280                 285

Lys Lys Pro Leu Cys Leu Gln Arg Glu Ala Lys Val Pro His Leu Pro
    290                 295                 300

Ala Asp Lys Ala Arg Gly Thr Gln Gly Pro Glu Gln Gln His Leu Leu
305                 310                 315                 320

Ile Thr Ala Pro Ser Ser Ser Ser Ser Ser Leu Glu Ser Ser Ala Ser
                325                 330                 335

Ala Leu Asp Arg Arg Ala Pro Thr Arg Asn Gln Pro Gln Ala Pro Gly
            340                 345                 350

Val Glu Ala Ser Gly Ala Gly Glu Ala Arg Ala Ser Thr Gly Ser Ser
        355                 360                 365
```

Asp Ser Ser Pro Gly Gly His Gly Thr Gln Val Asn Val Thr Cys Ile
    370                 375                 380

Val Asn Val Cys Ser Ser Ser Asp His Ser Ser Gln Cys Ser Ser Gln
385                 390                 395                 400

Ala Ser Ser Thr Met Gly Asp Thr Asp Ser Ser Pro Ser Glu Ser Pro
                405                 410                 415

Lys Asp Glu Gln Val Pro Phe Ser Lys Glu Glu Cys Ala Phe Arg Ser
            420                 425                 430

Gln Leu Glu Thr Pro Glu Thr Leu Leu Gly Ser Thr Glu Glu Lys Pro
        435                 440                 445

Leu Pro Leu Gly Val Pro Asp Ala Gly Met Lys Pro Ser
    450                 455                 460

<210> 70
<211> 466
<212> PRT
<213> Artificial Sequence

<220>
<223> VIME_HUMAN

<400> 70
Met Ser Thr Arg Ser Val Ser Ser Ser Ser Tyr Arg Arg Met Phe Gly
1               5                   10                  15

Gly Pro Gly Thr Ala Ser Arg Pro Ser Ser Ser Arg Ser Tyr Val Thr
            20                  25                  30

Thr Ser Thr Arg Thr Tyr Ser Leu Gly Ser Ala Leu Arg Pro Ser Thr
        35                  40                  45

Ser Arg Ser Leu Tyr Ala Ser Ser Pro Gly Gly Val Tyr Ala Thr Arg
    50                  55                  60

Ser Ser Ala Val Arg Leu Arg Ser Ser Val Pro Gly Val Arg Leu Leu
65                  70                  75                  80

Gln Asp Ser Val Asp Phe Ser Leu Ala Asp Ala Ile Asn Thr Glu Phe
                85                  90                  95

Lys Asn Thr Arg Thr Asn Glu Lys Val Glu Leu Gln Glu Leu Asn Asp
            100                 105                 110

Arg Phe Ala Asn Tyr Ile Asp Lys Val Arg Phe Leu Glu Gln Gln Asn
        115                 120                 125

Lys Ile Leu Leu Ala Glu Leu Glu Gln Leu Lys Gly Gln Gly Lys Ser
    130                 135                 140

Arg Leu Gly Asp Leu Tyr Glu Glu Glu Met Arg Glu Leu Arg Arg Gln
145                 150                 155                 160

Val Asp Gln Leu Thr Asn Asp Lys Ala Arg Val Glu Val Glu Arg Asp
                165                 170                 175

Asn Leu Ala Glu Asp Ile Met Arg Leu Arg Glu Lys Leu Gln Glu Glu
180 185 190

Met Leu Gln Arg Glu Glu Ala Glu Asn Thr Leu Gln Ser Phe Arg Gln
195 200 205

Asp Val Asp Asn Ala Ser Leu Ala Arg Leu Asp Leu Glu Arg Lys Val
210 215 220

Glu Ser Leu Gln Glu Glu Ile Ala Phe Leu Lys Lys Leu His Glu Glu
225 230 235 240

Glu Ile Gln Glu Leu Gln Ala Gln Ile Gln Glu Gln His Val Gln Ile
245 250 255

Asp Val Asp Val Ser Lys Pro Asp Leu Thr Ala Ala Leu Arg Asp Val
260 265 270

Arg Gln Gln Tyr Glu Ser Val Ala Ala Lys Asn Leu Gln Glu Ala Glu
275 280 285

Glu Trp Tyr Lys Ser Lys Phe Ala Asp Leu Ser Glu Ala Ala Asn Arg
290 295 300

Asn Asn Asp Ala Leu Arg Gln Ala Lys Gln Glu Ser Thr Glu Tyr Arg
305 310 315 320

Arg Gln Val Gln Ser Leu Thr Cys Glu Val Asp Ala Leu Lys Gly Thr
325 330 335

Asn Glu Ser Leu Glu Arg Gln Met Arg Glu Met Glu Glu Asn Phe Ala
340 345 350

Val Glu Ala Ala Asn Tyr Gln Asp Thr Ile Gly Arg Leu Gln Asp Glu
355 360 365

Ile Gln Asn Met Lys Glu Glu Met Ala Arg His Leu Arg Glu Tyr Gln
370 375 380

Asp Leu Leu Asn Val Lys Met Ala Leu Asp Ile Glu Ile Ala Thr Tyr
385 390 395 400

Arg Lys Leu Leu Glu Gly Glu Glu Ser Arg Ile Ser Leu Pro Leu Pro
405 410 415

Asn Phe Ser Ser Leu Asn Leu Arg Glu Thr Asn Leu Asp Ser Leu Pro
420 425 430

Leu Val Asp Thr His Ser Lys Arg Thr Leu Leu Ile Lys Thr Val Glu
435 440 445

Thr Arg Asp Gly Gln Val Ile Asn Glu Thr Ser Gln His His Asp Asp
450 455 460

Leu Glu
465

<210> 71
<211> 468
<212> PRT
<213> Artificial Sequence

<220>
<223> SCG3_HUMAN

<400> 71

```
Met Gly Phe Leu Gly Thr Gly Thr Trp Ile Leu Val Leu Val Leu Pro
1               5                   10                  15

Ile Gln Ala Phe Pro Lys Pro Gly Gly Ser Gln Asp Lys Ser Leu His
            20                  25                  30

Asn Arg Glu Leu Ser Ala Glu Arg Pro Leu Asn Glu Gln Ile Ala Glu
        35                  40                  45

Ala Glu Glu Asp Lys Ile Lys Lys Thr Tyr Pro Pro Glu Asn Lys Pro
    50                  55                  60

Gly Gln Ser Asn Tyr Ser Phe Val Asp Asn Leu Asn Leu Leu Lys Ala
65                  70                  75                  80

Ile Thr Glu Lys Glu Lys Ile Glu Lys Glu Arg Gln Ser Ile Arg Ser
                85                  90                  95

Ser Pro Leu Asp Asn Lys Leu Asn Val Glu Asp Val Asp Ser Thr Lys
            100                 105                 110

Asn Arg Lys Leu Ile Asp Asp Tyr Asp Ser Thr Lys Ser Gly Leu Asp
        115                 120                 125

His Lys Phe Gln Asp Asp Pro Asp Gly Leu His Gln Leu Asp Gly Thr
    130                 135                 140

Pro Leu Thr Ala Glu Asp Ile Val His Lys Ile Ala Ala Arg Ile Tyr
145                 150                 155                 160

Glu Glu Asn Asp Arg Ala Val Phe Asp Lys Ile Val Ser Lys Leu Leu
                165                 170                 175

Asn Leu Gly Leu Ile Thr Glu Ser Gln Ala His Thr Leu Glu Asp Glu
            180                 185                 190

Val Ala Glu Val Leu Gln Lys Leu Ile Ser Lys Glu Ala Asn Asn Tyr
        195                 200                 205

Glu Glu Asp Pro Asn Lys Pro Thr Ser Trp Thr Glu Asn Gln Ala Gly
    210                 215                 220

Lys Ile Pro Glu Lys Val Thr Pro Met Ala Ala Ile Gln Asp Gly Leu
225                 230                 235                 240

Ala Lys Gly Glu Asn Asp Glu Thr Val Ser Asn Thr Leu Thr Leu Thr
                245                 250                 255

Asn Gly Leu Glu Arg Arg Thr Lys Thr Tyr Ser Glu Asp Asn Phe Glu
            260                 265                 270

Glu Leu Gln Tyr Phe Pro Asn Phe Tyr Ala Leu Leu Lys Ser Ile Asp
        275                 280                 285

Ser Glu Lys Glu Ala Lys Glu Lys Glu Thr Leu Ile Thr Ile Met Lys
    290                 295                 300

Thr Leu Ile Asp Phe Val Lys Met Met Val Lys Tyr Gly Thr Ile Ser
```

305 310 315 320

Pro Glu Glu Gly Val Ser Tyr Leu Glu Asn Leu Asp Glu Met Ile Ala
325 330 335

Leu Gln Thr Lys Asn Lys Leu Glu Lys Asn Ala Thr Asp Asn Ile Ser
340 345 350

Lys Leu Phe Pro Ala Pro Ser Glu Lys Ser His Glu Glu Thr Asp Ser
355 360 365

Thr Lys Glu Glu Ala Ala Lys Met Glu Lys Glu Tyr Gly Ser Leu Lys
370 375 380

Asp Ser Thr Lys Asp Asp Asn Ser Asn Pro Gly Gly Lys Thr Asp Glu
385 390 395 400

Pro Lys Gly Lys Thr Glu Ala Tyr Leu Glu Ala Ile Arg Lys Asn Ile
405 410 415

Glu Trp Leu Lys Lys His Asp Lys Lys Gly Asn Lys Glu Asp Tyr Asp
420 425 430

Leu Ser Lys Met Arg Asp Phe Ile Asn Lys Gln Ala Asp Ala Tyr Val
435 440 445

Glu Lys Gly Ile Leu Asp Lys Glu Glu Ala Glu Ala Ile Lys Arg Ile
450 455 460

Tyr Ser Ser Leu
465

<210> 72
<211> 473
<212> PRT
<213> Artificial Sequence

<220>
<223> KCC4_HUMAN

<400> 72
Met Leu Lys Val Thr Val Pro Ser Cys Ser Ala Ser Ser Cys Ser Ser
1 5 10 15

Val Thr Ala Ser Ala Ala Pro Gly Thr Ala Ser Leu Val Pro Asp Tyr
20 25 30

Trp Ile Asp Gly Ser Asn Arg Asp Ala Leu Ser Asp Phe Phe Glu Val
35 40 45

Glu Ser Glu Leu Gly Arg Gly Ala Thr Ser Ile Val Tyr Arg Cys Lys
50 55 60

Gln Lys Gly Thr Gln Lys Pro Tyr Ala Leu Lys Val Leu Lys Lys Thr
65 70 75 80

Val Asp Lys Lys Ile Val Arg Thr Glu Ile Gly Val Leu Leu Arg Leu
85 90 95

Ser His Pro Asn Ile Ile Lys Leu Lys Glu Ile Phe Glu Thr Pro Thr
100 105 110

Glu Ile Ser Leu Val Leu Glu Leu Val Thr Gly Gly Glu Leu Phe Asp
115 120 125

Arg Ile Val Glu Lys Gly Tyr Tyr Ser Glu Arg Asp Ala Ala Asp Ala
130 135 140

Val Lys Gln Ile Leu Glu Ala Val Ala Tyr Leu His Glu Asn Gly Ile
145 150 155 160

Val His Arg Asp Leu Lys Pro Glu Asn Leu Leu Tyr Ala Thr Pro Ala
165 170 175

Pro Asp Ala Pro Leu Lys Ile Ala Asp Phe Gly Leu Ser Lys Ile Val
180 185 190

Glu His Gln Val Leu Met Lys Thr Val Cys Gly Thr Pro Gly Tyr Cys
195 200 205

Ala Pro Glu Ile Leu Arg Gly Cys Ala Tyr Gly Pro Glu Val Asp Met
210 215 220

Trp Ser Val Gly Ile Ile Thr Tyr Ile Leu Leu Cys Gly Phe Glu Pro
225 230 235 240

Phe Tyr Asp Glu Arg Gly Asp Gln Phe Met Phe Arg Arg Ile Leu Asn
245 250 255

Cys Glu Tyr Tyr Phe Ile Ser Pro Trp Trp Asp Glu Val Ser Leu Asn
260 265 270

Ala Lys Asp Leu Val Arg Lys Leu Ile Val Leu Asp Pro Lys Lys Arg
275 280 285

Leu Thr Thr Phe Gln Ala Leu Gln His Pro Trp Val Thr Gly Lys Ala
290 295 300

Ala Asn Phe Val His Met Asp Thr Ala Gln Lys Lys Leu Gln Glu Phe
305 310 315 320

Asn Ala Arg Arg Lys Leu Lys Ala Ala Val Lys Ala Val Val Ala Ser
325 330 335

Ser Arg Leu Gly Ser Ala Ser Ser Ser His Gly Ser Ile Gln Glu Ser
340 345 350

His Lys Ala Ser Arg Asp Pro Ser Pro Ile Gln Asp Gly Asn Glu Asp
355 360 365

Met Lys Ala Ile Pro Glu Gly Glu Lys Ile Gln Gly Asp Gly Ala Gln
370 375 380

Ala Ala Val Lys Gly Ala Gln Ala Glu Leu Met Lys Val Gln Ala Leu
385 390 395 400

Glu Lys Val Lys Gly Ala Asp Ile Asn Ala Glu Glu Ala Pro Lys Met
405 410 415

Val Pro Lys Ala Val Glu Asp Gly Ile Lys Val Ala Asp Leu Glu Leu
420 425 430

Glu Glu Gly Leu Ala Glu Glu Lys Leu Lys Thr Val Glu Glu Ala Ala
435 440 445

Ala Pro Arg Glu Gly Gln Gly Ser Ser Ala Val Gly Phe Glu Val Pro
450 455 460

Gln Gln Asp Val Ile Leu Pro Glu Tyr
465 470

<210> 73
<211> 480
<212> PRT
<213> Artificial Sequence

<220>
<223> AKT1_HUMAN

<400> 73
Met Ser Asp Val Ala Ile Val Lys Glu Gly Trp Leu His Lys Arg Gly
1 5 10 15

Glu Tyr Ile Lys Thr Trp Arg Pro Arg Tyr Phe Leu Leu Lys Asn Asp
20 25 30

Gly Thr Phe Ile Gly Tyr Lys Glu Arg Pro Gln Asp Val Asp Gln Arg
35 40 45

Glu Ala Pro Leu Asn Asn Phe Ser Val Ala Gln Cys Gln Leu Met Lys
50 55 60

Thr Glu Arg Pro Arg Pro Asn Thr Phe Ile Ile Arg Cys Leu Gln Trp
65 70 75 80

Thr Thr Val Ile Glu Arg Thr Phe His Val Glu Thr Pro Glu Glu Arg
85 90 95

Glu Glu Trp Thr Thr Ala Ile Gln Thr Val Ala Asp Gly Leu Lys Lys
100 105 110

Gln Glu Glu Glu Glu Met Asp Phe Arg Ser Gly Ser Pro Ser Asp Asn
115 120 125

Ser Gly Ala Glu Glu Met Glu Val Ser Leu Ala Lys Pro Lys His Arg
130 135 140

Val Thr Met Asn Glu Phe Glu Tyr Leu Lys Leu Leu Gly Lys Gly Thr
145 150 155 160

Phe Gly Lys Val Ile Leu Val Lys Glu Lys Ala Thr Gly Arg Tyr Tyr
165 170 175

Ala Met Lys Ile Leu Lys Lys Glu Val Ile Val Ala Lys Asp Glu Val
180 185 190

Ala His Thr Leu Thr Glu Asn Arg Val Leu Gln Asn Ser Arg His Pro
195 200 205

Phe Leu Thr Ala Leu Lys Tyr Ser Phe Gln Thr His Asp Arg Leu Cys
210 215 220

Phe Val Met Glu Tyr Ala Asn Gly Gly Glu Leu Phe Phe His Leu Ser
225 230 235 240

Arg Glu Arg Val Phe Ser Glu Asp Arg Ala Arg Phe Tyr Gly Ala Glu
245 250 255

Ile Val Ser Ala Leu Asp Tyr Leu His Ser Glu Lys Asn Val Val Tyr
260 265 270

Arg Asp Leu Lys Leu Glu Asn Leu Met Leu Asp Lys Asp Gly His Ile
275 280 285

Lys Ile Thr Asp Phe Gly Leu Cys Lys Glu Gly Ile Lys Asp Gly Ala
290 295 300

Thr Met Lys Thr Phe Cys Gly Thr Pro Glu Tyr Leu Ala Pro Glu Val
305 310 315 320

Leu Glu Asp Asn Asp Tyr Gly Arg Ala Val Asp Trp Trp Gly Leu Gly
325 330 335

Val Val Met Tyr Glu Met Met Cys Gly Arg Leu Pro Phe Tyr Asn Gln
340 345 350

Asp His Glu Lys Leu Phe Glu Leu Ile Leu Met Glu Glu Ile Arg Phe
355 360 365

Pro Arg Thr Leu Gly Pro Glu Ala Lys Ser Leu Leu Ser Gly Leu Leu
370 375 380

Lys Lys Asp Pro Lys Gln Arg Leu Gly Gly Gly Ser Glu Asp Ala Lys
385 390 395 400

Glu Ile Met Gln His Arg Phe Phe Ala Gly Ile Val Trp Gln His Val
405 410 415

Tyr Glu Lys Lys Leu Ser Pro Pro Phe Lys Pro Gln Val Thr Ser Glu
420 425 430

Thr Asp Thr Arg Tyr Phe Asp Glu Glu Phe Thr Ala Gln Met Ile Thr
435 440 445

Ile Thr Pro Pro Asp Gln Asp Asp Ser Met Glu Cys Val Asp Ser Glu
450 455 460

Arg Arg Pro His Phe Pro Gln Phe Ser Tyr Ser Ala Ser Gly Thr Ala
465 470 475 480

<210> 74
<211> 481
<212> PRT
<213> Artificial Sequence

<220>
<223> AKT2_HUMAN

<400> 74
Met Asn Glu Val Ser Val Ile Lys Glu Gly Trp Leu His Lys Arg Gly
1 5 10 15

Glu Tyr Ile Lys Thr Trp Arg Pro Arg Tyr Phe Leu Leu Lys Ser Asp
20 25 30

Gly Ser Phe Ile Gly Tyr Lys Glu Arg Pro Glu Ala Pro Asp Gln Thr

35 40 45

Leu Pro Pro Leu Asn Asn Phe Ser Val Ala Glu Cys Gln Leu Met Lys
50 55 60

Thr Glu Arg Pro Arg Pro Asn Thr Phe Val Ile Arg Cys Leu Gln Trp
65 70 75 80

Thr Thr Val Ile Glu Arg Thr Phe His Val Asp Ser Pro Asp Glu Arg
85 90 95

Glu Glu Trp Met Arg Ala Ile Gln Met Val Ala Asn Ser Leu Lys Gln
100 105 110

Arg Ala Pro Gly Glu Asp Pro Met Asp Tyr Lys Cys Gly Ser Pro Ser
115 120 125

Asp Ser Ser Thr Thr Glu Glu Met Glu Val Ala Val Ser Lys Ala Arg
130 135 140

Ala Lys Val Thr Met Asn Asp Phe Asp Tyr Leu Lys Leu Leu Gly Lys
145 150 155 160

Gly Thr Phe Gly Lys Val Ile Leu Val Arg Glu Lys Ala Thr Gly Arg
165 170 175

Tyr Tyr Ala Met Lys Ile Leu Arg Lys Glu Val Ile Ile Ala Lys Asp
180 185 190

Glu Val Ala His Thr Val Thr Glu Ser Arg Val Leu Gln Asn Thr Arg
195 200 205

His Pro Phe Leu Thr Ala Leu Lys Tyr Ala Phe Gln Thr His Asp Arg
210 215 220

Leu Cys Phe Val Met Glu Tyr Ala Asn Gly Gly Glu Leu Phe Phe His
225 230 235 240

Leu Ser Arg Glu Arg Val Phe Thr Glu Glu Arg Ala Arg Phe Tyr Gly
245 250 255

Ala Glu Ile Val Ser Ala Leu Glu Tyr Leu His Ser Arg Asp Val Val
260 265 270

Tyr Arg Asp Ile Lys Leu Glu Asn Leu Met Leu Asp Lys Asp Gly His
275 280 285

Ile Lys Ile Thr Asp Phe Gly Leu Cys Lys Glu Gly Ile Ser Asp Gly
290 295 300

Ala Thr Met Lys Thr Phe Cys Gly Thr Pro Glu Tyr Leu Ala Pro Glu
305 310 315 320

Val Leu Glu Asp Asn Asp Tyr Gly Arg Ala Val Asp Trp Trp Gly Leu
325 330 335

Gly Val Val Met Tyr Glu Met Met Cys Gly Arg Leu Pro Phe Tyr Asn
340 345 350

Gln Asp His Glu Arg Leu Phe Glu Leu Ile Leu Met Glu Glu Ile Arg
355 360 365

Phe Pro Arg Thr Leu Ser Pro Glu Ala Lys Ser Leu Leu Ala Gly Leu

370 375 380

Leu Lys Lys Asp Pro Lys Gln Arg Leu Gly Gly Gly Pro Ser Asp Ala
385 390 395 400

Lys Glu Val Met Glu His Arg Phe Phe Leu Ser Ile Asn Trp Gln Asp
405 410 415

Val Val Gln Lys Lys Leu Leu Pro Pro Phe Lys Pro Gln Val Thr Ser
420 425 430

Glu Val Asp Thr Arg Tyr Phe Asp Asp Glu Phe Thr Ala Gln Ser Ile
435 440 445

Thr Ile Thr Pro Pro Asp Arg Tyr Asp Ser Leu Gly Leu Leu Glu Leu
450 455 460

Asp Gln Arg Thr His Phe Pro Gln Phe Ser Tyr Ser Ala Ser Ile Arg
465 470 475 480

Glu

<210> 75
<211> 481
<212> PRT
<213> Artificial Sequence

<220>
<223> G37L1_HUMAN

<400> 75
Met Arg Trp Leu Trp Pro Leu Ala Val Ser Leu Ala Val Ile Leu Ala
1 5 10 15

Val Gly Leu Ser Arg Val Ser Gly Gly Ala Pro Leu His Leu Gly Arg
20 25 30

His Arg Ala Glu Thr Gln Glu Gln Gln Ser Arg Ser Lys Arg Gly Thr
35 40 45

Glu Asp Glu Glu Ala Lys Gly Val Gln Gln Tyr Val Pro Glu Glu Trp
50 55 60

Ala Glu Tyr Pro Arg Pro Ile His Pro Ala Gly Leu Gln Pro Thr Lys
65 70 75 80

Pro Leu Val Ala Thr Ser Pro Asn Pro Gly Lys Asp Gly Gly Thr Pro
85 90 95

Asp Ser Gly Gln Glu Leu Arg Gly Asn Leu Thr Gly Ala Pro Gly Gln
100 105 110

Arg Leu Gln Ile Gln Asn Pro Leu Tyr Pro Val Thr Glu Ser Ser Tyr
115 120 125

Ser Ala Tyr Ala Ile Met Leu Leu Ala Leu Val Val Phe Ala Val Gly
130 135 140

Ile Val Gly Asn Leu Ser Val Met Cys Ile Val Trp His Ser Tyr Tyr
145 150 155 160

Leu Lys Ser Ala Trp Asn Ser Ile Leu Ala Ser Leu Ala Leu Trp Asp
165 170 175

Phe Leu Val Leu Phe Phe Cys Leu Pro Ile Val Ile Phe Asn Glu Ile
180 185 190

Thr Lys Gln Arg Leu Leu Gly Asp Val Ser Cys Arg Ala Val Pro Phe
195 200 205

Met Glu Val Ser Ser Leu Gly Val Thr Thr Phe Ser Leu Cys Ala Leu
210 215 220

Gly Ile Asp Arg Phe His Val Ala Thr Ser Thr Leu Pro Lys Val Arg
225 230 235 240

Pro Ile Glu Arg Cys Gln Ser Ile Leu Ala Lys Leu Ala Val Ile Trp
245 250 255

Val Gly Ser Met Thr Leu Ala Val Pro Glu Leu Leu Leu Trp Gln Leu
260 265 270

Ala Gln Glu Pro Ala Pro Thr Met Gly Thr Leu Asp Ser Cys Ile Met
275 280 285

Lys Pro Ser Ala Ser Leu Pro Glu Ser Leu Tyr Ser Leu Val Met Thr
290 295 300

Tyr Gln Asn Ala Arg Met Trp Trp Tyr Phe Gly Cys Tyr Phe Cys Leu
305 310 315 320

Pro Ile Leu Phe Thr Val Thr Cys Gln Leu Val Thr Trp Arg Val Arg
325 330 335

Gly Pro Pro Gly Arg Lys Ser Glu Cys Arg Ala Ser Lys His Glu Gln
340 345 350

Cys Glu Ser Gln Leu Asn Ser Thr Val Val Gly Leu Thr Val Val Tyr
355 360 365

Ala Phe Cys Thr Leu Pro Glu Asn Val Cys Asn Ile Val Val Ala Tyr
370 375 380

Leu Ser Thr Glu Leu Thr Arg Gln Thr Leu Asp Leu Leu Gly Leu Ile
385 390 395 400

Asn Gln Phe Ser Thr Phe Phe Lys Gly Ala Ile Thr Pro Val Leu Leu
405 410 415

Leu Cys Ile Cys Arg Pro Leu Gly Gln Ala Phe Leu Asp Cys Cys Cys
420 425 430

Cys Cys Cys Cys Glu Glu Cys Gly Gly Ala Ser Glu Ala Ser Ala Ala
435 440 445

Asn Gly Ser Asp Asn Lys Leu Lys Thr Glu Val Ser Ser Ser Ile Tyr
450 455 460

Phe His Lys Pro Arg Glu Ser Pro Pro Leu Leu Pro Leu Gly Thr Pro
465 470 475 480

Cys

<210> 76
<211> 490
<212> PRT
<213> Artificial Sequence

<220>
<223> TEKT3_HUMAN

<400> 76
Met Glu Arg Val Gly Cys Thr Leu Thr Thr Thr Tyr Ala His Pro Arg
1 5 10 15

Pro Thr Pro Thr Asn Phe Leu Pro Ala Ile Ser Thr Met Ala Ser Ser
20 25 30

Tyr Arg Asp Arg Phe Pro His Ser Asn Leu Thr His Ser Leu Ser Leu
35 40 45

Pro Trp Arg Pro Ser Thr Tyr Tyr Lys Val Ala Ser Asn Ser Pro Ser
50 55 60

Val Ala Pro Tyr Cys Thr Arg Ser Gln Arg Val Ser Glu Asn Thr Met
65 70 75 80

Leu Pro Phe Val Ser Asn Arg Thr Thr Phe Phe Thr Arg Tyr Thr Pro
85 90 95

Asp Asp Trp Tyr Arg Ser Asn Leu Thr Asn Tyr Gln Glu Ser Asn Thr
100 105 110

Ser Arg His Asn Ser Glu Lys Leu Arg Val Asp Thr Ser Arg Leu Ile
115 120 125

Gln Asp Lys Tyr Gln Gln Thr Arg Lys Thr Gln Ala Asp Thr Thr Gln
130 135 140

Asn Leu Gly Glu Arg Val Asn Asp Ile Gly Phe Trp Lys Ser Glu Ile
145 150 155 160

Ile His Glu Leu Asp Glu Met Ile Gly Glu Thr Asn Ala Leu Thr Asp
165 170 175

Val Lys Lys Arg Leu Glu Arg Ala Leu Met Glu Thr Glu Ala Pro Leu
180 185 190

Gln Val Ala Arg Glu Cys Leu Phe His Arg Glu Lys Arg Met Gly Ile
195 200 205

Asp Leu Val His Asp Glu Val Glu Ala Gln Leu Leu Thr Glu Val Asp
210 215 220

Thr Ile Leu Cys Cys Gln Glu Arg Met Lys Leu His Leu Asp Lys Ala
225 230 235 240

Ile Ala Gln Leu Ala Ala Asn Arg Ala Ser Gln His Glu Leu Glu Lys
245 250 255

Asp Leu Ser Asp Lys Gln Thr Ala Tyr Arg Ile Asp Asp Lys Cys His
260 265 270

His Leu Arg Asn Thr Ser Asp Gly Val Gly Tyr Phe Arg Gly Val Glu
        275                 280                 285

Arg Val Asp Ala Thr Val Ser Val Pro Glu Ser Trp Ala Lys Phe Thr
    290                 295                 300

Asp Asp Asn Ile Leu Arg Ser Gln Ser Glu Arg Ala Ala Ser Ala Lys
305                 310                 315                 320

Leu Arg Asp Asp Ile Glu Asn Leu Leu Val Val Thr Ala Asn Glu Met
                325                 330                 335

Trp Asn Gln Phe Asn Lys Val Asn Leu Ser Phe Thr Asn Arg Ile Ala
            340                 345                 350

Glu Thr Ala Asp Ala Lys Asn Lys Ile Gln Thr His Leu Ala Lys Thr
        355                 360                 365

Leu Gln Glu Ile Phe Gln Thr Glu Met Thr Ile Glu Ser Ile Lys Lys
    370                 375                 380

Ala Ile Lys Asp Lys Thr Ala Phe Leu Lys Val Ala Gln Thr Arg Leu
385                 390                 395                 400

Asp Glu Arg Thr Arg Arg Pro Asn Ile Glu Leu Cys Arg Asp Met Ala
                405                 410                 415

Gln Leu Arg Leu Val Asn Glu Val His Glu Val Asp Asp Thr Ile Gln
            420                 425                 430

Thr Leu Gln Gln Arg Leu Arg Asp Ala Glu Asp Thr Leu Gln Ser Leu
        435                 440                 445

Val His Ile Lys Ala Thr Leu Glu Tyr Asp Leu Ala Val Lys Ala Asn
    450                 455                 460

Ser Leu Tyr Ile Asp Gln Glu Lys Cys Met Ser Met Arg Lys Ser Tyr
465                 470                 475                 480

Pro Asn Thr Leu Arg Leu Val Gly Phe Cys
                485                 490

<210> 77
<211> 500
<212> PRT
<213> Artificial Sequence

<220>
<223> IC1_HUMAN

<400> 77
Met Ala Ser Arg Leu Thr Leu Leu Thr Leu Leu Leu Leu Leu Leu Ala
1               5                   10                  15

Gly Asp Arg Ala Ser Ser Asn Pro Asn Ala Thr Ser Ser Ser Ser Gln
            20                  25                  30

Asp Pro Glu Ser Leu Gln Asp Arg Gly Glu Gly Lys Val Ala Thr Thr
        35                  40                  45

Val Ile Ser Lys Met Leu Phe Val Glu Pro Ile Leu Glu Val Ser Ser

50 55 60

Leu Pro Thr Thr Asn Ser Thr Thr Asn Ser Ala Thr Lys Ile Thr Ala
65 70 75 80

Asn Thr Thr Asp Glu Pro Thr Thr Gln Pro Thr Thr Glu Pro Thr Thr
85 90 95

Gln Pro Thr Ile Gln Pro Thr Gln Pro Thr Thr Gln Leu Pro Thr Asp
100 105 110

Ser Pro Thr Gln Pro Thr Thr Gly Ser Phe Cys Pro Gly Pro Val Thr
115 120 125

Leu Cys Ser Asp Leu Glu Ser His Ser Thr Glu Ala Val Leu Gly Asp
130 135 140

Ala Leu Val Asp Phe Ser Leu Lys Leu Tyr His Ala Phe Ser Ala Met
145 150 155 160

Lys Lys Val Glu Thr Asn Met Ala Phe Ser Pro Phe Ser Ile Ala Ser
165 170 175

Leu Leu Thr Gln Val Leu Leu Gly Ala Gly Glu Asn Thr Lys Thr Asn
180 185 190

Leu Glu Ser Ile Leu Ser Tyr Pro Lys Asp Phe Thr Cys Val His Gln
195 200 205

Ala Leu Lys Gly Phe Thr Thr Lys Gly Val Thr Ser Val Ser Gln Ile
210 215 220

Phe His Ser Pro Asp Leu Ala Ile Arg Asp Thr Phe Val Asn Ala Ser
225 230 235 240

Arg Thr Leu Tyr Ser Ser Ser Pro Arg Val Leu Ser Asn Asn Ser Asp
245 250 255

Ala Asn Leu Glu Leu Ile Asn Thr Trp Val Ala Lys Asn Thr Asn Asn
260 265 270

Lys Ile Ser Arg Leu Leu Asp Ser Leu Pro Ser Asp Thr Arg Leu Val
275 280 285

Leu Leu Asn Ala Ile Tyr Leu Ser Ala Lys Trp Lys Thr Thr Phe Asp
290 295 300

Pro Lys Lys Thr Arg Met Glu Pro Phe His Phe Lys Asn Ser Val Ile
305 310 315 320

Lys Val Pro Met Met Asn Ser Lys Lys Tyr Pro Val Ala His Phe Ile
325 330 335

Asp Gln Thr Leu Lys Ala Lys Val Gly Gln Leu Gln Leu Ser His Asn
340 345 350

Leu Ser Leu Val Ile Leu Val Pro Gln Asn Leu Lys His Arg Leu Glu
355 360 365

Asp Met Glu Gln Ala Leu Ser Pro Ser Val Phe Lys Ala Ile Met Glu
370 375 380

Lys Leu Glu Met Ser Lys Phe Gln Pro Thr Leu Leu Thr Leu Pro Arg

385 390 395 400

Ile Lys Val Thr Thr Ser Gln Asp Met Leu Ser Ile Met Glu Lys Leu
405 410 415

Glu Phe Phe Asp Phe Ser Tyr Asp Leu Asn Leu Cys Gly Leu Thr Glu
420 425 430

Asp Pro Asp Leu Gln Val Ser Ala Met Gln His Gln Thr Val Leu Glu
435 440 445

Leu Thr Glu Thr Gly Val Glu Ala Ala Ala Ala Ser Ala Ile Ser Val
450 455 460

Ala Arg Thr Leu Leu Val Phe Glu Val Gln Gln Pro Phe Leu Phe Val
465 470 475 480

Leu Trp Asp Gln Gln His Lys Phe Pro Val Phe Met Gly Arg Val Tyr
485 490 495

Asp Pro Arg Ala
500

<210> 78
<211> 508
<212> PRT
<213> Artificial Sequence

<220>
<223> SRF_HUMAN

<400> 78
Met Leu Pro Thr Gln Ala Gly Ala Ala Ala Ala Leu Gly Arg Gly Ser
1 5 10 15

Ala Leu Gly Gly Ser Leu Asn Arg Thr Pro Thr Gly Arg Pro Gly Gly
20 25 30

Gly Gly Gly Thr Arg Gly Ala Asn Gly Gly Arg Val Pro Gly Asn Gly
35 40 45

Ala Gly Leu Gly Pro Gly Arg Leu Glu Arg Glu Ala Ala Ala Ala Ala
50 55 60

Ala Thr Thr Pro Ala Pro Thr Ala Gly Ala Leu Tyr Ser Gly Ser Glu
65 70 75 80

Gly Asp Ser Glu Ser Gly Glu Glu Glu Glu Leu Gly Ala Glu Arg Arg
85 90 95

Gly Leu Lys Arg Ser Leu Ser Glu Met Glu Ile Gly Met Val Val Gly
100 105 110

Gly Pro Glu Ala Ser Ala Ala Ala Thr Gly Gly Tyr Gly Pro Val Ser
115 120 125

Gly Ala Val Ser Gly Ala Lys Pro Gly Lys Lys Thr Arg Gly Arg Val
130 135 140

Lys Ile Lys Met Glu Phe Ile Asp Asn Lys Leu Arg Arg Tyr Thr Thr
145 150 155 160

```
Phe Ser Lys Arg Lys Thr Gly Ile Met Lys Lys Ala Tyr Glu Leu Ser
                165                 170                 175

Thr Leu Thr Gly Thr Gln Val Leu Leu Leu Val Ala Ser Glu Thr Gly
            180                 185                 190

His Val Tyr Thr Phe Ala Thr Arg Lys Leu Gln Pro Met Ile Thr Ser
        195                 200                 205

Glu Thr Gly Lys Ala Leu Ile Gln Thr Cys Leu Asn Ser Pro Asp Ser
    210                 215                 220

Pro Pro Arg Ser Asp Pro Thr Thr Asp Gln Arg Met Ser Ala Thr Gly
225                 230                 235                 240

Phe Glu Glu Thr Asp Leu Thr Tyr Gln Val Ser Glu Ser Asp Ser Ser
                245                 250                 255

Gly Glu Thr Lys Asp Thr Leu Lys Pro Ala Phe Thr Val Thr Asn Leu
            260                 265                 270

Pro Gly Thr Thr Ser Thr Ile Gln Thr Ala Pro Ser Thr Ser Thr Thr
        275                 280                 285

Met Gln Val Ser Ser Gly Pro Ser Phe Pro Ile Thr Asn Tyr Leu Ala
    290                 295                 300

Pro Val Ser Ala Ser Val Ser Pro Ser Ala Val Ser Ser Ala Asn Gly
305                 310                 315                 320

Thr Val Leu Lys Ser Thr Gly Ser Gly Pro Val Ser Ser Gly Gly Leu
                325                 330                 335

Met Gln Leu Pro Thr Ser Phe Thr Leu Met Pro Gly Gly Ala Val Ala
            340                 345                 350

Gln Gln Val Pro Val Gln Ala Ile Gln Val His Gln Ala Pro Gln Gln
        355                 360                 365

Ala Ser Pro Ser Arg Asp Ser Ser Thr Asp Leu Thr Gln Thr Ser Ser
    370                 375                 380

Ser Gly Thr Val Thr Leu Pro Ala Thr Ile Met Thr Ser Ser Val Pro
385                 390                 395                 400

Thr Thr Val Gly Gly His Met Met Tyr Pro Ser Pro His Ala Val Met
                405                 410                 415

Tyr Ala Pro Thr Ser Gly Leu Gly Asp Gly Ser Leu Thr Val Leu Asn
            420                 425                 430

Ala Phe Ser Gln Ala Pro Ser Thr Met Gln Val Ser His Ser Gln Val
        435                 440                 445

Gln Glu Pro Gly Gly Val Pro Gln Val Phe Leu Thr Ala Ser Ser Gly
    450                 455                 460

Thr Val Gln Ile Pro Val Ser Ala Val Gln Leu His Gln Met Ala Val
465                 470                 475                 480

Ile Gly Gln Gln Ala Gly Ser Ser Ser Asn Leu Thr Glu Leu Gln Val
                485                 490                 495
```

Val Asn Leu Asp Thr Ala His Ser Thr Lys Ser Glu
500 505

<210> 79
<211> 512
<212> PRT
<213> Artificial Sequence

<220>
<223> IGD_HUMAN

<400> 79
Arg Leu Gln Leu Gln Glu Ser Gly Pro Gly Leu Val Lys Pro Ser Glu
1 5 10 15

Thr Leu Ser Leu Thr Cys Ile Val Ser Gly Gly Pro Ile Arg Arg Thr
20 25 30

Gly Tyr Tyr Trp Gly Trp Ile Arg Gln Pro Pro Gly Lys Gly Leu Glu
35 40 45

Trp Ile Gly Gly Val Tyr Tyr Thr Gly Ser Ile Tyr Tyr Asn Pro Ser
50 55 60

Leu Arg Gly Arg Val Thr Ile Ser Val Asp Thr Ser Arg Asn Gln Phe
65 70 75 80

Ser Leu Asn Leu Arg Ser Met Ser Ala Ala Asp Thr Ala Met Tyr Tyr
85 90 95

Cys Ala Arg Gly Asn Pro Pro Pro Tyr Tyr Asp Ile Gly Thr Gly Ser
100 105 110

Asp Asp Gly Ile Asp Val Trp Gly Gln Gly Thr Thr Val His Val Ser
115 120 125

Ser Ala Pro Thr Lys Ala Pro Asp Val Phe Pro Ile Ile Ser Gly Cys
130 135 140

Arg His Pro Lys Asp Asn Ser Pro Val Val Leu Ala Cys Leu Ile Thr
145 150 155 160

Gly Tyr His Pro Thr Ser Val Thr Val Thr Trp Tyr Met Gly Thr Gln
165 170 175

Ser Gln Pro Gln Arg Thr Phe Pro Glu Ile Gln Arg Arg Asp Ser Tyr
180 185 190

Tyr Met Thr Ser Ser Gln Leu Ser Thr Pro Leu Gln Gln Trp Arg Gln
195 200 205

Gly Glu Tyr Lys Cys Val Val Gln His Thr Ala Ser Lys Ser Lys Lys
210 215 220

Glu Ile Phe Arg Trp Pro Glu Ser Pro Lys Ala Gln Ala Ser Ser Val
225 230 235 240

Pro Thr Ala Gln Pro Gln Ala Glu Gly Ser Leu Ala Lys Ala Thr Thr
245 250 255

```
Ala Pro Ala Thr Thr Arg Asn Thr Gly Arg Gly Gly Glu Glu Lys Lys
            260                 265                 270

Lys Glu Lys Glu Lys Glu Glu Gln Glu Glu Arg Glu Thr Lys Thr Pro
        275                 280                 285

Glu Cys Pro Ser His Thr Gln Pro Leu Gly Val Tyr Leu Leu Thr Pro
    290                 295                 300

Ala Val Gln Asp Leu Trp Leu Arg Asp Lys Ala Thr Phe Thr Cys Phe
305                 310                 315                 320

Val Val Gly Ser Asp Leu Lys Asp Ala His Leu Thr Trp Glu Val Ala
                325                 330                 335

Gly Lys Val Pro Thr Gly Gly Val Glu Glu Gly Leu Leu Glu Arg His
            340                 345                 350

Ser Asn Gly Ser Gln Ser Gln His Ser Arg Leu Thr Leu Pro Arg Ser
        355                 360                 365

Leu Trp Asn Ala Gly Thr Ser Val Thr Cys Thr Leu Asn His Pro Ser
    370                 375                 380

Leu Pro Pro Gln Arg Leu Met Ala Leu Arg Glu Pro Ala Ala Gln Ala
385                 390                 395                 400

Pro Val Lys Leu Ser Leu Asn Leu Leu Ala Ser Ser Asp Pro Pro Glu
                405                 410                 415

Ala Ala Ser Trp Leu Leu Cys Glu Val Ser Gly Phe Ser Pro Pro Asn
            420                 425                 430

Ile Leu Leu Met Trp Leu Glu Asp Gln Arg Glu Val Asn Thr Ser Gly
        435                 440                 445

Phe Ala Pro Ala Arg Pro Pro Pro Gln Pro Gly Ser Thr Thr Phe Trp
    450                 455                 460

Ala Trp Ser Val Leu Arg Val Pro Ala Pro Pro Ser Pro Gln Pro Ala
465                 470                 475                 480

Thr Tyr Thr Cys Val Val Ser His Glu Asp Ser Arg Thr Leu Leu Asn
                485                 490                 495

Ala Ser Arg Ser Leu Glu Val Ser Tyr Val Thr Asp His Gly Pro Met
            500                 505                 510
```

```
<210>  80
<211>  556
<212>  PRT
<213>  Artificial Sequence

<220>
<223>  GPC4_HUMAN

<400>  80
Met Ala Arg Phe Gly Leu Pro Ala Leu Leu Cys Thr Leu Ala Val Leu
1               5                   10                  15
```

Ser Ala Ala Leu Leu Ala Ala Glu Leu Lys Ser Lys Ser Cys Ser Glu
20 25 30

Val Arg Arg Leu Tyr Val Ser Lys Gly Phe Asn Lys Asn Asp Ala Pro
35 40 45

Leu His Glu Ile Asn Gly Asp His Leu Lys Ile Cys Pro Gln Gly Ser
50 55 60

Thr Cys Cys Ser Gln Glu Met Glu Glu Lys Tyr Ser Leu Gln Ser Lys
65 70 75 80

Asp Asp Phe Lys Ser Val Val Ser Glu Gln Cys Asn His Leu Gln Ala
85 90 95

Val Phe Ala Ser Arg Tyr Lys Lys Phe Asp Glu Phe Phe Lys Glu Leu
100 105 110

Leu Glu Asn Ala Glu Lys Ser Leu Asn Asp Met Phe Val Lys Thr Tyr
115 120 125

Gly His Leu Tyr Met Gln Asn Ser Glu Leu Phe Lys Asp Leu Phe Val
130 135 140

Glu Leu Lys Arg Tyr Tyr Val Val Gly Asn Val Asn Leu Glu Glu Met
145 150 155 160

Leu Asn Asp Phe Trp Ala Arg Leu Leu Glu Arg Met Phe Arg Leu Val
165 170 175

Asn Ser Gln Tyr His Phe Thr Asp Glu Tyr Leu Glu Cys Val Ser Lys
180 185 190

Tyr Thr Glu Gln Leu Lys Pro Phe Gly Asp Val Pro Arg Lys Leu Lys
195 200 205

Leu Gln Val Thr Arg Ala Phe Val Ala Ala Arg Thr Phe Ala Gln Gly
210 215 220

Leu Ala Val Ala Gly Asp Val Val Ser Lys Val Ser Val Val Asn Pro
225 230 235 240

Thr Ala Gln Cys Thr His Ala Leu Leu Lys Met Ile Tyr Cys Ser His
245 250 255

Cys Arg Gly Leu Val Thr Val Lys Pro Cys Tyr Asn Tyr Cys Ser Asn
260 265 270

Ile Met Arg Gly Cys Leu Ala Asn Gln Gly Asp Leu Asp Phe Glu Trp
275 280 285

Asn Asn Phe Ile Asp Ala Met Leu Met Val Ala Glu Arg Leu Glu Gly
290 295 300

Pro Phe Asn Ile Glu Ser Val Met Asp Pro Ile Asp Val Lys Ile Ser
305 310 315 320

Asp Ala Ile Met Asn Met Gln Asp Asn Ser Val Gln Val Ser Gln Lys
325 330 335

Val Phe Gln Gly Cys Gly Pro Pro Lys Pro Leu Pro Ala Gly Arg Ile
340 345 350

Ser Arg Ser Ile Ser Glu Ser Ala Phe Ser Ala Arg Phe Arg Pro His
355 360 365

His Pro Glu Glu Arg Pro Thr Thr Ala Ala Gly Thr Ser Leu Asp Arg
370 375 380

Leu Val Thr Asp Val Lys Glu Lys Leu Lys Gln Ala Lys Lys Phe Trp
385 390 395 400

Ser Ser Leu Pro Ser Asn Val Cys Asn Asp Glu Arg Met Ala Ala Gly
405 410 415

Asn Gly Asn Glu Asp Asp Cys Trp Asn Gly Lys Gly Lys Ser Arg Tyr
420 425 430

Leu Phe Ala Val Thr Gly Asn Gly Leu Ala Asn Gln Gly Asn Asn Pro
435 440 445

Glu Val Gln Val Asp Thr Ser Lys Pro Asp Ile Leu Ile Leu Arg Gln
450 455 460

Ile Met Ala Leu Arg Val Met Thr Ser Lys Met Lys Asn Ala Tyr Asn
465 470 475 480

Gly Asn Asp Val Asp Phe Phe Asp Ile Ser Asp Glu Ser Ser Gly Glu
485 490 495

Gly Ser Gly Ser Gly Cys Glu Tyr Gln Gln Cys Pro Ser Glu Phe Asp
500 505 510

Tyr Asn Ala Thr Asp His Ala Gly Lys Ser Ala Asn Glu Lys Ala Asp
515 520 525

Ser Ala Gly Val Arg Pro Gly Ala Gln Ala Tyr Leu Leu Thr Val Phe
530 535 540

Cys Ile Leu Phe Leu Val Met Gln Arg Glu Trp Arg
545 550 555

<210> 81
<211> 558
<212> PRT
<213> Artificial Sequence

<220>
<223> GPC1_HUMAN

<400> 81
Met Glu Leu Arg Ala Arg Gly Trp Trp Leu Leu Cys Ala Ala Ala Ala
1 5 10 15

Leu Val Ala Cys Ala Arg Gly Asp Pro Ala Ser Lys Ser Arg Ser Cys
20 25 30

Gly Glu Val Arg Gln Ile Tyr Gly Ala Lys Gly Phe Ser Leu Ser Asp
35 40 45

Val Pro Gln Ala Glu Ile Ser Gly Glu His Leu Arg Ile Cys Pro Gln
50 55 60

```
Gly Tyr Thr Cys Cys Thr Ser Glu Met Glu Glu Asn Leu Ala Asn Arg
 65                  70                  75                  80

Ser His Ala Glu Leu Glu Thr Ala Leu Arg Asp Ser Ser Arg Val Leu
                85                  90                  95

Gln Ala Met Leu Ala Thr Gln Leu Arg Ser Phe Asp Asp His Phe Gln
            100                 105                 110

His Leu Leu Asn Asp Ser Glu Arg Thr Leu Gln Ala Thr Phe Pro Gly
        115                 120                 125

Ala Phe Gly Glu Leu Tyr Thr Gln Asn Ala Arg Ala Phe Arg Asp Leu
    130                 135                 140

Tyr Ser Glu Leu Arg Leu Tyr Tyr Arg Gly Ala Asn Leu His Leu Glu
145                 150                 155                 160

Glu Thr Leu Ala Glu Phe Trp Ala Arg Leu Leu Glu Arg Leu Phe Lys
                165                 170                 175

Gln Leu His Pro Gln Leu Leu Leu Pro Asp Asp Tyr Leu Asp Cys Leu
            180                 185                 190

Gly Lys Gln Ala Glu Ala Leu Arg Pro Phe Gly Glu Ala Pro Arg Glu
        195                 200                 205

Leu Arg Leu Arg Ala Thr Arg Ala Phe Val Ala Ala Arg Ser Phe Val
    210                 215                 220

Gln Gly Leu Gly Val Ala Ser Asp Val Val Arg Lys Val Ala Gln Val
225                 230                 235                 240

Pro Leu Gly Pro Glu Cys Ser Arg Ala Val Met Lys Leu Val Tyr Cys
                245                 250                 255

Ala His Cys Leu Gly Val Pro Gly Ala Arg Pro Cys Pro Asp Tyr Cys
            260                 265                 270

Arg Asn Val Leu Lys Gly Cys Leu Ala Asn Gln Ala Asp Leu Asp Ala
        275                 280                 285

Glu Trp Arg Asn Leu Leu Asp Ser Met Val Leu Ile Thr Asp Lys Phe
    290                 295                 300

Trp Gly Thr Ser Gly Val Glu Ser Val Ile Gly Ser Val His Thr Trp
305                 310                 315                 320

Leu Ala Glu Ala Ile Asn Ala Leu Gln Asp Asn Arg Asp Thr Leu Thr
                325                 330                 335

Ala Lys Val Ile Gln Gly Cys Gly Asn Pro Lys Val Asn Pro Gln Gly
            340                 345                 350

Pro Gly Pro Glu Glu Lys Arg Arg Arg Gly Lys Leu Ala Pro Arg Glu
        355                 360                 365

Arg Pro Pro Ser Gly Thr Leu Glu Lys Leu Val Ser Glu Ala Lys Ala
    370                 375                 380

Gln Leu Arg Asp Val Gln Asp Phe Trp Ile Ser Leu Pro Gly Thr Leu
385                 390                 395                 400
```

Cys Ser Glu Lys Met Ala Leu Ser Thr Ala Ser Asp Asp Arg Cys Trp
405 410 415

Asn Gly Met Ala Arg Gly Arg Tyr Leu Pro Glu Val Met Gly Asp Gly
420 425 430

Leu Ala Asn Gln Ile Asn Asn Pro Glu Val Glu Val Asp Ile Thr Lys
435 440 445

Pro Asp Met Thr Ile Arg Gln Gln Ile Met Gln Leu Lys Ile Met Thr
450 455 460

Asn Arg Leu Arg Ser Ala Tyr Asn Gly Asn Asp Val Asp Phe Gln Asp
465 470 475 480

Ala Ser Asp Asp Gly Ser Gly Ser Gly Ser Gly Asp Gly Cys Leu Asp
485 490 495

Asp Leu Cys Ser Arg Lys Val Ser Arg Lys Ser Ser Ser Ser Arg Thr
500 505 510

Pro Leu Thr His Ala Leu Pro Gly Leu Ser Glu Gln Glu Gly Gln Lys
515 520 525

Thr Ser Ala Ala Ser Cys Pro Gln Pro Pro Thr Phe Leu Leu Pro Leu
530 535 540

Leu Leu Phe Leu Ala Leu Thr Val Ala Arg Pro Arg Trp Arg
545 550 555

<210> 82
<211> 572
<212> PRT
<213> Artificial Sequence

<220>
<223> GPC5_HUMAN

<400> 82
Met Asp Ala Gln Thr Trp Pro Val Gly Phe Arg Cys Leu Leu Leu Leu
1 5 10 15

Ala Leu Val Gly Ser Ala Arg Ser Glu Gly Val Gln Thr Cys Glu Glu
20 25 30

Val Arg Lys Leu Phe Gln Trp Arg Leu Leu Gly Ala Val Arg Gly Leu
35 40 45

Pro Asp Ser Pro Arg Ala Gly Pro Asp Leu Gln Val Cys Ile Ser Lys
50 55 60

Lys Pro Thr Cys Cys Thr Arg Lys Met Glu Glu Arg Tyr Gln Ile Ala
65 70 75 80

Ala Arg Gln Asp Met Gln Gln Phe Leu Gln Thr Ser Ser Ser Thr Leu
85 90 95

Lys Phe Leu Ile Ser Arg Asn Ala Ala Ala Phe Gln Glu Thr Leu Glu
100 105 110

Thr Leu Ile Lys Gln Ala Glu Asn Tyr Thr Ser Ile Leu Phe Cys Ser

115 120 125

Thr Tyr Arg Asn Met Ala Leu Glu Ala Ala Ala Ser Val Gln Glu Phe
130 135 140

Phe Thr Asp Val Gly Leu Tyr Leu Phe Gly Ala Asp Val Asn Pro Glu
145 150 155 160

Glu Phe Val Asn Arg Phe Phe Asp Ser Leu Phe Pro Leu Val Tyr Asn
165 170 175

His Leu Ile Asn Pro Gly Val Thr Asp Ser Ser Leu Glu Tyr Ser Glu
180 185 190

Cys Ile Arg Met Ala Arg Arg Asp Val Ser Pro Phe Gly Asn Ile Pro
195 200 205

Gln Arg Val Met Gly Gln Met Gly Arg Ser Leu Leu Pro Ser Arg Thr
210 215 220

Phe Leu Gln Ala Leu Asn Leu Gly Ile Glu Val Ile Asn Thr Thr Asp
225 230 235 240

Tyr Leu His Phe Ser Lys Glu Cys Ser Arg Ala Leu Leu Lys Met Gln
245 250 255

Tyr Cys Pro His Cys Gln Gly Leu Ala Leu Thr Lys Pro Cys Met Gly
260 265 270

Tyr Cys Leu Asn Val Met Arg Gly Cys Leu Ala His Met Ala Glu Leu
275 280 285

Asn Pro His Trp His Ala Tyr Ile Arg Ser Leu Glu Glu Leu Ser Asp
290 295 300

Ala Met His Gly Thr Tyr Asp Ile Gly His Val Leu Leu Asn Phe His
305 310 315 320

Leu Leu Val Asn Asp Ala Val Leu Gln Ala His Leu Asn Gly Gln Lys
325 330 335

Leu Leu Glu Gln Val Asn Arg Ile Cys Gly Arg Pro Val Arg Thr Pro
340 345 350

Thr Gln Ser Pro Arg Cys Ser Phe Asp Gln Ser Lys Glu Lys His Gly
355 360 365

Met Lys Thr Thr Thr Arg Asn Ser Glu Glu Thr Leu Ala Asn Arg Arg
370 375 380

Lys Glu Phe Ile Asn Ser Leu Arg Leu Tyr Arg Ser Phe Tyr Gly Gly
385 390 395 400

Leu Ala Asp Gln Leu Cys Ala Asn Glu Leu Ala Ala Ala Asp Gly Leu
405 410 415

Pro Cys Trp Asn Gly Glu Asp Ile Val Lys Ser Tyr Thr Gln Arg Val
420 425 430

Val Gly Asn Gly Ile Lys Ala Gln Ser Gly Asn Pro Glu Val Lys Val
435 440 445

Lys Gly Ile Asp Pro Val Ile Asn Gln Ile Ile Asp Lys Leu Lys His

450 455 460

Val Val Gln Leu Leu Gln Gly Arg Ser Pro Lys Pro Asp Lys Trp Glu
465 470 475 480

Leu Leu Gln Leu Gly Ser Gly Gly Gly Met Val Glu Gln Val Ser Gly
485 490 495

Asp Cys Asp Asp Glu Asp Gly Cys Gly Gly Ser Gly Ser Gly Glu Val
500 505 510

Lys Arg Thr Leu Lys Ile Thr Asp Trp Met Pro Asp Asp Met Asn Phe
515 520 525

Ser Asp Val Lys Gln Ile His Gln Thr Asp Thr Gly Ser Thr Leu Asp
530 535 540

Thr Thr Gly Ala Gly Cys Ala Val Ala Thr Glu Ser Met Thr Phe Thr
545 550 555 560

Leu Ile Ser Val Val Met Leu Leu Pro Gly Ile Trp
565 570

<210> 83
<211> 579
<212> PRT
<213> Artificial Sequence

<220>
<223> GPC2_HUMAN

<400> 83
Met Ser Ala Leu Arg Pro Leu Leu Leu Leu Leu Leu Pro Leu Cys Pro
1 5 10 15

Gly Pro Gly Pro Gly Pro Gly Ser Glu Ala Lys Val Thr Arg Ser Cys
20 25 30

Ala Glu Thr Arg Gln Val Leu Gly Ala Arg Gly Tyr Ser Leu Asn Leu
35 40 45

Ile Pro Pro Ala Leu Ile Ser Gly Glu His Leu Arg Val Cys Pro Gln
50 55 60

Glu Tyr Thr Cys Cys Ser Ser Glu Thr Glu Gln Arg Leu Ile Arg Glu
65 70 75 80

Thr Glu Ala Thr Phe Arg Gly Leu Val Glu Asp Ser Gly Ser Phe Leu
85 90 95

Val His Thr Leu Ala Ala Arg His Arg Lys Phe Asp Glu Phe Phe Leu
100 105 110

Glu Met Leu Ser Val Ala Gln His Ser Leu Thr Gln Leu Phe Ser His
115 120 125

Ser Tyr Gly Arg Leu Tyr Ala Gln His Ala Leu Ile Phe Asn Gly Leu
130 135 140

Phe Ser Arg Leu Arg Asp Phe Tyr Gly Glu Ser Gly Glu Gly Leu Asp
145 150 155 160

```
Asp Thr Leu Ala Asp Phe Trp Ala Gln Leu Leu Glu Arg Val Phe Pro
                165                 170                 175

Leu Leu His Pro Gln Tyr Ser Phe Pro Pro Asp Tyr Leu Leu Cys Leu
            180                 185                 190

Ser Arg Leu Ala Ser Ser Thr Asp Gly Ser Leu Gln Pro Phe Gly Asp
        195                 200                 205

Ser Pro Arg Arg Leu Arg Leu Gln Ile Thr Arg Thr Leu Val Ala Ala
    210                 215                 220

Arg Ala Phe Val Gln Gly Leu Glu Thr Gly Arg Asn Val Val Ser Glu
225                 230                 235                 240

Ala Leu Lys Val Pro Val Ser Glu Gly Cys Ser Gln Ala Leu Met Arg
                245                 250                 255

Leu Ile Gly Cys Pro Leu Cys Arg Gly Val Pro Ser Leu Met Pro Cys
            260                 265                 270

Gln Gly Phe Cys Leu Asn Val Val Arg Gly Cys Leu Ser Ser Arg Gly
        275                 280                 285

Leu Glu Pro Asp Trp Gly Asn Tyr Leu Asp Gly Leu Leu Ile Leu Ala
    290                 295                 300

Asp Lys Leu Gln Gly Pro Phe Ser Phe Glu Leu Thr Ala Glu Ser Ile
305                 310                 315                 320

Gly Val Lys Ile Ser Glu Gly Leu Met Tyr Leu Gln Glu Asn Ser Ala
                325                 330                 335

Lys Val Ser Ala Gln Val Phe Gln Glu Cys Gly Pro Pro Asp Pro Val
            340                 345                 350

Pro Ala Arg Asn Arg Arg Ala Pro Pro Pro Arg Glu Glu Ala Gly Arg
        355                 360                 365

Leu Trp Ser Met Val Thr Glu Glu Glu Arg Pro Thr Thr Ala Ala Gly
    370                 375                 380

Thr Asn Leu His Arg Leu Val Trp Glu Leu Arg Glu Arg Leu Ala Arg
385                 390                 395                 400

Met Arg Gly Phe Trp Ala Arg Leu Ser Leu Thr Val Cys Gly Asp Ser
                405                 410                 415

Arg Met Ala Ala Asp Ala Ser Leu Glu Ala Ala Pro Cys Trp Thr Gly
            420                 425                 430

Ala Gly Arg Gly Arg Tyr Leu Pro Pro Val Val Gly Gly Ser Pro Ala
        435                 440                 445

Glu Gln Val Asn Asn Pro Glu Leu Lys Val Asp Ala Ser Gly Pro Asp
    450                 455                 460

Val Pro Thr Arg Arg Arg Arg Leu Gln Leu Arg Ala Ala Thr Ala Arg
465                 470                 475                 480

Met Lys Thr Ala Ala Leu Gly His Asp Leu Asp Gly Gln Asp Ala Asp
                485                 490                 495
```

Glu Asp Ala Ser Gly Ser Gly Gly Gly Gln Gln Tyr Ala Asp Asp Trp
500 505 510

Met Ala Gly Ala Val Ala Pro Pro Ala Arg Pro Pro Arg Pro Pro Tyr
515 520 525

Pro Pro Arg Arg Asp Gly Ser Gly Gly Lys Gly Gly Gly Gly Ser Ala
530 535 540

Arg Tyr Asn Gln Gly Arg Ser Arg Ser Gly Gly Ala Ser Ile Gly Phe
545 550 555 560

His Thr Gln Thr Ile Leu Ile Leu Ser Leu Ser Ala Leu Ala Leu Leu
565 570 575

Gly Pro Arg

<210> 84
<211> 615
<212> PRT
<213> Artificial Sequence

<220>
<223> FA12_HUMAN

<400> 84
Met Arg Ala Leu Leu Leu Leu Gly Phe Leu Leu Val Ser Leu Glu Ser
1 5 10 15

Thr Leu Ser Ile Pro Pro Trp Glu Ala Pro Lys Glu His Lys Tyr Lys
20 25 30

Ala Glu Glu His Thr Val Val Leu Thr Val Thr Gly Glu Pro Cys His
35 40 45

Phe Pro Phe Gln Tyr His Arg Gln Leu Tyr His Lys Cys Thr His Lys
50 55 60

Gly Arg Pro Gly Pro Gln Pro Trp Cys Ala Thr Thr Pro Asn Phe Asp
65 70 75 80

Gln Asp Gln Arg Trp Gly Tyr Cys Leu Glu Pro Lys Lys Val Lys Asp
85 90 95

His Cys Ser Lys His Ser Pro Cys Gln Lys Gly Gly Thr Cys Val Asn
100 105 110

Met Pro Ser Gly Pro His Cys Leu Cys Pro Gln His Leu Thr Gly Asn
115 120 125

His Cys Gln Lys Glu Lys Cys Phe Glu Pro Gln Leu Leu Arg Phe Phe
130 135 140

His Lys Asn Glu Ile Trp Tyr Arg Thr Glu Gln Ala Ala Val Ala Arg
145 150 155 160

Cys Gln Cys Lys Gly Pro Asp Ala His Cys Gln Arg Leu Ala Ser Gln
165 170 175

Ala Cys Arg Thr Asn Pro Cys Leu His Gly Gly Arg Cys Leu Glu Val
180 185 190

Glu Gly His Arg Leu Cys His Cys Pro Val Gly Tyr Thr Gly Ala Phe
195 200 205

Cys Asp Val Asp Thr Lys Ala Ser Cys Tyr Asp Gly Arg Gly Leu Ser
210 215 220

Tyr Arg Gly Leu Ala Arg Thr Thr Leu Ser Gly Ala Pro Cys Gln Pro
225 230 235 240

Trp Ala Ser Glu Ala Thr Tyr Arg Asn Val Thr Ala Glu Gln Ala Arg
245 250 255

Asn Trp Gly Leu Gly Gly His Ala Phe Cys Arg Asn Pro Asp Asn Asp
260 265 270

Ile Arg Pro Trp Cys Phe Val Leu Asn Arg Asp Arg Leu Ser Trp Glu
275 280 285

Tyr Cys Asp Leu Ala Gln Cys Gln Thr Pro Thr Gln Ala Ala Pro Pro
290 295 300

Thr Pro Val Ser Pro Arg Leu His Val Pro Leu Met Pro Ala Gln Pro
305 310 315 320

Ala Pro Pro Lys Pro Gln Pro Thr Thr Arg Thr Pro Pro Gln Ser Gln
325 330 335

Thr Pro Gly Ala Leu Pro Ala Lys Arg Glu Gln Pro Pro Ser Leu Thr
340 345 350

Arg Asn Gly Pro Leu Ser Cys Gly Gln Arg Leu Arg Lys Ser Leu Ser
355 360 365

Ser Met Thr Arg Val Val Gly Gly Leu Val Ala Leu Arg Gly Ala His
370 375 380

Pro Tyr Ile Ala Ala Leu Tyr Trp Gly His Ser Phe Cys Ala Gly Ser
385 390 395 400

Leu Ile Ala Pro Cys Trp Val Leu Thr Ala Ala His Cys Leu Gln Asp
405 410 415

Arg Pro Ala Pro Glu Asp Leu Thr Val Val Leu Gly Gln Glu Arg Arg
420 425 430

Asn His Ser Cys Glu Pro Cys Gln Thr Leu Ala Val Arg Ser Tyr Arg
435 440 445

Leu His Glu Ala Phe Ser Pro Val Ser Tyr Gln His Asp Leu Ala Leu
450 455 460

Leu Arg Leu Gln Glu Asp Ala Asp Gly Ser Cys Ala Leu Leu Ser Pro
465 470 475 480

Tyr Val Gln Pro Val Cys Leu Pro Ser Gly Ala Ala Arg Pro Ser Glu
485 490 495

Thr Thr Leu Cys Gln Val Ala Gly Trp Gly His Gln Phe Glu Gly Ala
500 505 510

Glu Glu Tyr Ala Ser Phe Leu Gln Glu Ala Gln Val Pro Phe Leu Ser
515 520 525

Leu Glu Arg Cys Ser Ala Pro Asp Val His Gly Ser Ser Ile Leu Pro
530 535 540

Gly Met Leu Cys Ala Gly Phe Leu Glu Gly Gly Thr Asp Ala Cys Gln
545 550 555 560

Gly Asp Ser Gly Gly Pro Leu Val Cys Glu Asp Gln Ala Ala Glu Arg
565 570 575

Arg Leu Thr Leu Gln Gly Ile Ile Ser Trp Gly Ser Gly Cys Gly Asp
580 585 590

Arg Asn Lys Pro Gly Val Tyr Thr Asp Val Ala Tyr Tyr Leu Ala Trp
595 600 605

Ile Arg Glu His Thr Val Ser
610 615

<210> 85
<211> 644
<212> PRT
<213> Artificial Sequence

<220>
<223> KNG1_HUMAN

<400> 85
Met Lys Leu Ile Thr Ile Leu Phe Leu Cys Ser Arg Leu Leu Leu Ser
1 5 10 15

Leu Thr Gln Glu Ser Gln Ser Glu Glu Ile Asp Cys Asn Asp Lys Asp
20 25 30

Leu Phe Lys Ala Val Asp Ala Ala Leu Lys Lys Tyr Asn Ser Gln Asn
35 40 45

Gln Ser Asn Asn Gln Phe Val Leu Tyr Arg Ile Thr Glu Ala Thr Lys
50 55 60

Thr Val Gly Ser Asp Thr Phe Tyr Ser Phe Lys Tyr Glu Ile Lys Glu
65 70 75 80

Gly Asp Cys Pro Val Gln Ser Gly Lys Thr Trp Gln Asp Cys Glu Tyr
85 90 95

Lys Asp Ala Ala Lys Ala Ala Thr Gly Glu Cys Thr Ala Thr Val Gly
100 105 110

Lys Arg Ser Ser Thr Lys Phe Ser Val Ala Thr Gln Thr Cys Gln Ile
115 120 125

Thr Pro Ala Glu Gly Pro Val Val Thr Ala Gln Tyr Asp Cys Leu Gly
130 135 140

Cys Val His Pro Ile Ser Thr Gln Ser Pro Asp Leu Glu Pro Ile Leu
145 150 155 160

Arg His Gly Ile Gln Tyr Phe Asn Asn Asn Thr Gln His Ser Ser Leu

165 170 175

Phe Met Leu Asn Glu Val Lys Arg Ala Gln Arg Gln Val Val Ala Gly
180 185 190

Leu Asn Phe Arg Ile Thr Tyr Ser Ile Val Gln Thr Asn Cys Ser Lys
195 200 205

Glu Asn Phe Leu Phe Leu Thr Pro Asp Cys Lys Ser Leu Trp Asn Gly
210 215 220

Asp Thr Gly Glu Cys Thr Asp Asn Ala Tyr Ile Asp Ile Gln Leu Arg
225 230 235 240

Ile Ala Ser Phe Ser Gln Asn Cys Asp Ile Tyr Pro Gly Lys Asp Phe
245 250 255

Val Gln Pro Pro Thr Lys Ile Cys Val Gly Cys Pro Arg Asp Ile Pro
260 265 270

Thr Asn Ser Pro Glu Leu Glu Glu Thr Leu Thr His Thr Ile Thr Lys
275 280 285

Leu Asn Ala Glu Asn Asn Ala Thr Phe Tyr Phe Lys Ile Asp Asn Val
290 295 300

Lys Lys Ala Arg Val Gln Val Val Ala Gly Lys Lys Tyr Phe Ile Asp
305 310 315 320

Phe Val Ala Arg Glu Thr Thr Cys Ser Lys Glu Ser Asn Glu Glu Leu
325 330 335

Thr Glu Ser Cys Glu Thr Lys Lys Leu Gly Gln Ser Leu Asp Cys Asn
340 345 350

Ala Glu Val Tyr Val Val Pro Trp Glu Lys Lys Ile Tyr Pro Thr Val
355 360 365

Asn Cys Gln Pro Leu Gly Met Ile Ser Leu Met Lys Arg Pro Pro Gly
370 375 380

Phe Ser Pro Phe Arg Ser Ser Arg Ile Gly Glu Ile Lys Glu Glu Thr
385 390 395 400

Thr Val Ser Pro Pro His Thr Ser Met Ala Pro Ala Gln Asp Glu Glu
405 410 415

Arg Asp Ser Gly Lys Glu Gln Gly His Thr Arg Arg His Asp Trp Gly
420 425 430

His Glu Lys Gln Arg Lys His Asn Leu Gly His Gly His Lys His Glu
435 440 445

Arg Asp Gln Gly His Gly His Gln Arg Gly His Gly Leu Gly His Gly
450 455 460

His Glu Gln Gln His Gly Leu Gly His Gly His Lys Phe Lys Leu Asp
465 470 475 480

Asp Asp Leu Glu His Gln Gly Gly His Val Leu Asp His Gly His Lys
485 490 495

His Lys His Gly His Gly His Gly Lys His Lys Asn Lys Gly Lys Lys

500 505 510

Asn Gly Lys His Asn Gly Trp Lys Thr Glu His Leu Ala Ser Ser Ser
515 520 525

Glu Asp Ser Thr Thr Pro Ser Ala Gln Thr Gln Glu Lys Thr Glu Gly
530 535 540

Pro Thr Pro Ile Pro Ser Leu Ala Lys Pro Gly Val Thr Val Thr Phe
545 550 555 560

Ser Asp Phe Gln Asp Ser Asp Leu Ile Ala Thr Met Met Pro Pro Ile
565 570 575

Ser Pro Ala Pro Ile Gln Ser Asp Asp Asp Trp Ile Pro Asp Ile Gln
580 585 590

Ile Asp Pro Asn Gly Leu Ser Phe Asn Pro Ile Ser Asp Phe Pro Asp
595 600 605

Thr Thr Ser Pro Lys Cys Pro Gly Arg Pro Trp Lys Ser Val Ser Glu
610 615 620

Ile Asn Pro Thr Thr Gln Met Lys Glu Ser Tyr Tyr Phe Asp Leu Thr
625 630 635 640

Asp Gly Leu Ser

<210> 86
<211> 650
<212> PRT
<213> Artificial Sequence

<220>
<223> APLP1_HUMAN

<400> 86
Met Gly Pro Ala Ser Pro Ala Ala Arg Gly Leu Ser Arg Arg Pro Gly
1 5 10 15

Gln Pro Pro Leu Pro Leu Leu Leu Pro Leu Leu Leu Leu Leu Leu Arg
20 25 30

Ala Gln Pro Ala Ile Gly Ser Leu Ala Gly Gly Ser Pro Gly Ala Ala
35 40 45

Glu Ala Pro Gly Ser Ala Gln Val Ala Gly Leu Cys Gly Arg Leu Thr
50 55 60

Leu His Arg Asp Leu Arg Thr Gly Arg Trp Glu Pro Asp Pro Gln Arg
65 70 75 80

Ser Arg Arg Cys Leu Arg Asp Pro Gln Arg Val Leu Glu Tyr Cys Arg
85 90 95

Gln Met Tyr Pro Glu Leu Gln Ile Ala Arg Val Glu Gln Ala Thr Gln
100 105 110

Ala Ile Pro Met Glu Arg Trp Cys Gly Gly Ser Arg Ser Gly Ser Cys
115 120 125

Ala His Pro His His Gln Val Val Pro Phe Arg Cys Leu Pro Gly Glu
130 135 140

Phe Val Ser Glu Ala Leu Leu Val Pro Glu Gly Cys Arg Phe Leu His
145 150 155 160

Gln Glu Arg Met Asp Gln Cys Glu Ser Ser Thr Arg Arg His Gln Glu
165 170 175

Ala Gln Glu Ala Cys Ser Ser Gln Gly Leu Ile Leu His Gly Ser Gly
180 185 190

Met Leu Leu Pro Cys Gly Ser Asp Arg Phe Arg Gly Val Glu Tyr Val
195 200 205

Cys Cys Pro Pro Pro Gly Thr Pro Asp Pro Ser Gly Thr Ala Val Gly
210 215 220

Asp Pro Ser Thr Arg Ser Trp Pro Pro Gly Ser Arg Val Glu Gly Ala
225 230 235 240

Glu Asp Glu Glu Glu Glu Glu Ser Phe Pro Gln Pro Val Asp Asp Tyr
245 250 255

Phe Val Glu Pro Pro Gln Ala Glu Glu Glu Glu Glu Thr Val Pro Pro
260 265 270

Pro Ser Ser His Thr Leu Ala Val Val Gly Lys Val Thr Pro Thr Pro
275 280 285

Arg Pro Thr Asp Gly Val Asp Ile Tyr Phe Gly Met Pro Gly Glu Ile
290 295 300

Ser Glu His Glu Gly Phe Leu Arg Ala Lys Met Asp Leu Glu Glu Arg
305 310 315 320

Arg Met Arg Gln Ile Asn Glu Val Met Arg Glu Trp Ala Met Ala Asp
325 330 335

Asn Gln Ser Lys Asn Leu Pro Lys Ala Asp Arg Gln Ala Leu Asn Glu
340 345 350

His Phe Gln Ser Ile Leu Gln Thr Leu Glu Glu Gln Val Ser Gly Glu
355 360 365

Arg Gln Arg Leu Val Glu Thr His Ala Thr Arg Val Ile Ala Leu Ile
370 375 380

Asn Asp Gln Arg Arg Ala Ala Leu Glu Gly Phe Leu Ala Ala Leu Gln
385 390 395 400

Ala Asp Pro Pro Gln Ala Glu Arg Val Leu Leu Ala Leu Arg Arg Tyr
405 410 415

Leu Arg Ala Glu Gln Lys Glu Gln Arg His Thr Leu Arg His Tyr Gln
420 425 430

His Val Ala Ala Val Asp Pro Glu Lys Ala Gln Gln Met Arg Phe Gln
435 440 445

Val His Thr His Leu Gln Val Ile Glu Glu Arg Val Asn Gln Ser Leu
450 455 460

Gly Leu Leu Asp Gln Asn Pro His Leu Ala Gln Glu Leu Arg Pro Gln
465 470 475 480

Ile Gln Glu Leu Leu His Ser Glu His Leu Gly Pro Ser Glu Leu Glu
485 490 495

Ala Pro Ala Pro Gly Gly Ser Ser Glu Asp Lys Gly Gly Leu Gln Pro
500 505 510

Pro Asp Ser Lys Asp Asp Thr Pro Met Thr Leu Pro Lys Gly Ser Thr
515 520 525

Glu Gln Asp Ala Ala Ser Pro Glu Lys Glu Lys Met Asn Pro Leu Glu
530 535 540

Gln Tyr Glu Arg Lys Val Asn Ala Ser Val Pro Arg Gly Phe Pro Phe
545 550 555 560

His Ser Ser Glu Ile Gln Arg Asp Glu Leu Ala Pro Ala Gly Thr Gly
565 570 575

Val Ser Arg Glu Ala Val Ser Gly Leu Leu Ile Met Gly Ala Gly Gly
580 585 590

Gly Ser Leu Ile Val Leu Ser Met Leu Leu Leu Arg Arg Lys Lys Pro
595 600 605

Tyr Gly Ala Ile Ser His Gly Val Val Glu Val Asp Pro Met Leu Thr
610 615 620

Leu Glu Glu Gln Gln Leu Arg Glu Leu Gln Arg His Gly Tyr Glu Asn
625 630 635 640

Pro Thr Tyr Arg Phe Leu Glu Glu Arg Pro
645 650

<210> 87
<211> 661
<212> PRT
<213> Artificial Sequence

<220>
<223> CRAC1_HUMAN

<400> 87
Met Ala Pro Ser Ala Asp Pro Gly Met Ser Arg Met Leu Pro Phe Leu
1 5 10 15

Leu Leu Leu Trp Phe Leu Pro Ile Thr Glu Gly Ser Gln Arg Ala Glu
20 25 30

Pro Met Phe Thr Ala Val Thr Asn Ser Val Leu Pro Pro Asp Tyr Asp
35 40 45

Ser Asn Pro Thr Gln Leu Asn Tyr Gly Val Ala Val Thr Asp Val Asp
50 55 60

His Asp Gly Asp Phe Glu Ile Val Val Ala Gly Tyr Asn Gly Pro Asn
65 70 75 80

```
Leu Val Leu Lys Tyr Asp Arg Ala Gln Lys Arg Leu Val Asn Ile Ala
                85                  90                  95

Val Asp Glu Arg Ser Ser Pro Tyr Tyr Ala Leu Arg Asp Arg Gln Gly
            100                 105                 110

Asn Ala Ile Gly Val Thr Ala Cys Asp Ile Asp Gly Asp Gly Arg Glu
        115                 120                 125

Glu Ile Tyr Phe Leu Asn Thr Asn Asn Ala Phe Ser Gly Val Ala Thr
    130                 135                 140

Tyr Thr Asp Lys Leu Phe Lys Phe Arg Asn Asn Arg Trp Glu Asp Ile
145                 150                 155                 160

Leu Ser Asp Glu Val Asn Val Ala Arg Gly Val Ala Ser Leu Phe Ala
                165                 170                 175

Gly Arg Ser Val Ala Cys Val Asp Arg Lys Gly Ser Gly Arg Tyr Ser
            180                 185                 190

Ile Tyr Ile Ala Asn Tyr Ala Tyr Gly Asn Val Gly Pro Asp Ala Leu
        195                 200                 205

Ile Glu Met Asp Pro Glu Ala Ser Asp Leu Ser Arg Gly Ile Leu Ala
    210                 215                 220

Leu Arg Asp Val Ala Ala Glu Ala Gly Val Ser Lys Tyr Thr Gly Gly
225                 230                 235                 240

Arg Gly Val Ser Val Gly Pro Ile Leu Ser Ser Ser Ala Ser Asp Ile
                245                 250                 255

Phe Cys Asp Asn Glu Asn Gly Pro Asn Phe Leu Phe His Asn Arg Gly
            260                 265                 270

Asp Gly Thr Phe Val Asp Ala Ala Ala Ser Ala Gly Val Asp Asp Pro
        275                 280                 285

His Gln His Gly Arg Gly Val Ala Leu Ala Asp Phe Asn Arg Asp Gly
    290                 295                 300

Lys Val Asp Ile Val Tyr Gly Asn Trp Asn Gly Pro His Arg Leu Tyr
305                 310                 315                 320

Leu Gln Met Ser Thr His Gly Lys Val Arg Phe Arg Asp Ile Ala Ser
                325                 330                 335

Pro Lys Phe Ser Met Pro Ser Pro Val Arg Thr Val Ile Thr Ala Asp
            340                 345                 350

Phe Asp Asn Asp Gln Glu Leu Glu Ile Phe Phe Asn Asn Ile Ala Tyr
        355                 360                 365

Arg Ser Ser Ser Ala Asn Arg Leu Phe Arg Val Ile Arg Arg Glu His
    370                 375                 380

Gly Asp Pro Leu Ile Glu Glu Leu Asn Pro Gly Asp Ala Leu Glu Pro
385                 390                 395                 400

Glu Gly Arg Gly Thr Gly Gly Val Val Thr Asp Phe Asp Gly Asp Gly
                405                 410                 415
```

Met Leu Asp Leu Ile Leu Ser His Gly Glu Ser Met Ala Gln Pro Leu
420 425 430

Ser Val Phe Arg Gly Asn Gln Gly Phe Asn Asn Asn Trp Leu Arg Val
435 440 445

Val Pro Arg Thr Arg Phe Gly Ala Phe Ala Arg Gly Ala Lys Val Val
450 455 460

Leu Tyr Thr Lys Lys Ser Gly Ala His Leu Arg Ile Ile Asp Gly Gly
465 470 475 480

Ser Gly Tyr Leu Cys Glu Met Glu Pro Val Ala His Phe Gly Leu Gly
485 490 495

Lys Asp Glu Ala Ser Ser Val Glu Val Thr Trp Pro Asp Gly Lys Met
500 505 510

Val Ser Arg Asn Val Ala Ser Gly Glu Met Asn Ser Val Leu Glu Ile
515 520 525

Leu Tyr Pro Arg Asp Glu Asp Thr Leu Gln Asp Pro Ala Pro Leu Glu
530 535 540

Cys Gly Gln Gly Phe Ser Gln Gln Glu Asn Gly His Cys Met Asp Thr
545 550 555 560

Asn Glu Cys Ile Gln Phe Pro Phe Val Cys Pro Arg Asp Lys Pro Val
565 570 575

Cys Val Asn Thr Tyr Gly Ser Tyr Arg Cys Arg Thr Asn Lys Lys Cys
580 585 590

Ser Arg Gly Tyr Glu Pro Asn Glu Asp Gly Thr Ala Cys Val Gly Thr
595 600 605

Leu Gly Gln Ser Pro Gly Pro Arg Pro Thr Thr Pro Thr Ala Ala Ala
610 615 620

Ala Thr Ala Ala Ala Ala Ala Ala Ala Gly Ala Ala Thr Ala Ala Pro
625 630 635 640

Val Leu Val Asp Gly Asp Leu Asn Leu Gly Ser Val Val Lys Glu Ser
645 650 655

Cys Glu Pro Ser Cys
660

<210> 88
<211> 664
<212> PRT
<213> Artificial Sequence

<220>
<223> SPRL1_HUMAN

<400> 88
Met Lys Thr Gly Leu Phe Phe Leu Cys Leu Leu Gly Thr Ala Ala Ala
1 5 10 15

Ile Pro Thr Asn Ala Arg Leu Leu Ser Asp His Ser Lys Pro Thr Ala

```
            20                  25                  30

Glu Thr Val Ala Pro Asp Asn Thr Ala Ile Pro Ser Leu Arg Ala Glu
        35                  40                  45

Ala Glu Glu Asn Glu Lys Glu Thr Ala Val Ser Thr Glu Asp Asp Ser
    50                  55                  60

His His Lys Ala Glu Lys Ser Ser Val Leu Lys Ser Lys Glu Glu Ser
65                  70                  75                  80

His Glu Gln Ser Ala Glu Gln Gly Lys Ser Ser Ser Gln Glu Leu Gly
                85                  90                  95

Leu Lys Asp Gln Glu Asp Ser Asp Gly His Leu Ser Val Asn Leu Glu
            100                 105                 110

Tyr Ala Pro Thr Glu Gly Thr Leu Asp Ile Lys Glu Asp Met Ser Glu
        115                 120                 125

Pro Gln Glu Lys Lys Leu Ser Glu Asn Thr Asp Phe Leu Ala Pro Gly
    130                 135                 140

Val Ser Ser Phe Thr Asp Ser Asn Gln Gln Glu Ser Ile Thr Lys Arg
145                 150                 155                 160

Glu Glu Asn Gln Glu Gln Pro Arg Asn Tyr Ser His His Gln Leu Asn
                165                 170                 175

Arg Ser Ser Lys His Ser Gln Gly Leu Arg Asp Gln Gly Asn Gln Glu
            180                 185                 190

Gln Asp Pro Asn Ile Ser Asn Gly Glu Glu Glu Glu Glu Lys Glu Pro
        195                 200                 205

Gly Glu Val Gly Thr His Asn Asp Asn Gln Glu Arg Lys Thr Glu Leu
    210                 215                 220

Pro Arg Glu His Ala Asn Ser Lys Gln Glu Glu Asp Asn Thr Gln Ser
225                 230                 235                 240

Asp Asp Ile Leu Glu Glu Ser Asp Gln Pro Thr Gln Val Ser Lys Met
                245                 250                 255

Gln Glu Asp Glu Phe Asp Gln Gly Asn Gln Glu Gln Glu Asp Asn Ser
            260                 265                 270

Asn Ala Glu Met Glu Glu Glu Asn Ala Ser Asn Val Asn Lys His Ile
        275                 280                 285

Gln Glu Thr Glu Trp Gln Ser Gln Glu Gly Lys Thr Gly Leu Glu Ala
    290                 295                 300

Ile Ser Asn His Lys Glu Thr Glu Glu Lys Thr Val Ser Glu Ala Leu
305                 310                 315                 320

Leu Met Glu Pro Thr Asp Asp Gly Asn Thr Thr Pro Arg Asn His Gly
                325                 330                 335

Val Asp Asp Asp Gly Asp Asp Asp Gly Asp Asp Gly Gly Thr Asp Gly
            340                 345                 350

Pro Arg His Ser Ala Ser Asp Asp Tyr Phe Ile Pro Ser Gln Ala Phe
```

355 360 365

Leu Glu Ala Glu Arg Ala Gln Ser Ile Ala Tyr His Leu Lys Ile Glu
370 375 380

Glu Gln Arg Glu Lys Val His Glu Asn Glu Asn Ile Gly Thr Thr Glu
385 390 395 400

Pro Gly Glu His Gln Glu Ala Lys Lys Ala Glu Asn Ser Ser Asn Glu
405 410 415

Glu Glu Thr Ser Ser Glu Gly Asn Met Arg Val His Ala Val Asp Ser
420 425 430

Cys Met Ser Phe Gln Cys Lys Arg Gly His Ile Cys Lys Ala Asp Gln
435 440 445

Gln Gly Lys Pro His Cys Val Cys Gln Asp Pro Val Thr Cys Pro Pro
450 455 460

Thr Lys Pro Leu Asp Gln Val Cys Gly Thr Asp Asn Gln Thr Tyr Ala
465 470 475 480

Ser Ser Cys His Leu Phe Ala Thr Lys Cys Arg Leu Glu Gly Thr Lys
485 490 495

Lys Gly His Gln Leu Gln Leu Asp Tyr Phe Gly Ala Cys Lys Ser Ile
500 505 510

Pro Thr Cys Thr Asp Phe Glu Val Ile Gln Phe Pro Leu Arg Met Arg
515 520 525

Asp Trp Leu Lys Asn Ile Leu Met Gln Leu Tyr Glu Ala Asn Ser Glu
530 535 540

His Ala Gly Tyr Leu Asn Glu Lys Gln Arg Asn Lys Val Lys Lys Ile
545 550 555 560

Tyr Leu Asp Glu Lys Arg Leu Leu Ala Gly Asp His Pro Ile Asp Leu
565 570 575

Leu Leu Arg Asp Phe Lys Lys Asn Tyr His Met Tyr Val Tyr Pro Val
580 585 590

His Trp Gln Phe Ser Glu Leu Asp Gln His Pro Met Asp Arg Val Leu
595 600 605

Thr His Ser Glu Leu Ala Pro Leu Arg Ala Ser Leu Val Pro Met Glu
610 615 620

His Cys Ile Thr Arg Phe Phe Glu Glu Cys Asp Pro Asn Lys Asp Lys
625 630 635 640

His Ile Thr Leu Lys Glu Trp Gly His Cys Phe Gly Ile Lys Glu Glu
645 650 655

Asp Ile Asp Glu Asn Leu Leu Phe
660

<210> 89
<211> 701
<212> PRT

<213> Artificial Sequence

<220>
<223> MEP1B_HUMAN

<400> 89

```
Met Asp Leu Trp Asn Leu Ser Trp Phe Leu Phe Leu Asp Ala Leu Leu
1               5                   10                  15

Val Ile Ser Gly Leu Ala Thr Pro Glu Asn Phe Asp Val Asp Gly Gly
            20                  25                  30

Met Asp Gln Asp Ile Phe Asp Ile Asn Glu Gly Leu Gly Leu Asp Leu
        35                  40                  45

Phe Glu Gly Asp Ile Arg Leu Asp Arg Ala Gln Ile Arg Asn Ser Ile
    50                  55                  60

Ile Gly Glu Lys Tyr Arg Trp Pro His Thr Ile Pro Tyr Val Leu Glu
65                  70                  75                  80

Asp Ser Leu Glu Met Asn Ala Lys Gly Val Ile Leu Asn Ala Phe Glu
                85                  90                  95

Arg Tyr Arg Leu Lys Thr Cys Ile Asp Phe Lys Pro Trp Ala Gly Glu
            100                 105                 110

Thr Asn Tyr Ile Ser Val Phe Lys Gly Ser Gly Cys Trp Ser Ser Val
        115                 120                 125

Gly Asn Arg Arg Val Gly Lys Gln Glu Leu Ser Ile Gly Ala Asn Cys
    130                 135                 140

Asp Arg Ile Ala Thr Val Gln His Glu Phe Leu His Ala Leu Gly Phe
145                 150                 155                 160

Trp His Glu Gln Ser Arg Ser Asp Arg Asp Asp Tyr Val Arg Ile Met
                165                 170                 175

Trp Asp Arg Ile Leu Ser Gly Arg Glu His Asn Phe Asn Thr Tyr Ser
            180                 185                 190

Asp Asp Ile Ser Asp Ser Leu Asn Val Pro Tyr Asp Tyr Thr Ser Val
        195                 200                 205

Met His Tyr Ser Lys Thr Ala Phe Gln Asn Gly Thr Glu Pro Thr Ile
    210                 215                 220

Val Thr Arg Ile Ser Asp Phe Glu Asp Val Ile Gly Gln Arg Met Asp
225                 230                 235                 240

Phe Ser Asp Ser Asp Leu Leu Lys Leu Asn Gln Leu Tyr Asn Cys Ser
                245                 250                 255

Ser Ser Leu Ser Phe Met Asp Ser Cys Ser Phe Glu Leu Glu Asn Val
            260                 265                 270

Cys Gly Met Ile Gln Ser Ser Gly Asp Asn Ala Asp Trp Gln Arg Val
        275                 280                 285

Ser Gln Val Pro Arg Gly Pro Glu Ser Asp His Ser Asn Met Gly Gln
    290                 295                 300
```

Cys Gln Gly Ser Gly Phe Phe Met His Phe Asp Ser Ser Ser Val Asn
305 310 315 320

Val Gly Ala Thr Ala Val Leu Glu Ser Arg Thr Leu Tyr Pro Lys Arg
325 330 335

Gly Phe Gln Cys Leu Gln Phe Tyr Leu Tyr Asn Ser Gly Ser Glu Ser
340 345 350

Asp Gln Leu Asn Ile Tyr Ile Arg Glu Tyr Ser Ala Asp Asn Val Asp
355 360 365

Gly Asn Leu Thr Leu Val Glu Glu Ile Lys Glu Ile Pro Thr Gly Ser
370 375 380

Trp Gln Leu Tyr His Val Thr Leu Lys Val Thr Lys Lys Phe Arg Val
385 390 395 400

Val Phe Glu Gly Arg Lys Gly Ser Gly Ala Ser Leu Gly Gly Leu Ser
405 410 415

Ile Asp Asp Ile Asn Leu Ser Glu Thr Arg Cys Pro His His Ile Trp
420 425 430

His Ile Arg Asn Phe Thr Gln Phe Ile Gly Ser Pro Asn Gly Thr Leu
435 440 445

Tyr Ser Pro Pro Phe Tyr Ser Ser Lys Gly Tyr Ala Phe Gln Ile Tyr
450 455 460

Leu Asn Leu Ala His Val Thr Asn Ala Gly Ile Tyr Phe His Leu Ile
465 470 475 480

Ser Gly Ala Asn Asp Asp Gln Leu Gln Trp Pro Cys Pro Trp Gln Gln
485 490 495

Ala Thr Met Thr Leu Leu Asp Gln Asn Pro Asp Ile Arg Gln Arg Met
500 505 510

Ser Asn Gln Arg Ser Ile Thr Thr Asp Pro Phe Met Thr Thr Asp Asn
515 520 525

Gly Asn Tyr Phe Trp Asp Arg Pro Ser Lys Val Gly Thr Val Ala Leu
530 535 540

Phe Ser Asn Gly Thr Gln Phe Arg Arg Gly Gly Gly Tyr Gly Thr Ser
545 550 555 560

Ala Phe Ile Thr His Glu Arg Leu Lys Ser Arg Asp Phe Ile Lys Gly
565 570 575

Asp Asp Val Tyr Ile Leu Leu Thr Val Glu Asp Ile Ser His Leu Asn
580 585 590

Ser Thr Gln Ile Gln Leu Thr Pro Ala Pro Ser Val Gln Asp Leu Cys
595 600 605

Ser Lys Thr Thr Cys Lys Asn Asp Gly Val Cys Thr Val Arg Asp Gly
610 615 620

Lys Ala Glu Cys Arg Cys Gln Ser Gly Glu Asp Trp Trp Tyr Met Gly
625 630 635 640

Glu Arg Cys Glu Lys Arg Gly Ser Thr Arg Asp Thr Ile Val Ile Ala
645 650 655

Val Ser Ser Thr Val Ala Val Phe Ala Leu Met Leu Ile Ile Thr Leu
660 665 670

Val Ser Val Tyr Cys Thr Arg Lys Lys Tyr Arg Glu Arg Met Ser Ser
675 680 685

Asn Arg Pro Asn Leu Thr Pro Gln Asn Gln His Ala Phe
690 695 700

<210> 90
<211> 705
<212> PRT
<213> Artificial Sequence

<220>
<223> SYN1_HUMAN

<400> 90
Met Asn Tyr Leu Arg Arg Arg Leu Ser Asp Ser Asn Phe Met Ala Asn
1 5 10 15

Leu Pro Asn Gly Tyr Met Thr Asp Leu Gln Arg Pro Gln Pro Pro Pro
20 25 30

Pro Pro Pro Gly Ala His Ser Pro Gly Ala Thr Pro Gly Pro Gly Thr
35 40 45

Ala Thr Ala Glu Arg Ser Ser Gly Val Ala Pro Ala Ala Ser Pro Ala
50 55 60

Ala Pro Ser Pro Gly Ser Ser Gly Gly Gly Gly Phe Phe Ser Ser Leu
65 70 75 80

Ser Asn Ala Val Lys Gln Thr Thr Ala Ala Ala Ala Ala Thr Phe Ser
85 90 95

Glu Gln Val Gly Gly Gly Ser Gly Gly Ala Gly Arg Gly Gly Ala Ala
100 105 110

Ser Arg Val Leu Leu Val Ile Asp Glu Pro His Thr Asp Trp Ala Lys
115 120 125

Tyr Phe Lys Gly Lys Lys Ile His Gly Glu Ile Asp Ile Lys Val Glu
130 135 140

Gln Ala Glu Phe Ser Asp Leu Asn Leu Val Ala His Ala Asn Gly Gly
145 150 155 160

Phe Ser Val Asp Met Glu Val Leu Arg Asn Gly Val Lys Val Val Arg
165 170 175

Ser Leu Lys Pro Asp Phe Val Leu Ile Arg Gln His Ala Phe Ser Met
180 185 190

Ala Arg Asn Gly Asp Tyr Arg Ser Leu Val Ile Gly Leu Gln Tyr Ala
195 200 205

Gly Ile Pro Ser Val Asn Ser Leu His Ser Val Tyr Asn Phe Cys Asp
    210                 215                 220

Lys Pro Trp Val Phe Ala Gln Met Val Arg Leu His Lys Lys Leu Gly
225                 230                 235                 240

Thr Glu Glu Phe Pro Leu Ile Asp Gln Thr Phe Tyr Pro Asn His Lys
                245                 250                 255

Glu Met Leu Ser Ser Thr Thr Tyr Pro Val Val Val Lys Met Gly His
            260                 265                 270

Ala His Ser Gly Met Gly Lys Val Lys Val Asp Asn Gln His Asp Phe
        275                 280                 285

Gln Asp Ile Ala Ser Val Val Ala Leu Thr Lys Thr Tyr Ala Thr Ala
    290                 295                 300

Glu Pro Phe Ile Asp Ala Lys Tyr Asp Val Arg Val Gln Lys Ile Gly
305                 310                 315                 320

Gln Asn Tyr Lys Ala Tyr Met Arg Thr Ser Val Ser Gly Asn Trp Lys
                325                 330                 335

Thr Asn Thr Gly Ser Ala Met Leu Glu Gln Ile Ala Met Ser Asp Arg
            340                 345                 350

Tyr Lys Leu Trp Val Asp Thr Cys Ser Glu Ile Phe Gly Gly Leu Asp
        355                 360                 365

Ile Cys Ala Val Glu Ala Leu His Gly Lys Asp Gly Arg Asp His Ile
    370                 375                 380

Ile Glu Val Val Gly Ser Ser Met Pro Leu Ile Gly Asp His Gln Asp
385                 390                 395                 400

Glu Asp Lys Gln Leu Ile Val Glu Leu Val Val Asn Lys Met Ala Gln
                405                 410                 415

Ala Leu Pro Arg Gln Arg Gln Arg Asp Ala Ser Pro Gly Arg Gly Ser
            420                 425                 430

His Gly Gln Thr Pro Ser Pro Gly Ala Leu Pro Leu Gly Arg Gln Thr
        435                 440                 445

Ser Gln Gln Pro Ala Gly Pro Pro Ala Gln Gln Arg Pro Pro Pro Gln
    450                 455                 460

Gly Gly Pro Pro Gln Pro Gly Pro Gly Pro Gln Arg Gln Gly Pro Pro
465                 470                 475                 480

Leu Gln Gln Arg Pro Pro Pro Gln Gly Gln Gln His Leu Ser Gly Leu
                485                 490                 495

Gly Pro Pro Ala Gly Ser Pro Leu Pro Gln Arg Leu Pro Ser Pro Thr
            500                 505                 510

Ser Ala Pro Gln Gln Pro Ala Ser Gln Ala Ala Pro Pro Thr Gln Gly
        515                 520                 525

Gln Gly Arg Gln Ser Arg Pro Val Ala Gly Gly Pro Gly Ala Pro Pro
    530                 535                 540

Ala Ala Arg Pro Pro Ala Ser Pro Ser Pro Gln Arg Gln Ala Gly Pro
545 550 555 560

Pro Gln Ala Thr Arg Gln Thr Ser Val Ser Gly Pro Ala Pro Pro Lys
565 570 575

Ala Ser Gly Ala Pro Pro Gly Gly Gln Gln Arg Gln Gly Pro Pro Gln
580 585 590

Lys Pro Pro Gly Pro Ala Gly Pro Thr Arg Gln Ala Ser Gln Ala Gly
595 600 605

Pro Val Pro Arg Thr Gly Pro Pro Thr Thr Gln Gln Pro Arg Pro Ser
610 615 620

Gly Pro Gly Pro Ala Gly Arg Pro Lys Pro Gln Leu Ala Gln Lys Pro
625 630 635 640

Ser Gln Asp Val Pro Pro Pro Ala Thr Ala Ala Ala Gly Gly Pro Pro
645 650 655

His Pro Gln Leu Asn Lys Ser Gln Ser Leu Thr Asn Ala Phe Asn Leu
660 665 670

Pro Glu Pro Ala Pro Pro Arg Pro Ser Leu Ser Gln Asp Glu Val Lys
675 680 685

Ala Glu Thr Ile Arg Ser Leu Arg Lys Ser Phe Ala Ser Leu Phe Ser
690 695 700

Asp
705

<210> 91
<211> 753
<212> PRT
<213> Artificial Sequence

<220>
<223> CEL_HUMAN

<400> 91
Met Gly Arg Leu Gln Leu Val Val Leu Gly Leu Thr Cys Cys Trp Ala
1 5 10 15

Val Ala Ser Ala Ala Lys Leu Gly Ala Val Tyr Thr Glu Gly Gly Phe
20 25 30

Val Glu Gly Val Asn Lys Lys Leu Gly Leu Leu Gly Asp Ser Val Asp
35 40 45

Ile Phe Lys Gly Ile Pro Phe Ala Ala Pro Thr Lys Ala Leu Glu Asn
50 55 60

Pro Gln Pro His Pro Gly Trp Gln Gly Thr Leu Lys Ala Lys Asn Phe
65 70 75 80

Lys Lys Arg Cys Leu Gln Ala Thr Ile Thr Gln Asp Ser Thr Tyr Gly
85 90 95

Asp Glu Asp Cys Leu Tyr Leu Asn Ile Trp Val Pro Gln Gly Arg Lys
100 105 110

Gln Val Ser Arg Asp Leu Pro Val Met Ile Trp Ile Tyr Gly Gly Ala
115 120 125

Phe Leu Met Gly Ser Gly His Gly Ala Asn Phe Leu Asn Asn Tyr Leu
130 135 140

Tyr Asp Gly Glu Glu Ile Ala Thr Arg Gly Asn Val Ile Val Val Thr
145 150 155 160

Phe Asn Tyr Arg Val Gly Pro Leu Gly Phe Leu Ser Thr Gly Asp Ala
165 170 175

Asn Leu Pro Gly Asn Tyr Gly Leu Arg Asp Gln His Met Ala Ile Ala
180 185 190

Trp Val Lys Arg Asn Ile Ala Ala Phe Gly Gly Asp Pro Asn Asn Ile
195 200 205

Thr Leu Phe Gly Glu Ser Ala Gly Gly Ala Ser Val Ser Leu Gln Thr
210 215 220

Leu Ser Pro Tyr Asn Lys Gly Leu Ile Arg Arg Ala Ile Ser Gln Ser
225 230 235 240

Gly Val Ala Leu Ser Pro Trp Val Ile Gln Lys Asn Pro Leu Phe Trp
245 250 255

Ala Lys Lys Val Ala Glu Lys Val Gly Cys Pro Val Gly Asp Ala Ala
260 265 270

Arg Met Ala Gln Cys Leu Lys Val Thr Asp Pro Arg Ala Leu Thr Leu
275 280 285

Ala Tyr Lys Val Pro Leu Ala Gly Leu Glu Tyr Pro Met Leu His Tyr
290 295 300

Val Gly Phe Val Pro Val Ile Asp Gly Asp Phe Ile Pro Ala Asp Pro
305 310 315 320

Ile Asn Leu Tyr Ala Asn Ala Ala Asp Ile Asp Tyr Ile Ala Gly Thr
325 330 335

Asn Asn Met Asp Gly His Ile Phe Ala Ser Ile Asp Met Pro Ala Ile
340 345 350

Asn Lys Gly Asn Lys Lys Val Thr Glu Glu Asp Phe Tyr Lys Leu Val
355 360 365

Ser Glu Phe Thr Ile Thr Lys Gly Leu Arg Gly Ala Lys Thr Thr Phe
370 375 380

Asp Val Tyr Thr Glu Ser Trp Ala Gln Asp Pro Ser Gln Glu Asn Lys
385 390 395 400

Lys Lys Thr Val Val Asp Phe Glu Thr Asp Val Leu Phe Leu Val Pro
405 410 415

Thr Glu Ile Ala Leu Ala Gln His Arg Ala Asn Ala Lys Ser Ala Lys
420 425 430

```
Thr Tyr Ala Tyr Leu Phe Ser His Pro Ser Arg Met Pro Val Tyr Pro
        435                 440                 445

Lys Trp Val Gly Ala Asp His Ala Asp Asp Ile Gln Tyr Val Phe Gly
    450                 455                 460

Lys Pro Phe Ala Thr Pro Thr Gly Tyr Arg Pro Gln Asp Arg Thr Val
465                 470                 475                 480

Ser Lys Ala Met Ile Ala Tyr Trp Thr Asn Phe Ala Lys Thr Gly Asp
                485                 490                 495

Pro Asn Met Gly Asp Ser Ala Val Pro Thr His Trp Glu Pro Tyr Thr
            500                 505                 510

Thr Glu Asn Ser Gly Tyr Leu Glu Ile Thr Lys Lys Met Gly Ser Ser
        515                 520                 525

Ser Met Lys Arg Ser Leu Arg Thr Asn Phe Leu Arg Tyr Trp Thr Leu
    530                 535                 540

Thr Tyr Leu Ala Leu Pro Thr Val Thr Asp Gln Glu Ala Thr Pro Val
545                 550                 555                 560

Pro Pro Thr Gly Asp Ser Glu Ala Thr Pro Val Pro Pro Thr Gly Asp
                565                 570                 575

Ser Glu Thr Ala Pro Val Pro Pro Thr Gly Asp Ser Gly Ala Pro Pro
            580                 585                 590

Val Pro Pro Thr Gly Asp Ser Gly Ala Pro Pro Val Pro Pro Thr Gly
        595                 600                 605

Asp Ser Gly Ala Pro Pro Val Pro Pro Thr Gly Asp Ser Gly Ala Pro
    610                 615                 620

Pro Val Pro Pro Thr Gly Asp Ser Gly Ala Pro Pro Val Pro Pro Thr
625                 630                 635                 640

Gly Asp Ser Gly Ala Pro Pro Val Pro Pro Thr Gly Asp Ser Gly Ala
                645                 650                 655

Pro Pro Val Pro Pro Thr Gly Asp Ser Gly Ala Pro Pro Val Pro Pro
            660                 665                 670

Thr Gly Asp Ala Gly Pro Pro Pro Val Pro Pro Thr Gly Asp Ser Gly
        675                 680                 685

Ala Pro Pro Val Pro Pro Thr Gly Asp Ser Gly Ala Pro Pro Val Thr
    690                 695                 700

Pro Thr Gly Asp Ser Glu Thr Ala Pro Val Pro Pro Thr Gly Asp Ser
705                 710                 715                 720

Gly Ala Pro Pro Val Pro Pro Thr Gly Asp Ser Glu Ala Ala Pro Val
                725                 730                 735

Pro Pro Thr Asp Asp Ser Lys Glu Ala Gln Met Pro Ala Val Ile Arg
            740                 745                 750

Phe
```

```
<210>    92
<211>    757
<212>    PRT
<213>    Artificial Sequence

<220>
<223>    CD248_HUMAN


<400>    92
Met Leu Leu Arg Leu Leu Leu Ala Trp Ala Ala Ala Gly Pro Thr Leu
1               5                   10                  15

Gly Gln Asp Pro Trp Ala Ala Glu Pro Arg Ala Ala Cys Gly Pro Ser
            20                  25                  30

Ser Cys Tyr Ala Leu Phe Pro Arg Arg Arg Thr Phe Leu Glu Ala Trp
        35                  40                  45

Arg Ala Cys Arg Glu Leu Gly Gly Asp Leu Ala Thr Pro Arg Thr Pro
    50                  55                  60

Glu Glu Ala Gln Arg Val Asp Ser Leu Val Gly Ala Gly Pro Ala Ser
65                  70                  75                  80

Arg Leu Leu Trp Ile Gly Leu Gln Arg Gln Ala Arg Gln Cys Gln Leu
                85                  90                  95

Gln Arg Pro Leu Arg Gly Phe Thr Trp Thr Thr Gly Asp Gln Asp Thr
            100                 105                 110

Ala Phe Thr Asn Trp Ala Gln Pro Ala Ser Gly Gly Pro Cys Pro Ala
        115                 120                 125

Gln Arg Cys Val Ala Leu Glu Ala Ser Gly Glu His Arg Trp Leu Glu
    130                 135                 140

Gly Ser Cys Thr Leu Ala Val Asp Gly Tyr Leu Cys Gln Phe Gly Phe
145                 150                 155                 160

Glu Gly Ala Cys Pro Ala Leu Gln Asp Glu Ala Gly Gln Ala Gly Pro
                165                 170                 175

Ala Val Tyr Thr Thr Pro Phe His Leu Val Ser Thr Glu Phe Glu Trp
            180                 185                 190

Leu Pro Phe Gly Ser Val Ala Ala Val Gln Cys Gln Ala Gly Arg Gly
        195                 200                 205

Ala Ser Leu Leu Cys Val Lys Gln Pro Glu Gly Gly Val Gly Trp Ser
    210                 215                 220

Arg Ala Gly Pro Leu Cys Leu Gly Thr Gly Cys Ser Pro Asp Asn Gly
225                 230                 235                 240

Gly Cys Glu His Glu Cys Val Glu Glu Val Asp Gly His Val Ser Cys
                245                 250                 255

Arg Cys Thr Glu Gly Phe Arg Leu Ala Ala Asp Gly Arg Ser Cys Glu
            260                 265                 270

Asp Pro Cys Ala Gln Ala Pro Cys Glu Gln Gln Cys Glu Pro Gly Gly
```

```
        275                 280                 285

Pro Gln Gly Tyr Ser Cys His Cys Arg Leu Gly Phe Arg Pro Ala Glu
    290                 295                 300

Asp Asp Pro His Arg Cys Val Asp Thr Asp Glu Cys Gln Ile Ala Gly
305                 310                 315                 320

Val Cys Gln Gln Met Cys Val Asn Tyr Val Gly Gly Phe Glu Cys Tyr
                325                 330                 335

Cys Ser Glu Gly His Glu Leu Glu Ala Asp Gly Ile Ser Cys Ser Pro
            340                 345                 350

Ala Gly Ala Met Gly Ala Gln Ala Ser Gln Asp Leu Gly Asp Glu Leu
        355                 360                 365

Leu Asp Asp Gly Glu Asp Glu Glu Asp Glu Asp Glu Ala Trp Lys Ala
    370                 375                 380

Phe Asn Gly Gly Trp Thr Glu Met Pro Gly Ile Leu Trp Met Glu Pro
385                 390                 395                 400

Thr Gln Pro Pro Asp Phe Ala Leu Ala Tyr Arg Pro Ser Phe Pro Glu
                405                 410                 415

Asp Arg Glu Pro Gln Ile Pro Tyr Pro Glu Pro Thr Trp Pro Pro Pro
            420                 425                 430

Leu Ser Ala Pro Arg Val Pro Tyr His Ser Ser Val Leu Ser Val Thr
        435                 440                 445

Arg Pro Val Val Val Ser Ala Thr His Pro Thr Leu Pro Ser Ala His
    450                 455                 460

Gln Pro Pro Val Ile Pro Ala Thr His Pro Ala Leu Ser Arg Asp His
465                 470                 475                 480

Gln Ile Pro Val Ile Ala Ala Asn Tyr Pro Asp Leu Pro Ser Ala Tyr
                485                 490                 495

Gln Pro Gly Ile Leu Ser Val Ser His Ser Ala Gln Pro Pro Ala His
            500                 505                 510

Gln Pro Pro Met Ile Ser Thr Lys Tyr Pro Glu Leu Phe Pro Ala His
        515                 520                 525

Gln Ser Pro Met Phe Pro Asp Thr Arg Val Ala Gly Thr Gln Thr Thr
    530                 535                 540

Thr His Leu Pro Gly Ile Pro Pro Asn His Ala Pro Leu Val Thr Thr
545                 550                 555                 560

Leu Gly Ala Gln Leu Pro Pro Gln Ala Pro Asp Ala Leu Val Leu Arg
                565                 570                 575

Thr Gln Ala Thr Gln Leu Pro Ile Ile Pro Thr Ala Gln Pro Ser Leu
            580                 585                 590

Thr Thr Thr Ser Arg Ser Pro Val Ser Pro Ala His Gln Ile Ser Val
        595                 600                 605

Pro Ala Ala Thr Gln Pro Ala Ala Leu Pro Thr Leu Leu Pro Ser Gln
```

```
    610                 615                 620

Ser Pro Thr Asn Gln Thr Ser Pro Ile Ser Pro Thr His Pro His Ser
625                 630                 635                 640

Lys Ala Pro Gln Ile Pro Arg Glu Asp Gly Pro Ser Pro Lys Leu Ala
                645                 650                 655

Leu Trp Leu Pro Ser Pro Ala Pro Thr Ala Ala Pro Thr Ala Leu Gly
            660                 665                 670

Glu Ala Gly Leu Ala Glu His Ser Gln Arg Asp Asp Arg Trp Leu Leu
        675                 680                 685

Val Ala Leu Leu Val Pro Thr Cys Val Phe Leu Val Val Leu Leu Ala
    690                 695                 700

Leu Gly Ile Val Tyr Cys Thr Arg Cys Gly Pro His Ala Pro Asn Lys
705                 710                 715                 720

Arg Ile Thr Asp Cys Tyr Arg Trp Val Ile His Ala Gly Ser Lys Ser
                725                 730                 735

Pro Thr Glu Pro Met Pro Pro Arg Gly Ser Leu Thr Gly Val Gln Thr
            740                 745                 750

Cys Arg Thr Ser Val
        755


<210>  93
<211>  770
<212>  PRT
<213>  Artificial Sequence

<220>
<223>  A4_HUMAN


<400>  93
Met Leu Pro Gly Leu Ala Leu Leu Leu Leu Ala Ala Trp Thr Ala Arg
  1               5                  10                  15

Ala Leu Glu Val Pro Thr Asp Gly Asn Ala Gly Leu Leu Ala Glu Pro
             20                  25                  30

Gln Ile Ala Met Phe Cys Gly Arg Leu Asn Met His Met Asn Val Gln
         35                  40                  45

Asn Gly Lys Trp Asp Ser Asp Pro Ser Gly Thr Lys Thr Cys Ile Asp
     50                  55                  60

Thr Lys Glu Gly Ile Leu Gln Tyr Cys Gln Glu Val Tyr Pro Glu Leu
 65                  70                  75                  80

Gln Ile Thr Asn Val Val Glu Ala Asn Gln Pro Val Thr Ile Gln Asn
                 85                  90                  95

Trp Cys Lys Arg Gly Arg Lys Gln Cys Lys Thr His Pro His Phe Val
            100                 105                 110

Ile Pro Tyr Arg Cys Leu Val Gly Glu Phe Val Ser Asp Ala Leu Leu
        115                 120                 125
```

Val Pro Asp Lys Cys Lys Phe Leu His Gln Glu Arg Met Asp Val Cys
    130                 135                 140

Glu Thr His Leu His Trp His Thr Val Ala Lys Glu Thr Cys Ser Glu
145                 150                 155                 160

Lys Ser Thr Asn Leu His Asp Tyr Gly Met Leu Leu Pro Cys Gly Ile
                165                 170                 175

Asp Lys Phe Arg Gly Val Glu Phe Val Cys Cys Pro Leu Ala Glu Glu
            180                 185                 190

Ser Asp Asn Val Asp Ser Ala Asp Ala Glu Glu Asp Asp Ser Asp Val
        195                 200                 205

Trp Trp Gly Gly Ala Asp Thr Asp Tyr Ala Asp Gly Ser Glu Asp Lys
    210                 215                 220

Val Val Glu Val Ala Glu Glu Glu Glu Val Ala Glu Val Glu Glu Glu
225                 230                 235                 240

Glu Ala Asp Asp Asp Glu Asp Asp Glu Asp Gly Asp Glu Val Glu Glu
                245                 250                 255

Glu Ala Glu Glu Pro Tyr Glu Glu Ala Thr Glu Arg Thr Thr Ser Ile
            260                 265                 270

Ala Thr Thr Thr Thr Thr Thr Thr Glu Ser Val Glu Glu Val Val Arg
        275                 280                 285

Glu Val Cys Ser Glu Gln Ala Glu Thr Gly Pro Cys Arg Ala Met Ile
    290                 295                 300

Ser Arg Trp Tyr Phe Asp Val Thr Glu Gly Lys Cys Ala Pro Phe Phe
305                 310                 315                 320

Tyr Gly Gly Cys Gly Gly Asn Arg Asn Asn Phe Asp Thr Glu Glu Tyr
                325                 330                 335

Cys Met Ala Val Cys Gly Ser Ala Met Ser Gln Ser Leu Leu Lys Thr
            340                 345                 350

Thr Gln Glu Pro Leu Ala Arg Asp Pro Val Lys Leu Pro Thr Thr Ala
        355                 360                 365

Ala Ser Thr Pro Asp Ala Val Asp Lys Tyr Leu Glu Thr Pro Gly Asp
    370                 375                 380

Glu Asn Glu His Ala His Phe Gln Lys Ala Lys Glu Arg Leu Glu Ala
385                 390                 395                 400

Lys His Arg Glu Arg Met Ser Gln Val Met Arg Glu Trp Glu Glu Ala
                405                 410                 415

Glu Arg Gln Ala Lys Asn Leu Pro Lys Ala Asp Lys Lys Ala Val Ile
            420                 425                 430

Gln His Phe Gln Glu Lys Val Glu Ser Leu Glu Gln Glu Ala Ala Asn
        435                 440                 445

Glu Arg Gln Gln Leu Val Glu Thr His Met Ala Arg Val Glu Ala Met
    450                 455                 460

```
Leu Asn Asp Arg Arg Arg Leu Ala Leu Glu Asn Tyr Ile Thr Ala Leu
465                 470                 475                 480

Gln Ala Val Pro Pro Arg Pro Arg His Val Phe Asn Met Leu Lys Lys
                485                 490                 495

Tyr Val Arg Ala Glu Gln Lys Asp Arg Gln His Thr Leu Lys His Phe
            500                 505                 510

Glu His Val Arg Met Val Asp Pro Lys Lys Ala Ala Gln Ile Arg Ser
        515                 520                 525

Gln Val Met Thr His Leu Arg Val Ile Tyr Glu Arg Met Asn Gln Ser
    530                 535                 540

Leu Ser Leu Leu Tyr Asn Val Pro Ala Val Ala Glu Glu Ile Gln Asp
545                 550                 555                 560

Glu Val Asp Glu Leu Leu Gln Lys Glu Gln Asn Tyr Ser Asp Asp Val
                565                 570                 575

Leu Ala Asn Met Ile Ser Glu Pro Arg Ile Ser Tyr Gly Asn Asp Ala
            580                 585                 590

Leu Met Pro Ser Leu Thr Glu Thr Lys Thr Thr Val Glu Leu Leu Pro
        595                 600                 605

Val Asn Gly Glu Phe Ser Leu Asp Asp Leu Gln Pro Trp His Ser Phe
    610                 615                 620

Gly Ala Asp Ser Val Pro Ala Asn Thr Glu Asn Glu Val Glu Pro Val
625                 630                 635                 640

Asp Ala Arg Pro Ala Ala Asp Arg Gly Leu Thr Thr Arg Pro Gly Ser
                645                 650                 655

Gly Leu Thr Asn Ile Lys Thr Glu Glu Ile Ser Glu Val Lys Met Asp
            660                 665                 670

Ala Glu Phe Arg His Asp Ser Gly Tyr Glu Val His His Gln Lys Leu
        675                 680                 685

Val Phe Phe Ala Glu Asp Val Gly Ser Asn Lys Gly Ala Ile Ile Gly
    690                 695                 700

Leu Met Val Gly Gly Val Val Ile Ala Thr Val Ile Val Ile Thr Leu
705                 710                 715                 720

Val Met Leu Lys Lys Lys Gln Tyr Thr Ser Ile His His Gly Val Val
                725                 730                 735

Glu Val Asp Ala Ala Val Thr Pro Glu Glu Arg His Leu Ser Lys Met
            740                 745                 750

Gln Gln Asn Gly Tyr Glu Asn Pro Thr Tyr Lys Phe Phe Glu Gln Met
        755                 760                 765

Gln Asn
    770
```

<210> 94

<211> 780
<212> PRT
<213> Artificial Sequence

<220>
<223> ASAH2_HUMAN

<400> 94

```
Met Ala Lys Arg Thr Phe Ser Asn Leu Glu Thr Phe Leu Ile Phe Leu
 1               5                  10                  15

Leu Val Met Met Ser Ala Ile Thr Val Ala Leu Leu Ser Leu Leu Phe
            20                  25                  30

Ile Thr Ser Gly Thr Ile Glu Asn His Lys Asp Leu Gly Gly His Phe
        35                  40                  45

Phe Ser Thr Thr Gln Ser Pro Pro Ala Thr Gln Gly Ser Thr Ala Ala
    50                  55                  60

Gln Arg Ser Thr Ala Thr Gln His Ser Thr Ala Thr Gln Ser Ser Thr
65                  70                  75                  80

Ala Thr Gln Thr Ser Pro Val Pro Leu Thr Pro Glu Ser Pro Leu Phe
                85                  90                  95

Gln Asn Phe Ser Gly Tyr His Ile Gly Val Gly Arg Ala Asp Cys Thr
            100                 105                 110

Gly Gln Val Ala Asp Ile Asn Leu Met Gly Tyr Gly Lys Ser Gly Gln
        115                 120                 125

Asn Ala Gln Gly Ile Leu Thr Arg Leu Tyr Ser Arg Ala Phe Ile Met
    130                 135                 140

Ala Glu Pro Asp Gly Ser Asn Arg Thr Val Phe Val Ser Ile Asp Ile
145                 150                 155                 160

Gly Met Val Ser Gln Arg Leu Arg Leu Glu Val Leu Asn Arg Leu Gln
                165                 170                 175

Ser Lys Tyr Gly Ser Leu Tyr Arg Arg Asp Asn Val Ile Leu Ser Gly
            180                 185                 190

Thr His Thr His Ser Gly Pro Ala Gly Tyr Phe Gln Tyr Thr Val Phe
        195                 200                 205

Val Ile Ala Ser Glu Gly Phe Ser Asn Gln Thr Phe Gln His Met Val
    210                 215                 220

Thr Gly Ile Leu Lys Ser Ile Asp Ile Ala His Thr Asn Met Lys Pro
225                 230                 235                 240

Gly Lys Ile Phe Ile Asn Lys Gly Asn Val Asp Gly Val Gln Ile Asn
                245                 250                 255

Arg Ser Pro Tyr Ser Tyr Leu Gln Asn Pro Gln Ser Glu Arg Ala Arg
            260                 265                 270

Tyr Ser Ser Asn Thr Asp Lys Glu Met Ile Val Leu Lys Met Val Asp
        275                 280                 285
```

Leu Asn Gly Asp Asp Leu Gly Leu Ile Ser Trp Phe Ala Ile His Pro
290 295 300

Val Ser Met Asn Asn Ser Asn His Leu Val Asn Ser Asp Asn Val Gly
305 310 315 320

Tyr Ala Ser Tyr Leu Leu Glu Gln Glu Lys Asn Lys Gly Tyr Leu Pro
325 330 335

Gly Gln Gly Pro Phe Val Ala Ala Phe Ala Ser Ser Asn Leu Gly Asp
340 345 350

Val Ser Pro Asn Ile Leu Gly Pro Arg Cys Ile Asn Thr Gly Glu Ser
355 360 365

Cys Asp Asn Ala Asn Ser Thr Cys Pro Ile Gly Gly Pro Ser Met Cys
370 375 380

Ile Ala Lys Gly Pro Gly Gln Asp Met Phe Asp Ser Thr Gln Ile Ile
385 390 395 400

Gly Arg Ala Met Tyr Gln Arg Ala Lys Glu Leu Tyr Ala Ser Ala Ser
405 410 415

Gln Glu Val Thr Gly Pro Leu Ala Ser Ala His Gln Trp Val Asp Met
420 425 430

Thr Asp Val Thr Val Trp Leu Asn Ser Thr His Ala Ser Lys Thr Cys
435 440 445

Lys Pro Ala Leu Gly Tyr Ser Phe Ala Ala Gly Thr Ile Asp Gly Val
450 455 460

Gly Gly Leu Asn Phe Thr Gln Gly Lys Thr Glu Gly Asp Pro Phe Trp
465 470 475 480

Asp Thr Ile Arg Asp Gln Ile Leu Gly Lys Pro Ser Glu Glu Ile Lys
485 490 495

Glu Cys His Lys Pro Lys Pro Ile Leu Leu His Thr Gly Glu Leu Ser
500 505 510

Lys Pro His Pro Trp His Pro Asp Ile Val Asp Val Gln Ile Ile Thr
515 520 525

Leu Gly Ser Leu Ala Ile Thr Ala Ile Pro Gly Glu Phe Thr Thr Met
530 535 540

Ser Gly Arg Arg Leu Arg Glu Ala Val Gln Ala Glu Phe Ala Ser His
545 550 555 560

Gly Met Gln Asn Met Thr Val Val Ile Ser Gly Leu Cys Asn Val Tyr
565 570 575

Thr His Tyr Ile Thr Thr Tyr Glu Glu Tyr Gln Ala Gln Arg Tyr Glu
580 585 590

Ala Ala Ser Thr Ile Tyr Gly Pro His Thr Leu Ser Ala Tyr Ile Gln
595 600 605

Leu Phe Arg Asn Leu Ala Lys Ala Ile Ala Thr Asp Thr Val Ala Asn
610 615 620

Leu Ser Arg Gly Pro Glu Pro Pro Phe Phe Lys Gln Leu Ile Val Pro
625 630 635 640

Leu Ile Pro Ser Ile Val Asp Arg Ala Pro Lys Gly Arg Thr Phe Gly
645 650 655

Asp Val Leu Gln Pro Ala Lys Pro Glu Tyr Arg Val Gly Glu Val Ala
660 665 670

Glu Val Ile Phe Val Gly Ala Asn Pro Lys Asn Ser Val Gln Asn Gln
675 680 685

Thr His Gln Thr Phe Leu Thr Val Glu Lys Tyr Glu Ala Thr Ser Thr
690 695 700

Ser Trp Gln Ile Val Cys Asn Asp Ala Ser Trp Glu Thr Arg Phe Tyr
705 710 715 720

Trp His Lys Gly Leu Leu Gly Leu Ser Asn Ala Thr Val Glu Trp His
725 730 735

Ile Pro Asp Thr Ala Gln Pro Gly Ile Tyr Arg Ile Arg Tyr Phe Gly
740 745 750

His Asn Arg Lys Gln Asp Ile Leu Lys Pro Ala Val Ile Leu Ser Phe
755 760 765

Glu Gly Thr Ser Pro Ala Phe Glu Val Val Thr Ile
770 775 780

<210> 95
<211> 785
<212> PRT
<213> Artificial Sequence

<220>
<223> SP1_HUMAN

<400> 95
Met Ser Asp Gln Asp His Ser Met Asp Glu Met Thr Ala Val Val Lys
1 5 10 15

Ile Glu Lys Gly Val Gly Gly Asn Asn Gly Gly Asn Gly Asn Gly Gly
20 25 30

Gly Ala Phe Ser Gln Ala Arg Ser Ser Ser Thr Gly Ser Ser Ser Ser
35 40 45

Thr Gly Gly Gly Gly Gln Glu Ser Gln Pro Ser Pro Leu Ala Leu Leu
50 55 60

Ala Ala Thr Cys Ser Arg Ile Glu Ser Pro Asn Glu Asn Ser Asn Asn
65 70 75 80

Ser Gln Gly Pro Ser Gln Ser Gly Gly Thr Gly Glu Leu Asp Leu Thr
85 90 95

Ala Thr Gln Leu Ser Gln Gly Ala Asn Gly Trp Gln Ile Ile Ser Ser
100 105 110

Ser Ser Gly Ala Thr Pro Thr Ser Lys Glu Gln Ser Gly Ser Ser Thr

115 120 125

Asn Gly Ser Asn Gly Ser Glu Ser Ser Lys Asn Arg Thr Val Ser Gly
130 135 140

Gly Gln Tyr Val Val Ala Ala Ala Pro Asn Leu Gln Asn Gln Gln Val
145 150 155 160

Leu Thr Gly Leu Pro Gly Val Met Pro Asn Ile Gln Tyr Gln Val Ile
165 170 175

Pro Gln Phe Gln Thr Val Asp Gly Gln Gln Leu Gln Phe Ala Ala Thr
180 185 190

Gly Ala Gln Val Gln Gln Asp Gly Ser Gly Gln Ile Gln Ile Ile Pro
195 200 205

Gly Ala Asn Gln Gln Ile Ile Thr Asn Arg Gly Ser Gly Gly Asn Ile
210 215 220

Ile Ala Ala Met Pro Asn Leu Leu Gln Gln Ala Val Pro Leu Gln Gly
225 230 235 240

Leu Ala Asn Asn Val Leu Ser Gly Gln Thr Gln Tyr Val Thr Asn Val
245 250 255

Pro Val Ala Leu Asn Gly Asn Ile Thr Leu Leu Pro Val Asn Ser Val
260 265 270

Ser Ala Ala Thr Leu Thr Pro Ser Ser Gln Ala Val Thr Ile Ser Ser
275 280 285

Ser Gly Ser Gln Glu Ser Gly Ser Gln Pro Val Thr Ser Gly Thr Thr
290 295 300

Ile Ser Ser Ala Ser Leu Val Ser Ser Gln Ala Ser Ser Ser Ser Phe
305 310 315 320

Phe Thr Asn Ala Asn Ser Tyr Ser Thr Thr Thr Thr Thr Ser Asn Met
325 330 335

Gly Ile Met Asn Phe Thr Thr Ser Gly Ser Ser Gly Thr Asn Ser Gln
340 345 350

Gly Gln Thr Pro Gln Arg Val Ser Gly Leu Gln Gly Ser Asp Ala Leu
355 360 365

Asn Ile Gln Gln Asn Gln Thr Ser Gly Gly Ser Leu Gln Ala Gly Gln
370 375 380

Gln Lys Glu Gly Glu Gln Asn Gln Gln Thr Gln Gln Gln Gln Ile Leu
385 390 395 400

Ile Gln Pro Gln Leu Val Gln Gly Gly Gln Ala Leu Gln Ala Leu Gln
405 410 415

Ala Ala Pro Leu Ser Gly Gln Thr Phe Thr Thr Gln Ala Ile Ser Gln
420 425 430

Glu Thr Leu Gln Asn Leu Gln Leu Gln Ala Val Pro Asn Ser Gly Pro
435 440 445

Ile Ile Ile Arg Thr Pro Thr Val Gly Pro Asn Gly Gln Val Ser Trp

450 455 460

Gln Thr Leu Gln Leu Gln Asn Leu Gln Val Gln Asn Pro Gln Ala Gln
465 470 475 480

Thr Ile Thr Leu Ala Pro Met Gln Gly Val Ser Leu Gly Gln Thr Ser
485 490 495

Ser Ser Asn Thr Thr Leu Thr Pro Ile Ala Ser Ala Ala Ser Ile Pro
500 505 510

Ala Gly Thr Val Thr Val Asn Ala Ala Gln Leu Ser Ser Met Pro Gly
515 520 525

Leu Gln Thr Ile Asn Leu Ser Ala Leu Gly Thr Ser Gly Ile Gln Val
530 535 540

His Pro Ile Gln Gly Leu Pro Leu Ala Ile Ala Asn Ala Pro Gly Asp
545 550 555 560

His Gly Ala Gln Leu Gly Leu His Gly Ala Gly Gly Asp Gly Ile His
565 570 575

Asp Asp Thr Ala Gly Gly Glu Glu Gly Glu Asn Ser Pro Asp Ala Gln
580 585 590

Pro Gln Ala Gly Arg Arg Thr Arg Arg Glu Ala Cys Thr Cys Pro Tyr
595 600 605

Cys Lys Asp Ser Glu Gly Arg Gly Ser Gly Asp Pro Gly Lys Lys Lys
610 615 620

Gln His Ile Cys His Ile Gln Gly Cys Gly Lys Val Tyr Gly Lys Thr
625 630 635 640

Ser His Leu Arg Ala His Leu Arg Trp His Thr Gly Glu Arg Pro Phe
645 650 655

Met Cys Thr Trp Ser Tyr Cys Gly Lys Arg Phe Thr Arg Ser Asp Glu
660 665 670

Leu Gln Arg His Lys Arg Thr His Thr Gly Glu Lys Lys Phe Ala Cys
675 680 685

Pro Glu Cys Pro Lys Arg Phe Met Arg Ser Asp His Leu Ser Lys His
690 695 700

Ile Lys Thr His Gln Asn Lys Lys Gly Gly Pro Gly Val Ala Leu Ser
705 710 715 720

Val Gly Thr Leu Pro Leu Asp Ser Gly Ala Gly Ser Glu Gly Ser Gly
725 730 735

Thr Ala Thr Pro Ser Ala Leu Ile Thr Thr Asn Met Val Ala Met Glu
740 745 750

Ala Ile Cys Pro Glu Gly Ile Ala Arg Leu Ala Asn Ser Gly Ile Asn
755 760 765

Val Met Gln Val Ala Asp Leu Gln Ser Ile Asn Ile Ser Gly Asn Gly
770 775 780

Phe

785

<210> 96
<211> 797
<212> PRT
<213> Artificial Sequence

<220>
<223> IMPG1_HUMAN

<400> 96

```
Met Tyr Leu Glu Thr Arg Arg Ala Ile Phe Val Phe Trp Ile Phe Leu
1               5                   10                  15

Gln Val Gln Gly Thr Lys Asp Ile Ser Ile Asn Ile Tyr His Ser Glu
            20                  25                  30

Thr Lys Asp Ile Asp Asn Pro Pro Arg Asn Glu Thr Thr Glu Ser Thr
        35                  40                  45

Glu Lys Met Tyr Lys Met Ser Thr Met Arg Arg Ile Phe Asp Leu Ala
    50                  55                  60

Lys His Arg Thr Lys Arg Ser Ala Phe Phe Pro Thr Gly Val Lys Val
65                  70                  75                  80

Cys Pro Gln Glu Ser Met Lys Gln Ile Leu Asp Ser Leu Gln Ala Tyr
                85                  90                  95

Tyr Arg Leu Arg Val Cys Gln Glu Ala Val Trp Glu Ala Tyr Arg Ile
            100                 105                 110

Phe Leu Asp Arg Ile Pro Asp Thr Gly Glu Tyr Gln Asp Trp Val Ser
        115                 120                 125

Ile Cys Gln Gln Glu Thr Phe Cys Leu Phe Asp Ile Gly Lys Asn Phe
    130                 135                 140

Ser Asn Ser Gln Glu His Leu Asp Leu Leu Gln Gln Arg Ile Lys Gln
145                 150                 155                 160

Arg Ser Phe Pro Asp Arg Lys Asp Glu Ile Ser Ala Glu Lys Thr Leu
                165                 170                 175

Gly Glu Pro Gly Glu Thr Ile Val Ile Ser Thr Asp Val Ala Asn Val
            180                 185                 190

Ser Leu Gly Pro Phe Pro Leu Thr Pro Asp Asp Thr Leu Leu Asn Glu
        195                 200                 205

Ile Leu Asp Asn Thr Leu Asn Asp Thr Lys Met Pro Thr Thr Glu Arg
    210                 215                 220

Glu Thr Glu Phe Ala Val Leu Glu Glu Gln Arg Val Glu Leu Ser Val
225                 230                 235                 240

Ser Leu Val Asn Gln Lys Phe Lys Ala Glu Leu Ala Asp Ser Gln Ser
                245                 250                 255

Pro Tyr Tyr Gln Glu Leu Ala Gly Lys Ser Gln Leu Gln Met Gln Lys
            260                 265                 270
```

```
Ile Phe Lys Lys Leu Pro Gly Phe Lys Lys Ile His Val Leu Gly Phe
        275                 280                 285

Arg Pro Lys Lys Glu Lys Asp Gly Ser Ser Ser Thr Glu Met Gln Leu
    290                 295                 300

Thr Ala Ile Phe Lys Arg His Ser Ala Glu Ala Lys Ser Pro Ala Ser
305                 310                 315                 320

Asp Leu Leu Ser Phe Asp Ser Asn Lys Ile Glu Ser Glu Glu Val Tyr
                325                 330                 335

His Gly Thr Met Glu Glu Asp Lys Gln Pro Glu Ile Tyr Leu Thr Ala
            340                 345                 350

Thr Asp Leu Lys Arg Leu Ile Ser Lys Ala Leu Glu Glu Glu Gln Ser
        355                 360                 365

Leu Asp Val Gly Thr Ile Gln Phe Thr Asp Glu Ile Ala Gly Ser Leu
    370                 375                 380

Pro Ala Phe Gly Pro Asp Thr Gln Ser Glu Leu Pro Thr Ser Phe Ala
385                 390                 395                 400

Val Ile Thr Glu Asp Ala Thr Leu Ser Pro Glu Leu Pro Pro Val Glu
                405                 410                 415

Pro Gln Leu Glu Thr Val Asp Gly Ala Glu His Gly Leu Pro Asp Thr
            420                 425                 430

Ser Trp Ser Pro Pro Ala Met Ala Ser Thr Ser Leu Ser Glu Ala Pro
        435                 440                 445

Pro Phe Phe Met Ala Ser Ser Ile Phe Ser Leu Thr Asp Gln Gly Thr
    450                 455                 460

Thr Asp Thr Met Ala Thr Asp Gln Thr Met Leu Val Pro Gly Leu Thr
465                 470                 475                 480

Ile Pro Thr Ser Asp Tyr Ser Ala Ile Ser Gln Leu Ala Leu Gly Ile
                485                 490                 495

Ser His Pro Pro Ala Ser Ser Asp Asp Ser Arg Ser Ser Ala Gly Gly
            500                 505                 510

Glu Asp Met Val Arg His Leu Asp Glu Met Asp Leu Ser Asp Thr Pro
        515                 520                 525

Ala Pro Ser Glu Val Pro Glu Leu Ser Glu Tyr Val Ser Val Pro Asp
    530                 535                 540

His Phe Leu Glu Asp Thr Thr Pro Val Ser Ala Leu Gln Tyr Ile Thr
545                 550                 555                 560

Thr Ser Ser Met Thr Ile Ala Pro Lys Gly Arg Glu Leu Val Val Phe
                565                 570                 575

Phe Ser Leu Arg Val Ala Asn Met Ala Phe Ser Asn Asp Leu Phe Asn
            580                 585                 590

Lys Ser Ser Leu Glu Tyr Arg Ala Leu Glu Gln Gln Phe Thr Gln Leu
        595                 600                 605
```

Leu Val Pro Tyr Leu Arg Ser Asn Leu Thr Gly Phe Lys Gln Leu Glu
610 615 620

Ile Leu Asn Phe Arg Asn Gly Ser Val Ile Val Asn Ser Lys Met Lys
625 630 635 640

Phe Ala Lys Ser Val Pro Tyr Asn Leu Thr Lys Ala Val His Gly Val
645 650 655

Leu Glu Asp Phe Arg Ser Ala Ala Ala Gln Gln Leu His Leu Glu Ile
660 665 670

Asp Ser Tyr Ser Leu Asn Ile Glu Pro Ala Asp Gln Ala Asp Pro Cys
675 680 685

Lys Phe Leu Ala Cys Gly Glu Phe Ala Gln Cys Val Lys Asn Glu Arg
690 695 700

Thr Glu Glu Ala Glu Cys Arg Cys Lys Pro Gly Tyr Asp Ser Gln Gly
705 710 715 720

Ser Leu Asp Gly Leu Glu Pro Gly Leu Cys Gly Pro Gly Thr Lys Glu
725 730 735

Cys Glu Val Leu Gln Gly Lys Gly Ala Pro Cys Arg Leu Pro Asp His
740 745 750

Ser Glu Asn Gln Ala Tyr Lys Thr Ser Val Lys Lys Phe Gln Asn Gln
755 760 765

Gln Asn Asn Lys Val Ile Ser Lys Arg Asn Ser Glu Leu Leu Thr Val
770 775 780

Glu Tyr Glu Glu Phe Asn His Gln Asp Trp Glu Gly Asn
785 790 795

<210> 97
<211> 815
<212> PRT
<213> Artificial Sequence

<220>
<223> SL9A1_HUMAN

<400> 97
Met Val Leu Arg Ser Gly Ile Cys Gly Leu Ser Pro His Arg Ile Phe
1 5 10 15

Pro Ser Leu Leu Val Val Val Ala Leu Val Gly Leu Leu Pro Val Leu
20 25 30

Arg Ser His Gly Leu Gln Leu Ser Pro Thr Ala Ser Thr Ile Arg Ser
35 40 45

Ser Glu Pro Pro Arg Glu Arg Ser Ile Gly Asp Val Thr Thr Ala Pro
50 55 60

Pro Glu Val Thr Pro Glu Ser Arg Pro Val Asn His Ser Val Thr Asp
65 70 75 80

```
His Gly Met Lys Pro Arg Lys Ala Phe Pro Val Leu Gly Ile Asp Tyr
                85                  90                  95

Thr His Val Arg Thr Pro Phe Glu Ile Ser Leu Trp Ile Leu Leu Ala
            100                 105                 110

Cys Leu Met Lys Ile Gly Phe His Val Ile Pro Thr Ile Ser Ser Ile
        115                 120                 125

Val Pro Glu Ser Cys Leu Leu Ile Val Val Gly Leu Leu Val Gly Gly
    130                 135                 140

Leu Ile Lys Gly Val Gly Glu Thr Pro Pro Phe Leu Gln Ser Asp Val
145                 150                 155                 160

Phe Phe Leu Phe Leu Leu Pro Pro Ile Ile Leu Asp Ala Gly Tyr Phe
                165                 170                 175

Leu Pro Leu Arg Gln Phe Thr Glu Asn Leu Gly Thr Ile Leu Ile Phe
            180                 185                 190

Ala Val Val Gly Thr Leu Trp Asn Ala Phe Phe Leu Gly Gly Leu Met
        195                 200                 205

Tyr Ala Val Cys Leu Val Gly Gly Glu Gln Ile Asn Asn Ile Gly Leu
    210                 215                 220

Leu Asp Asn Leu Leu Phe Gly Ser Ile Ile Ser Ala Val Asp Pro Val
225                 230                 235                 240

Ala Val Leu Ala Val Phe Glu Glu Ile His Ile Asn Glu Leu Leu His
                245                 250                 255

Ile Leu Val Phe Gly Glu Ser Leu Leu Asn Asp Ala Val Thr Val Val
            260                 265                 270

Leu Tyr His Leu Phe Glu Glu Phe Ala Asn Tyr Glu His Val Gly Ile
        275                 280                 285

Val Asp Ile Phe Leu Gly Phe Leu Ser Phe Phe Val Val Ala Leu Gly
    290                 295                 300

Gly Val Leu Val Gly Val Val Tyr Gly Val Ile Ala Ala Phe Thr Ser
305                 310                 315                 320

Arg Phe Thr Ser His Ile Arg Val Ile Glu Pro Leu Phe Val Phe Leu
                325                 330                 335

Tyr Ser Tyr Met Ala Tyr Leu Ser Ala Glu Leu Phe His Leu Ser Gly
            340                 345                 350

Ile Met Ala Leu Ile Ala Ser Gly Val Val Met Arg Pro Tyr Val Glu
        355                 360                 365

Ala Asn Ile Ser His Lys Ser His Thr Thr Ile Lys Tyr Phe Leu Lys
    370                 375                 380

Met Trp Ser Ser Val Ser Glu Thr Leu Ile Phe Ile Phe Leu Gly Val
385                 390                 395                 400

Ser Thr Val Ala Gly Ser His His Trp Asn Trp Thr Phe Val Ile Ser
                405                 410                 415
```

Thr Leu Leu Phe Cys Leu Ile Ala Arg Val Leu Gly Val Leu Gly Leu
420 425 430

Thr Trp Phe Ile Asn Lys Phe Arg Ile Val Lys Leu Thr Pro Lys Asp
435 440 445

Gln Phe Ile Ile Ala Tyr Gly Gly Leu Arg Gly Ala Ile Ala Phe Ser
450 455 460

Leu Gly Tyr Leu Leu Asp Lys Lys His Phe Pro Met Cys Asp Leu Phe
465 470 475 480

Leu Thr Ala Ile Ile Thr Val Ile Phe Phe Thr Val Phe Val Gln Gly
485 490 495

Met Thr Ile Arg Pro Leu Val Asp Leu Leu Ala Val Lys Lys Lys Gln
500 505 510

Glu Thr Lys Arg Ser Ile Asn Glu Glu Ile His Thr Gln Phe Leu Asp
515 520 525

His Leu Leu Thr Gly Ile Glu Asp Ile Cys Gly His Tyr Gly His His
530 535 540

His Trp Lys Asp Lys Leu Asn Arg Phe Asn Lys Lys Tyr Val Lys Lys
545 550 555 560

Cys Leu Ile Ala Gly Glu Arg Ser Lys Glu Pro Gln Leu Ile Ala Phe
565 570 575

Tyr His Lys Met Glu Met Lys Gln Ala Ile Glu Leu Val Glu Ser Gly
580 585 590

Gly Met Gly Lys Ile Pro Ser Ala Val Ser Thr Val Ser Met Gln Asn
595 600 605

Ile His Pro Lys Ser Leu Pro Ser Glu Arg Ile Leu Pro Ala Leu Ser
610 615 620

Lys Asp Lys Glu Glu Glu Ile Arg Lys Ile Leu Arg Asn Asn Leu Gln
625 630 635 640

Lys Thr Arg Gln Arg Leu Arg Ser Tyr Asn Arg His Thr Leu Val Ala
645 650 655

Asp Pro Tyr Glu Glu Ala Trp Asn Gln Met Leu Leu Arg Arg Gln Lys
660 665 670

Ala Arg Gln Leu Glu Gln Lys Ile Asn Asn Tyr Leu Thr Val Pro Ala
675 680 685

His Lys Leu Asp Ser Pro Thr Met Ser Arg Ala Arg Ile Gly Ser Asp
690 695 700

Pro Leu Ala Tyr Glu Pro Lys Glu Asp Leu Pro Val Ile Thr Ile Asp
705 710 715 720

Pro Ala Ser Pro Gln Ser Pro Glu Ser Val Asp Leu Val Asn Glu Glu
725 730 735

Leu Lys Gly Lys Val Leu Gly Leu Ser Arg Asp Pro Ala Lys Val Ala
740 745 750

Glu Glu Asp Glu Asp Asp Asp Gly Gly Ile Met Met Arg Ser Lys Glu
755 760 765

Thr Ser Ser Pro Gly Thr Asp Asp Val Phe Thr Pro Ala Pro Ser Asp
770 775 780

Ser Pro Ser Ser Gln Arg Ile Gln Arg Cys Leu Ser Asp Pro Gly Pro
785 790 795 800

His Pro Glu Pro Gly Glu Gly Glu Pro Phe Phe Pro Lys Gly Gln
805 810 815

<210> 98
<211> 882
<212> PRT
<213> Artificial Sequence

<220>
<223> CADH1_HUMAN

<400> 98
Met Gly Pro Trp Ser Arg Ser Leu Ser Ala Leu Leu Leu Leu Leu Gln
1 5 10 15

Val Ser Ser Trp Leu Cys Gln Glu Pro Glu Pro Cys His Pro Gly Phe
20 25 30

Asp Ala Glu Ser Tyr Thr Phe Thr Val Pro Arg Arg His Leu Glu Arg
35 40 45

Gly Arg Val Leu Gly Arg Val Asn Phe Glu Asp Cys Thr Gly Arg Gln
50 55 60

Arg Thr Ala Tyr Phe Ser Leu Asp Thr Arg Phe Lys Val Gly Thr Asp
65 70 75 80

Gly Val Ile Thr Val Lys Arg Pro Leu Arg Phe His Asn Pro Gln Ile
85 90 95

His Phe Leu Val Tyr Ala Trp Asp Ser Thr Tyr Arg Lys Phe Ser Thr
100 105 110

Lys Val Thr Leu Asn Thr Val Gly His His His Arg Pro Pro Pro His
115 120 125

Gln Ala Ser Val Ser Gly Ile Gln Ala Glu Leu Leu Thr Phe Pro Asn
130 135 140

Ser Ser Pro Gly Leu Arg Arg Gln Lys Arg Asp Trp Val Ile Pro Pro
145 150 155 160

Ile Ser Cys Pro Glu Asn Glu Lys Gly Pro Phe Pro Lys Asn Leu Val
165 170 175

Gln Ile Lys Ser Asn Lys Asp Lys Glu Gly Lys Val Phe Tyr Ser Ile
180 185 190

Thr Gly Gln Gly Ala Asp Thr Pro Pro Val Gly Val Phe Ile Ile Glu
195 200 205

Arg Glu Thr Gly Trp Leu Lys Val Thr Glu Pro Leu Asp Arg Glu Arg

```
            210                 215                 220

Ile Ala Thr Tyr Thr Leu Phe Ser His Ala Val Ser Ser Asn Gly Asn
225                 230                 235                 240

Ala Val Glu Asp Pro Met Glu Ile Leu Ile Thr Val Thr Asp Gln Asn
                245                 250                 255

Asp Asn Lys Pro Glu Phe Thr Gln Glu Val Phe Lys Gly Ser Val Met
            260                 265                 270

Glu Gly Ala Leu Pro Gly Thr Ser Val Met Glu Val Thr Ala Thr Asp
        275                 280                 285

Ala Asp Asp Asp Val Asn Thr Tyr Asn Ala Ala Ile Ala Tyr Thr Ile
    290                 295                 300

Leu Ser Gln Asp Pro Glu Leu Pro Asp Lys Asn Met Phe Thr Ile Asn
305                 310                 315                 320

Arg Asn Thr Gly Val Ile Ser Val Val Thr Thr Gly Leu Asp Arg Glu
                325                 330                 335

Ser Phe Pro Thr Tyr Thr Leu Val Val Gln Ala Ala Asp Leu Gln Gly
            340                 345                 350

Glu Gly Leu Ser Thr Thr Ala Thr Ala Val Ile Thr Val Thr Asp Thr
        355                 360                 365

Asn Asp Asn Pro Pro Ile Phe Asn Pro Thr Thr Tyr Lys Gly Gln Val
    370                 375                 380

Pro Glu Asn Glu Ala Asn Val Val Ile Thr Thr Leu Lys Val Thr Asp
385                 390                 395                 400

Ala Asp Ala Pro Asn Thr Pro Ala Trp Glu Ala Val Tyr Thr Ile Leu
                405                 410                 415

Asn Asp Asp Gly Gly Gln Phe Val Val Thr Thr Asn Pro Val Asn Asn
            420                 425                 430

Asp Gly Ile Leu Lys Thr Ala Lys Gly Leu Asp Phe Glu Ala Lys Gln
        435                 440                 445

Gln Tyr Ile Leu His Val Ala Val Thr Asn Val Val Pro Phe Glu Val
    450                 455                 460

Ser Leu Thr Thr Ser Thr Ala Thr Val Thr Val Asp Val Leu Asp Val
465                 470                 475                 480

Asn Glu Ala Pro Ile Phe Val Pro Pro Glu Lys Arg Val Glu Val Ser
                485                 490                 495

Glu Asp Phe Gly Val Gly Gln Glu Ile Thr Ser Tyr Thr Ala Gln Glu
            500                 505                 510

Pro Asp Thr Phe Met Glu Gln Lys Ile Thr Tyr Arg Ile Trp Arg Asp
        515                 520                 525

Thr Ala Asn Trp Leu Glu Ile Asn Pro Asp Thr Gly Ala Ile Ser Thr
    530                 535                 540

Arg Ala Glu Leu Asp Arg Glu Asp Phe Glu His Val Lys Asn Ser Thr
```

```
545                 550                 555                 560

Tyr Thr Ala Leu Ile Ile Ala Thr Asp Asn Gly Ser Pro Val Ala Thr
                565                 570                 575

Gly Thr Gly Thr Leu Leu Leu Ile Leu Ser Asp Val Asn Asp Asn Ala
            580                 585                 590

Pro Ile Pro Glu Pro Arg Thr Ile Phe Phe Cys Glu Arg Asn Pro Lys
        595                 600                 605

Pro Gln Val Ile Asn Ile Ile Asp Ala Asp Leu Pro Pro Asn Thr Ser
    610                 615                 620

Pro Phe Thr Ala Glu Leu Thr His Gly Ala Ser Ala Asn Trp Thr Ile
625                 630                 635                 640

Gln Tyr Asn Asp Pro Thr Gln Glu Ser Ile Ile Leu Lys Pro Lys Met
                645                 650                 655

Ala Leu Glu Val Gly Asp Tyr Lys Ile Asn Leu Lys Leu Met Asp Asn
            660                 665                 670

Gln Asn Lys Asp Gln Val Thr Thr Leu Glu Val Ser Val Cys Asp Cys
        675                 680                 685

Glu Gly Ala Ala Gly Val Cys Arg Lys Ala Gln Pro Val Glu Ala Gly
    690                 695                 700

Leu Gln Ile Pro Ala Ile Leu Gly Ile Leu Gly Gly Ile Leu Ala Leu
705                 710                 715                 720

Leu Ile Leu Ile Leu Leu Leu Leu Leu Phe Leu Arg Arg Arg Ala Val
                725                 730                 735

Val Lys Glu Pro Leu Leu Pro Pro Glu Asp Asp Thr Arg Asp Asn Val
            740                 745                 750

Tyr Tyr Tyr Asp Glu Glu Gly Gly Gly Glu Glu Asp Gln Asp Phe Asp
        755                 760                 765

Leu Ser Gln Leu His Arg Gly Leu Asp Ala Arg Pro Glu Val Thr Arg
    770                 775                 780

Asn Asp Val Ala Pro Thr Leu Met Ser Val Pro Arg Tyr Leu Pro Arg
785                 790                 795                 800

Pro Ala Asn Pro Asp Glu Ile Gly Asn Phe Ile Asp Glu Asn Leu Lys
                805                 810                 815

Ala Ala Asp Thr Asp Pro Thr Ala Pro Pro Tyr Asp Ser Leu Leu Val
            820                 825                 830

Phe Asp Tyr Glu Gly Ser Gly Ser Glu Ala Ala Ser Leu Ser Ser Leu
        835                 840                 845

Asn Ser Ser Glu Ser Asp Lys Asp Gln Asp Tyr Asp Tyr Leu Asn Glu
    850                 855                 860

Trp Gly Asn Arg Phe Lys Lys Leu Ala Asp Met Tyr Gly Gly Gly Glu
865                 870                 875                 880

Asp Asp
```

<210> 99
<211> 895
<212> PRT
<213> Artificial Sequence

<220>
<223> DAG1_HUMAN

<400> 99

```
Met Arg Met Ser Val Gly Leu Ser Leu Leu Leu Pro Leu Ser Gly Arg
  1               5                  10                  15

Thr Phe Leu Leu Leu Leu Ser Val Val Met Ala Gln Ser His Trp Pro
             20                  25                  30

Ser Glu Pro Ser Glu Ala Val Arg Asp Trp Glu Asn Gln Leu Glu Ala
        35                  40                  45

Ser Met His Ser Val Leu Ser Asp Leu His Glu Ala Val Pro Thr Val
    50                  55                  60

Val Gly Ile Pro Asp Gly Thr Ala Val Val Gly Arg Ser Phe Arg Val
 65                  70                  75                  80

Thr Ile Pro Thr Asp Leu Ile Ala Ser Ser Gly Asp Ile Ile Lys Val
                 85                  90                  95

Ser Ala Ala Gly Lys Glu Ala Leu Pro Ser Trp Leu His Trp Asp Ser
            100                 105                 110

Gln Ser His Thr Leu Glu Gly Leu Pro Leu Asp Thr Asp Lys Gly Val
        115                 120                 125

His Tyr Ile Ser Val Ser Ala Thr Arg Leu Gly Ala Asn Gly Ser His
    130                 135                 140

Ile Pro Gln Thr Ser Ser Val Phe Ser Ile Glu Val Tyr Pro Glu Asp
145                 150                 155                 160

His Ser Glu Leu Gln Ser Val Arg Thr Ala Ser Pro Asp Pro Gly Glu
                165                 170                 175

Val Val Ser Ser Ala Cys Ala Ala Asp Glu Pro Val Thr Val Leu Thr
            180                 185                 190

Val Ile Leu Asp Ala Asp Leu Thr Lys Met Thr Pro Lys Gln Arg Ile
        195                 200                 205

Asp Leu Leu His Arg Met Arg Ser Phe Ser Glu Val Glu Leu His Asn
    210                 215                 220

Met Lys Leu Val Pro Val Val Asn Asn Arg Leu Phe Asp Met Ser Ala
225                 230                 235                 240

Phe Met Ala Gly Pro Gly Asn Ala Lys Lys Val Val Glu Asn Gly Ala
                245                 250                 255

Leu Leu Ser Trp Lys Leu Gly Cys Ser Leu Asn Gln Asn Ser Val Pro
            260                 265                 270
```

```
Asp Ile His Gly Val Glu Ala Pro Ala Arg Glu Gly Ala Met Ser Ala
        275                 280                 285

Gln Leu Gly Tyr Pro Val Val Gly Trp His Ile Ala Asn Lys Lys Pro
    290                 295                 300

Pro Leu Pro Lys Arg Val Arg Arg Gln Ile His Ala Thr Pro Thr Pro
305                 310                 315                 320

Val Thr Ala Ile Gly Pro Pro Thr Thr Ala Ile Gln Glu Pro Pro Ser
                325                 330                 335

Arg Ile Val Pro Thr Pro Thr Ser Pro Ala Ile Ala Pro Pro Thr Glu
            340                 345                 350

Thr Met Ala Pro Pro Val Arg Asp Pro Val Pro Gly Lys Pro Thr Val
        355                 360                 365

Thr Ile Arg Thr Arg Gly Ala Ile Ile Gln Thr Pro Thr Leu Gly Pro
    370                 375                 380

Ile Gln Pro Thr Arg Val Ser Glu Ala Gly Thr Thr Val Pro Gly Gln
385                 390                 395                 400

Ile Arg Pro Thr Met Thr Ile Pro Gly Tyr Val Glu Pro Thr Ala Val
                405                 410                 415

Ala Thr Pro Pro Thr Thr Thr Thr Lys Lys Pro Arg Val Ser Thr Pro
            420                 425                 430

Lys Pro Ala Thr Pro Ser Thr Asp Ser Thr Thr Thr Thr Thr Arg Arg
        435                 440                 445

Pro Thr Lys Lys Pro Arg Thr Pro Arg Pro Val Pro Arg Val Thr Thr
    450                 455                 460

Lys Val Ser Ile Thr Arg Leu Glu Thr Ala Ser Pro Pro Thr Arg Ile
465                 470                 475                 480

Arg Thr Thr Thr Ser Gly Val Pro Arg Gly Gly Glu Pro Asn Gln Arg
                485                 490                 495

Pro Glu Leu Lys Asn His Ile Asp Arg Val Asp Ala Trp Val Gly Thr
            500                 505                 510

Tyr Phe Glu Val Lys Ile Pro Ser Asp Thr Phe Tyr Asp His Glu Asp
        515                 520                 525

Thr Thr Thr Asp Lys Leu Lys Leu Thr Leu Lys Leu Arg Glu Gln Gln
    530                 535                 540

Leu Val Gly Glu Lys Ser Trp Val Gln Phe Asn Ser Asn Ser Gln Leu
545                 550                 555                 560

Met Tyr Gly Leu Pro Asp Ser Ser His Val Gly Lys His Glu Tyr Phe
                565                 570                 575

Met His Ala Thr Asp Lys Gly Gly Leu Ser Ala Val Asp Ala Phe Glu
            580                 585                 590

Ile His Val His Arg Arg Pro Gln Gly Asp Arg Ala Pro Ala Arg Phe
        595                 600                 605
```

Lys Ala Lys Phe Val Gly Asp Pro Ala Leu Val Leu Asn Asp Ile His
    610                 615                 620

Lys Lys Ile Ala Leu Val Lys Lys Leu Ala Phe Ala Phe Gly Asp Arg
625                 630                 635                 640

Asn Cys Ser Thr Ile Thr Leu Gln Asn Ile Thr Arg Gly Ser Ile Val
                645                 650                 655

Val Glu Trp Thr Asn Asn Thr Leu Pro Leu Glu Pro Cys Pro Lys Glu
            660                 665                 670

Gln Ile Ala Gly Leu Ser Arg Arg Ile Ala Glu Asp Asp Gly Lys Pro
        675                 680                 685

Arg Pro Ala Phe Ser Asn Ala Leu Glu Pro Asp Phe Lys Ala Thr Ser
    690                 695                 700

Ile Thr Val Thr Gly Ser Gly Ser Cys Arg His Leu Gln Phe Ile Pro
705                 710                 715                 720

Val Val Pro Pro Arg Arg Val Pro Ser Glu Ala Pro Pro Thr Glu Val
                725                 730                 735

Pro Asp Arg Asp Pro Glu Lys Ser Ser Glu Asp Asp Val Tyr Leu His
            740                 745                 750

Thr Val Ile Pro Ala Val Val Val Ala Ala Ile Leu Leu Ile Ala Gly
        755                 760                 765

Ile Ile Ala Met Ile Cys Tyr Arg Lys Lys Arg Lys Gly Lys Leu Thr
    770                 775                 780

Leu Glu Asp Gln Ala Thr Phe Ile Lys Lys Gly Val Pro Ile Ile Phe
785                 790                 795                 800

Ala Asp Glu Leu Asp Asp Ser Lys Pro Pro Pro Ser Ser Ser Met Pro
                805                 810                 815

Leu Ile Leu Gln Glu Glu Lys Ala Pro Leu Pro Pro Pro Glu Tyr Pro
            820                 825                 830

Asn Gln Ser Val Pro Glu Thr Thr Pro Leu Asn Gln Asp Thr Met Gly
        835                 840                 845

Glu Tyr Thr Pro Leu Arg Asp Glu Asp Pro Asn Ala Pro Pro Tyr Gln
    850                 855                 860

Pro Pro Pro Pro Phe Thr Ala Pro Met Glu Gly Lys Gly Ser Arg Pro
865                 870                 875                 880

Lys Asn Met Thr Pro Tyr Arg Ser Pro Pro Pro Tyr Val Pro Pro
                885                 890                 895

<210> 100
<211> 930
<212> PRT
<213> Artificial Sequence

<220>
<223> ITIH4_HUMAN

<400> 100

Met Lys Pro Pro Arg Pro Val Arg Thr Cys Ser Lys Val Leu Val Leu
1 5 10 15

Leu Ser Leu Leu Ala Ile His Gln Thr Thr Thr Ala Glu Lys Asn Gly
20 25 30

Ile Asp Ile Tyr Ser Leu Thr Val Asp Ser Arg Val Ser Ser Arg Phe
35 40 45

Ala His Thr Val Val Thr Ser Arg Val Val Asn Arg Ala Asn Thr Val
50 55 60

Gln Glu Ala Thr Phe Gln Met Glu Leu Pro Lys Lys Ala Phe Ile Thr
65 70 75 80

Asn Phe Ser Met Ile Ile Asp Gly Met Thr Tyr Pro Gly Ile Ile Lys
85 90 95

Glu Lys Ala Glu Ala Gln Ala Gln Tyr Ser Ala Ala Val Ala Lys Gly
100 105 110

Lys Ser Ala Gly Leu Val Lys Ala Thr Gly Arg Asn Met Glu Gln Phe
115 120 125

Gln Val Ser Val Ser Val Ala Pro Asn Ala Lys Ile Thr Phe Glu Leu
130 135 140

Val Tyr Glu Glu Leu Leu Lys Arg Arg Leu Gly Val Tyr Glu Leu Leu
145 150 155 160

Leu Lys Val Arg Pro Gln Gln Leu Val Lys His Leu Gln Met Asp Ile
165 170 175

His Ile Phe Glu Pro Gln Gly Ile Ser Phe Leu Glu Thr Glu Ser Thr
180 185 190

Phe Met Thr Asn Gln Leu Val Asp Ala Leu Thr Thr Trp Gln Asn Lys
195 200 205

Thr Lys Ala His Ile Arg Phe Lys Pro Thr Leu Ser Gln Gln Gln Lys
210 215 220

Ser Pro Glu Gln Gln Glu Thr Val Leu Asp Gly Asn Leu Ile Ile Arg
225 230 235 240

Tyr Asp Val Asp Arg Ala Ile Ser Gly Gly Ser Ile Gln Ile Glu Asn
245 250 255

Gly Tyr Phe Val His Tyr Phe Ala Pro Glu Gly Leu Thr Thr Met Pro
260 265 270

Lys Asn Val Val Phe Val Ile Asp Lys Ser Gly Ser Met Ser Gly Arg
275 280 285

Lys Ile Gln Gln Thr Arg Glu Ala Leu Ile Lys Ile Leu Asp Asp Leu
290 295 300

Ser Pro Arg Asp Gln Phe Asn Leu Ile Val Phe Ser Thr Glu Ala Thr
305 310 315 320

Gln Trp Arg Pro Ser Leu Val Pro Ala Ser Ala Glu Asn Val Asn Lys
325 330 335

Ala Arg Ser Phe Ala Ala Gly Ile Gln Ala Leu Gly Gly Thr Asn Ile
340 345 350

Asn Asp Ala Met Leu Met Ala Val Gln Leu Leu Asp Ser Ser Asn Gln
355 360 365

Glu Glu Arg Leu Pro Glu Gly Ser Val Ser Leu Ile Ile Leu Leu Thr
370 375 380

Asp Gly Asp Pro Thr Val Gly Glu Thr Asn Pro Arg Ser Ile Gln Asn
385 390 395 400

Asn Val Arg Glu Ala Val Ser Gly Arg Tyr Ser Leu Phe Cys Leu Gly
405 410 415

Phe Gly Phe Asp Val Ser Tyr Ala Phe Leu Glu Lys Leu Ala Leu Asp
420 425 430

Asn Gly Gly Leu Ala Arg Arg Ile His Glu Asp Ser Asp Ser Ala Leu
435 440 445

Gln Leu Gln Asp Phe Tyr Gln Glu Val Ala Asn Pro Leu Leu Thr Ala
450 455 460

Val Thr Phe Glu Tyr Pro Ser Asn Ala Val Glu Glu Val Thr Gln Asn
465 470 475 480

Asn Phe Arg Leu Leu Phe Lys Gly Ser Glu Met Val Val Ala Gly Lys
485 490 495

Leu Gln Asp Arg Gly Pro Asp Val Leu Thr Ala Thr Val Ser Gly Lys
500 505 510

Leu Pro Thr Gln Asn Ile Thr Phe Gln Thr Glu Ser Ser Val Ala Glu
515 520 525

Gln Glu Ala Glu Phe Gln Ser Pro Lys Tyr Ile Phe His Asn Phe Met
530 535 540

Glu Arg Leu Trp Ala Tyr Leu Thr Ile Gln Gln Leu Leu Glu Gln Thr
545 550 555 560

Val Ser Ala Ser Asp Ala Asp Gln Gln Ala Leu Arg Asn Gln Ala Leu
565 570 575

Asn Leu Ser Leu Ala Tyr Ser Phe Val Thr Pro Leu Thr Ser Met Val
580 585 590

Val Thr Lys Pro Asp Asp Gln Glu Gln Ser Gln Val Ala Glu Lys Pro
595 600 605

Met Glu Gly Glu Ser Arg Asn Arg Asn Val His Ser Gly Ser Thr Phe
610 615 620

Phe Lys Tyr Tyr Leu Gln Gly Ala Lys Ile Pro Lys Pro Glu Ala Ser
625 630 635 640

Phe Ser Pro Arg Arg Gly Trp Asn Arg Gln Ala Gly Ala Ala Gly Ser
645 650 655

Arg Met Asn Phe Arg Pro Gly Val Leu Ser Ser Arg Gln Leu Gly Leu
660 665 670

Pro Gly Pro Pro Asp Val Pro Asp His Ala Ala Tyr His Pro Phe Arg
675 680 685

Arg Leu Ala Ile Leu Pro Ala Ser Ala Pro Pro Ala Thr Ser Asn Pro
690 695 700

Asp Pro Ala Val Ser Arg Val Met Asn Met Lys Ile Glu Glu Thr Thr
705 710 715 720

Met Thr Thr Gln Thr Pro Ala Pro Ile Gln Ala Pro Ser Ala Ile Leu
725 730 735

Pro Leu Pro Gly Gln Ser Val Glu Arg Leu Cys Val Asp Pro Arg His
740 745 750

Arg Gln Gly Pro Val Asn Leu Leu Ser Asp Pro Glu Gln Gly Val Glu
755 760 765

Val Thr Gly Gln Tyr Glu Arg Glu Lys Ala Gly Phe Ser Trp Ile Glu
770 775 780

Val Thr Phe Lys Asn Pro Leu Val Trp Val His Ala Ser Pro Glu His
785 790 795 800

Val Val Val Thr Arg Asn Arg Arg Ser Ser Ala Tyr Lys Trp Lys Glu
805 810 815

Thr Leu Phe Ser Val Met Pro Gly Leu Lys Met Thr Met Asp Lys Thr
820 825 830

Gly Leu Leu Leu Leu Ser Asp Pro Asp Lys Val Thr Ile Gly Leu Leu
835 840 845

Phe Trp Asp Gly Arg Gly Glu Gly Leu Arg Leu Leu Leu Arg Asp Thr
850 855 860

Asp Arg Phe Ser Ser His Val Gly Gly Thr Leu Gly Gln Phe Tyr Gln
865 870 875 880

Glu Val Leu Trp Gly Ser Pro Ala Ala Ser Asp Asp Gly Arg Arg Thr
885 890 895

Leu Arg Val Gln Gly Asn Asp His Ser Ala Thr Arg Glu Arg Arg Leu
900 905 910

Asp Tyr Gln Glu Gly Pro Pro Gly Val Glu Ile Ser Cys Trp Ser Val
915 920 925

Glu Leu
930

<210> 101
<211> 946
<212> PRT
<213> Artificial Sequence

<220>
<223> ITIH2_HUMAN

<400> 101

```
Met Lys Arg Leu Thr Cys Phe Phe Ile Cys Phe Phe Leu Ser Glu Val
1               5                   10                  15

Ser Gly Phe Glu Ile Pro Ile Asn Gly Leu Ser Glu Phe Val Asp Tyr
            20                  25                  30

Glu Asp Leu Val Glu Leu Ala Pro Gly Lys Phe Gln Leu Val Ala Glu
        35                  40                  45

Asn Arg Arg Tyr Gln Arg Ser Leu Pro Gly Glu Ser Glu Glu Met Met
    50                  55                  60

Glu Glu Val Asp Gln Val Thr Leu Tyr Ser Tyr Lys Val Gln Ser Thr
65                  70                  75                  80

Ile Thr Ser Arg Met Ala Thr Thr Met Ile Gln Ser Lys Val Val Asn
                85                  90                  95

Asn Ser Pro Gln Pro Gln Asn Val Val Phe Asp Val Gln Ile Pro Lys
            100                 105                 110

Gly Ala Phe Ile Ser Asn Phe Ser Met Thr Val Asp Gly Lys Thr Phe
        115                 120                 125

Arg Ser Ser Ile Lys Glu Lys Thr Val Gly Arg Ala Leu Tyr Ala Gln
    130                 135                 140

Ala Arg Ala Lys Gly Lys Thr Ala Gly Leu Val Arg Ser Ser Ala Leu
145                 150                 155                 160

Asp Met Glu Asn Phe Arg Thr Glu Val Asn Val Leu Pro Gly Ala Lys
                165                 170                 175

Val Gln Phe Glu Leu His Tyr Gln Glu Val Lys Trp Arg Lys Leu Gly
            180                 185                 190

Ser Tyr Glu His Arg Ile Tyr Leu Gln Pro Gly Arg Leu Ala Lys His
        195                 200                 205

Leu Glu Val Asp Val Trp Val Ile Glu Pro Gln Gly Leu Arg Phe Leu
    210                 215                 220

His Val Pro Asp Thr Phe Glu Gly His Phe Asp Gly Val Pro Val Ile
225                 230                 235                 240

Ser Lys Gly Gln Gln Lys Ala His Val Ser Phe Lys Pro Thr Val Ala
                245                 250                 255

Gln Gln Arg Ile Cys Pro Asn Cys Arg Glu Thr Ala Val Asp Gly Glu
            260                 265                 270

Leu Val Val Leu Tyr Asp Val Lys Arg Glu Glu Lys Ala Gly Glu Leu
        275                 280                 285

Glu Val Phe Asn Gly Tyr Phe Val His Phe Phe Ala Pro Asp Asn Leu
    290                 295                 300

Asp Pro Ile Pro Lys Asn Ile Leu Phe Val Ile Asp Val Ser Gly Ser
305                 310                 315                 320

Met Trp Gly Val Lys Met Lys Gln Thr Val Glu Ala Met Lys Thr Ile
```

325 330 335

Leu Asp Asp Leu Arg Ala Glu Asp His Phe Ser Val Ile Asp Phe Asn
340 345 350

Gln Asn Ile Arg Thr Trp Arg Asn Asp Leu Ile Ser Ala Thr Lys Thr
355 360 365

Gln Val Ala Asp Ala Lys Arg Tyr Ile Glu Lys Ile Gln Pro Ser Gly
370 375 380

Gly Thr Asn Ile Asn Glu Ala Leu Leu Arg Ala Ile Phe Ile Leu Asn
385 390 395 400

Glu Ala Asn Asn Leu Gly Leu Leu Asp Pro Asn Ser Val Ser Leu Ile
405 410 415

Ile Leu Val Ser Asp Gly Asp Pro Thr Val Gly Glu Leu Lys Leu Ser
420 425 430

Lys Ile Gln Lys Asn Val Lys Glu Asn Ile Gln Asp Asn Ile Ser Leu
435 440 445

Phe Ser Leu Gly Met Gly Phe Asp Val Asp Tyr Asp Phe Leu Lys Arg
450 455 460

Leu Ser Asn Glu Asn His Gly Ile Ala Gln Arg Ile Tyr Gly Asn Gln
465 470 475 480

Asp Thr Ser Ser Gln Leu Lys Lys Phe Tyr Asn Gln Val Ser Thr Pro
485 490 495

Leu Leu Arg Asn Val Gln Phe Asn Tyr Pro His Thr Ser Val Thr Asp
500 505 510

Val Thr Gln Asn Asn Phe His Asn Tyr Phe Gly Gly Ser Glu Ile Val
515 520 525

Val Ala Gly Lys Phe Asp Pro Ala Lys Leu Asp Gln Ile Glu Ser Val
530 535 540

Ile Thr Ala Thr Ser Ala Asn Thr Gln Leu Val Leu Glu Thr Leu Ala
545 550 555 560

Gln Met Asp Asp Leu Gln Asp Phe Leu Ser Lys Asp Lys His Ala Asp
565 570 575

Pro Asp Phe Thr Arg Lys Leu Trp Ala Tyr Leu Thr Ile Asn Gln Leu
580 585 590

Leu Ala Glu Arg Ser Leu Ala Pro Thr Ala Ala Ala Lys Arg Arg Ile
595 600 605

Thr Arg Ser Ile Leu Gln Met Ser Leu Asp His His Ile Val Thr Pro
610 615 620

Leu Thr Ser Leu Val Ile Glu Asn Glu Ala Gly Asp Glu Arg Met Leu
625 630 635 640

Ala Asp Ala Pro Pro Gln Asp Pro Ser Cys Cys Ser Gly Ala Leu Tyr
645 650 655

Tyr Gly Ser Lys Val Val Pro Asp Ser Thr Pro Ser Trp Ala Asn Pro

660 665 670

Ser Pro Thr Pro Val Ile Ser Met Leu Ala Gln Gly Ser Gln Val Leu
675 680 685

Glu Ser Thr Pro Pro Pro His Val Met Arg Val Glu Asn Asp Pro His
690 695 700

Phe Ile Ile Tyr Leu Pro Lys Ser Gln Lys Asn Ile Cys Phe Asn Ile
705 710 715 720

Asp Ser Glu Pro Gly Lys Ile Leu Asn Leu Val Ser Asp Pro Glu Ser
725 730 735

Gly Ile Val Val Asn Gly Gln Leu Val Gly Ala Lys Lys Pro Asn Asn
740 745 750

Gly Lys Leu Ser Thr Tyr Phe Gly Lys Leu Gly Phe Tyr Phe Gln Ser
755 760 765

Glu Asp Ile Lys Ile Glu Ile Ser Thr Glu Thr Ile Thr Leu Ser His
770 775 780

Gly Ser Ser Thr Phe Ser Leu Ser Trp Ser Asp Thr Ala Gln Val Thr
785 790 795 800

Asn Gln Arg Val Gln Ile Ser Val Lys Lys Glu Lys Val Val Thr Ile
805 810 815

Thr Leu Asp Lys Glu Met Ser Phe Ser Val Leu Leu His Arg Val Trp
820 825 830

Lys Lys His Pro Val Asn Val Asp Phe Leu Gly Ile Tyr Ile Pro Pro
835 840 845

Thr Asn Lys Phe Ser Pro Lys Ala His Gly Leu Ile Gly Gln Phe Met
850 855 860

Gln Glu Pro Lys Ile His Ile Phe Asn Glu Arg Pro Gly Lys Asp Pro
865 870 875 880

Glu Lys Pro Glu Ala Ser Met Glu Val Lys Gly Gln Lys Leu Ile Ile
885 890 895

Thr Arg Gly Leu Gln Lys Asp Tyr Arg Thr Asp Leu Val Phe Gly Thr
900 905 910

Asp Val Thr Cys Trp Phe Val His Asn Ser Gly Lys Gly Phe Ile Asp
915 920 925

Gly His Tyr Lys Asp Tyr Phe Val Pro Gln Leu Tyr Ser Phe Leu Lys
930 935 940

Arg Pro
945

<210> 102
<211> 953
<212> PRT
<213> Artificial Sequence

<220>

<223> TRAK1_HUMAN

<400> 102

```
Met Ala Leu Val Phe Gln Phe Gly Gln Pro Val Arg Ala Gln Pro Leu
1               5                   10                  15

Pro Gly Leu Cys His Gly Lys Leu Ile Arg Thr Asn Ala Cys Asp Val
            20                  25                  30

Cys Asn Ser Thr Asp Leu Pro Glu Val Glu Ile Ile Ser Leu Leu Glu
        35                  40                  45

Glu Gln Leu Pro His Tyr Lys Leu Arg Ala Asp Thr Ile Tyr Gly Tyr
    50                  55                  60

Asp His Asp Asp Trp Leu His Thr Pro Leu Ile Ser Pro Asp Ala Asn
65                  70                  75                  80

Ile Asp Leu Thr Thr Glu Gln Ile Glu Glu Thr Leu Lys Tyr Phe Leu
                85                  90                  95

Leu Cys Ala Glu Arg Val Gly Gln Met Thr Lys Thr Tyr Asn Asp Ile
            100                 105                 110

Asp Ala Val Thr Arg Leu Leu Glu Glu Lys Glu Arg Asp Leu Glu Leu
        115                 120                 125

Ala Ala Arg Ile Gly Gln Ser Leu Leu Lys Lys Asn Lys Thr Leu Thr
    130                 135                 140

Glu Arg Asn Glu Leu Leu Glu Glu Gln Val Glu His Ile Arg Glu Glu
145                 150                 155                 160

Val Ser Gln Leu Arg His Glu Leu Ser Met Lys Asp Glu Leu Leu Gln
                165                 170                 175

Phe Tyr Thr Ser Ala Ala Glu Glu Ser Glu Pro Glu Ser Val Cys Ser
            180                 185                 190

Thr Pro Leu Lys Arg Asn Glu Ser Ser Ser Ser Val Gln Asn Tyr Phe
        195                 200                 205

His Leu Asp Ser Leu Gln Lys Lys Leu Lys Asp Leu Glu Glu Glu Asn
    210                 215                 220

Val Val Leu Arg Ser Glu Ala Ser Gln Leu Lys Thr Glu Thr Ile Thr
225                 230                 235                 240

Tyr Glu Glu Lys Glu Gln Gln Leu Val Asn Asp Cys Val Lys Glu Leu
                245                 250                 255

Arg Asp Ala Asn Val Gln Ile Ala Ser Ile Ser Glu Glu Leu Ala Lys
            260                 265                 270

Lys Thr Glu Asp Ala Ala Arg Gln Gln Glu Glu Ile Thr His Leu Leu
        275                 280                 285

Ser Gln Ile Val Asp Leu Gln Lys Lys Ala Lys Ala Cys Ala Val Glu
    290                 295                 300

Asn Glu Glu Leu Val Gln His Leu Gly Ala Ala Lys Asp Ala Gln Arg
305                 310                 315                 320
```

```
Gln Leu Thr Ala Glu Leu Arg Glu Leu Glu Asp Lys Tyr Ala Glu Cys
                325                 330                 335

Met Glu Met Leu His Glu Ala Gln Glu Glu Leu Lys Asn Leu Arg Asn
            340                 345                 350

Lys Thr Met Pro Asn Thr Thr Ser Arg Arg Tyr His Ser Leu Gly Leu
        355                 360                 365

Phe Pro Met Asp Ser Leu Ala Ala Glu Ile Glu Gly Thr Met Arg Lys
    370                 375                 380

Glu Leu Gln Leu Glu Glu Ala Glu Ser Pro Asp Ile Thr His Gln Lys
385                 390                 395                 400

Arg Val Phe Glu Thr Val Arg Asn Ile Asn Gln Val Val Lys Gln Arg
                405                 410                 415

Ser Leu Thr Pro Ser Pro Met Asn Ile Pro Gly Ser Asn Gln Ser Ser
            420                 425                 430

Ala Met Asn Ser Leu Leu Ser Ser Cys Val Ser Thr Pro Arg Ser Ser
        435                 440                 445

Phe Tyr Gly Ser Asp Ile Gly Asn Val Val Leu Asp Asn Lys Thr Asn
    450                 455                 460

Ser Ile Ile Leu Glu Thr Glu Ala Ala Asp Leu Gly Asn Asp Glu Arg
465                 470                 475                 480

Ser Lys Lys Pro Gly Thr Pro Gly Thr Pro Gly Ser His Asp Leu Glu
                485                 490                 495

Thr Ala Leu Arg Arg Leu Ser Leu Arg Arg Glu Asn Tyr Leu Ser Glu
            500                 505                 510

Arg Arg Phe Phe Glu Glu Glu Gln Glu Arg Lys Leu Gln Glu Leu Ala
        515                 520                 525

Glu Lys Gly Glu Leu Arg Ser Gly Ser Leu Thr Pro Thr Glu Ser Ile
    530                 535                 540

Met Ser Leu Gly Thr His Ser Arg Phe Ser Glu Phe Thr Gly Phe Ser
545                 550                 555                 560

Gly Met Ser Phe Ser Ser Arg Ser Tyr Leu Pro Glu Lys Leu Gln Ile
                565                 570                 575

Val Lys Pro Leu Glu Gly Ser Ala Thr Leu His His Trp Gln Gln Leu
            580                 585                 590

Ala Gln Pro His Leu Gly Gly Ile Leu Asp Pro Arg Pro Gly Val Val
        595                 600                 605

Thr Lys Gly Phe Arg Thr Leu Asp Val Asp Leu Asp Glu Val Tyr Cys
    610                 615                 620

Leu Asn Asp Phe Glu Glu Asp Asp Thr Gly Asp His Ile Ser Leu Pro
625                 630                 635                 640

Arg Leu Ala Thr Ser Thr Pro Val Gln His Pro Glu Thr Ser Ala His
                645                 650                 655
```

His Pro Gly Lys Cys Met Ser Gln Thr Asn Ser Thr Phe Thr Phe Thr
660 665 670

Thr Cys Arg Ile Leu His Pro Ser Asp Glu Leu Thr Arg Val Thr Pro
675 680 685

Ser Leu Asn Ser Ala Pro Thr Pro Ala Cys Gly Ser Thr Ser His Leu
690 695 700

Lys Ser Thr Pro Val Ala Thr Pro Cys Thr Pro Arg Arg Leu Ser Leu
705 710 715 720

Ala Glu Ser Phe Thr Asn Thr Arg Glu Ser Thr Thr Thr Met Ser Thr
725 730 735

Ser Leu Gly Leu Val Trp Leu Leu Lys Glu Arg Gly Ile Ser Ala Ala
740 745 750

Val Tyr Asp Pro Gln Ser Trp Asp Arg Ala Gly Arg Gly Ser Leu Leu
755 760 765

His Ser Tyr Thr Pro Lys Met Ala Val Ile Pro Ser Thr Pro Pro Asn
770 775 780

Ser Pro Met Gln Thr Pro Thr Ser Ser Pro Pro Ser Phe Glu Phe Lys
785 790 795 800

Cys Thr Ser Pro Pro Tyr Asp Asn Phe Leu Ala Ser Lys Pro Ala Ser
805 810 815

Ser Ile Leu Arg Glu Val Arg Glu Lys Asn Val Arg Ser Ser Glu Ser
820 825 830

Gln Thr Asp Val Ser Val Ser Asn Leu Asn Leu Val Asp Lys Val Arg
835 840 845

Arg Phe Gly Val Ala Lys Val Val Asn Ser Gly Arg Ala His Val Pro
850 855 860

Thr Leu Thr Glu Glu Gln Gly Pro Leu Leu Cys Gly Pro Pro Gly Pro
865 870 875 880

Ala Pro Ala Leu Val Pro Arg Gly Leu Val Pro Glu Gly Leu Pro Leu
885 890 895

Arg Cys Pro Thr Val Thr Ser Ala Ile Gly Gly Leu Gln Leu Asn Ser
900 905 910

Gly Ile Arg Arg Asn Arg Ser Phe Pro Thr Met Val Gly Ser Ser Met
915 920 925

Gln Met Lys Ala Pro Val Thr Leu Thr Ser Gly Ile Leu Met Gly Ala
930 935 940

Lys Leu Ser Lys Gln Thr Ser Leu Arg
945 950

<210> 103
<211> 1255
<212> PRT
<213> Artificial Sequence

<220>
<223> MUC1_HUMAN

<400> 103
Met Thr Pro Gly Thr Gln Ser Pro Phe Phe Leu Leu Leu Leu Leu Thr
1 5 10 15

Val Leu Thr Val Val Thr Gly Ser Gly His Ala Ser Ser Thr Pro Gly
20 25 30

Gly Glu Lys Glu Thr Ser Ala Thr Gln Arg Ser Ser Val Pro Ser Ser
35 40 45

Thr Glu Lys Asn Ala Val Ser Met Thr Ser Ser Val Leu Ser Ser His
50 55 60

Ser Pro Gly Ser Gly Ser Ser Thr Thr Gln Gly Gln Asp Val Thr Leu
65 70 75 80

Ala Pro Ala Thr Glu Pro Ala Ser Gly Ser Ala Ala Thr Trp Gly Gln
85 90 95

Asp Val Thr Ser Val Pro Val Thr Arg Pro Ala Leu Gly Ser Thr Thr
100 105 110

Pro Pro Ala His Asp Val Thr Ser Ala Pro Asp Asn Lys Pro Ala Pro
115 120 125

Gly Ser Thr Ala Pro Pro Ala His Gly Val Thr Ser Ala Pro Asp Thr
130 135 140

Arg Pro Ala Pro Gly Ser Thr Ala Pro Pro Ala His Gly Val Thr Ser
145 150 155 160

Ala Pro Asp Thr Arg Pro Ala Pro Gly Ser Thr Ala Pro Pro Ala His
165 170 175

Gly Val Thr Ser Ala Pro Asp Thr Arg Pro Ala Pro Gly Ser Thr Ala
180 185 190

Pro Pro Ala His Gly Val Thr Ser Ala Pro Asp Thr Arg Pro Ala Pro
195 200 205

Gly Ser Thr Ala Pro Pro Ala His Gly Val Thr Ser Ala Pro Asp Thr
210 215 220

Arg Pro Ala Pro Gly Ser Thr Ala Pro Pro Ala His Gly Val Thr Ser
225 230 235 240

Ala Pro Asp Thr Arg Pro Ala Pro Gly Ser Thr Ala Pro Pro Ala His
245 250 255

Gly Val Thr Ser Ala Pro Asp Thr Arg Pro Ala Pro Gly Ser Thr Ala
260 265 270

Pro Pro Ala His Gly Val Thr Ser Ala Pro Asp Thr Arg Pro Ala Pro
275 280 285

Gly Ser Thr Ala Pro Pro Ala His Gly Val Thr Ser Ala Pro Asp Thr
290 295 300

Arg Pro Ala Pro Gly Ser Thr Ala Pro Pro Ala His Gly Val Thr Ser
305 310 315 320

Ala Pro Asp Thr Arg Pro Ala Pro Gly Ser Thr Ala Pro Pro Ala His
325 330 335

Gly Val Thr Ser Ala Pro Asp Thr Arg Pro Ala Pro Gly Ser Thr Ala
340 345 350

Pro Pro Ala His Gly Val Thr Ser Ala Pro Asp Thr Arg Pro Ala Pro
355 360 365

Gly Ser Thr Ala Pro Pro Ala His Gly Val Thr Ser Ala Pro Asp Thr
370 375 380

Arg Pro Ala Pro Gly Ser Thr Ala Pro Pro Ala His Gly Val Thr Ser
385 390 395 400

Ala Pro Asp Thr Arg Pro Ala Pro Gly Ser Thr Ala Pro Pro Ala His
405 410 415

Gly Val Thr Ser Ala Pro Asp Thr Arg Pro Ala Pro Gly Ser Thr Ala
420 425 430

Pro Pro Ala His Gly Val Thr Ser Ala Pro Asp Thr Arg Pro Ala Pro
435 440 445

Gly Ser Thr Ala Pro Pro Ala His Gly Val Thr Ser Ala Pro Asp Thr
450 455 460

Arg Pro Ala Pro Gly Ser Thr Ala Pro Pro Ala His Gly Val Thr Ser
465 470 475 480

Ala Pro Asp Thr Arg Pro Ala Pro Gly Ser Thr Ala Pro Pro Ala His
485 490 495

Gly Val Thr Ser Ala Pro Asp Thr Arg Pro Ala Pro Gly Ser Thr Ala
500 505 510

Pro Pro Ala His Gly Val Thr Ser Ala Pro Asp Thr Arg Pro Ala Pro
515 520 525

Gly Ser Thr Ala Pro Pro Ala His Gly Val Thr Ser Ala Pro Asp Thr
530 535 540

Arg Pro Ala Pro Gly Ser Thr Ala Pro Pro Ala His Gly Val Thr Ser
545 550 555 560

Ala Pro Asp Thr Arg Pro Ala Pro Gly Ser Thr Ala Pro Pro Ala His
565 570 575

Gly Val Thr Ser Ala Pro Asp Thr Arg Pro Ala Pro Gly Ser Thr Ala
580 585 590

Pro Pro Ala His Gly Val Thr Ser Ala Pro Asp Thr Arg Pro Ala Pro
595 600 605

Gly Ser Thr Ala Pro Pro Ala His Gly Val Thr Ser Ala Pro Asp Thr
610 615 620

Arg Pro Ala Pro Gly Ser Thr Ala Pro Pro Ala His Gly Val Thr Ser
625 630 635 640

```
Ala Pro Asp Thr Arg Pro Ala Pro Gly Ser Thr Ala Pro Pro Ala His
                645                 650                 655

Gly Val Thr Ser Ala Pro Asp Thr Arg Pro Ala Pro Gly Ser Thr Ala
            660                 665                 670

Pro Pro Ala His Gly Val Thr Ser Ala Pro Asp Thr Arg Pro Ala Pro
        675                 680                 685

Gly Ser Thr Ala Pro Pro Ala His Gly Val Thr Ser Ala Pro Asp Thr
    690                 695                 700

Arg Pro Ala Pro Gly Ser Thr Ala Pro Pro Ala His Gly Val Thr Ser
705                 710                 715                 720

Ala Pro Asp Thr Arg Pro Ala Pro Gly Ser Thr Ala Pro Pro Ala His
                725                 730                 735

Gly Val Thr Ser Ala Pro Asp Thr Arg Pro Ala Pro Gly Ser Thr Ala
            740                 745                 750

Pro Pro Ala His Gly Val Thr Ser Ala Pro Asp Thr Arg Pro Ala Pro
        755                 760                 765

Gly Ser Thr Ala Pro Pro Ala His Gly Val Thr Ser Ala Pro Asp Thr
    770                 775                 780

Arg Pro Ala Pro Gly Ser Thr Ala Pro Pro Ala His Gly Val Thr Ser
785                 790                 795                 800

Ala Pro Asp Thr Arg Pro Ala Pro Gly Ser Thr Ala Pro Pro Ala His
                805                 810                 815

Gly Val Thr Ser Ala Pro Asp Thr Arg Pro Ala Pro Gly Ser Thr Ala
            820                 825                 830

Pro Pro Ala His Gly Val Thr Ser Ala Pro Asp Thr Arg Pro Ala Pro
        835                 840                 845

Gly Ser Thr Ala Pro Pro Ala His Gly Val Thr Ser Ala Pro Asp Thr
    850                 855                 860

Arg Pro Ala Pro Gly Ser Thr Ala Pro Pro Ala His Gly Val Thr Ser
865                 870                 875                 880

Ala Pro Asp Thr Arg Pro Ala Pro Gly Ser Thr Ala Pro Pro Ala His
                885                 890                 895

Gly Val Thr Ser Ala Pro Asp Thr Arg Pro Ala Pro Gly Ser Thr Ala
            900                 905                 910

Pro Pro Ala His Gly Val Thr Ser Ala Pro Asp Thr Arg Pro Ala Pro
        915                 920                 925

Gly Ser Thr Ala Pro Pro Ala His Gly Val Thr Ser Ala Pro Asp Asn
    930                 935                 940

Arg Pro Ala Leu Gly Ser Thr Ala Pro Pro Val His Asn Val Thr Ser
945                 950                 955                 960

Ala Ser Gly Ser Ala Ser Gly Ser Ala Ser Thr Leu Val His Asn Gly
                965                 970                 975
```

Thr Ser Ala Arg Ala Thr Thr Thr Pro Ala Ser Lys Ser Thr Pro Phe
980 985 990

Ser Ile Pro Ser His His Ser Asp Thr Pro Thr Thr Leu Ala Ser His
995 1000 1005

Ser Thr Lys Thr Asp Ala Ser Ser Thr His His Ser Ser Val Pro Pro
1010 1015 1020

Leu Thr Ser Ser Asn His Ser Thr Ser Pro Gln Leu Ser Thr Gly Val
1025 1030 1035 1040

Ser Phe Phe Phe Leu Ser Phe His Ile Ser Asn Leu Gln Phe Asn Ser
1045 1050 1055

Ser Leu Glu Asp Pro Ser Thr Asp Tyr Tyr Gln Glu Leu Gln Arg Asp
1060 1065 1070

Ile Ser Glu Met Phe Leu Gln Ile Tyr Lys Gln Gly Gly Phe Leu Gly
1075 1080 1085

Leu Ser Asn Ile Lys Phe Arg Pro Gly Ser Val Val Val Gln Leu Thr
1090 1095 1100

Leu Ala Phe Arg Glu Gly Thr Ile Asn Val His Asp Val Glu Thr Gln
1105 1110 1115 1120

Phe Asn Gln Tyr Lys Thr Glu Ala Ala Ser Arg Tyr Asn Leu Thr Ile
1125 1130 1135

Ser Asp Val Ser Val Ser Asp Val Pro Phe Pro Phe Ser Ala Gln Ser
1140 1145 1150

Gly Ala Gly Val Pro Gly Trp Gly Ile Ala Leu Leu Val Leu Val Cys
1155 1160 1165

Val Leu Val Ala Leu Ala Ile Val Tyr Leu Ile Ala Leu Ala Val Cys
1170 1175 1180

Gln Cys Arg Arg Lys Asn Tyr Gly Gln Leu Asp Ile Phe Pro Ala Arg
1185 1190 1195 1200

Asp Thr Tyr His Pro Met Ser Glu Tyr Pro Thr Tyr His Thr His Gly
1205 1210 1215

Arg Tyr Val Pro Pro Ser Ser Thr Asp Arg Ser Pro Tyr Glu Lys Val
1220 1225 1230

Ser Ala Gly Asn Gly Gly Ser Ser Leu Ser Tyr Thr Asn Pro Ala Val
1235 1240 1245

Ala Ala Thr Ser Ala Asn Leu
1250 1255

<210> 104
<211> 1322
<212> PRT
<213> Artificial Sequence

<220>
<223> EMSY_HUMAN

<400> 104

Met Pro Val Val Trp Pro Thr Leu Leu Asp Leu Ser Arg Asp Glu Cys
1 5 10 15

Lys Arg Ile Leu Arg Lys Leu Glu Leu Glu Ala Tyr Ala Gly Val Ile
20 25 30

Ser Ala Leu Arg Ala Gln Gly Asp Leu Thr Lys Glu Lys Lys Asp Leu
35 40 45

Leu Gly Glu Leu Ser Lys Val Leu Ser Ile Ser Thr Glu Arg His Arg
50 55 60

Ala Glu Val Arg Arg Ala Val Asn Asp Glu Arg Leu Thr Thr Ile Ala
65 70 75 80

His Asn Met Ser Gly Pro Asn Ser Ser Ser Glu Trp Ser Ile Glu Gly
85 90 95

Arg Arg Leu Val Pro Leu Met Pro Arg Leu Val Pro Gln Thr Ala Phe
100 105 110

Thr Val Thr Ala Asn Ala Val Ala Asn Ala Ala Ile Gln His Asn Ala
115 120 125

Ser Leu Pro Val Pro Ala Glu Thr Gly Ser Lys Glu Val Val Cys Tyr
130 135 140

Ser Tyr Thr Ser Thr Thr Ser Thr Pro Thr Ser Thr Pro Val Pro Ser
145 150 155 160

Gly Ser Ile Ala Thr Val Lys Ser Pro Arg Pro Ala Ser Pro Ala Ser
165 170 175

Asn Val Val Val Leu Pro Ser Gly Ser Thr Val Tyr Val Lys Ser Val
180 185 190

Ser Cys Ser Asp Glu Asp Glu Lys Pro Arg Lys Arg Arg Arg Thr Asn
195 200 205

Ser Ser Ser Ser Ser Pro Val Val Leu Lys Glu Val Pro Lys Ala Val
210 215 220

Val Pro Val Ser Lys Thr Ile Thr Val Pro Val Ser Gly Ser Pro Lys
225 230 235 240

Met Ser Asn Ile Met Gln Ser Ile Ala Asn Ser Leu Pro Pro His Met
245 250 255

Ser Pro Val Lys Ile Thr Phe Thr Lys Pro Ser Thr Gln Thr Thr Asn
260 265 270

Thr Thr Thr Gln Lys Val Ile Ile Val Thr Thr Ser Pro Ser Ser Thr
275 280 285

Phe Val Pro Asn Ile Leu Ser Lys Ser His Asn Tyr Ala Ala Val Thr
290 295 300

Lys Leu Val Pro Thr Ser Val Ile Ala Ser Thr Thr Gln Lys Pro Pro
305 310 315 320

Val Val Ile Thr Ala Ser Gln Ser Ser Leu Val Ser Asn Ser Ser Ser

325 330 335

Gly Ser Ser Ser Ser Thr Pro Ser Pro Ile Pro Asn Thr Val Ala Val
340 345 350

Thr Ala Val Val Ser Ser Thr Pro Ser Val Val Met Ser Thr Val Ala
355 360 365

Gln Gly Val Ser Thr Ser Ala Ile Lys Met Ala Ser Thr Arg Leu Pro
370 375 380

Ser Pro Lys Ser Leu Val Ser Ala Pro Thr Gln Ile Leu Ala Gln Phe
385 390 395 400

Pro Lys Gln His Gln Gln Ser Pro Lys Gln Gln Leu Tyr Gln Val Gln
405 410 415

Gln Gln Thr Gln Gln Gln Val Ala Gln Pro Ser Pro Val Ser His Gln
420 425 430

Gln Gln Pro Gln Gln Ser Pro Leu Pro Pro Gly Ile Lys Pro Thr Ile
435 440 445

Gln Ile Lys Gln Glu Ser Gly Val Lys Ile Ile Thr Gln Gln Val Gln
450 455 460

Pro Ser Lys Ile Leu Pro Lys Pro Val Thr Ala Thr Leu Pro Thr Ser
465 470 475 480

Ser Asn Ser Pro Ile Met Val Val Ser Ser Asn Gly Ala Ile Met Thr
485 490 495

Thr Lys Leu Val Thr Thr Pro Thr Gly Thr Gln Ala Thr Tyr Thr Arg
500 505 510

Pro Thr Val Ser Pro Ser Ile Gly Arg Met Ala Ala Thr Pro Gly Ala
515 520 525

Ala Thr Tyr Val Lys Thr Thr Ser Gly Ser Ile Ile Thr Val Val Pro
530 535 540

Lys Ser Leu Ala Thr Leu Gly Gly Lys Ile Ile Ser Ser Asn Ile Val
545 550 555 560

Ser Gly Thr Thr Thr Lys Ile Thr Thr Ile Pro Met Thr Ser Lys Pro
565 570 575

Asn Val Ile Val Val Gln Lys Thr Thr Gly Lys Gly Thr Thr Ile Gln
580 585 590

Gly Leu Pro Gly Lys Asn Val Val Thr Thr Leu Leu Asn Ala Gly Gly
595 600 605

Glu Lys Thr Ile Gln Thr Val Pro Thr Gly Ala Lys Pro Ala Ile Leu
610 615 620

Thr Ala Thr Arg Pro Ile Thr Lys Met Ile Val Thr Gln Pro Lys Gly
625 630 635 640

Ile Gly Ser Thr Val Gln Pro Ala Ala Lys Ile Ile Pro Thr Lys Ile
645 650 655

Val Tyr Gly Gln Gln Gly Lys Thr Gln Val Leu Ile Lys Pro Lys Pro

660 665 670

Val Thr Phe Gln Ala Thr Val Val Ser Glu Gln Thr Arg Gln Leu Val
675 680 685

Thr Glu Thr Leu Gln Gln Ala Ser Arg Val Ala Glu Ala Gly Asn Ser
690 695 700

Ser Ile Gln Glu Gly Lys Glu Glu Pro Gln Asn Tyr Thr Asp Ser Ser
705 710 715 720

Ser Ser Ser Thr Glu Ser Ser Gln Ser Ser Gln Asp Ser Gln Pro Val
725 730 735

Val His Val Ile Ala Ser Arg Arg Gln Asp Trp Ser Glu His Glu Ile
740 745 750

Ala Met Glu Thr Ser Pro Thr Ile Ile Tyr Gln Asp Val Ser Ser Glu
755 760 765

Ser Gln Ser Ala Thr Ser Thr Ile Lys Ala Leu Leu Glu Leu Gln Gln
770 775 780

Thr Thr Val Lys Glu Lys Leu Glu Ser Lys Pro Arg Gln Pro Thr Ile
785 790 795 800

Asp Leu Ser Gln Met Ala Val Pro Ile Gln Met Thr Gln Glu Lys Arg
805 810 815

His Ser Pro Glu Ser Pro Ser Ile Ala Val Val Glu Ser Glu Leu Val
820 825 830

Ala Glu Tyr Ile Thr Thr Glu Arg Thr Asp Glu Gly Thr Glu Val Ala
835 840 845

Phe Pro Leu Leu Val Ser His Arg Ser Gln Pro Gln Gln Pro Ser Gln
850 855 860

Pro Gln Arg Thr Leu Leu Gln His Val Ala Gln Ser Gln Thr Ala Thr
865 870 875 880

Gln Thr Ser Val Val Val Lys Ser Ile Pro Ala Ser Ser Pro Gly Ala
885 890 895

Ile Thr His Ile Met Gln Gln Ala Leu Ser Ser His Thr Ala Phe Thr
900 905 910

Lys His Ser Glu Glu Leu Gly Thr Glu Glu Gly Glu Val Glu Glu Met
915 920 925

Asp Thr Leu Asp Pro Gln Thr Gly Leu Phe Tyr Arg Ser Ala Leu Thr
930 935 940

Gln Ser Gln Ser Ala Lys Gln Gln Lys Leu Ser Gln Pro Pro Leu Glu
945 950 955 960

Gln Thr Gln Leu Gln Val Lys Thr Leu Gln Cys Phe Gln Thr Lys Gln
965 970 975

Lys Gln Thr Ile His Leu Gln Ala Asp Gln Leu Gln His Lys Leu Pro
980 985 990

Gln Met Pro Gln Leu Ser Ile Arg His Gln Lys Leu Thr Pro Leu Gln

995 1000 1005

Gln Glu Gln Ala Gln Pro Lys Pro Asp Val Gln His Thr Gln His Pro
1010 1015 1020

Met Val Ala Lys Asp Arg Gln Leu Pro Thr Leu Met Ala Gln Pro Pro
1025 1030 1035 1040

Gln Thr Val Val Gln Val Leu Ala Val Lys Thr Thr Gln Gln Leu Pro
1045 1050 1055

Lys Leu Gln Gln Ala Pro Asn Gln Pro Lys Ile Tyr Val Gln Pro Gln
1060 1065 1070

Thr Pro Gln Ser Gln Met Ser Leu Pro Ala Ser Ser Glu Lys Gln Thr
1075 1080 1085

Ala Ser Gln Val Glu Gln Pro Ile Ile Thr Gln Gly Ser Ser Val Thr
1090 1095 1100

Lys Ile Thr Phe Glu Gly Arg Gln Pro Pro Thr Val Thr Lys Ile Thr
1105 1110 1115 1120

Gly Gly Ser Ser Val Pro Lys Leu Thr Ser Pro Val Thr Ser Ile Ser
1125 1130 1135

Pro Ile Gln Ala Ser Glu Lys Thr Ala Val Ser Asp Ile Leu Lys Met
1140 1145 1150

Ser Leu Met Glu Ala Gln Ile Asp Thr Asn Val Glu His Met Ile Val
1155 1160 1165

Asp Pro Pro Lys Lys Ala Leu Ala Thr Ser Met Leu Thr Gly Glu Ala
1170 1175 1180

Gly Ser Leu Pro Ser Thr His Met Val Val Ala Gly Met Ala Asn Ser
1185 1190 1195 1200

Thr Pro Gln Gln Gln Lys Cys Arg Glu Ser Cys Ser Ser Pro Ser Thr
1205 1210 1215

Val Gly Ser Ser Leu Thr Thr Arg Lys Ile Asp Pro Pro Ala Val Pro
1220 1225 1230

Ala Thr Gly Gln Phe Met Arg Ile Gln Asn Val Gly Gln Lys Lys Ala
1235 1240 1245

Glu Glu Ser Pro Ala Glu Ile Ile Ile Gln Ala Ile Pro Gln Tyr Ala
1250 1255 1260

Ile Pro Cys His Ser Ser Ser Asn Val Val Val Glu Pro Ser Gly Leu
1265 1270 1275 1280

Leu Glu Leu Asn Asn Phe Thr Ser Gln Gln Leu Asp Asp Glu Glu Thr
1285 1290 1295

Ala Met Glu Gln Asp Ile Asp Ser Ser Thr Glu Asp Gly Thr Glu Pro
1300 1305 1310

Ser Pro Ser Gln Ser Ser Ala Glu Arg Ser
1315 1320

<210> 105
<211> 1404
<212> PRT
<213> Artificial Sequence

<220>
<223> PRG4_HUMAN

<400> 105

```
Met Ala Trp Lys Thr Leu Pro Ile Tyr Leu Leu Leu Leu Leu Ser Val
1               5                   10                  15

Phe Val Ile Gln Gln Val Ser Ser Gln Asp Leu Ser Ser Cys Ala Gly
            20                  25                  30

Arg Cys Gly Glu Gly Tyr Ser Arg Asp Ala Thr Cys Asn Cys Asp Tyr
        35                  40                  45

Asn Cys Gln His Tyr Met Glu Cys Cys Pro Asp Phe Lys Arg Val Cys
    50                  55                  60

Thr Ala Glu Leu Ser Cys Lys Gly Arg Cys Phe Glu Ser Phe Glu Arg
65                  70                  75                  80

Gly Arg Glu Cys Asp Cys Asp Ala Gln Cys Lys Lys Tyr Asp Lys Cys
                85                  90                  95

Cys Pro Asp Tyr Glu Ser Phe Cys Ala Glu Val His Asn Pro Thr Ser
            100                 105                 110

Pro Pro Ser Ser Lys Lys Ala Pro Pro Pro Ser Gly Ala Ser Gln Thr
        115                 120                 125

Ile Lys Ser Thr Thr Lys Arg Ser Pro Lys Pro Pro Asn Lys Lys Lys
    130                 135                 140

Thr Lys Lys Val Ile Glu Ser Glu Glu Ile Thr Glu Glu His Ser Val
145                 150                 155                 160

Ser Glu Asn Gln Glu Ser Ser Ser Ser Ser Ser Ser Ser Ser Ser Ser
                165                 170                 175

Ser Thr Ile Arg Lys Ile Lys Ser Ser Lys Asn Ser Ala Ala Asn Arg
            180                 185                 190

Glu Leu Gln Lys Lys Leu Lys Val Lys Asp Asn Lys Lys Asn Arg Thr
        195                 200                 205

Lys Lys Lys Pro Thr Pro Lys Pro Pro Val Val Asp Glu Ala Gly Ser
    210                 215                 220

Gly Leu Asp Asn Gly Asp Phe Lys Val Thr Thr Pro Asp Thr Ser Thr
225                 230                 235                 240

Thr Gln His Asn Lys Val Ser Thr Ser Pro Lys Ile Thr Thr Ala Lys
                245                 250                 255

Pro Ile Asn Pro Arg Pro Ser Leu Pro Pro Asn Ser Asp Thr Ser Lys
            260                 265                 270

Glu Thr Ser Leu Thr Val Asn Lys Glu Thr Thr Val Glu Thr Lys Glu
        275                 280                 285
```

Thr Thr Thr Thr Asn Lys Gln Thr Ser Thr Asp Gly Lys Glu Lys Thr
290 295 300

Thr Ser Ala Lys Glu Thr Gln Ser Ile Glu Lys Thr Ser Ala Lys Asp
305 310 315 320

Leu Ala Pro Thr Ser Lys Val Leu Ala Lys Pro Thr Pro Lys Ala Glu
325 330 335

Thr Thr Thr Lys Gly Pro Ala Leu Thr Thr Pro Lys Glu Pro Thr Pro
340 345 350

Thr Thr Pro Lys Glu Pro Ala Ser Thr Thr Pro Lys Glu Pro Thr Pro
355 360 365

Thr Thr Ile Lys Ser Ala Pro Thr Thr Pro Lys Glu Pro Ala Pro Thr
370 375 380

Thr Thr Lys Ser Ala Pro Thr Thr Pro Lys Glu Pro Ala Pro Thr Thr
385 390 395 400

Thr Lys Glu Pro Ala Pro Thr Thr Pro Lys Glu Pro Ala Pro Thr Thr
405 410 415

Thr Lys Glu Pro Ala Pro Thr Thr Thr Lys Ser Ala Pro Thr Thr Pro
420 425 430

Lys Glu Pro Ala Pro Thr Thr Pro Lys Lys Pro Ala Pro Thr Thr Pro
435 440 445

Lys Glu Pro Ala Pro Thr Thr Pro Lys Glu Pro Thr Pro Thr Thr Pro
450 455 460

Lys Glu Pro Ala Pro Thr Thr Lys Glu Pro Ala Pro Thr Thr Pro Lys
465 470 475 480

Glu Pro Ala Pro Thr Ala Pro Lys Lys Pro Ala Pro Thr Thr Pro Lys
485 490 495

Glu Pro Ala Pro Thr Thr Pro Lys Glu Pro Ala Pro Thr Thr Thr Lys
500 505 510

Glu Pro Ser Pro Thr Thr Pro Lys Glu Pro Ala Pro Thr Thr Thr Lys
515 520 525

Ser Ala Pro Thr Thr Thr Lys Glu Pro Ala Pro Thr Thr Thr Lys Ser
530 535 540

Ala Pro Thr Thr Pro Lys Glu Pro Ser Pro Thr Thr Thr Lys Glu Pro
545 550 555 560

Ala Pro Thr Thr Pro Lys Glu Pro Ala Pro Thr Thr Pro Lys Lys Pro
565 570 575

Ala Pro Thr Thr Pro Lys Glu Pro Ala Pro Thr Thr Pro Lys Glu Pro
580 585 590

Ala Pro Thr Thr Thr Lys Lys Pro Ala Pro Thr Thr Pro Lys Glu Pro
595 600 605

Ala Pro Thr Thr Pro Lys Glu Thr Ala Pro Thr Thr Pro Lys Lys Leu
610 615 620

Thr Pro Thr Thr Pro Glu Lys Leu Ala Pro Thr Thr Pro Glu Lys Pro
625 630 635 640

Ala Pro Thr Thr Pro Glu Glu Leu Ala Pro Thr Thr Pro Glu Glu Pro
645 650 655

Thr Pro Thr Thr Pro Glu Glu Pro Ala Pro Thr Thr Pro Lys Ala Ala
660 665 670

Ala Pro Asn Thr Pro Lys Glu Pro Ala Pro Thr Thr Pro Lys Glu Pro
675 680 685

Ala Pro Thr Thr Pro Lys Glu Pro Ala Pro Thr Thr Pro Lys Glu Thr
690 695 700

Ala Pro Thr Thr Pro Lys Gly Thr Ala Pro Thr Thr Leu Lys Glu Pro
705 710 715 720

Ala Pro Thr Thr Pro Lys Lys Pro Ala Pro Lys Glu Leu Ala Pro Thr
725 730 735

Thr Thr Lys Glu Pro Thr Ser Thr Thr Ser Asp Lys Pro Ala Pro Thr
740 745 750

Thr Pro Lys Gly Thr Ala Pro Thr Thr Pro Lys Glu Pro Ala Pro Thr
755 760 765

Thr Pro Lys Glu Pro Ala Pro Thr Thr Pro Lys Gly Thr Ala Pro Thr
770 775 780

Thr Leu Lys Glu Pro Ala Pro Thr Thr Pro Lys Lys Pro Ala Pro Lys
785 790 795 800

Glu Leu Ala Pro Thr Thr Thr Lys Gly Pro Thr Ser Thr Thr Ser Asp
805 810 815

Lys Pro Ala Pro Thr Thr Pro Lys Glu Thr Ala Pro Thr Thr Pro Lys
820 825 830

Glu Pro Ala Pro Thr Thr Pro Lys Lys Pro Ala Pro Thr Thr Pro Glu
835 840 845

Thr Pro Pro Pro Thr Thr Ser Glu Val Ser Thr Pro Thr Thr Thr Lys
850 855 860

Glu Pro Thr Thr Ile His Lys Ser Pro Asp Glu Ser Thr Pro Glu Leu
865 870 875 880

Ser Ala Glu Pro Thr Pro Lys Ala Leu Glu Asn Ser Pro Lys Glu Pro
885 890 895

Gly Val Pro Thr Thr Lys Thr Pro Ala Ala Thr Lys Pro Glu Met Thr
900 905 910

Thr Thr Ala Lys Asp Lys Thr Thr Glu Arg Asp Leu Arg Thr Thr Pro
915 920 925

Glu Thr Thr Thr Ala Ala Pro Lys Met Thr Lys Glu Thr Ala Thr Thr
930 935 940

Thr Glu Lys Thr Thr Glu Ser Lys Ile Thr Ala Thr Thr Thr Gln Val
945 950 955 960

```
Thr Ser Thr Thr Thr Gln Asp Thr Thr Pro Phe Lys Ile Thr Thr Leu
                965                 970                 975

Lys Thr Thr Thr Leu Ala Pro Lys Val Thr Thr Thr Lys Lys Thr Ile
            980                 985                 990

Thr Thr Thr Glu Ile Met Asn Lys Pro Glu Glu Thr Ala Lys Pro Lys
        995                 1000                1005

Asp Arg Ala Thr Asn Ser Lys Ala Thr Thr Pro Lys Pro Gln Lys Pro
    1010                1015                1020

Thr Lys Ala Pro Lys Lys Pro Thr Ser Thr Lys Lys Pro Lys Thr Met
1025                1030                1035                1040

Pro Arg Val Arg Lys Pro Lys Thr Thr Pro Thr Pro Arg Lys Met Thr
                1045                1050                1055

Ser Thr Met Pro Glu Leu Asn Pro Thr Ser Arg Ile Ala Glu Ala Met
            1060                1065                1070

Leu Gln Thr Thr Thr Arg Pro Asn Gln Thr Pro Asn Ser Lys Leu Val
        1075                1080                1085

Glu Val Asn Pro Lys Ser Glu Asp Ala Gly Gly Ala Glu Gly Glu Thr
    1090                1095                1100

Pro His Met Leu Leu Arg Pro His Val Phe Met Pro Glu Val Thr Pro
1105                1110                1115                1120

Asp Met Asp Tyr Leu Pro Arg Val Pro Asn Gln Gly Ile Ile Ile Asn
                1125                1130                1135

Pro Met Leu Ser Asp Glu Thr Asn Ile Cys Asn Gly Lys Pro Val Asp
            1140                1145                1150

Gly Leu Thr Thr Leu Arg Asn Gly Thr Leu Val Ala Phe Arg Gly His
        1155                1160                1165

Tyr Phe Trp Met Leu Ser Pro Phe Ser Pro Pro Ser Pro Ala Arg Arg
    1170                1175                1180

Ile Thr Glu Val Trp Gly Ile Pro Ser Pro Ile Asp Thr Val Phe Thr
1185                1190                1195                1200

Arg Cys Asn Cys Glu Gly Lys Thr Phe Phe Phe Lys Asp Ser Gln Tyr
                1205                1210                1215

Trp Arg Phe Thr Asn Asp Ile Lys Asp Ala Gly Tyr Pro Lys Pro Ile
            1220                1225                1230

Phe Lys Gly Phe Gly Gly Leu Thr Gly Gln Ile Val Ala Ala Leu Ser
        1235                1240                1245

Thr Ala Lys Tyr Lys Asn Trp Pro Glu Ser Val Tyr Phe Phe Lys Arg
    1250                1255                1260

Gly Gly Ser Ile Gln Gln Tyr Ile Tyr Lys Gln Glu Pro Val Gln Lys
1265                1270                1275                1280

Cys Pro Gly Arg Arg Pro Ala Leu Asn Tyr Pro Val Tyr Gly Glu Thr
                1285                1290                1295
```

Thr Gln Val Arg Arg Arg Arg Phe Glu Arg Ala Ile Gly Pro Ser Gln
1300 1305 1310

Thr His Thr Ile Arg Ile Gln Tyr Ser Pro Ala Arg Leu Ala Tyr Gln
1315 1320 1325

Asp Lys Gly Val Leu His Asn Glu Val Lys Val Ser Ile Leu Trp Arg
1330 1335 1340

Gly Leu Pro Asn Val Val Thr Ser Ala Ile Ser Leu Pro Asn Ile Arg
1345 1350 1355 1360

Lys Pro Asp Gly Tyr Asp Tyr Tyr Ala Phe Ser Lys Asp Gln Tyr Tyr
1365 1370 1375

Asn Ile Asp Val Pro Ser Arg Thr Ala Arg Ala Ile Thr Thr Arg Ser
1380 1385 1390

Gly Gln Thr Leu Ser Lys Val Trp Tyr Asn Cys Pro
1395 1400

<210> 106
<211> 1427
<212> PRT
<213> Artificial Sequence

<220>
<223> ATS13_HUMAN

<400> 106
Met His Gln Arg His Pro Arg Ala Arg Cys Pro Pro Leu Cys Val Ala
1 5 10 15

Gly Ile Leu Ala Cys Gly Phe Leu Leu Gly Cys Trp Gly Pro Ser His
20 25 30

Phe Gln Gln Ser Cys Leu Gln Ala Leu Glu Pro Gln Ala Val Ser Ser
35 40 45

Tyr Leu Ser Pro Gly Ala Pro Leu Lys Gly Arg Pro Pro Ser Pro Gly
50 55 60

Phe Gln Arg Gln Arg Gln Arg Gln Arg Arg Ala Ala Gly Gly Ile Leu
65 70 75 80

His Leu Glu Leu Leu Val Ala Val Gly Pro Asp Val Phe Gln Ala His
85 90 95

Gln Glu Asp Thr Glu Arg Tyr Val Leu Thr Asn Leu Asn Ile Gly Ala
100 105 110

Glu Leu Leu Arg Asp Pro Ser Leu Gly Ala Gln Phe Arg Val His Leu
115 120 125

Val Lys Met Val Ile Leu Thr Glu Pro Glu Gly Ala Pro Asn Ile Thr
130 135 140

Ala Asn Leu Thr Ser Ser Leu Leu Ser Val Cys Gly Trp Ser Gln Thr
145 150 155 160

```
Ile Asn Pro Glu Asp Asp Thr Asp Pro Gly His Ala Asp Leu Val Leu
                165                 170                 175

Tyr Ile Thr Arg Phe Asp Leu Glu Leu Pro Asp Gly Asn Arg Gln Val
            180                 185                 190

Arg Gly Val Thr Gln Leu Gly Gly Ala Cys Ser Pro Thr Trp Ser Cys
        195                 200                 205

Leu Ile Thr Glu Asp Thr Gly Phe Asp Leu Gly Val Thr Ile Ala His
    210                 215                 220

Glu Ile Gly His Ser Phe Gly Leu Glu His Asp Gly Ala Pro Gly Ser
225                 230                 235                 240

Gly Cys Gly Pro Ser Gly His Val Met Ala Ser Asp Gly Ala Ala Pro
                245                 250                 255

Arg Ala Gly Leu Ala Trp Ser Pro Cys Ser Arg Arg Gln Leu Leu Ser
            260                 265                 270

Leu Leu Ser Ala Gly Arg Ala Arg Cys Val Trp Asp Pro Pro Arg Pro
        275                 280                 285

Gln Pro Gly Ser Ala Gly His Pro Pro Asp Ala Gln Pro Gly Leu Tyr
    290                 295                 300

Tyr Ser Ala Asn Glu Gln Cys Arg Val Ala Phe Gly Pro Lys Ala Val
305                 310                 315                 320

Ala Cys Thr Phe Ala Arg Glu His Leu Asp Met Cys Gln Ala Leu Ser
                325                 330                 335

Cys His Thr Asp Pro Leu Asp Gln Ser Ser Cys Ser Arg Leu Leu Val
            340                 345                 350

Pro Leu Leu Asp Gly Thr Glu Cys Gly Val Glu Lys Trp Cys Ser Lys
        355                 360                 365

Gly Arg Cys Arg Ser Leu Val Glu Leu Thr Pro Ile Ala Ala Val His
    370                 375                 380

Gly Arg Trp Ser Ser Trp Gly Pro Arg Ser Pro Cys Ser Arg Ser Cys
385                 390                 395                 400

Gly Gly Gly Val Val Thr Arg Arg Arg Gln Cys Asn Asn Pro Arg Pro
                405                 410                 415

Ala Phe Gly Gly Arg Ala Cys Val Gly Ala Asp Leu Gln Ala Glu Met
            420                 425                 430

Cys Asn Thr Gln Ala Cys Glu Lys Thr Gln Leu Glu Phe Met Ser Gln
        435                 440                 445

Gln Cys Ala Arg Thr Asp Gly Gln Pro Leu Arg Ser Ser Pro Gly Gly
    450                 455                 460

Ala Ser Phe Tyr His Trp Gly Ala Ala Val Pro His Ser Gln Gly Asp
465                 470                 475                 480

Ala Leu Cys Arg His Met Cys Arg Ala Ile Gly Glu Ser Phe Ile Met
                485                 490                 495
```

Lys Arg Gly Asp Ser Phe Leu Asp Gly Thr Arg Cys Met Pro Ser Gly
500 505 510

Pro Arg Glu Asp Gly Thr Leu Ser Leu Cys Val Ser Gly Ser Cys Arg
515 520 525

Thr Phe Gly Cys Asp Gly Arg Met Asp Ser Gln Gln Val Trp Asp Arg
530 535 540

Cys Gln Val Cys Gly Gly Asp Asn Ser Thr Cys Ser Pro Arg Lys Gly
545 550 555 560

Ser Phe Thr Ala Gly Arg Ala Arg Glu Tyr Val Thr Phe Leu Thr Val
565 570 575

Thr Pro Asn Leu Thr Ser Val Tyr Ile Ala Asn His Arg Pro Leu Phe
580 585 590

Thr His Leu Ala Val Arg Ile Gly Gly Arg Tyr Val Val Ala Gly Lys
595 600 605

Met Ser Ile Ser Pro Asn Thr Thr Tyr Pro Ser Leu Leu Glu Asp Gly
610 615 620

Arg Val Glu Tyr Arg Val Ala Leu Thr Glu Asp Arg Leu Pro Arg Leu
625 630 635 640

Glu Glu Ile Arg Ile Trp Gly Pro Leu Gln Glu Asp Ala Asp Ile Gln
645 650 655

Val Tyr Arg Arg Tyr Gly Glu Glu Tyr Gly Asn Leu Thr Arg Pro Asp
660 665 670

Ile Thr Phe Thr Tyr Phe Gln Pro Lys Pro Arg Gln Ala Trp Val Trp
675 680 685

Ala Ala Val Arg Gly Pro Cys Ser Val Ser Cys Gly Ala Gly Leu Arg
690 695 700

Trp Val Asn Tyr Ser Cys Leu Asp Gln Ala Arg Lys Glu Leu Val Glu
705 710 715 720

Thr Val Gln Cys Gln Gly Ser Gln Gln Pro Pro Ala Trp Pro Glu Ala
725 730 735

Cys Val Leu Glu Pro Cys Pro Pro Tyr Trp Ala Val Gly Asp Phe Gly
740 745 750

Pro Cys Ser Ala Ser Cys Gly Gly Gly Leu Arg Glu Arg Pro Val Arg
755 760 765

Cys Val Glu Ala Gln Gly Ser Leu Leu Lys Thr Leu Pro Pro Ala Arg
770 775 780

Cys Arg Ala Gly Ala Gln Gln Pro Ala Val Ala Leu Glu Thr Cys Asn
785 790 795 800

Pro Gln Pro Cys Pro Ala Arg Trp Glu Val Ser Glu Pro Ser Ser Cys
805 810 815

Thr Ser Ala Gly Gly Ala Gly Leu Ala Leu Glu Asn Glu Thr Cys Val
820 825 830

Pro Gly Ala Asp Gly Leu Glu Ala Pro Val Thr Glu Gly Pro Gly Ser
835 840 845

Val Asp Glu Lys Leu Pro Ala Pro Glu Pro Cys Val Gly Met Ser Cys
850 855 860

Pro Pro Gly Trp Gly His Leu Asp Ala Thr Ser Ala Gly Glu Lys Ala
865 870 875 880

Pro Ser Pro Trp Gly Ser Ile Arg Thr Gly Ala Gln Ala Ala His Val
885 890 895

Trp Thr Pro Ala Ala Gly Ser Cys Ser Val Ser Cys Gly Arg Gly Leu
900 905 910

Met Glu Leu Arg Phe Leu Cys Met Asp Ser Ala Leu Arg Val Pro Val
915 920 925

Gln Glu Glu Leu Cys Gly Leu Ala Ser Lys Pro Gly Ser Arg Arg Glu
930 935 940

Val Cys Gln Ala Val Pro Cys Pro Ala Arg Trp Gln Tyr Lys Leu Ala
945 950 955 960

Ala Cys Ser Val Ser Cys Gly Arg Gly Val Val Arg Arg Ile Leu Tyr
965 970 975

Cys Ala Arg Ala His Gly Glu Asp Asp Gly Glu Glu Ile Leu Leu Asp
980 985 990

Thr Gln Cys Gln Gly Leu Pro Arg Pro Glu Pro Gln Glu Ala Cys Ser
995 1000 1005

Leu Glu Pro Cys Pro Pro Arg Trp Lys Val Met Ser Leu Gly Pro Cys
1010 1015 1020

Ser Ala Ser Cys Gly Leu Gly Thr Ala Arg Arg Ser Val Ala Cys Val
1025 1030 1035 1040

Gln Leu Asp Gln Gly Gln Asp Val Glu Val Asp Glu Ala Ala Cys Ala
1045 1050 1055

Ala Leu Val Arg Pro Glu Ala Ser Val Pro Cys Leu Ile Ala Asp Cys
1060 1065 1070

Thr Tyr Arg Trp His Val Gly Thr Trp Met Glu Cys Ser Val Ser Cys
1075 1080 1085

Gly Asp Gly Ile Gln Arg Arg Arg Asp Thr Cys Leu Gly Pro Gln Ala
1090 1095 1100

Gln Ala Pro Val Pro Ala Asp Phe Cys Gln His Leu Pro Lys Pro Val
1105 1110 1115 1120

Thr Val Arg Gly Cys Trp Ala Gly Pro Cys Val Gly Gln Gly Thr Pro
1125 1130 1135

Ser Leu Val Pro His Glu Glu Ala Ala Ala Pro Gly Arg Thr Thr Ala
1140 1145 1150

Thr Pro Ala Gly Ala Ser Leu Glu Trp Ser Gln Ala Arg Gly Leu Leu
1155 1160 1165

Phe Ser Pro Ala Pro Gln Pro Arg Arg Leu Leu Pro Gly Pro Gln Glu
1170 1175 1180

Asn Ser Val Gln Ser Ser Ala Cys Gly Arg Gln His Leu Glu Pro Thr
1185 1190 1195 1200

Gly Thr Ile Asp Met Arg Gly Pro Gly Gln Ala Asp Cys Ala Val Ala
1205 1210 1215

Ile Gly Arg Pro Leu Gly Glu Val Val Thr Leu Arg Val Leu Glu Ser
1220 1225 1230

Ser Leu Asn Cys Ser Ala Gly Asp Met Leu Leu Leu Trp Gly Arg Leu
1235 1240 1245

Thr Trp Arg Lys Met Cys Arg Lys Leu Leu Asp Met Thr Phe Ser Ser
1250 1255 1260

Lys Thr Asn Thr Leu Val Val Arg Gln Arg Cys Gly Arg Pro Gly Gly
1265 1270 1275 1280

Gly Val Leu Leu Arg Tyr Gly Ser Gln Leu Ala Pro Glu Thr Phe Tyr
1285 1290 1295

Arg Glu Cys Asp Met Gln Leu Phe Gly Pro Trp Gly Glu Ile Val Ser
1300 1305 1310

Pro Ser Leu Ser Pro Ala Thr Ser Asn Ala Gly Gly Cys Arg Leu Phe
1315 1320 1325

Ile Asn Val Ala Pro His Ala Arg Ile Ala Ile His Ala Leu Ala Thr
1330 1335 1340

Asn Met Gly Ala Gly Thr Glu Gly Ala Asn Ala Ser Tyr Ile Leu Ile
1345 1350 1355 1360

Arg Asp Thr His Ser Leu Arg Thr Thr Ala Phe His Gly Gln Gln Val
1365 1370 1375

Leu Tyr Trp Glu Ser Glu Ser Ser Gln Ala Glu Met Glu Phe Ser Glu
1380 1385 1390

Gly Phe Leu Lys Ala Gln Ala Ser Leu Arg Gly Gln Tyr Trp Thr Leu
1395 1400 1405

Gln Ser Trp Val Pro Glu Met Gln Asp Pro Gln Ser Trp Lys Gly Lys
1410 1415 1420

Glu Gly Thr
1425

<210> 107
<211> 1475
<212> PRT
<213> Artificial Sequence

<220>
<223> NU153_HUMAN

<400> 107
Met Ala Ser Gly Ala Gly Gly Val Gly Gly Gly Gly Gly Gly Lys Ile

1 5 10 15

Arg Thr Arg Arg Cys His Gln Gly Pro Ile Lys Pro Tyr Gln Gln Gly
20 25 30

Arg Gln Gln His Gln Gly Ile Leu Ser Arg Val Thr Glu Ser Val Lys
35 40 45

Asn Ile Val Pro Gly Trp Leu Gln Arg Tyr Phe Asn Lys Asn Glu Asp
50 55 60

Val Cys Ser Cys Ser Thr Asp Thr Ser Glu Val Pro Arg Trp Pro Glu
65 70 75 80

Asn Lys Glu Asp His Leu Val Tyr Ala Asp Glu Glu Ser Ser Asn Ile
85 90 95

Thr Asp Gly Arg Ile Thr Pro Glu Pro Ala Val Ser Asn Thr Glu Glu
100 105 110

Pro Ser Thr Thr Ser Thr Ala Ser Asn Tyr Pro Asp Val Leu Thr Arg
115 120 125

Pro Ser Leu His Arg Ser His Leu Asn Phe Ser Met Leu Glu Ser Pro
130 135 140

Ala Leu His Cys Gln Pro Ser Thr Ser Ser Ala Phe Pro Ile Gly Ser
145 150 155 160

Ser Gly Phe Ser Leu Val Lys Glu Ile Lys Asp Ser Thr Ser Gln His
165 170 175

Asp Asp Asp Asn Ile Ser Thr Thr Ser Gly Phe Ser Ser Arg Ala Ser
180 185 190

Asp Lys Asp Ile Thr Val Ser Lys Asn Thr Ser Leu Pro Pro Leu Trp
195 200 205

Ser Pro Glu Ala Glu Arg Ser His Ser Leu Ser Gln His Thr Ala Thr
210 215 220

Ser Ser Lys Lys Pro Ala Phe Asn Leu Ser Ala Phe Gly Thr Leu Ser
225 230 235 240

Pro Ser Leu Gly Asn Ser Ser Ile Leu Lys Thr Ser Gln Leu Gly Asp
245 250 255

Ser Pro Phe Tyr Pro Gly Lys Thr Thr Tyr Gly Gly Ala Ala Ala Ala
260 265 270

Val Arg Gln Ser Lys Leu Arg Asn Thr Pro Tyr Gln Ala Pro Val Arg
275 280 285

Arg Gln Met Lys Ala Lys Gln Leu Ser Ala Gln Ser Tyr Gly Val Thr
290 295 300

Ser Ser Thr Ala Arg Arg Ile Leu Gln Ser Leu Glu Lys Met Ser Ser
305 310 315 320

Pro Leu Ala Asp Ala Lys Arg Ile Pro Ser Ile Val Ser Ser Pro Leu
325 330 335

Asn Ser Pro Leu Asp Arg Ser Gly Ile Asp Ile Thr Asp Phe Gln Ala

340 345 350

Lys Arg Glu Lys Val Asp Ser Gln Tyr Pro Pro Val Gln Arg Leu Met
355 360 365

Thr Pro Lys Pro Val Ser Ile Ala Thr Asn Arg Ser Val Tyr Phe Lys
370 375 380

Pro Ser Leu Thr Pro Ser Gly Glu Phe Arg Lys Thr Asn Gln Arg Ile
385 390 395 400

Asp Asn Lys Cys Ser Thr Gly Tyr Glu Lys Asn Met Thr Pro Gly Gln
405 410 415

Asn Arg Glu Gln Arg Glu Ser Gly Phe Ser Tyr Pro Asn Phe Ser Leu
420 425 430

Pro Ala Ala Asn Gly Leu Ser Ser Gly Val Gly Gly Gly Gly Gly Lys
435 440 445

Met Arg Arg Glu Arg Thr Arg Phe Val Ala Ser Lys Pro Leu Glu Glu
450 455 460

Glu Glu Met Glu Val Pro Val Leu Pro Lys Ile Ser Leu Pro Ile Thr
465 470 475 480

Ser Ser Ser Leu Pro Thr Phe Asn Phe Ser Ser Pro Glu Ile Thr Thr
485 490 495

Ser Ser Pro Ser Pro Ile Asn Ser Ser Gln Ala Leu Thr Asn Lys Val
500 505 510

Gln Met Thr Ser Pro Ser Ser Thr Gly Ser Pro Met Phe Lys Phe Ser
515 520 525

Ser Pro Ile Val Lys Ser Thr Glu Ala Asn Val Leu Pro Pro Ser Ser
530 535 540

Ile Gly Phe Thr Phe Ser Val Pro Val Ala Lys Thr Ala Glu Leu Ser
545 550 555 560

Gly Ser Ser Ser Thr Leu Glu Pro Ile Ile Ser Ser Ser Ala His His
565 570 575

Val Thr Thr Val Asn Ser Thr Asn Cys Lys Lys Thr Pro Pro Glu Asp
580 585 590

Cys Glu Gly Pro Phe Arg Pro Ala Glu Ile Leu Lys Glu Gly Ser Val
595 600 605

Leu Asp Ile Leu Lys Ser Pro Gly Phe Ala Ser Pro Lys Ile Asp Ser
610 615 620

Val Ala Ala Gln Pro Thr Ala Thr Ser Pro Val Val Tyr Thr Arg Pro
625 630 635 640

Ala Ile Ser Ser Phe Ser Ser Ser Gly Ile Gly Phe Gly Glu Ser Leu
645 650 655

Lys Ala Gly Ser Ser Trp Gln Cys Asp Thr Cys Leu Leu Gln Asn Lys
660 665 670

Val Thr Asp Asn Lys Cys Ile Ala Cys Gln Ala Ala Lys Leu Ser Pro

675 680 685

Arg Asp Thr Ala Lys Gln Thr Gly Ile Glu Thr Pro Asn Lys Ser Gly
690 695 700

Lys Thr Thr Leu Ser Ala Ser Gly Thr Gly Phe Gly Asp Lys Phe Lys
705 710 715 720

Pro Val Ile Gly Thr Trp Asp Cys Asp Thr Cys Leu Val Gln Asn Lys
725 730 735

Pro Glu Ala Ile Lys Cys Val Ala Cys Glu Thr Pro Lys Pro Gly Thr
740 745 750

Cys Val Lys Arg Ala Leu Thr Leu Thr Val Val Ser Glu Ser Ala Glu
755 760 765

Thr Met Thr Ala Ser Ser Ser Ser Cys Thr Val Thr Thr Gly Thr Leu
770 775 780

Gly Phe Gly Asp Lys Phe Lys Arg Pro Ile Gly Ser Trp Glu Cys Ser
785 790 795 800

Val Cys Cys Val Ser Asn Asn Ala Glu Asp Asn Lys Cys Val Ser Cys
805 810 815

Met Ser Glu Lys Pro Gly Ser Ser Val Pro Ala Ser Ser Ser Ser Thr
820 825 830

Val Pro Val Ser Leu Pro Ser Gly Gly Ser Leu Gly Leu Glu Lys Phe
835 840 845

Lys Lys Pro Glu Gly Ser Trp Asp Cys Glu Leu Cys Leu Val Gln Asn
850 855 860

Lys Ala Asp Ser Thr Lys Cys Leu Ala Cys Glu Ser Ala Lys Pro Gly
865 870 875 880

Thr Lys Ser Gly Phe Lys Gly Phe Asp Thr Ser Ser Ser Ser Ser Asn
885 890 895

Ser Ala Ala Ser Ser Ser Phe Lys Phe Gly Val Ser Ser Ser Ser Ser
900 905 910

Gly Pro Ser Gln Thr Leu Thr Ser Thr Gly Asn Phe Lys Phe Gly Asp
915 920 925

Gln Gly Gly Phe Lys Ile Gly Val Ser Ser Asp Ser Gly Ser Ile Asn
930 935 940

Pro Met Ser Glu Gly Phe Lys Phe Ser Lys Pro Ile Gly Asp Phe Lys
945 950 955 960

Phe Gly Val Ser Ser Glu Ser Lys Pro Glu Glu Val Lys Lys Asp Ser
965 970 975

Lys Asn Asp Asn Phe Lys Phe Gly Leu Ser Ser Gly Leu Ser Asn Pro
980 985 990

Val Ser Leu Thr Pro Phe Gln Phe Gly Val Ser Asn Leu Gly Gln Glu
995 1000 1005

Glu Lys Lys Glu Glu Leu Pro Lys Ser Ser Ser Ala Gly Phe Ser Phe

1010 1015 1020

Gly Thr Gly Val Ile Asn Ser Thr Pro Ala Pro Ala Asn Thr Ile Val
1025 1030 1035 1040

Thr Ser Glu Asn Lys Ser Ser Phe Asn Leu Gly Thr Ile Glu Thr Lys
1045 1050 1055

Ser Ala Ser Val Ala Pro Phe Thr Cys Lys Thr Ser Glu Ala Lys Lys
1060 1065 1070

Glu Glu Met Pro Ala Thr Lys Gly Gly Phe Ser Phe Gly Asn Val Glu
1075 1080 1085

Pro Ala Ser Leu Pro Ser Ala Ser Val Phe Val Leu Gly Arg Thr Glu
1090 1095 1100

Glu Lys Gln Gln Glu Pro Val Thr Ser Thr Ser Leu Val Phe Gly Lys
1105 1110 1115 1120

Lys Ala Asp Asn Glu Glu Pro Lys Cys Gln Pro Val Phe Ser Phe Gly
1125 1130 1135

Asn Ser Glu Gln Thr Lys Asp Glu Asn Ser Ser Lys Ser Thr Phe Ser
1140 1145 1150

Phe Ser Met Thr Lys Pro Ser Glu Lys Glu Ser Glu Gln Pro Ala Lys
1155 1160 1165

Ala Thr Phe Ala Phe Gly Ala Gln Thr Ser Thr Thr Ala Asp Gln Gly
1170 1175 1180

Ala Ala Lys Pro Val Phe Ser Phe Leu Asn Asn Ser Ser Ser Ser Ser
1185 1190 1195 1200

Ser Thr Pro Ala Thr Ser Ala Gly Gly Gly Ile Phe Gly Ser Ser Thr
1205 1210 1215

Ser Ser Ser Asn Pro Pro Val Ala Thr Phe Val Phe Gly Gln Ser Ser
1220 1225 1230

Asn Pro Val Ser Ser Ser Ala Phe Gly Asn Thr Ala Glu Ser Ser Thr
1235 1240 1245

Ser Gln Ser Leu Leu Phe Ser Gln Asp Ser Lys Leu Ala Thr Thr Ser
1250 1255 1260

Ser Thr Gly Thr Ala Val Thr Pro Phe Val Phe Gly Pro Gly Ala Ser
1265 1270 1275 1280

Ser Asn Asn Thr Thr Thr Ser Gly Phe Gly Phe Gly Ala Thr Thr Thr
1285 1290 1295

Ser Ser Ser Ala Gly Ser Ser Phe Val Phe Gly Thr Gly Pro Ser Ala
1300 1305 1310

Pro Ser Ala Ser Pro Ala Phe Gly Ala Asn Gln Thr Pro Thr Phe Gly
1315 1320 1325

Gln Ser Gln Gly Ala Ser Gln Pro Asn Pro Pro Gly Phe Gly Ser Ile
1330 1335 1340

Ser Ser Ser Thr Ala Leu Phe Pro Thr Gly Ser Gln Pro Ala Pro Pro

1345 1350 1355 1360

Thr Phe Gly Thr Val Ser Ser Ser Ser Gln Pro Pro Val Phe Gly Gln
1365 1370 1375

Gln Pro Ser Gln Ser Ala Phe Gly Ser Gly Thr Thr Pro Asn Ser Ser
1380 1385 1390

Ser Ala Phe Gln Phe Gly Ser Ser Thr Thr Asn Phe Asn Phe Thr Asn
1395 1400 1405

Asn Ser Pro Ser Gly Val Phe Thr Phe Gly Ala Asn Ser Ser Thr Pro
1410 1415 1420

Ala Ala Ser Ala Gln Pro Ser Gly Ser Gly Gly Phe Pro Phe Asn Gln
1425 1430 1435 1440

Ser Pro Ala Ala Phe Thr Val Gly Ser Asn Gly Lys Asn Val Phe Ser
1445 1450 1455

Ser Ser Gly Thr Ser Phe Ser Gly Arg Lys Ile Lys Thr Ala Val Arg
1460 1465 1470

Arg Arg Lys
1475

<210> 108
<211> 1500
<212> PRT
<213> Artificial Sequence

<220>
<223> CPSM_HUMAN

<400> 108
Met Thr Arg Ile Leu Thr Ala Phe Lys Val Val Arg Thr Leu Lys Thr
1 5 10 15

Gly Phe Gly Phe Thr Asn Val Thr Ala His Gln Lys Trp Lys Phe Ser
20 25 30

Arg Pro Gly Ile Arg Leu Leu Ser Val Lys Ala Gln Thr Ala His Ile
35 40 45

Val Leu Glu Asp Gly Thr Lys Met Lys Gly Tyr Ser Phe Gly His Pro
50 55 60

Ser Ser Val Ala Gly Glu Val Val Phe Asn Thr Gly Leu Gly Gly Tyr
65 70 75 80

Pro Glu Ala Ile Thr Asp Pro Ala Tyr Lys Gly Gln Ile Leu Thr Met
85 90 95

Ala Asn Pro Ile Ile Gly Asn Gly Gly Ala Pro Asp Thr Thr Ala Leu
100 105 110

Asp Glu Leu Gly Leu Ser Lys Tyr Leu Glu Ser Asn Gly Ile Lys Val
115 120 125

Ser Gly Leu Leu Val Leu Asp Tyr Ser Lys Asp Tyr Asn His Trp Leu
130 135 140

```
Ala Thr Lys Ser Leu Gly Gln Trp Leu Gln Glu Glu Lys Val Pro Ala
145                 150                 155                 160

Ile Tyr Gly Val Asp Thr Arg Met Leu Thr Lys Ile Ile Arg Asp Lys
                165                 170                 175

Gly Thr Met Leu Gly Lys Ile Glu Phe Glu Gly Gln Pro Val Asp Phe
            180                 185                 190

Val Asp Pro Asn Lys Gln Asn Leu Ile Ala Glu Val Ser Thr Lys Asp
        195                 200                 205

Val Lys Val Tyr Gly Lys Gly Asn Pro Thr Lys Val Val Ala Val Asp
    210                 215                 220

Cys Gly Ile Lys Asn Asn Val Ile Arg Leu Leu Val Lys Arg Gly Ala
225                 230                 235                 240

Glu Val His Leu Val Pro Trp Asn His Asp Phe Thr Lys Met Glu Tyr
                245                 250                 255

Asp Gly Ile Leu Ile Ala Gly Gly Pro Gly Asn Pro Ala Leu Ala Glu
            260                 265                 270

Pro Leu Ile Gln Asn Val Arg Lys Ile Leu Glu Ser Asp Arg Lys Glu
        275                 280                 285

Pro Leu Phe Gly Ile Ser Thr Gly Asn Leu Ile Thr Gly Leu Ala Ala
    290                 295                 300

Gly Ala Lys Thr Tyr Lys Met Ser Met Ala Asn Arg Gly Gln Asn Gln
305                 310                 315                 320

Pro Val Leu Asn Ile Thr Asn Lys Gln Ala Phe Ile Thr Ala Gln Asn
                325                 330                 335

His Gly Tyr Ala Leu Asp Asn Thr Leu Pro Ala Gly Trp Lys Pro Leu
            340                 345                 350

Phe Val Asn Val Asn Asp Gln Thr Asn Glu Gly Ile Met His Glu Ser
        355                 360                 365

Lys Pro Phe Phe Ala Val Gln Phe His Pro Glu Val Thr Pro Gly Pro
    370                 375                 380

Ile Asp Thr Glu Tyr Leu Phe Asp Ser Phe Phe Ser Leu Ile Lys Lys
385                 390                 395                 400

Gly Lys Ala Thr Thr Ile Thr Ser Val Leu Pro Lys Pro Ala Leu Val
                405                 410                 415

Ala Ser Arg Val Glu Val Ser Lys Val Leu Ile Leu Gly Ser Gly Gly
            420                 425                 430

Leu Ser Ile Gly Gln Ala Gly Glu Phe Asp Tyr Ser Gly Ser Gln Ala
        435                 440                 445

Val Lys Ala Met Lys Glu Glu Asn Val Lys Thr Val Leu Met Asn Pro
    450                 455                 460

Asn Ile Ala Ser Val Gln Thr Asn Glu Val Gly Leu Lys Gln Ala Asp
465                 470                 475                 480
```

```
Thr Val Tyr Phe Leu Pro Ile Thr Pro Gln Phe Val Thr Glu Val Ile
                485                 490                 495

Lys Ala Glu Gln Pro Asp Gly Leu Ile Leu Gly Met Gly Gly Gln Thr
            500                 505                 510

Ala Leu Asn Cys Gly Val Glu Leu Phe Lys Arg Gly Val Leu Lys Glu
        515                 520                 525

Tyr Gly Val Lys Val Leu Gly Thr Ser Val Glu Ser Ile Met Ala Thr
    530                 535                 540

Glu Asp Arg Gln Leu Phe Ser Asp Lys Leu Asn Glu Ile Asn Glu Lys
545                 550                 555                 560

Ile Ala Pro Ser Phe Ala Val Glu Ser Ile Glu Asp Ala Leu Lys Ala
                565                 570                 575

Ala Asp Thr Ile Gly Tyr Pro Val Met Ile Arg Ser Ala Tyr Ala Leu
            580                 585                 590

Gly Gly Leu Gly Ser Gly Ile Cys Pro Asn Arg Glu Thr Leu Met Asp
        595                 600                 605

Leu Ser Thr Lys Ala Phe Ala Met Thr Asn Gln Ile Leu Val Glu Lys
    610                 615                 620

Ser Val Thr Gly Trp Lys Glu Ile Glu Tyr Glu Val Val Arg Asp Ala
625                 630                 635                 640

Asp Asp Asn Cys Val Thr Val Cys Asn Met Glu Asn Val Asp Ala Met
                645                 650                 655

Gly Val His Thr Gly Asp Ser Val Val Val Ala Pro Ala Gln Thr Leu
            660                 665                 670

Ser Asn Ala Glu Phe Gln Met Leu Arg Arg Thr Ser Ile Asn Val Val
        675                 680                 685

Arg His Leu Gly Ile Val Gly Glu Cys Asn Ile Gln Phe Ala Leu His
    690                 695                 700

Pro Thr Ser Met Glu Tyr Cys Ile Ile Glu Val Asn Ala Arg Leu Ser
705                 710                 715                 720

Arg Ser Ser Ala Leu Ala Ser Lys Ala Thr Gly Tyr Pro Leu Ala Phe
                725                 730                 735

Ile Ala Ala Lys Ile Ala Leu Gly Ile Pro Leu Pro Glu Ile Lys Asn
            740                 745                 750

Val Val Ser Gly Lys Thr Ser Ala Cys Phe Glu Pro Ser Leu Asp Tyr
        755                 760                 765

Met Val Thr Lys Ile Pro Arg Trp Asp Leu Asp Arg Phe His Gly Thr
    770                 775                 780

Ser Ser Arg Ile Gly Ser Ser Met Lys Ser Val Gly Glu Val Met Ala
785                 790                 795                 800

Ile Gly Arg Thr Phe Glu Glu Ser Phe Gln Lys Ala Leu Arg Met Cys
                805                 810                 815
```

His Pro Ser Ile Glu Gly Phe Thr Pro Arg Leu Pro Met Asn Lys Glu
820 825 830

Trp Pro Ser Asn Leu Asp Leu Arg Lys Glu Leu Ser Glu Pro Ser Ser
835 840 845

Thr Arg Ile Tyr Ala Ile Ala Lys Ala Ile Asp Asp Asn Met Ser Leu
850 855 860

Asp Glu Ile Glu Lys Leu Thr Tyr Ile Asp Lys Trp Phe Leu Tyr Lys
865 870 875 880

Met Arg Asp Ile Leu Asn Met Glu Lys Thr Leu Lys Gly Leu Asn Ser
885 890 895

Glu Ser Met Thr Glu Glu Thr Leu Lys Arg Ala Lys Glu Ile Gly Phe
900 905 910

Ser Asp Lys Gln Ile Ser Lys Cys Leu Gly Leu Thr Glu Ala Gln Thr
915 920 925

Arg Glu Leu Arg Leu Lys Lys Asn Ile His Pro Trp Val Lys Gln Ile
930 935 940

Asp Thr Leu Ala Ala Glu Tyr Pro Ser Val Thr Asn Tyr Leu Tyr Val
945 950 955 960

Thr Tyr Asn Gly Gln Glu His Asp Val Asn Phe Asp Asp His Gly Met
965 970 975

Met Val Leu Gly Cys Gly Pro Tyr His Ile Gly Ser Ser Val Glu Phe
980 985 990

Asp Trp Cys Ala Val Ser Ser Ile Arg Thr Leu Arg Gln Leu Gly Lys
995 1000 1005

Lys Thr Val Val Val Asn Cys Asn Pro Glu Thr Val Ser Thr Asp Phe
1010 1015 1020

Asp Glu Cys Asp Lys Leu Tyr Phe Glu Glu Leu Ser Leu Glu Arg Ile
1025 1030 1035 1040

Leu Asp Ile Tyr His Gln Glu Ala Cys Gly Gly Cys Ile Ile Ser Val
1045 1050 1055

Gly Gly Gln Ile Pro Asn Asn Leu Ala Val Pro Leu Tyr Lys Asn Gly
1060 1065 1070

Val Lys Ile Met Gly Thr Ser Pro Leu Gln Ile Asp Arg Ala Glu Asp
1075 1080 1085

Arg Ser Ile Phe Ser Ala Val Leu Asp Glu Leu Lys Val Ala Gln Ala
1090 1095 1100

Pro Trp Lys Ala Val Asn Thr Leu Asn Glu Ala Leu Glu Phe Ala Lys
1105 1110 1115 1120

Ser Val Asp Tyr Pro Cys Leu Leu Arg Pro Ser Tyr Val Leu Ser Gly
1125 1130 1135

Ser Ala Met Asn Val Val Phe Ser Glu Asp Glu Met Lys Lys Phe Leu
1140 1145 1150

Glu Glu Ala Thr Arg Val Ser Gln Glu His Pro Val Val Leu Thr Lys
1155 1160 1165

Phe Val Glu Gly Ala Arg Glu Val Glu Met Asp Ala Val Gly Lys Asp
1170 1175 1180

Gly Arg Val Ile Ser His Ala Ile Ser Glu His Val Glu Asp Ala Gly
1185 1190 1195 1200

Val His Ser Gly Asp Ala Thr Leu Met Leu Pro Thr Gln Thr Ile Ser
1205 1210 1215

Gln Gly Ala Ile Glu Lys Val Lys Asp Ala Thr Arg Lys Ile Ala Lys
1220 1225 1230

Ala Phe Ala Ile Ser Gly Pro Phe Asn Val Gln Phe Leu Val Lys Gly
1235 1240 1245

Asn Asp Val Leu Val Ile Glu Cys Asn Leu Arg Ala Ser Arg Ser Phe
1250 1255 1260

Pro Phe Val Ser Lys Thr Leu Gly Val Asp Phe Ile Asp Val Ala Thr
1265 1270 1275 1280

Lys Val Met Ile Gly Glu Asn Val Asp Glu Lys His Leu Pro Thr Leu
1285 1290 1295

Asp His Pro Ile Ile Pro Ala Asp Tyr Val Ala Ile Lys Ala Pro Met
1300 1305 1310

Phe Ser Trp Pro Arg Leu Arg Asp Ala Asp Pro Ile Leu Arg Cys Glu
1315 1320 1325

Met Ala Ser Thr Gly Glu Val Ala Cys Phe Gly Glu Gly Ile His Thr
1330 1335 1340

Ala Phe Leu Lys Ala Met Leu Ser Thr Gly Phe Lys Ile Pro Gln Lys
1345 1350 1355 1360

Gly Ile Leu Ile Gly Ile Gln Gln Ser Phe Arg Pro Arg Phe Leu Gly
1365 1370 1375

Val Ala Glu Gln Leu His Asn Glu Gly Phe Lys Leu Phe Ala Thr Glu
1380 1385 1390

Ala Thr Ser Asp Trp Leu Asn Ala Asn Asn Val Pro Ala Thr Pro Val
1395 1400 1405

Ala Trp Pro Ser Gln Glu Gly Gln Asn Pro Ser Leu Ser Ser Ile Arg
1410 1415 1420

Lys Leu Ile Arg Asp Gly Ser Ile Asp Leu Val Ile Asn Leu Pro Asn
1425 1430 1435 1440

Asn Asn Thr Lys Phe Val His Asp Asn Tyr Val Ile Arg Arg Thr Ala
1445 1450 1455

Val Asp Ser Gly Ile Pro Leu Leu Thr Asn Phe Gln Val Thr Lys Leu
1460 1465 1470

Phe Ala Glu Ala Val Gln Lys Ser Arg Lys Val Asp Ser Lys Ser Leu
1475 1480 1485

Phe His Tyr Arg Gln Tyr Ser Ala Gly Lys Ala Ala
1490 1495 1500

<210> 109
<211> 1762
<212> PRT
<213> Artificial Sequence

<220>
<223> ATL1_HUMAN

<400> 109
Met Glu Cys Cys Arg Arg Ala Thr Pro Gly Thr Leu Leu Leu Phe Leu
1 5 10 15

Ala Phe Leu Leu Leu Ser Ser Arg Thr Ala Arg Ser Glu Glu Asp Arg
20 25 30

Asp Gly Leu Trp Asp Ala Trp Gly Pro Trp Ser Glu Cys Ser Arg Thr
35 40 45

Cys Gly Gly Gly Ala Ser Tyr Ser Leu Arg Arg Cys Leu Ser Ser Lys
50 55 60

Ser Cys Glu Gly Arg Asn Ile Arg Tyr Arg Thr Cys Ser Asn Val Asp
65 70 75 80

Cys Pro Pro Glu Ala Gly Asp Phe Arg Ala Gln Gln Cys Ser Ala His
85 90 95

Asn Asp Val Lys His His Gly Gln Phe Tyr Glu Trp Leu Pro Val Ser
100 105 110

Asn Asp Pro Asp Asn Pro Cys Ser Leu Lys Cys Gln Ala Lys Gly Thr
115 120 125

Thr Leu Val Val Glu Leu Ala Pro Lys Val Leu Asp Gly Thr Arg Cys
130 135 140

Tyr Thr Glu Ser Leu Asp Met Cys Ile Ser Gly Leu Cys Gln Ile Val
145 150 155 160

Gly Cys Asp His Gln Leu Gly Ser Thr Val Lys Glu Asp Asn Cys Gly
165 170 175

Val Cys Asn Gly Asp Gly Ser Thr Cys Arg Leu Val Arg Gly Gln Tyr
180 185 190

Lys Ser Gln Leu Ser Ala Thr Lys Ser Asp Asp Thr Val Val Ala Ile
195 200 205

Pro Tyr Gly Ser Arg His Ile Arg Leu Val Leu Lys Gly Pro Asp His
210 215 220

Leu Tyr Leu Glu Thr Lys Thr Leu Gln Gly Thr Lys Gly Glu Asn Ser
225 230 235 240

Leu Ser Ser Thr Gly Thr Phe Leu Val Asp Asn Ser Ser Val Asp Phe
245 250 255

Gln Lys Phe Pro Asp Lys Glu Ile Leu Arg Met Ala Gly Pro Leu Thr
260 265 270

Ala Asp Phe Ile Val Lys Ile Arg Asn Ser Gly Ser Ala Asp Ser Thr
275 280 285

Val Gln Phe Ile Phe Tyr Gln Pro Ile Ile His Arg Trp Arg Glu Thr
290 295 300

Asp Phe Phe Pro Cys Ser Ala Thr Cys Gly Gly Gly Tyr Gln Leu Thr
305 310 315 320

Ser Ala Glu Cys Tyr Asp Leu Arg Ser Asn Arg Val Val Ala Asp Gln
325 330 335

Tyr Cys His Tyr Tyr Pro Glu Asn Ile Lys Pro Lys Pro Lys Leu Gln
340 345 350

Glu Cys Asn Leu Asp Pro Cys Pro Ala Ser Asp Gly Tyr Lys Gln Ile
355 360 365

Met Pro Tyr Asp Leu Tyr His Pro Leu Pro Arg Trp Glu Ala Thr Pro
370 375 380

Trp Thr Ala Cys Ser Ser Ser Cys Gly Gly Gly Ile Gln Ser Arg Ala
385 390 395 400

Val Ser Cys Val Glu Glu Asp Ile Gln Gly His Val Thr Ser Val Glu
405 410 415

Glu Trp Lys Cys Met Tyr Thr Pro Lys Met Pro Ile Ala Gln Pro Cys
420 425 430

Asn Ile Phe Asp Cys Pro Lys Trp Leu Ala Gln Glu Trp Ser Pro Cys
435 440 445

Thr Val Thr Cys Gly Gln Gly Leu Arg Tyr Arg Val Val Leu Cys Ile
450 455 460

Asp His Arg Gly Met His Thr Gly Gly Cys Ser Pro Lys Thr Lys Pro
465 470 475 480

His Ile Lys Glu Glu Cys Ile Val Pro Thr Pro Cys Tyr Lys Pro Lys
485 490 495

Glu Lys Leu Pro Val Glu Ala Lys Leu Pro Trp Phe Lys Gln Ala Gln
500 505 510

Glu Leu Glu Glu Gly Ala Ala Val Ser Glu Glu Pro Ser Phe Ile Pro
515 520 525

Glu Ala Trp Ser Ala Cys Thr Val Thr Cys Gly Val Gly Thr Gln Val
530 535 540

Arg Ile Val Arg Cys Gln Val Leu Leu Ser Phe Ser Gln Ser Val Ala
545 550 555 560

Asp Leu Pro Ile Asp Glu Cys Glu Gly Pro Lys Pro Ala Ser Gln Arg
565 570 575

Ala Cys Tyr Ala Gly Pro Cys Ser Gly Glu Ile Pro Glu Phe Asn Pro
580 585 590

```
Asp Glu Thr Asp Gly Leu Phe Gly Gly Leu Gln Asp Phe Asp Glu Leu
        595                 600                 605

Tyr Asp Trp Glu Tyr Glu Gly Phe Thr Lys Cys Ser Glu Ser Cys Gly
    610                 615                 620

Gly Gly Val Gln Glu Ala Val Val Ser Cys Leu Asn Lys Gln Thr Arg
625                 630                 635                 640

Glu Pro Ala Glu Glu Asn Leu Cys Val Thr Ser Arg Arg Pro Pro Gln
                645                 650                 655

Leu Leu Lys Ser Cys Asn Leu Asp Pro Cys Pro Ala Arg Trp Glu Ile
            660                 665                 670

Gly Lys Trp Ser Pro Cys Ser Leu Thr Cys Gly Val Gly Leu Gln Thr
        675                 680                 685

Arg Asp Val Phe Cys Ser His Leu Leu Ser Arg Glu Met Asn Glu Thr
    690                 695                 700

Val Ile Leu Ala Asp Glu Leu Cys Arg Gln Pro Lys Pro Ser Thr Val
705                 710                 715                 720

Gln Ala Cys Asn Arg Phe Asn Cys Pro Pro Ala Trp Tyr Pro Ala Gln
                725                 730                 735

Trp Gln Pro Cys Ser Arg Thr Cys Gly Gly Gly Val Gln Lys Arg Glu
            740                 745                 750

Val Leu Cys Lys Gln Arg Met Ala Asp Gly Ser Phe Leu Glu Leu Pro
        755                 760                 765

Glu Thr Phe Cys Ser Ala Ser Lys Pro Ala Cys Gln Gln Ala Cys Lys
    770                 775                 780

Lys Asp Asp Cys Pro Ser Glu Trp Leu Leu Ser Asp Trp Thr Glu Cys
785                 790                 795                 800

Ser Thr Ser Cys Gly Glu Gly Thr Gln Thr Arg Ser Ala Ile Cys Arg
                805                 810                 815

Lys Met Leu Lys Thr Gly Leu Ser Thr Val Val Asn Ser Thr Leu Cys
            820                 825                 830

Pro Pro Leu Pro Phe Ser Ser Ser Ile Arg Pro Cys Met Leu Ala Thr
        835                 840                 845

Cys Ala Arg Pro Gly Arg Pro Ser Thr Lys His Ser Pro His Ile Ala
    850                 855                 860

Ala Ala Arg Lys Val Tyr Ile Gln Thr Arg Arg Gln Arg Lys Leu His
865                 870                 875                 880

Phe Val Val Gly Gly Phe Ala Tyr Leu Leu Pro Lys Thr Ala Val Val
                885                 890                 895

Leu Arg Cys Pro Ala Arg Arg Val Arg Lys Pro Leu Ile Thr Trp Glu
            900                 905                 910

Lys Asp Gly Gln His Leu Ile Ser Ser Thr His Val Thr Val Ala Pro
        915                 920                 925
```

Phe Gly Tyr Leu Lys Ile His Arg Leu Lys Pro Ser Asp Ala Gly Val
930 935 940

Tyr Thr Cys Ser Ala Gly Pro Ala Arg Glu His Phe Val Ile Lys Leu
945 950 955 960

Ile Gly Gly Asn Arg Lys Leu Val Ala Arg Pro Leu Ser Pro Arg Ser
965 970 975

Glu Glu Glu Val Leu Ala Gly Arg Lys Gly Gly Pro Lys Glu Ala Leu
980 985 990

Gln Thr His Lys His Gln Asn Gly Ile Phe Ser Asn Gly Ser Lys Ala
995 1000 1005

Glu Lys Arg Gly Leu Ala Ala Asn Pro Gly Ser Arg Tyr Asp Asp Leu
1010 1015 1020

Val Ser Arg Leu Leu Glu Gln Gly Gly Trp Pro Gly Glu Leu Leu Ala
1025 1030 1035 1040

Ser Trp Glu Ala Gln Asp Ser Ala Glu Arg Asn Thr Thr Ser Glu Glu
1045 1050 1055

Asp Pro Gly Ala Glu Gln Val Leu Leu His Leu Pro Phe Thr Met Val
1060 1065 1070

Thr Glu Gln Arg Arg Leu Asp Asp Ile Leu Gly Asn Leu Ser Gln Gln
1075 1080 1085

Pro Glu Glu Leu Arg Asp Leu Tyr Ser Lys His Leu Val Ala Gln Leu
1090 1095 1100

Ala Gln Glu Ile Phe Arg Ser His Leu Glu His Gln Asp Thr Leu Leu
1105 1110 1115 1120

Lys Pro Ser Glu Arg Arg Thr Ser Pro Val Thr Leu Ser Pro His Lys
1125 1130 1135

His Val Ser Gly Phe Ser Ser Ser Leu Arg Thr Ser Ser Thr Gly Asp
1140 1145 1150

Ala Gly Gly Gly Ser Arg Arg Pro His Arg Lys Pro Thr Ile Leu Arg
1155 1160 1165

Lys Ile Ser Ala Ala Gln Gln Leu Ser Ala Ser Glu Val Val Thr His
1170 1175 1180

Leu Gly Gln Thr Val Ala Leu Ala Ser Gly Thr Leu Ser Val Leu Leu
1185 1190 1195 1200

His Cys Glu Ala Ile Gly His Pro Arg Pro Thr Ile Ser Trp Ala Arg
1205 1210 1215

Asn Gly Glu Glu Val Gln Phe Ser Asp Arg Ile Leu Leu Gln Pro Asp
1220 1225 1230

Asp Ser Leu Gln Ile Leu Ala Pro Val Glu Ala Asp Val Gly Phe Tyr
1235 1240 1245

Thr Cys Asn Ala Thr Asn Ala Leu Gly Tyr Asp Ser Val Ser Ile Ala
1250 1255 1260

Val Thr Leu Ala Gly Lys Pro Leu Val Lys Thr Ser Arg Met Thr Val
1265 1270 1275 1280

Ile Asn Thr Glu Lys Pro Ala Val Thr Val Asp Ile Gly Ser Thr Ile
1285 1290 1295

Lys Thr Val Gln Gly Val Asn Val Thr Ile Asn Cys Gln Val Ala Gly
1300 1305 1310

Val Pro Glu Ala Glu Val Thr Trp Phe Arg Asn Lys Ser Lys Leu Gly
1315 1320 1325

Ser Pro His His Leu His Glu Gly Ser Leu Leu Leu Thr Asn Val Ser
1330 1335 1340

Ser Ser Asp Gln Gly Leu Tyr Ser Cys Arg Ala Ala Asn Leu His Gly
1345 1350 1355 1360

Glu Leu Thr Glu Ser Thr Gln Leu Leu Ile Leu Asp Pro Pro Gln Val
1365 1370 1375

Pro Thr Gln Leu Glu Asp Ile Arg Ala Leu Leu Ala Ala Thr Gly Pro
1380 1385 1390

Asn Leu Pro Ser Val Leu Thr Ser Pro Leu Gly Thr Gln Leu Val Leu
1395 1400 1405

Asp Pro Gly Asn Ser Ala Leu Leu Gly Cys Pro Ile Lys Gly His Pro
1410 1415 1420

Val Pro Asn Ile Thr Trp Phe His Gly Gly Gln Pro Ile Val Thr Ala
1425 1430 1435 1440

Thr Gly Leu Thr His His Ile Leu Ala Ala Gly Gln Ile Leu Gln Val
1445 1450 1455

Ala Asn Leu Ser Gly Gly Ser Gln Gly Glu Phe Ser Cys Leu Ala Gln
1460 1465 1470

Asn Glu Ala Gly Val Leu Met Gln Lys Ala Ser Leu Val Ile Gln Asp
1475 1480 1485

Tyr Trp Trp Ser Val Asp Arg Leu Ala Thr Cys Ser Ala Ser Cys Gly
1490 1495 1500

Asn Arg Gly Val Gln Gln Pro Arg Leu Arg Cys Leu Leu Asn Ser Thr
1505 1510 1515 1520

Glu Val Asn Pro Ala His Cys Ala Gly Lys Val Arg Pro Ala Val Gln
1525 1530 1535

Pro Ile Ala Cys Asn Arg Arg Asp Cys Pro Ser Arg Trp Met Val Thr
1540 1545 1550

Ser Trp Ser Ala Cys Thr Arg Ser Cys Gly Gly Gly Val Gln Thr Arg
1555 1560 1565

Arg Val Thr Cys Gln Lys Leu Lys Ala Ser Gly Ile Ser Thr Pro Val
1570 1575 1580

Ser Asn Asp Met Cys Thr Gln Val Ala Lys Arg Pro Val Asp Thr Gln
1585 1590 1595 1600

Ala Cys Asn Gln Gln Leu Cys Val Glu Trp Ala Phe Ser Ser Trp Gly
1605 1610 1615

Gln Cys Asn Gly Pro Cys Ile Gly Pro His Leu Ala Val Gln His Arg
1620 1625 1630

Gln Val Phe Cys Gln Thr Arg Asp Gly Ile Thr Leu Pro Ser Glu Gln
1635 1640 1645

Cys Ser Ala Leu Pro Arg Pro Val Ser Thr Gln Asn Cys Trp Ser Glu
1650 1655 1660

Ala Cys Ser Val His Trp Arg Val Ser Leu Trp Thr Leu Cys Thr Ala
1665 1670 1675 1680

Thr Cys Gly Asn Tyr Gly Phe Gln Ser Arg Arg Val Glu Cys Val His
1685 1690 1695

Ala Arg Thr Asn Lys Ala Val Pro Glu His Leu Cys Ser Trp Gly Pro
1700 1705 1710

Arg Pro Ala Asn Trp Gln Arg Cys Asn Ile Thr Pro Cys Glu Asn Met
1715 1720 1725

Glu Cys Arg Asp Thr Thr Arg Tyr Cys Glu Lys Val Lys Gln Leu Lys
1730 1735 1740

Leu Cys Gln Leu Ser Gln Phe Lys Ser Arg Cys Cys Gly Thr Cys Gly
1745 1750 1755 1760

Lys Ala

<210> 110
<211> 2555
<212> PRT
<213> Artificial Sequence

<220>
<223> NOTC1_HUMAN

<400> 110
Met Pro Pro Leu Leu Ala Pro Leu Leu Cys Leu Ala Leu Leu Pro Ala
1 5 10 15

Leu Ala Ala Arg Gly Pro Arg Cys Ser Gln Pro Gly Glu Thr Cys Leu
20 25 30

Asn Gly Gly Lys Cys Glu Ala Ala Asn Gly Thr Glu Ala Cys Val Cys
35 40 45

Gly Gly Ala Phe Val Gly Pro Arg Cys Gln Asp Pro Asn Pro Cys Leu
50 55 60

Ser Thr Pro Cys Lys Asn Ala Gly Thr Cys His Val Val Asp Arg Arg
65 70 75 80

Gly Val Ala Asp Tyr Ala Cys Ser Cys Ala Leu Gly Phe Ser Gly Pro
85 90 95

Leu Cys Leu Thr Pro Leu Asp Asn Ala Cys Leu Thr Asn Pro Cys Arg

100 105 110

Asn Gly Gly Thr Cys Asp Leu Leu Thr Leu Thr Glu Tyr Lys Cys Arg
115 120 125

Cys Pro Pro Gly Trp Ser Gly Lys Ser Cys Gln Gln Ala Asp Pro Cys
130 135 140

Ala Ser Asn Pro Cys Ala Asn Gly Gly Gln Cys Leu Pro Phe Glu Ala
145 150 155 160

Ser Tyr Ile Cys His Cys Pro Pro Ser Phe His Gly Pro Thr Cys Arg
165 170 175

Gln Asp Val Asn Glu Cys Gly Gln Lys Pro Gly Leu Cys Arg His Gly
180 185 190

Gly Thr Cys His Asn Glu Val Gly Ser Tyr Arg Cys Val Cys Arg Ala
195 200 205

Thr His Thr Gly Pro Asn Cys Glu Arg Pro Tyr Val Pro Cys Ser Pro
210 215 220

Ser Pro Cys Gln Asn Gly Gly Thr Cys Arg Pro Thr Gly Asp Val Thr
225 230 235 240

His Glu Cys Ala Cys Leu Pro Gly Phe Thr Gly Gln Asn Cys Glu Glu
245 250 255

Asn Ile Asp Asp Cys Pro Gly Asn Asn Cys Lys Asn Gly Gly Ala Cys
260 265 270

Val Asp Gly Val Asn Thr Tyr Asn Cys Arg Cys Pro Pro Glu Trp Thr
275 280 285

Gly Gln Tyr Cys Thr Glu Asp Val Asp Glu Cys Gln Leu Met Pro Asn
290 295 300

Ala Cys Gln Asn Gly Gly Thr Cys His Asn Thr His Gly Gly Tyr Asn
305 310 315 320

Cys Val Cys Val Asn Gly Trp Thr Gly Glu Asp Cys Ser Glu Asn Ile
325 330 335

Asp Asp Cys Ala Ser Ala Ala Cys Phe His Gly Ala Thr Cys His Asp
340 345 350

Arg Val Ala Ser Phe Tyr Cys Glu Cys Pro His Gly Arg Thr Gly Leu
355 360 365

Leu Cys His Leu Asn Asp Ala Cys Ile Ser Asn Pro Cys Asn Glu Gly
370 375 380

Ser Asn Cys Asp Thr Asn Pro Val Asn Gly Lys Ala Ile Cys Thr Cys
385 390 395 400

Pro Ser Gly Tyr Thr Gly Pro Ala Cys Ser Gln Asp Val Asp Glu Cys
405 410 415

Ser Leu Gly Ala Asn Pro Cys Glu His Ala Gly Lys Cys Ile Asn Thr
420 425 430

Leu Gly Ser Phe Glu Cys Gln Cys Leu Gln Gly Tyr Thr Gly Pro Arg

435 440 445

Cys Glu Ile Asp Val Asn Glu Cys Val Ser Asn Pro Cys Gln Asn Asp
450 455 460

Ala Thr Cys Leu Asp Gln Ile Gly Glu Phe Gln Cys Ile Cys Met Pro
465 470 475 480

Gly Tyr Glu Gly Val His Cys Glu Val Asn Thr Asp Glu Cys Ala Ser
485 490 495

Ser Pro Cys Leu His Asn Gly Arg Cys Leu Asp Lys Ile Asn Glu Phe
500 505 510

Gln Cys Glu Cys Pro Thr Gly Phe Thr Gly His Leu Cys Gln Tyr Asp
515 520 525

Val Asp Glu Cys Ala Ser Thr Pro Cys Lys Asn Gly Ala Lys Cys Leu
530 535 540

Asp Gly Pro Asn Thr Tyr Thr Cys Val Cys Thr Glu Gly Tyr Thr Gly
545 550 555 560

Thr His Cys Glu Val Asp Ile Asp Glu Cys Asp Pro Asp Pro Cys His
565 570 575

Tyr Gly Ser Cys Lys Asp Gly Val Ala Thr Phe Thr Cys Leu Cys Arg
580 585 590

Pro Gly Tyr Thr Gly His His Cys Glu Thr Asn Ile Asn Glu Cys Ser
595 600 605

Ser Gln Pro Cys Arg His Gly Gly Thr Cys Gln Asp Arg Asp Asn Ala
610 615 620

Tyr Leu Cys Phe Cys Leu Lys Gly Thr Thr Gly Pro Asn Cys Glu Ile
625 630 635 640

Asn Leu Asp Asp Cys Ala Ser Ser Pro Cys Asp Ser Gly Thr Cys Leu
645 650 655

Asp Lys Ile Asp Gly Tyr Glu Cys Ala Cys Glu Pro Gly Tyr Thr Gly
660 665 670

Ser Met Cys Asn Ile Asn Ile Asp Glu Cys Ala Gly Asn Pro Cys His
675 680 685

Asn Gly Gly Thr Cys Glu Asp Gly Ile Asn Gly Phe Thr Cys Arg Cys
690 695 700

Pro Glu Gly Tyr His Asp Pro Thr Cys Leu Ser Glu Val Asn Glu Cys
705 710 715 720

Asn Ser Asn Pro Cys Val His Gly Ala Cys Arg Asp Ser Leu Asn Gly
725 730 735

Tyr Lys Cys Asp Cys Asp Pro Gly Trp Ser Gly Thr Asn Cys Asp Ile
740 745 750

Asn Asn Asn Glu Cys Glu Ser Asn Pro Cys Val Asn Gly Gly Thr Cys
755 760 765

Lys Asp Met Thr Ser Gly Tyr Val Cys Thr Cys Arg Glu Gly Phe Ser

```
    770                 775                 780

Gly Pro Asn Cys Gln Thr Asn Ile Asn Glu Cys Ala Ser Asn Pro Cys
785                 790                 795                 800

Leu Asn Gln Gly Thr Cys Ile Asp Asp Val Ala Gly Tyr Lys Cys Asn
                805                 810                 815

Cys Leu Leu Pro Tyr Thr Gly Ala Thr Cys Glu Val Val Leu Ala Pro
            820                 825                 830

Cys Ala Pro Ser Pro Cys Arg Asn Gly Gly Glu Cys Arg Gln Ser Glu
        835                 840                 845

Asp Tyr Glu Ser Phe Ser Cys Val Cys Pro Thr Gly Trp Gln Gly Gln
    850                 855                 860

Thr Cys Glu Val Asp Ile Asn Glu Cys Val Leu Ser Pro Cys Arg His
865                 870                 875                 880

Gly Ala Ser Cys Gln Asn Thr His Gly Gly Tyr Arg Cys His Cys Gln
                885                 890                 895

Ala Gly Tyr Ser Gly Arg Asn Cys Glu Thr Asp Ile Asp Asp Cys Arg
            900                 905                 910

Pro Asn Pro Cys His Asn Gly Gly Ser Cys Thr Asp Gly Ile Asn Thr
        915                 920                 925

Ala Phe Cys Asp Cys Leu Pro Gly Phe Arg Gly Thr Phe Cys Glu Glu
    930                 935                 940

Asp Ile Asn Glu Cys Ala Ser Asp Pro Cys Arg Asn Gly Ala Asn Cys
945                 950                 955                 960

Thr Asp Cys Val Asp Ser Tyr Thr Cys Thr Cys Pro Ala Gly Phe Ser
                965                 970                 975

Gly Ile His Cys Glu Asn Asn Thr Pro Asp Cys Thr Glu Ser Ser Cys
            980                 985                 990

Phe Asn Gly Gly Thr Cys Val Asp Gly Ile Asn Ser Phe Thr Cys Leu
        995                 1000                1005

Cys Pro Pro Gly Phe Thr Gly Ser Tyr Cys Gln His Asp Val Asn Glu
   1010                1015                1020

Cys Asp Ser Gln Pro Cys Leu His Gly Gly Thr Cys Gln Asp Gly Cys
1025               1030                1035                1040

Gly Ser Tyr Arg Cys Thr Cys Pro Gln Gly Tyr Thr Gly Pro Asn Cys
               1045                1050                1055

Gln Asn Leu Val His Trp Cys Asp Ser Ser Pro Cys Lys Asn Gly Gly
           1060                1065                1070

Lys Cys Trp Gln Thr His Thr Gln Tyr Arg Cys Glu Cys Pro Ser Gly
       1075                1080                1085

Trp Thr Gly Leu Tyr Cys Asp Val Pro Ser Val Ser Cys Glu Val Ala
   1090                1095                1100

Ala Gln Arg Gln Gly Val Asp Val Ala Arg Leu Cys Gln His Gly Gly
```

1105 1110 1115 1120

Leu Cys Val Asp Ala Gly Asn Thr His His Cys Arg Cys Gln Ala Gly
1125 1130 1135

Tyr Thr Gly Ser Tyr Cys Glu Asp Leu Val Asp Glu Cys Ser Pro Ser
1140 1145 1150

Pro Cys Gln Asn Gly Ala Thr Cys Thr Asp Tyr Leu Gly Gly Tyr Ser
1155 1160 1165

Cys Lys Cys Val Ala Gly Tyr His Gly Val Asn Cys Ser Glu Glu Ile
1170 1175 1180

Asp Glu Cys Leu Ser His Pro Cys Gln Asn Gly Gly Thr Cys Leu Asp
1185 1190 1195 1200

Leu Pro Asn Thr Tyr Lys Cys Ser Cys Pro Arg Gly Thr Gln Gly Val
1205 1210 1215

His Cys Glu Ile Asn Val Asp Asp Cys Asn Pro Pro Val Asp Pro Val
1220 1225 1230

Ser Arg Ser Pro Lys Cys Phe Asn Asn Gly Thr Cys Val Asp Gln Val
1235 1240 1245

Gly Gly Tyr Ser Cys Thr Cys Pro Pro Gly Phe Val Gly Glu Arg Cys
1250 1255 1260

Glu Gly Asp Val Asn Glu Cys Leu Ser Asn Pro Cys Asp Ala Arg Gly
1265 1270 1275 1280

Thr Gln Asn Cys Val Gln Arg Val Asn Asp Phe His Cys Glu Cys Arg
1285 1290 1295

Ala Gly His Thr Gly Arg Arg Cys Glu Ser Val Ile Asn Gly Cys Lys
1300 1305 1310

Gly Lys Pro Cys Lys Asn Gly Gly Thr Cys Ala Val Ala Ser Asn Thr
1315 1320 1325

Ala Arg Gly Phe Ile Cys Lys Cys Pro Ala Gly Phe Glu Gly Ala Thr
1330 1335 1340

Cys Glu Asn Asp Ala Arg Thr Cys Gly Ser Leu Arg Cys Leu Asn Gly
1345 1350 1355 1360

Gly Thr Cys Ile Ser Gly Pro Arg Ser Pro Thr Cys Leu Cys Leu Gly
1365 1370 1375

Pro Phe Thr Gly Pro Glu Cys Gln Phe Pro Ala Ser Ser Pro Cys Leu
1380 1385 1390

Gly Gly Asn Pro Cys Tyr Asn Gln Gly Thr Cys Glu Pro Thr Ser Glu
1395 1400 1405

Ser Pro Phe Tyr Arg Cys Leu Cys Pro Ala Lys Phe Asn Gly Leu Leu
1410 1415 1420

Cys His Ile Leu Asp Tyr Ser Phe Gly Gly Gly Ala Gly Arg Asp Ile
1425 1430 1435 1440

Pro Pro Pro Leu Ile Glu Glu Ala Cys Glu Leu Pro Glu Cys Gln Glu

1445 1450 1455

Asp Ala Gly Asn Lys Val Cys Ser Leu Gln Cys Asn Asn His Ala Cys
1460 1465 1470

Gly Trp Asp Gly Gly Asp Cys Ser Leu Asn Phe Asn Asp Pro Trp Lys
1475 1480 1485

Asn Cys Thr Gln Ser Leu Gln Cys Trp Lys Tyr Phe Ser Asp Gly His
1490 1495 1500

Cys Asp Ser Gln Cys Asn Ser Ala Gly Cys Leu Phe Asp Gly Phe Asp
1505 1510 1515 1520

Cys Gln Arg Ala Glu Gly Gln Cys Asn Pro Leu Tyr Asp Gln Tyr Cys
1525 1530 1535

Lys Asp His Phe Ser Asp Gly His Cys Asp Gln Gly Cys Asn Ser Ala
1540 1545 1550

Glu Cys Glu Trp Asp Gly Leu Asp Cys Ala Glu His Val Pro Glu Arg
1555 1560 1565

Leu Ala Ala Gly Thr Leu Val Val Val Val Leu Met Pro Pro Glu Gln
1570 1575 1580

Leu Arg Asn Ser Ser Phe His Phe Leu Arg Glu Leu Ser Arg Val Leu
1585 1590 1595 1600

His Thr Asn Val Val Phe Lys Arg Asp Ala His Gly Gln Gln Met Ile
1605 1610 1615

Phe Pro Tyr Tyr Gly Arg Glu Glu Glu Leu Arg Lys His Pro Ile Lys
1620 1625 1630

Arg Ala Ala Glu Gly Trp Ala Ala Pro Asp Ala Leu Leu Gly Gln Val
1635 1640 1645

Lys Ala Ser Leu Leu Pro Gly Gly Ser Glu Gly Gly Arg Arg Arg Arg
1650 1655 1660

Glu Leu Asp Pro Met Asp Val Arg Gly Ser Ile Val Tyr Leu Glu Ile
1665 1670 1675 1680

Asp Asn Arg Gln Cys Val Gln Ala Ser Ser Gln Cys Phe Gln Ser Ala
1685 1690 1695

Thr Asp Val Ala Ala Phe Leu Gly Ala Leu Ala Ser Leu Gly Ser Leu
1700 1705 1710

Asn Ile Pro Tyr Lys Ile Glu Ala Val Gln Ser Glu Thr Val Glu Pro
1715 1720 1725

Pro Pro Pro Ala Gln Leu His Phe Met Tyr Val Ala Ala Ala Ala Phe
1730 1735 1740

Val Leu Leu Phe Phe Val Gly Cys Gly Val Leu Leu Ser Arg Lys Arg
1745 1750 1755 1760

Arg Arg Gln His Gly Gln Leu Trp Phe Pro Glu Gly Phe Lys Val Ser
1765 1770 1775

Glu Ala Ser Lys Lys Lys Arg Arg Glu Pro Leu Gly Glu Asp Ser Val

1780 1785 1790

Gly Leu Lys Pro Leu Lys Asn Ala Ser Asp Gly Ala Leu Met Asp Asp
1795 1800 1805

Asn Gln Asn Glu Trp Gly Asp Glu Asp Leu Glu Thr Lys Lys Phe Arg
1810 1815 1820

Phe Glu Glu Pro Val Val Leu Pro Asp Leu Asp Asp Gln Thr Asp His
1825 1830 1835 1840

Arg Gln Trp Thr Gln Gln His Leu Asp Ala Ala Asp Leu Arg Met Ser
1845 1850 1855

Ala Met Ala Pro Thr Pro Pro Gln Gly Glu Val Asp Ala Asp Cys Met
1860 1865 1870

Asp Val Asn Val Arg Gly Pro Asp Gly Phe Thr Pro Leu Met Ile Ala
1875 1880 1885

Ser Cys Ser Gly Gly Gly Leu Glu Thr Gly Asn Ser Glu Glu Glu Glu
1890 1895 1900

Asp Ala Pro Ala Val Ile Ser Asp Phe Ile Tyr Gln Gly Ala Ser Leu
1905 1910 1915 1920

His Asn Gln Thr Asp Arg Thr Gly Glu Thr Ala Leu His Leu Ala Ala
1925 1930 1935

Arg Tyr Ser Arg Ser Asp Ala Ala Lys Arg Leu Leu Glu Ala Ser Ala
1940 1945 1950

Asp Ala Asn Ile Gln Asp Asn Met Gly Arg Thr Pro Leu His Ala Ala
1955 1960 1965

Val Ser Ala Asp Ala Gln Gly Val Phe Gln Ile Leu Ile Arg Asn Arg
1970 1975 1980

Ala Thr Asp Leu Asp Ala Arg Met His Asp Gly Thr Thr Pro Leu Ile
1985 1990 1995 2000

Leu Ala Ala Arg Leu Ala Val Glu Gly Met Leu Glu Asp Leu Ile Asn
2005 2010 2015

Ser His Ala Asp Val Asn Ala Val Asp Asp Leu Gly Lys Ser Ala Leu
2020 2025 2030

His Trp Ala Ala Ala Val Asn Asn Val Asp Ala Ala Val Val Leu Leu
2035 2040 2045

Lys Asn Gly Ala Asn Lys Asp Met Gln Asn Asn Arg Glu Glu Thr Pro
2050 2055 2060

Leu Phe Leu Ala Ala Arg Glu Gly Ser Tyr Glu Thr Ala Lys Val Leu
2065 2070 2075 2080

Leu Asp His Phe Ala Asn Arg Asp Ile Thr Asp His Met Asp Arg Leu
2085 2090 2095

Pro Arg Asp Ile Ala Gln Glu Arg Met His His Asp Ile Val Arg Leu
2100 2105 2110

Leu Asp Glu Tyr Asn Leu Val Arg Ser Pro Gln Leu His Gly Ala Pro

2115 2120 2125

Leu Gly Gly Thr Pro Thr Leu Ser Pro Pro Leu Cys Ser Pro Asn Gly
2130 2135 2140

Tyr Leu Gly Ser Leu Lys Pro Gly Val Gln Gly Lys Lys Val Arg Lys
2145 2150 2155 2160

Pro Ser Ser Lys Gly Leu Ala Cys Gly Ser Lys Glu Ala Lys Asp Leu
2165 2170 2175

Lys Ala Arg Arg Lys Lys Ser Gln Asp Gly Lys Gly Cys Leu Leu Asp
2180 2185 2190

Ser Ser Gly Met Leu Ser Pro Val Asp Ser Leu Glu Ser Pro His Gly
2195 2200 2205

Tyr Leu Ser Asp Val Ala Ser Pro Pro Leu Leu Pro Ser Pro Phe Gln
2210 2215 2220

Gln Ser Pro Ser Val Pro Leu Asn His Leu Pro Gly Met Pro Asp Thr
2225 2230 2235 2240

His Leu Gly Ile Gly His Leu Asn Val Ala Ala Lys Pro Glu Met Ala
2245 2250 2255

Ala Leu Gly Gly Gly Gly Arg Leu Ala Phe Glu Thr Gly Pro Pro Arg
2260 2265 2270

Leu Ser His Leu Pro Val Ala Ser Gly Thr Ser Thr Val Leu Gly Ser
2275 2280 2285

Ser Ser Gly Gly Ala Leu Asn Phe Thr Val Gly Gly Ser Thr Ser Leu
2290 2295 2300

Asn Gly Gln Cys Glu Trp Leu Ser Arg Leu Gln Ser Gly Met Val Pro
2305 2310 2315 2320

Asn Gln Tyr Asn Pro Leu Arg Gly Ser Val Ala Pro Gly Pro Leu Ser
2325 2330 2335

Thr Gln Ala Pro Ser Leu Gln His Gly Met Val Gly Pro Leu His Ser
2340 2345 2350

Ser Leu Ala Ala Ser Ala Leu Ser Gln Met Met Ser Tyr Gln Gly Leu
2355 2360 2365

Pro Ser Thr Arg Leu Ala Thr Gln Pro His Leu Val Gln Thr Gln Gln
2370 2375 2380

Val Gln Pro Gln Asn Leu Gln Met Gln Gln Gln Asn Leu Gln Pro Ala
2385 2390 2395 2400

Asn Ile Gln Gln Gln Gln Ser Leu Gln Pro Pro Pro Pro Pro Pro Gln
2405 2410 2415

Pro His Leu Gly Val Ser Ser Ala Ala Ser Gly His Leu Gly Arg Ser
2420 2425 2430

Phe Leu Ser Gly Glu Pro Ser Gln Ala Asp Val Gln Pro Leu Gly Pro
2435 2440 2445

Ser Ser Leu Ala Val His Thr Ile Leu Pro Gln Glu Ser Pro Ala Leu

```
        2450                2455                2460

Pro Thr Ser Leu Pro Ser Ser Leu Val Pro Pro Val Thr Ala Ala Gln
2465                2470                2475                2480

Phe Leu Thr Pro Pro Ser Gln His Ser Tyr Ser Ser Pro Val Asp Asn
                2485                2490                2495

Thr Pro Ser His Gln Leu Gln Val Pro Glu His Pro Phe Leu Thr Pro
            2500                2505                2510

Ser Pro Glu Ser Pro Asp Gln Trp Ser Ser Ser Ser Pro His Ser Asn
        2515                2520                2525

Val Ser Asp Trp Ser Glu Gly Val Ser Ser Pro Pro Thr Ser Met Gln
    2530                2535                2540

Ser Gln Ile Ala Arg Ile Pro Glu Ala Phe Lys
2545                2550                2555


<210>   111
<211>   2813
<212>   PRT
<213>   Artificial Sequence

<220>
<223>   VWF_HUMAN


<400>   111
Met Ile Pro Ala Arg Phe Ala Gly Val Leu Leu Ala Leu Ala Leu Ile
1               5                   10                  15

Leu Pro Gly Thr Leu Cys Ala Glu Gly Thr Arg Gly Arg Ser Ser Thr
            20                  25                  30

Ala Arg Cys Ser Leu Phe Gly Ser Asp Phe Val Asn Thr Phe Asp Gly
        35                  40                  45

Ser Met Tyr Ser Phe Ala Gly Tyr Cys Ser Tyr Leu Leu Ala Gly Gly
    50                  55                  60

Cys Gln Lys Arg Ser Phe Ser Ile Ile Gly Asp Phe Gln Asn Gly Lys
65                  70                  75                  80

Arg Val Ser Leu Ser Val Tyr Leu Gly Glu Phe Phe Asp Ile His Leu
                85                  90                  95

Phe Val Asn Gly Thr Val Thr Gln Gly Asp Gln Arg Val Ser Met Pro
            100                 105                 110

Tyr Ala Ser Lys Gly Leu Tyr Leu Glu Thr Glu Ala Gly Tyr Tyr Lys
        115                 120                 125

Leu Ser Gly Glu Ala Tyr Gly Phe Val Ala Arg Ile Asp Gly Ser Gly
    130                 135                 140

Asn Phe Gln Val Leu Leu Ser Asp Arg Tyr Phe Asn Lys Thr Cys Gly
145                 150                 155                 160

Leu Cys Gly Asn Phe Asn Ile Phe Ala Glu Asp Asp Phe Met Thr Gln
                165                 170                 175
```

```
Glu Gly Thr Leu Thr Ser Asp Pro Tyr Asp Phe Ala Asn Ser Trp Ala
            180                 185                 190

Leu Ser Ser Gly Glu Gln Trp Cys Glu Arg Ala Ser Pro Pro Ser Ser
        195                 200                 205

Ser Cys Asn Ile Ser Ser Gly Glu Met Gln Lys Gly Leu Trp Glu Gln
    210                 215                 220

Cys Gln Leu Leu Lys Ser Thr Ser Val Phe Ala Arg Cys His Pro Leu
225                 230                 235                 240

Val Asp Pro Glu Pro Phe Val Ala Leu Cys Glu Lys Thr Leu Cys Glu
                245                 250                 255

Cys Ala Gly Gly Leu Glu Cys Ala Cys Pro Ala Leu Leu Glu Tyr Ala
            260                 265                 270

Arg Thr Cys Ala Gln Glu Gly Met Val Leu Tyr Gly Trp Thr Asp His
        275                 280                 285

Ser Ala Cys Ser Pro Val Cys Pro Ala Gly Met Glu Tyr Arg Gln Cys
    290                 295                 300

Val Ser Pro Cys Ala Arg Thr Cys Gln Ser Leu His Ile Asn Glu Met
305                 310                 315                 320

Cys Gln Glu Arg Cys Val Asp Gly Cys Ser Cys Pro Glu Gly Gln Leu
                325                 330                 335

Leu Asp Glu Gly Leu Cys Val Glu Ser Thr Glu Cys Pro Cys Val His
            340                 345                 350

Ser Gly Lys Arg Tyr Pro Pro Gly Thr Ser Leu Ser Arg Asp Cys Asn
        355                 360                 365

Thr Cys Ile Cys Arg Asn Ser Gln Trp Ile Cys Ser Asn Glu Glu Cys
    370                 375                 380

Pro Gly Glu Cys Leu Val Thr Gly Gln Ser His Phe Lys Ser Phe Asp
385                 390                 395                 400

Asn Arg Tyr Phe Thr Phe Ser Gly Ile Cys Gln Tyr Leu Leu Ala Arg
                405                 410                 415

Asp Cys Gln Asp His Ser Phe Ser Ile Val Ile Glu Thr Val Gln Cys
            420                 425                 430

Ala Asp Asp Arg Asp Ala Val Cys Thr Arg Ser Val Thr Val Arg Leu
        435                 440                 445

Pro Gly Leu His Asn Ser Leu Val Lys Leu Lys His Gly Ala Gly Val
    450                 455                 460

Ala Met Asp Gly Gln Asp Val Gln Leu Pro Leu Leu Lys Gly Asp Leu
465                 470                 475                 480

Arg Ile Gln His Thr Val Thr Ala Ser Val Arg Leu Ser Tyr Gly Glu
                485                 490                 495

Asp Leu Gln Met Asp Trp Asp Gly Arg Gly Arg Leu Leu Val Lys Leu
            500                 505                 510
```

Ser Pro Val Tyr Ala Gly Lys Thr Cys Gly Leu Cys Gly Asn Tyr Asn
515 520 525

Gly Asn Gln Gly Asp Asp Phe Leu Thr Pro Ser Gly Leu Ala Glu Pro
530 535 540

Arg Val Glu Asp Phe Gly Asn Ala Trp Lys Leu His Gly Asp Cys Gln
545 550 555 560

Asp Leu Gln Lys Gln His Ser Asp Pro Cys Ala Leu Asn Pro Arg Met
565 570 575

Thr Arg Phe Ser Glu Glu Ala Cys Ala Val Leu Thr Ser Pro Thr Phe
580 585 590

Glu Ala Cys His Arg Ala Val Ser Pro Leu Pro Tyr Leu Arg Asn Cys
595 600 605

Arg Tyr Asp Val Cys Ser Cys Ser Asp Gly Arg Glu Cys Leu Cys Gly
610 615 620

Ala Leu Ala Ser Tyr Ala Ala Ala Cys Ala Gly Arg Gly Val Arg Val
625 630 635 640

Ala Trp Arg Glu Pro Gly Arg Cys Glu Leu Asn Cys Pro Lys Gly Gln
645 650 655

Val Tyr Leu Gln Cys Gly Thr Pro Cys Asn Leu Thr Cys Arg Ser Leu
660 665 670

Ser Tyr Pro Asp Glu Glu Cys Asn Glu Ala Cys Leu Glu Gly Cys Phe
675 680 685

Cys Pro Pro Gly Leu Tyr Met Asp Glu Arg Gly Asp Cys Val Pro Lys
690 695 700

Ala Gln Cys Pro Cys Tyr Tyr Asp Gly Glu Ile Phe Gln Pro Glu Asp
705 710 715 720

Ile Phe Ser Asp His His Thr Met Cys Tyr Cys Glu Asp Gly Phe Met
725 730 735

His Cys Thr Met Ser Gly Val Pro Gly Ser Leu Leu Pro Asp Ala Val
740 745 750

Leu Ser Ser Pro Leu Ser His Arg Ser Lys Arg Ser Leu Ser Cys Arg
755 760 765

Pro Pro Met Val Lys Leu Val Cys Pro Ala Asp Asn Leu Arg Ala Glu
770 775 780

Gly Leu Glu Cys Thr Lys Thr Cys Gln Asn Tyr Asp Leu Glu Cys Met
785 790 795 800

Ser Met Gly Cys Val Ser Gly Cys Leu Cys Pro Pro Gly Met Val Arg
805 810 815

His Glu Asn Arg Cys Val Ala Leu Glu Arg Cys Pro Cys Phe His Gln
820 825 830

Gly Lys Glu Tyr Ala Pro Gly Glu Thr Val Lys Ile Gly Cys Asn Thr
835 840 845

Cys Val Cys Gln Asp Arg Lys Trp Asn Cys Thr Asp His Val Cys Asp
    850                 855                 860

Ala Thr Cys Ser Thr Ile Gly Met Ala His Tyr Leu Thr Phe Asp Gly
865                 870                 875                 880

Leu Lys Tyr Leu Phe Pro Gly Glu Cys Gln Tyr Val Leu Val Gln Asp
                885                 890                 895

Tyr Cys Gly Ser Asn Pro Gly Thr Phe Arg Ile Leu Val Gly Asn Lys
            900                 905                 910

Gly Cys Ser His Pro Ser Val Lys Cys Lys Lys Arg Val Thr Ile Leu
        915                 920                 925

Val Glu Gly Gly Glu Ile Glu Leu Phe Asp Gly Glu Val Asn Val Lys
    930                 935                 940

Arg Pro Met Lys Asp Glu Thr His Phe Glu Val Val Glu Ser Gly Arg
945                 950                 955                 960

Tyr Ile Ile Leu Leu Leu Gly Lys Ala Leu Ser Val Val Trp Asp Arg
                965                 970                 975

His Leu Ser Ile Ser Val Val Leu Lys Gln Thr Tyr Gln Glu Lys Val
            980                 985                 990

Cys Gly Leu Cys Gly Asn Phe Asp Gly Ile Gln Asn Asn Asp Leu Thr
        995                 1000                1005

Ser Ser Asn Leu Gln Val Glu Glu Asp Pro Val Asp Phe Gly Asn Ser
    1010                1015                1020

Trp Lys Val Ser Ser Gln Cys Ala Asp Thr Arg Lys Val Pro Leu Asp
1025                1030                1035                1040

Ser Ser Pro Ala Thr Cys His Asn Asn Ile Met Lys Gln Thr Met Val
                1045                1050                1055

Asp Ser Ser Cys Arg Ile Leu Thr Ser Asp Val Phe Gln Asp Cys Asn
            1060                1065                1070

Lys Leu Val Asp Pro Glu Pro Tyr Leu Asp Val Cys Ile Tyr Asp Thr
        1075                1080                1085

Cys Ser Cys Glu Ser Ile Gly Asp Cys Ala Cys Phe Cys Asp Thr Ile
    1090                1095                1100

Ala Ala Tyr Ala His Val Cys Ala Gln His Gly Lys Val Val Thr Trp
1105                1110                1115                1120

Arg Thr Ala Thr Leu Cys Pro Gln Ser Cys Glu Glu Arg Asn Leu Arg
                1125                1130                1135

Glu Asn Gly Tyr Glu Cys Glu Trp Arg Tyr Asn Ser Cys Ala Pro Ala
            1140                1145                1150

Cys Gln Val Thr Cys Gln His Pro Glu Pro Leu Ala Cys Pro Val Gln
        1155                1160                1165

Cys Val Glu Gly Cys His Ala His Cys Pro Pro Gly Lys Ile Leu Asp
    1170                1175                1180

Glu Leu Leu Gln Thr Cys Val Asp Pro Glu Asp Cys Pro Val Cys Glu
1185 1190 1195 1200

Val Ala Gly Arg Arg Phe Ala Ser Gly Lys Lys Val Thr Leu Asn Pro
1205 1210 1215

Ser Asp Pro Glu His Cys Gln Ile Cys His Cys Asp Val Val Asn Leu
1220 1225 1230

Thr Cys Glu Ala Cys Gln Glu Pro Gly Gly Leu Val Val Pro Pro Thr
1235 1240 1245

Asp Ala Pro Val Ser Pro Thr Thr Leu Tyr Val Glu Asp Ile Ser Glu
1250 1255 1260

Pro Pro Leu His Asp Phe Tyr Cys Ser Arg Leu Leu Asp Leu Val Phe
1265 1270 1275 1280

Leu Leu Asp Gly Ser Ser Arg Leu Ser Glu Ala Glu Phe Glu Val Leu
1285 1290 1295

Lys Ala Phe Val Val Asp Met Met Glu Arg Leu Arg Ile Ser Gln Lys
1300 1305 1310

Trp Val Arg Val Ala Val Val Glu Tyr His Asp Gly Ser His Ala Tyr
1315 1320 1325

Ile Gly Leu Lys Asp Arg Lys Arg Pro Ser Glu Leu Arg Arg Ile Ala
1330 1335 1340

Ser Gln Val Lys Tyr Ala Gly Ser Gln Val Ala Ser Thr Ser Glu Val
1345 1350 1355 1360

Leu Lys Tyr Thr Leu Phe Gln Ile Phe Ser Lys Ile Asp Arg Pro Glu
1365 1370 1375

Ala Ser Arg Ile Thr Leu Leu Leu Met Ala Ser Gln Glu Pro Gln Arg
1380 1385 1390

Met Ser Arg Asn Phe Val Arg Tyr Val Gln Gly Leu Lys Lys Lys Lys
1395 1400 1405

Val Ile Val Ile Pro Val Gly Ile Gly Pro His Ala Asn Leu Lys Gln
1410 1415 1420

Ile Arg Leu Ile Glu Lys Gln Ala Pro Glu Asn Lys Ala Phe Val Leu
1425 1430 1435 1440

Ser Ser Val Asp Glu Leu Glu Gln Gln Arg Asp Glu Ile Val Ser Tyr
1445 1450 1455

Leu Cys Asp Leu Ala Pro Glu Ala Pro Pro Pro Thr Leu Pro Pro Asp
1460 1465 1470

Met Ala Gln Val Thr Val Gly Pro Gly Leu Leu Gly Val Ser Thr Leu
1475 1480 1485

Gly Pro Lys Arg Asn Ser Met Val Leu Asp Val Ala Phe Val Leu Glu
1490 1495 1500

Gly Ser Asp Lys Ile Gly Glu Ala Asp Phe Asn Arg Ser Lys Glu Phe
1505 1510 1515 1520

Met Glu Glu Val Ile Gln Arg Met Asp Val Gly Gln Asp Ser Ile His
1525 1530 1535

Val Thr Val Leu Gln Tyr Ser Tyr Met Val Thr Val Glu Tyr Pro Phe
1540 1545 1550

Ser Glu Ala Gln Ser Lys Gly Asp Ile Leu Gln Arg Val Arg Glu Ile
1555 1560 1565

Arg Tyr Gln Gly Gly Asn Arg Thr Asn Thr Gly Leu Ala Leu Arg Tyr
1570 1575 1580

Leu Ser Asp His Ser Phe Leu Val Ser Gln Gly Asp Arg Glu Gln Ala
1585 1590 1595 1600

Pro Asn Leu Val Tyr Met Val Thr Gly Asn Pro Ala Ser Asp Glu Ile
1605 1610 1615

Lys Arg Leu Pro Gly Asp Ile Gln Val Val Pro Ile Gly Val Gly Pro
1620 1625 1630

Asn Ala Asn Val Gln Glu Leu Glu Arg Ile Gly Trp Pro Asn Ala Pro
1635 1640 1645

Ile Leu Ile Gln Asp Phe Glu Thr Leu Pro Arg Glu Ala Pro Asp Leu
1650 1655 1660

Val Leu Gln Arg Cys Cys Ser Gly Glu Gly Leu Gln Ile Pro Thr Leu
1665 1670 1675 1680

Ser Pro Ala Pro Asp Cys Ser Gln Pro Leu Asp Val Ile Leu Leu Leu
1685 1690 1695

Asp Gly Ser Ser Ser Phe Pro Ala Ser Tyr Phe Asp Glu Met Lys Ser
1700 1705 1710

Phe Ala Lys Ala Phe Ile Ser Lys Ala Asn Ile Gly Pro Arg Leu Thr
1715 1720 1725

Gln Val Ser Val Leu Gln Tyr Gly Ser Ile Thr Thr Ile Asp Val Pro
1730 1735 1740

Trp Asn Val Val Pro Glu Lys Ala His Leu Leu Ser Leu Val Asp Val
1745 1750 1755 1760

Met Gln Arg Glu Gly Gly Pro Ser Gln Ile Gly Asp Ala Leu Gly Phe
1765 1770 1775

Ala Val Arg Tyr Leu Thr Ser Glu Met His Gly Ala Arg Pro Gly Ala
1780 1785 1790

Ser Lys Ala Val Val Ile Leu Val Thr Asp Val Ser Val Asp Ser Val
1795 1800 1805

Asp Ala Ala Ala Asp Ala Ala Arg Ser Asn Arg Val Thr Val Phe Pro
1810 1815 1820

Ile Gly Ile Gly Asp Arg Tyr Asp Ala Ala Gln Leu Arg Ile Leu Ala
1825 1830 1835 1840

Gly Pro Ala Gly Asp Ser Asn Val Val Lys Leu Gln Arg Ile Glu Asp
1845 1850 1855

Leu Pro Thr Met Val Thr Leu Gly Asn Ser Phe Leu His Lys Leu Cys
1860 1865 1870

Ser Gly Phe Val Arg Ile Cys Met Asp Glu Asp Gly Asn Glu Lys Arg
1875 1880 1885

Pro Gly Asp Val Trp Thr Leu Pro Asp Gln Cys His Thr Val Thr Cys
1890 1895 1900

Gln Pro Asp Gly Gln Thr Leu Leu Lys Ser His Arg Val Asn Cys Asp
1905 1910 1915 1920

Arg Gly Leu Arg Pro Ser Cys Pro Asn Ser Gln Ser Pro Val Lys Val
1925 1930 1935

Glu Glu Thr Cys Gly Cys Arg Trp Thr Cys Pro Cys Val Cys Thr Gly
1940 1945 1950

Ser Ser Thr Arg His Ile Val Thr Phe Asp Gly Gln Asn Phe Lys Leu
1955 1960 1965

Thr Gly Ser Cys Ser Tyr Val Leu Phe Gln Asn Lys Glu Gln Asp Leu
1970 1975 1980

Glu Val Ile Leu His Asn Gly Ala Cys Ser Pro Gly Ala Arg Gln Gly
1985 1990 1995 2000

Cys Met Lys Ser Ile Glu Val Lys His Ser Ala Leu Ser Val Glu Leu
2005 2010 2015

His Ser Asp Met Glu Val Thr Val Asn Gly Arg Leu Val Ser Val Pro
2020 2025 2030

Tyr Val Gly Gly Asn Met Glu Val Asn Val Tyr Gly Ala Ile Met His
2035 2040 2045

Glu Val Arg Phe Asn His Leu Gly His Ile Phe Thr Phe Thr Pro Gln
2050 2055 2060

Asn Asn Glu Phe Gln Leu Gln Leu Ser Pro Lys Thr Phe Ala Ser Lys
2065 2070 2075 2080

Thr Tyr Gly Leu Cys Gly Ile Cys Asp Glu Asn Gly Ala Asn Asp Phe
2085 2090 2095

Met Leu Arg Asp Gly Thr Val Thr Thr Asp Trp Lys Thr Leu Val Gln
2100 2105 2110

Glu Trp Thr Val Gln Arg Pro Gly Gln Thr Cys Gln Pro Ile Leu Glu
2115 2120 2125

Glu Gln Cys Leu Val Pro Asp Ser Ser His Cys Gln Val Leu Leu Leu
2130 2135 2140

Pro Leu Phe Ala Glu Cys His Lys Val Leu Ala Pro Ala Thr Phe Tyr
2145 2150 2155 2160

Ala Ile Cys Gln Gln Asp Ser Cys His Gln Glu Gln Val Cys Glu Val
2165 2170 2175

Ile Ala Ser Tyr Ala His Leu Cys Arg Thr Asn Gly Val Cys Val Asp
2180 2185 2190

Trp Arg Thr Pro Asp Phe Cys Ala Met Ser Cys Pro Pro Ser Leu Val
2195 2200 2205

Tyr Asn His Cys Glu His Gly Cys Pro Arg His Cys Asp Gly Asn Val
2210 2215 2220

Ser Ser Cys Gly Asp His Pro Ser Glu Gly Cys Phe Cys Pro Pro Asp
2225 2230 2235 2240

Lys Val Met Leu Glu Gly Ser Cys Val Pro Glu Glu Ala Cys Thr Gln
2245 2250 2255

Cys Ile Gly Glu Asp Gly Val Gln His Gln Phe Leu Glu Ala Trp Val
2260 2265 2270

Pro Asp His Gln Pro Cys Gln Ile Cys Thr Cys Leu Ser Gly Arg Lys
2275 2280 2285

Val Asn Cys Thr Thr Gln Pro Cys Pro Thr Ala Lys Ala Pro Thr Cys
2290 2295 2300

Gly Leu Cys Glu Val Ala Arg Leu Arg Gln Asn Ala Asp Gln Cys Cys
2305 2310 2315 2320

Pro Glu Tyr Glu Cys Val Cys Asp Pro Val Ser Cys Asp Leu Pro Pro
2325 2330 2335

Val Pro His Cys Glu Arg Gly Leu Gln Pro Thr Leu Thr Asn Pro Gly
2340 2345 2350

Glu Cys Arg Pro Asn Phe Thr Cys Ala Cys Arg Lys Glu Glu Cys Lys
2355 2360 2365

Arg Val Ser Pro Pro Ser Cys Pro Pro His Arg Leu Pro Thr Leu Arg
2370 2375 2380

Lys Thr Gln Cys Cys Asp Glu Tyr Glu Cys Ala Cys Asn Cys Val Asn
2385 2390 2395 2400

Ser Thr Val Ser Cys Pro Leu Gly Tyr Leu Ala Ser Thr Ala Thr Asn
2405 2410 2415

Asp Cys Gly Cys Thr Thr Thr Thr Cys Leu Pro Asp Lys Val Cys Val
2420 2425 2430

His Arg Ser Thr Ile Tyr Pro Val Gly Gln Phe Trp Glu Glu Gly Cys
2435 2440 2445

Asp Val Cys Thr Cys Thr Asp Met Glu Asp Ala Val Met Gly Leu Arg
2450 2455 2460

Val Ala Gln Cys Ser Gln Lys Pro Cys Glu Asp Ser Cys Arg Ser Gly
2465 2470 2475 2480

Phe Thr Tyr Val Leu His Glu Gly Glu Cys Cys Gly Arg Cys Leu Pro
2485 2490 2495

Ser Ala Cys Glu Val Val Thr Gly Ser Pro Arg Gly Asp Ser Gln Ser
2500 2505 2510

Ser Trp Lys Ser Val Gly Ser Gln Trp Ala Ser Pro Glu Asn Pro Cys
2515 2520 2525

Leu Ile Asn Glu Cys Val Arg Val Lys Glu Glu Val Phe Ile Gln Gln
2530 2535 2540

Arg Asn Val Ser Cys Pro Gln Leu Glu Val Pro Val Cys Pro Ser Gly
2545 2550 2555 2560

Phe Gln Leu Ser Cys Lys Thr Ser Ala Cys Cys Pro Ser Cys Arg Cys
2565 2570 2575

Glu Arg Met Glu Ala Cys Met Leu Asn Gly Thr Val Ile Gly Pro Gly
2580 2585 2590

Lys Thr Val Met Ile Asp Val Cys Thr Thr Cys Arg Cys Met Val Gln
2595 2600 2605

Val Gly Val Ile Ser Gly Phe Lys Leu Glu Cys Arg Lys Thr Thr Cys
2610 2615 2620

Asn Pro Cys Pro Leu Gly Tyr Lys Glu Glu Asn Asn Thr Gly Glu Cys
2625 2630 2635 2640

Cys Gly Arg Cys Leu Pro Thr Ala Cys Thr Ile Gln Leu Arg Gly Gly
2645 2650 2655

Gln Ile Met Thr Leu Lys Arg Asp Glu Thr Leu Gln Asp Gly Cys Asp
2660 2665 2670

Thr His Phe Cys Lys Val Asn Glu Arg Gly Glu Tyr Phe Trp Glu Lys
2675 2680 2685

Arg Val Thr Gly Cys Pro Pro Phe Asp Glu His Lys Cys Leu Ala Glu
2690 2695 2700

Gly Gly Lys Ile Met Lys Ile Pro Gly Thr Cys Cys Asp Thr Cys Glu
2705 2710 2715 2720

Glu Pro Glu Cys Asn Asp Ile Thr Ala Arg Leu Gln Tyr Val Lys Val
2725 2730 2735

Gly Ser Cys Lys Ser Glu Val Glu Val Asp Ile His Tyr Cys Gln Gly
2740 2745 2750

Lys Cys Ala Ser Lys Ala Met Tyr Ser Ile Asp Ile Asn Asp Val Gln
2755 2760 2765

Asp Gln Cys Ser Cys Cys Ser Pro Thr Arg Thr Glu Pro Met Gln Val
2770 2775 2780

Ala Leu His Cys Thr Asn Gly Ser Val Val Tyr His Glu Val Leu Asn
2785 2790 2795 2800

Ala Met Glu Cys Lys Cys Ser Pro Arg Lys Cys Ser Lys
2805 2810

<210> 112
<211> 3926
<212> PRT
<213> Artificial Sequence

<220>
<223> BSN_HUMAN

<400> 112

Met Gly Asn Glu Val Ser Leu Glu Gly Gly Ala Gly Asp Gly Pro Leu
1 5 10 15

Pro Pro Gly Gly Ala Gly Pro Gly Pro Gly Pro Gly Pro Gly Pro Gly
20 25 30

Ala Gly Lys Pro Pro Ser Ala Pro Ala Gly Gly Gly Gln Leu Pro Ala
35 40 45

Ala Gly Ala Ala Arg Ser Thr Ala Val Pro Pro Val Pro Gly Pro Gly
50 55 60

Pro Gly Pro Gly Pro Gly Pro Gly Pro Gly Ser Thr Ser Arg Arg Leu
65 70 75 80

Asp Pro Lys Glu Pro Leu Gly Asn Gln Arg Ala Ala Ser Pro Thr Pro
85 90 95

Lys Gln Ala Ser Ala Thr Thr Pro Gly His Glu Ser Pro Arg Glu Thr
100 105 110

Arg Ala Gln Gly Pro Ala Gly Gln Glu Ala Asp Gly Pro Arg Arg Thr
115 120 125

Leu Gln Val Asp Ser Arg Thr Gln Arg Ser Gly Arg Ser Pro Ser Val
130 135 140

Ser Pro Asp Arg Gly Ser Thr Pro Thr Ser Pro Tyr Ser Val Pro Gln
145 150 155 160

Ile Ala Pro Leu Pro Ser Ser Thr Leu Cys Pro Ile Cys Lys Thr Ser
165 170 175

Asp Leu Thr Ser Thr Pro Ser Gln Pro Asn Phe Asn Thr Cys Thr Gln
180 185 190

Cys His Asn Lys Val Cys Asn Gln Cys Gly Phe Asn Pro Asn Pro His
195 200 205

Leu Thr Gln Val Lys Glu Trp Leu Cys Leu Asn Cys Gln Met Gln Arg
210 215 220

Ala Leu Gly Met Asp Met Thr Thr Ala Pro Arg Ser Lys Ser Gln Gln
225 230 235 240

Gln Leu His Ser Pro Ala Leu Ser Pro Ala His Ser Pro Ala Lys Gln
245 250 255

Pro Leu Gly Lys Pro Asp Gln Glu Arg Ser Arg Gly Pro Gly Gly Pro
260 265 270

Gln Pro Gly Ser Arg Gln Ala Glu Thr Ala Arg Ala Thr Ser Val Pro
275 280 285

Gly Pro Ala Gln Ala Ala Ala Pro Pro Glu Val Gly Arg Val Ser Pro
290 295 300

Gln Pro Pro Gln Pro Thr Lys Pro Ser Thr Ala Glu Pro Arg Pro Pro
305 310 315 320

```
Ala Gly Glu Ala Pro Ala Lys Ser Ala Thr Ala Val Pro Ala Gly Leu
                325                 330                 335

Gly Ala Thr Glu Gln Thr Gln Glu Gly Leu Thr Gly Lys Leu Phe Gly
            340                 345                 350

Leu Gly Ala Ser Leu Leu Thr Gln Ala Ser Thr Leu Met Ser Val Gln
        355                 360                 365

Pro Glu Ala Asp Thr Gln Gly Gln Pro Ala Pro Ser Lys Gly Thr Pro
    370                 375                 380

Lys Ile Val Phe Asn Asp Ala Ser Lys Glu Ala Gly Pro Lys Pro Leu
385                 390                 395                 400

Gly Ser Gly Pro Gly Pro Gly Pro Ala Pro Gly Ala Lys Thr Glu Pro
                405                 410                 415

Gly Ala Arg Met Gly Pro Gly Ser Gly Pro Gly Ala Leu Pro Lys Thr
            420                 425                 430

Gly Gly Thr Thr Ser Pro Lys His Gly Arg Ala Glu His Gln Ala Ala
        435                 440                 445

Ser Lys Ala Ala Ala Lys Pro Lys Thr Met Pro Lys Glu Arg Ala Ile
    450                 455                 460

Cys Pro Leu Cys Gln Ala Glu Leu Asn Val Gly Ser Lys Ser Pro Ala
465                 470                 475                 480

Asn Tyr Asn Thr Cys Thr Thr Cys Arg Leu Gln Val Cys Asn Leu Cys
                485                 490                 495

Gly Phe Asn Pro Thr Pro His Leu Val Glu Lys Thr Glu Trp Leu Cys
            500                 505                 510

Leu Asn Cys Gln Thr Lys Arg Leu Leu Glu Gly Ser Leu Gly Glu Pro
        515                 520                 525

Thr Pro Leu Pro Pro Pro Thr Ser Gln Gln Pro Pro Val Gly Ala Pro
    530                 535                 540

His Arg Ala Ser Gly Thr Ser Pro Leu Lys Gln Lys Gly Pro Gln Gly
545                 550                 555                 560

Leu Gly Gln Pro Ser Gly Pro Leu Pro Ala Lys Ala Ser Pro Leu Ser
                565                 570                 575

Thr Lys Ala Ser Pro Leu Pro Ser Lys Ala Ser Pro Gln Ala Lys Pro
            580                 585                 590

Leu Arg Ala Ser Glu Pro Ser Lys Thr Pro Ser Ser Val Gln Glu Lys
        595                 600                 605

Lys Thr Arg Val Pro Thr Lys Ala Glu Pro Met Pro Lys Pro Pro Pro
    610                 615                 620

Glu Thr Thr Pro Thr Pro Ala Thr Pro Lys Val Lys Ser Gly Val Arg
625                 630                 635                 640

Arg Ala Glu Pro Ala Thr Pro Val Val Lys Ala Val Pro Glu Ala Pro
                645                 650                 655
```

Lys Gly Gly Glu Ala Glu Asp Leu Val Gly Lys Pro Tyr Ser Gln Asp
660 665 670

Ala Ser Arg Ser Pro Gln Ser Leu Ser Asp Thr Gly Tyr Ser Ser Asp
675 680 685

Gly Ile Ser Ser Ser Gln Ser Glu Ile Thr Gly Val Val Gln Gln Glu
690 695 700

Val Glu Gln Leu Asp Ser Ala Gly Val Thr Gly Pro His Pro Pro Ser
705 710 715 720

Pro Ser Glu Ile His Lys Val Gly Ser Ser Met Arg Pro Leu Leu Gln
725 730 735

Ala Gln Gly Leu Ala Pro Ser Glu Arg Ser Lys Pro Leu Ser Ser Gly
740 745 750

Thr Gly Glu Glu Gln Lys Gln Arg Pro His Ser Leu Ser Ile Thr Pro
755 760 765

Glu Ala Phe Asp Ser Asp Glu Glu Leu Glu Asp Ile Leu Glu Glu Asp
770 775 780

Glu Asp Ser Ala Glu Trp Arg Arg Arg Arg Glu Gln Gln Asp Thr Ala
785 790 795 800

Glu Ser Ser Asp Asp Phe Gly Ser Gln Leu Arg His Asp Tyr Val Glu
805 810 815

Asp Ser Ser Glu Gly Gly Leu Ser Pro Leu Pro Pro Gln Pro Pro Ala
820 825 830

Arg Ala Ala Glu Leu Thr Asp Glu Asp Phe Met Arg Arg Gln Ile Leu
835 840 845

Glu Met Ser Ala Glu Glu Asp Asn Leu Glu Glu Asp Asp Thr Ala Thr
850 855 860

Ser Gly Arg Gly Leu Ala Lys His Gly Thr Gln Lys Gly Gly Pro Arg
865 870 875 880

Pro Arg Pro Glu Pro Ser Gln Glu Pro Ala Ala Leu Pro Lys Arg Arg
885 890 895

Leu Pro His Asn Ala Thr Thr Gly Tyr Glu Glu Leu Leu Pro Glu Gly
900 905 910

Gly Ser Ala Glu Ala Thr Asp Gly Ser Gly Thr Leu Gln Gly Gly Leu
915 920 925

Arg Arg Phe Lys Thr Ile Glu Leu Asn Ser Thr Gly Ser Tyr Gly His
930 935 940

Glu Leu Asp Leu Gly Gln Gly Pro Asp Pro Ser Leu Asp Arg Glu Pro
945 950 955 960

Glu Leu Glu Met Glu Ser Leu Thr Gly Ser Pro Glu Asp Arg Ser Arg
965 970 975

Gly Glu His Ser Ser Thr Leu Pro Ala Ser Thr Pro Ser Tyr Thr Ser
980 985 990

Gly Thr Ser Pro Thr Ser Leu Ser Ser Leu Glu Glu Asp Ser Asp Ser
995 1000 1005

Ser Pro Ser Arg Arg Gln Arg Leu Glu Glu Ala Lys Gln Gln Arg Lys
1010 1015 1020

Ala Arg His Arg Ser His Gly Pro Leu Leu Pro Thr Ile Glu Asp Ser
1025 1030 1035 1040

Ser Glu Glu Glu Glu Leu Arg Glu Glu Glu Glu Leu Leu Arg Glu Gln
1045 1050 1055

Glu Lys Met Arg Glu Val Glu Gln Gln Arg Ile Arg Ser Thr Ala Arg
1060 1065 1070

Lys Thr Arg Arg Asp Lys Glu Glu Leu Arg Ala Gln Arg Arg Arg Glu
1075 1080 1085

Arg Ser Lys Thr Pro Pro Ser Asn Leu Ser Pro Ile Glu Asp Ala Ser
1090 1095 1100

Pro Thr Glu Glu Leu Arg Gln Ala Ala Glu Met Glu Glu Leu His Arg
1105 1110 1115 1120

Ser Ser Cys Ser Glu Tyr Ser Pro Ser Pro Ser Leu Asp Ser Glu Ala
1125 1130 1135

Glu Ala Leu Asp Gly Gly Pro Ser Arg Leu Tyr Lys Ser Gly Ser Glu
1140 1145 1150

Tyr Asn Leu Pro Thr Phe Met Ser Leu Tyr Ser Pro Thr Glu Thr Pro
1155 1160 1165

Ser Gly Ser Ser Thr Thr Pro Ser Ser Gly Arg Pro Leu Lys Ser Ala
1170 1175 1180

Glu Glu Ala Tyr Glu Glu Met Met Arg Lys Ala Glu Leu Leu Gln Arg
1185 1190 1195 1200

Gln Gln Gly Gln Ala Ala Gly Ala Arg Gly Pro His Gly Gly Pro Ser
1205 1210 1215

Gln Pro Thr Gly Pro Arg Gly Leu Gly Ser Phe Glu Tyr Gln Asp Thr
1220 1225 1230

Thr Asp Arg Glu Tyr Gly Gln Ala Ala Gln Pro Ala Ala Glu Gly Thr
1235 1240 1245

Pro Ala Ser Leu Gly Ala Ala Val Tyr Glu Glu Ile Leu Gln Thr Ser
1250 1255 1260

Gln Ser Ile Val Arg Met Arg Gln Ala Ser Ser Arg Asp Leu Ala Phe
1265 1270 1275 1280

Ala Glu Asp Lys Lys Lys Glu Lys Gln Phe Leu Asn Ala Glu Ser Ala
1285 1290 1295

Tyr Met Asp Pro Met Lys Gln Asn Gly Gly Pro Leu Thr Pro Gly Thr
1300 1305 1310

Ser Pro Thr Gln Leu Ala Ala Pro Val Ser Phe Ser Thr Pro Thr Ser
1315 1320 1325

Ser Asp Ser Ser Gly Gly Arg Val Ile Pro Asp Val Arg Val Thr Gln
1330 1335 1340

His Phe Ala Lys Glu Thr Gln Asp Pro Leu Lys Leu His Ser Ser Pro
1345 1350 1355 1360

Ala Ser Pro Ser Ser Ala Ser Lys Glu Ile Gly Met Pro Phe Ser Gln
1365 1370 1375

Gly Pro Gly Thr Pro Ala Thr Thr Ala Val Ala Pro Cys Pro Ala Gly
1380 1385 1390

Leu Pro Arg Gly Tyr Met Thr Pro Ala Ser Pro Ala Gly Ser Glu Arg
1395 1400 1405

Ser Pro Ser Pro Ser Ser Thr Ala His Ser Tyr Gly His Ser Pro Thr
1410 1415 1420

Thr Ala Asn Tyr Gly Ser Gln Thr Glu Asp Leu Pro Gln Ala Pro Ser
1425 1430 1435 1440

Gly Leu Ala Ala Ala Gly Arg Ala Ala Arg Glu Lys Pro Leu Ser Ala
1445 1450 1455

Ser Asp Gly Glu Gly Gly Thr Pro Gln Pro Ser Arg Ala Tyr Ser Tyr
1460 1465 1470

Phe Ala Ser Ser Ser Pro Pro Leu Ser Pro Ser Ser Pro Ser Glu Ser
1475 1480 1485

Pro Thr Phe Ser Pro Gly Lys Met Gly Pro Arg Ala Thr Ala Glu Phe
1490 1495 1500

Ser Thr Gln Thr Pro Ser Pro Ala Pro Ala Ser Asp Met Pro Arg Ser
1505 1510 1515 1520

Pro Gly Ala Pro Thr Pro Ser Pro Met Val Ala Gln Gly Thr Gln Thr
1525 1530 1535

Pro His Arg Pro Ser Thr Pro Arg Leu Val Trp Gln Glu Ser Ser Gln
1540 1545 1550

Glu Ala Pro Phe Met Val Ile Thr Leu Ala Ser Asp Ala Ser Ser Gln
1555 1560 1565

Thr Arg Met Val His Ala Ser Ala Ser Thr Ser Pro Leu Cys Ser Pro
1570 1575 1580

Thr Glu Thr Gln Pro Thr Thr His Gly Tyr Ser Gln Thr Thr Pro Pro
1585 1590 1595 1600

Ser Val Ser Gln Leu Pro Pro Glu Pro Pro Gly Pro Pro Gly Phe Pro
1605 1610 1615

Arg Val Pro Ser Ala Gly Ala Asp Gly Pro Leu Ala Leu Tyr Gly Trp
1620 1625 1630

Gly Ala Leu Pro Ala Glu Asn Ile Ser Leu Cys Arg Ile Ser Ser Val
1635 1640 1645

Pro Gly Thr Ser Arg Val Glu Pro Gly Pro Arg Thr Pro Gly Thr Ala
1650 1655 1660

Val Val Asp Leu Arg Thr Ala Val Lys Pro Thr Pro Ile Ile Leu Thr
1665 1670 1675 1680

Asp Gln Gly Met Asp Leu Thr Ser Leu Ala Val Glu Ala Arg Lys Tyr
1685 1690 1695

Gly Leu Ala Leu Asp Pro Ile Pro Gly Arg Gln Ser Thr Ala Val Gln
1700 1705 1710

Pro Leu Val Ile Asn Leu Asn Ala Gln Glu His Thr Phe Leu Ala Thr
1715 1720 1725

Ala Thr Thr Val Ser Ile Thr Met Ala Ser Ser Val Phe Met Ala Gln
1730 1735 1740

Gln Lys Gln Pro Val Val Tyr Gly Asp Pro Tyr Gln Ser Arg Leu Asp
1745 1750 1755 1760

Phe Gly Gln Gly Gly Gly Ser Pro Val Cys Leu Ala Gln Val Lys Gln
1765 1770 1775

Val Glu Gln Ala Val Gln Thr Ala Pro Tyr Arg Ser Gly Pro Arg Gly
1780 1785 1790

Arg Pro Arg Glu Ala Lys Phe Ala Arg Tyr Asn Leu Pro Asn Gln Val
1795 1800 1805

Ala Pro Leu Ala Arg Arg Asp Val Leu Ile Thr Gln Met Gly Thr Ala
1810 1815 1820

Gln Ser Ile Gly Leu Lys Pro Gly Pro Val Pro Glu Pro Gly Ala Glu
1825 1830 1835 1840

Pro His Arg Ala Thr Pro Ala Glu Leu Arg Ser His Ala Leu Pro Gly
1845 1850 1855

Ala Arg Lys Pro His Thr Val Val Val Gln Met Gly Glu Gly Thr Ala
1860 1865 1870

Gly Thr Val Thr Thr Leu Leu Pro Glu Glu Pro Ala Gly Ala Leu Asp
1875 1880 1885

Leu Thr Gly Met Arg Pro Glu Ser Gln Leu Ala Cys Cys Asp Met Val
1890 1895 1900

Tyr Lys Leu Pro Phe Gly Ser Ser Cys Thr Gly Thr Phe His Pro Ala
1905 1910 1915 1920

Pro Ser Val Pro Glu Lys Ser Met Ala Asp Ala Ala Pro Pro Gly Gln
1925 1930 1935

Ser Ser Ser Pro Phe Tyr Gly Pro Arg Asp Pro Glu Pro Pro Glu Pro
1940 1945 1950

Pro Thr Tyr Arg Ala Gln Gly Val Val Gly Pro Gly Pro His Glu Glu
1955 1960 1965

Gln Arg Pro Tyr Pro Gln Gly Leu Pro Gly Arg Leu Tyr Ser Ser Met
1970 1975 1980

Ser Asp Thr Asn Leu Ala Glu Ala Gly Leu Asn Tyr His Ala Gln Arg
1985 1990 1995 2000

Ile Gly Gln Leu Phe Gln Gly Pro Gly Arg Asp Ser Ala Met Asp Leu
2005 2010 2015

Ser Ser Leu Lys His Ser Tyr Ser Leu Gly Phe Ala Asp Gly Arg Tyr
2020 2025 2030

Leu Gly Gln Gly Leu Gln Tyr Gly Ser Val Thr Asp Leu Arg His Pro
2035 2040 2045

Thr Asp Leu Leu Ala His Pro Leu Pro Met Arg Arg Tyr Ser Ser Val
2050 2055 2060

Ser Asn Ile Tyr Ser Asp His Arg Tyr Gly Pro Arg Gly Asp Ala Val
2065 2070 2075 2080

Gly Phe Gln Glu Ala Ser Leu Ala Gln Tyr Ser Ala Thr Thr Ala Arg
2085 2090 2095

Glu Ile Ser Arg Met Cys Ala Ala Leu Asn Ser Met Asp Gln Tyr Gly
2100 2105 2110

Gly Arg His Gly Ser Gly Gly Gly Gly Pro Asp Leu Val Gln Tyr Gln
2115 2120 2125

Pro Gln His Gly Pro Gly Leu Ser Ala Pro Gln Ser Leu Val Pro Leu
2130 2135 2140

Arg Pro Gly Leu Leu Gly Asn Pro Thr Phe Pro Glu Gly His Pro Ser
2145 2150 2155 2160

Pro Gly Asn Leu Ala Gln Tyr Gly Pro Ala Ala Gly Gln Gly Thr Ala
2165 2170 2175

Val Arg Gln Leu Leu Pro Ser Thr Ala Thr Val Arg Ala Ala Asp Gly
2180 2185 2190

Met Ile Tyr Ser Thr Ile Asn Thr Pro Ile Ala Ala Thr Leu Pro Ile
2195 2200 2205

Thr Thr Gln Pro Ala Ser Val Leu Arg Pro Met Val Arg Gly Gly Met
2210 2215 2220

Tyr Arg Pro Tyr Ala Ser Gly Gly Ile Thr Ala Val Pro Leu Thr Ser
2225 2230 2235 2240

Leu Thr Arg Val Pro Met Ile Ala Pro Arg Val Pro Leu Gly Pro Thr
2245 2250 2255

Gly Leu Tyr Arg Tyr Pro Ala Pro Ser Arg Phe Pro Ile Ala Ser Ser
2260 2265 2270

Val Pro Pro Ala Glu Gly Pro Val Tyr Leu Gly Lys Pro Ala Ala Ala
2275 2280 2285

Lys Ala Pro Gly Ala Gly Gly Pro Ser Arg Pro Glu Met Pro Val Gly
2290 2295 2300

Ala Ala Arg Glu Glu Pro Leu Pro Thr Thr Thr Pro Ala Ala Ile Lys
2305 2310 2315 2320

Glu Ala Ala Gly Ala Pro Ala Pro Ala Pro Leu Ala Gly Gln Lys Pro
2325 2330 2335

Pro Ala Asp Ala Ala Pro Gly Gly Gly Ser Gly Ala Leu Ser Arg Pro
2340 2345 2350

Gly Phe Glu Lys Glu Glu Ala Ser Gln Glu Glu Arg Gln Arg Lys Gln
2355 2360 2365

Gln Glu Gln Leu Leu Gln Leu Glu Arg Glu Arg Val Glu Leu Glu Lys
2370 2375 2380

Leu Arg Gln Leu Arg Leu Gln Glu Glu Leu Glu Arg Glu Arg Val Glu
2385 2390 2395 2400

Leu Gln Arg His Arg Glu Glu Glu Gln Leu Leu Val Gln Arg Glu Leu
2405 2410 2415

Gln Glu Leu Gln Thr Ile Lys His His Val Leu Gln Gln Gln Gln Glu
2420 2425 2430

Glu Arg Gln Ala Gln Phe Ala Leu Gln Arg Glu Gln Leu Ala Gln Gln
2435 2440 2445

Arg Leu Gln Leu Glu Gln Ile Gln Gln Leu Gln Gln Gln Leu Gln Gln
2450 2455 2460

Gln Leu Glu Glu Gln Lys Gln Arg Gln Lys Ala Pro Phe Pro Ala Ala
2465 2470 2475 2480

Cys Glu Ala Pro Gly Arg Gly Pro Pro Leu Ala Ala Ala Glu Leu Ala
2485 2490 2495

Gln Asn Gly Gln Tyr Trp Pro Pro Leu Thr His Ala Ala Phe Ile Ala
2500 2505 2510

Met Ala Gly Pro Glu Gly Leu Gly Gln Pro Arg Glu Pro Val Leu His
2515 2520 2525

Arg Gly Leu Pro Ser Ser Ala Ser Asp Met Ser Leu Gln Thr Glu Glu
2530 2535 2540

Gln Trp Glu Ala Ser Arg Ser Gly Ile Lys Lys Arg His Ser Met Pro
2545 2550 2555 2560

Arg Leu Arg Asp Ala Cys Glu Leu Glu Ser Gly Thr Glu Pro Cys Val
2565 2570 2575

Val Arg Arg Ile Ala Asp Ser Ser Val Gln Thr Asp Asp Glu Asp Gly
2580 2585 2590

Glu Ser Arg Tyr Leu Leu Ser Arg Arg Arg Arg Ala Arg Arg Ser Ala
2595 2600 2605

Asp Cys Ser Val Gln Thr Asp Asp Glu Asp Ser Ala Glu Trp Glu Gln
2610 2615 2620

Pro Val Arg Arg Arg Arg Ser Arg Leu Pro Arg His Ser Asp Ser Gly
2625 2630 2635 2640

Ser Asp Ser Lys His Asp Ala Thr Ala Ser Ser Ser Ser Ala Ala Ala
2645 2650 2655

Thr Val Arg Ala Met Ser Ser Val Gly Ile Gln Thr Ile Ser Asp Cys
2660 2665 2670

Ser Val Gln Thr Glu Pro Asp Gln Leu Pro Arg Val Ser Pro Ala Ile
2675 2680 2685

His Ile Thr Ala Ala Thr Asp Pro Lys Val Glu Ile Val Arg Tyr Ile
2690 2695 2700

Ser Ala Pro Glu Lys Thr Gly Arg Gly Glu Ser Leu Ala Cys Gln Thr
2705 2710 2715 2720

Glu Pro Asp Gly Gln Ala Gln Gly Val Ala Gly Pro Gln Leu Val Gly
2725 2730 2735

Pro Thr Ala Ile Ser Pro Tyr Leu Pro Gly Ile Gln Ile Val Thr Pro
2740 2745 2750

Gly Pro Leu Gly Arg Phe Glu Lys Lys Lys Pro Asp Pro Leu Glu Ile
2755 2760 2765

Gly Tyr Gln Ala His Leu Pro Pro Glu Ser Leu Ser Gln Leu Val Ser
2770 2775 2780

Arg Gln Pro Pro Lys Ser Pro Gln Val Leu Tyr Ser Pro Val Ser Pro
2785 2790 2795 2800

Leu Ser Pro His Arg Leu Leu Asp Thr Ser Phe Ala Ser Ser Glu Arg
2805 2810 2815

Leu Asn Lys Ala His Val Ser Pro Gln Lys His Phe Thr Ala Asp Ser
2820 2825 2830

Ala Leu Arg Gln Gln Thr Leu Pro Arg Pro Met Lys Thr Leu Gln Arg
2835 2840 2845

Ser Leu Ser Asp Pro Lys Pro Leu Ser Pro Thr Ala Glu Glu Ser Ala
2850 2855 2860

Lys Glu Arg Phe Ser Leu Tyr Gln His Gln Gly Gly Leu Gly Ser Gln
2865 2870 2875 2880

Val Ser Ala Leu Pro Pro Asn Ser Leu Val Arg Lys Val Lys Arg Thr
2885 2890 2895

Leu Pro Ser Pro Pro Pro Glu Glu Ala His Leu Pro Leu Ala Gly Gln
2900 2905 2910

Ala Ser Pro Gln Leu Tyr Ala Ala Ser Leu Leu Gln Arg Gly Leu Thr
2915 2920 2925

Gly Pro Thr Thr Val Pro Ala Thr Lys Ala Ser Leu Leu Arg Glu Leu
2930 2935 2940

Asp Arg Asp Leu Arg Leu Val Glu His Glu Ser Thr Lys Leu Arg Lys
2945 2950 2955 2960

Lys Gln Ala Glu Leu Asp Glu Glu Glu Lys Glu Ile Asp Ala Lys Leu
2965 2970 2975

Lys Tyr Leu Glu Leu Gly Ile Thr Gln Arg Lys Glu Ser Leu Ala Lys
2980 2985 2990

Asp Arg Gly Gly Arg Asp Tyr Pro Pro Leu Arg Gly Leu Gly Glu His
2995 3000 3005

```
Arg Asp Tyr Leu Ser Asp Ser Glu Leu Asn Gln Leu Arg Leu Gln Gly
   3010                3015                3020

Cys Thr Thr Pro Ala Gly Gln Phe Val Asp Phe Pro Ala Thr Ala Ala
3025                3030                3035                3040

Ala Pro Ala Thr Pro Ser Gly Pro Thr Ala Phe Gln Gln Pro Arg Phe
               3045                3050                3055

Gln Pro Pro Ala Pro Gln Tyr Ser Ala Gly Ser Gly Gly Pro Thr Gln
           3060                3065                3070

Asn Gly Phe Pro Ala His Gln Ala Pro Thr Tyr Pro Gly Pro Ser Thr
       3075                3080                3085

Tyr Pro Ala Pro Ala Phe Pro Pro Gly Ala Ser Tyr Pro Ala Glu Pro
   3090                3095                3100

Gly Leu Pro Asn Gln Gln Ala Phe Arg Pro Thr Gly His Tyr Ala Gly
3105                3110                3115                3120

Gln Thr Pro Met Pro Thr Thr Gln Ser Thr Leu Phe Pro Val Pro Ala
               3125                3130                3135

Asp Ser Arg Ala Pro Leu Gln Lys Pro Arg Gln Thr Ser Leu Ala Asp
           3140                3145                3150

Leu Glu Gln Lys Val Pro Thr Asn Tyr Glu Val Ile Ala Ser Pro Val
       3155                3160                3165

Val Pro Met Ser Ser Ala Pro Ser Glu Thr Ser Tyr Ser Gly Pro Ala
   3170                3175                3180

Val Ser Ser Gly Tyr Glu Gln Gly Lys Val Pro Glu Val Pro Arg Ala
3185                3190                3195                3200

Gly Asp Arg Gly Ser Val Ser Gln Ser Pro Ala Pro Thr Tyr Pro Ser
               3205                3210                3215

Asp Ser His Tyr Thr Ser Leu Glu Gln Asn Val Pro Arg Asn Tyr Val
           3220                3225                3230

Met Ile Asp Asp Ile Ser Glu Leu Thr Lys Asp Ser Thr Ser Thr Ala
       3235                3240                3245

Pro Asp Ser Gln Arg Leu Glu Pro Leu Gly Pro Gly Ser Ser Gly Arg
   3250                3255                3260

Pro Gly Lys Glu Pro Gly Glu Pro Gly Val Leu Asp Gly Pro Thr Leu
3265                3270                3275                3280

Pro Cys Cys Tyr Ala Arg Gly Glu Glu Glu Ser Glu Glu Asp Ser Tyr
               3285                3290                3295

Asp Pro Arg Gly Lys Gly Gly His Leu Arg Ser Met Glu Ser Asn Gly
           3300                3305                3310

Arg Pro Ala Ser Thr His Tyr Tyr Gly Asp Ser Asp Tyr Arg His Gly
       3315                3320                3325

Ala Arg Val Glu Lys Tyr Gly Pro Gly Pro Met Gly Pro Lys His Pro
   3330                3335                3340
```

Ser Lys Ser Leu Ala Pro Ala Ala Ile Ser Ser Lys Arg Ser Lys His
3345 3350 3355 3360

Arg Lys Gln Gly Met Glu Gln Lys Ile Ser Lys Phe Ser Pro Ile Glu
3365 3370 3375

Glu Ala Lys Asp Val Glu Ser Asp Leu Ala Ser Tyr Pro Pro Pro Ala
3380 3385 3390

Val Ser Ser Ser Leu Val Ser Arg Gly Arg Lys Phe Gln Asp Glu Ile
3395 3400 3405

Thr Tyr Gly Leu Lys Lys Asn Val Tyr Glu Gln Gln Lys Tyr Tyr Gly
3410 3415 3420

Met Ser Ser Arg Asp Ala Val Glu Asp Asp Arg Ile Tyr Gly Gly Ser
3425 3430 3435 3440

Ser Arg Ser Arg Ala Pro Ser Ala Tyr Ser Gly Glu Lys Leu Ser Ser
3445 3450 3455

His Asp Phe Ser Gly Trp Gly Lys Gly Tyr Glu Arg Glu Arg Glu Ala
3460 3465 3470

Val Glu Arg Leu Gln Lys Ala Gly Pro Lys Pro Ser Ser Leu Ser Met
3475 3480 3485

Ala His Ser Arg Val Arg Pro Pro Met Arg Ser Gln Ala Ser Glu Glu
3490 3495 3500

Glu Ser Pro Val Ser Pro Leu Gly Arg Pro Arg Pro Ala Gly Gly Pro
3505 3510 3515 3520

Leu Pro Pro Gly Gly Asp Thr Cys Pro Gln Phe Cys Ser Ser His Ser
3525 3530 3535

Met Pro Asp Val Gln Glu His Val Lys Asp Gly Pro Arg Ala His Ala
3540 3545 3550

Tyr Lys Arg Glu Glu Gly Tyr Ile Leu Asp Asp Ser His Cys Val Val
3555 3560 3565

Ser Asp Ser Glu Ala Tyr His Leu Gly Gln Glu Glu Thr Asp Trp Phe
3570 3575 3580

Asp Lys Pro Arg Asp Ala Arg Ser Asp Arg Phe Arg His His Gly Gly
3585 3590 3595 3600

His Ala Val Ser Ser Ser Ser Gln Lys Arg Gly Pro Ala Arg His Ser
3605 3610 3615

Tyr His Asp Tyr Asp Glu Pro Pro Glu Glu Gly Leu Trp Pro His Asp
3620 3625 3630

Glu Gly Gly Pro Gly Arg His Ala Ser Ala Lys Glu His Arg His Gly
3635 3640 3645

Asp His Gly Arg His Ser Gly Arg His Thr Gly Glu Glu Pro Gly Arg
3650 3655 3660

Arg Ala Ala Lys Pro His Ala Arg Asp Leu Gly Arg His Glu Ala Arg
3665 3670 3675 3680

Pro His Ser Gln Pro Ser Ser Ala Pro Ala Met Pro Lys Lys Gly Gln
3685 3690 3695

Pro Gly Tyr Pro Ser Ser Ala Glu Tyr Ser Gln Pro Ser Arg Ala Ser
3700 3705 3710

Ser Ala Tyr His His Ala Ser Asp Ser Lys Lys Gly Ser Arg Gln Ala
3715 3720 3725

His Ser Gly Pro Ala Ala Leu Gln Ser Lys Ala Glu Pro Gln Ala Gln
3730 3735 3740

Pro Gln Leu Gln Gly Arg Gln Ala Ala Pro Gly Pro Gln Gln Ser Gln
3745 3750 3755 3760

Ser Pro Ser Ser Arg Gln Ile Pro Ser Gly Ala Ala Ser Arg Gln Pro
3765 3770 3775

Gln Thr Gln Gln Gln Gln Gln Gly Leu Gly Leu Gln Pro Pro Gln Gln
3780 3785 3790

Ala Leu Thr Gln Ala Arg Leu Gln Gln Gln Ser Gln Pro Thr Thr Arg
3795 3800 3805

Gly Ser Ala Pro Ala Ala Ser Gln Pro Ala Gly Lys Pro Gln Pro Gly
3810 3815 3820

Pro Ser Thr Ala Thr Gly Pro Gln Pro Ala Gly Pro Pro Arg Ala Glu
3825 3830 3835 3840

Gln Thr Asn Gly Ser Lys Gly Thr Ala Lys Ala Pro Gln Gln Gly Arg
3845 3850 3855

Ala Pro Gln Ala Gln Pro Ala Pro Gly Pro Gly Pro Ala Gly Val Lys
3860 3865 3870

Ala Gly Ala Arg Pro Gly Gly Thr Pro Gly Ala Pro Ala Gly Gln Pro
3875 3880 3885

Gly Ala Asp Gly Glu Ser Val Phe Ser Lys Ile Leu Pro Gly Gly Ala
3890 3895 3900

Ala Glu Gln Ala Gly Lys Leu Thr Glu Ala Val Ser Ala Phe Gly Lys
3905 3910 3915 3920

Lys Phe Ser Ser Phe Trp
3925

<210> 113
<211> 4243
<212> PRT
<213> Artificial Sequence

<220>
<223> PKHL1_HUMAN

<400> 113
Met Gly His Leu Trp Leu Leu Gly Ile Trp Gly Leu Cys Gly Leu Leu
1 5 10 15

Leu Cys Ala Ala Asp Pro Ser Thr Asp Gly Ser Gln Ile Ile Pro Lys

20 25 30

Val Thr Glu Ile Ile Pro Lys Tyr Gly Ser Ile Asn Gly Ala Thr Arg
35 40 45

Leu Thr Ile Arg Gly Glu Gly Phe Ser Gln Ala Asn Gln Phe Asn Tyr
50 55 60

Gly Val Asp Asn Ala Glu Leu Gly Asn Ser Val Gln Leu Ile Ser Ser
65 70 75 80

Phe Gln Ser Ile Thr Cys Asp Val Glu Lys Asp Ala Ser His Ser Thr
85 90 95

Gln Ile Thr Cys Tyr Thr Arg Ala Met Pro Glu Asp Ser Tyr Thr Val
100 105 110

Arg Val Ser Val Asp Gly Val Pro Val Thr Glu Asn Asn Thr Cys Lys
115 120 125

Gly His Ile Asn Ser Trp Glu Cys Thr Phe Asn Ala Lys Ser Phe Arg
130 135 140

Thr Pro Thr Ile Arg Ser Ile Thr Pro Leu Ser Gly Thr Pro Gly Thr
145 150 155 160

Leu Ile Thr Ile Gln Gly Arg Ile Phe Thr Asp Val Tyr Gly Ser Asn
165 170 175

Ile Ala Leu Ser Ser Asn Gly Lys Asn Val Arg Ile Leu Arg Val Tyr
180 185 190

Ile Gly Gly Met Pro Cys Glu Leu Leu Ile Pro Gln Ser Asp Asn Leu
195 200 205

Tyr Gly Leu Lys Leu Asp His Pro Asn Gly Asp Met Gly Ser Met Val
210 215 220

Cys Lys Thr Thr Gly Thr Phe Ile Gly His His Asn Val Ser Phe Ile
225 230 235 240

Leu Asp Asn Asp Tyr Gly Arg Ser Phe Pro Gln Lys Met Ala Tyr Phe
245 250 255

Val Ser Ser Leu Asn Lys Ile Ala Met Phe Gln Thr Tyr Ala Glu Val
260 265 270

Thr Met Ile Phe Pro Ser Gln Gly Ser Ile Arg Gly Gly Thr Thr Leu
275 280 285

Thr Ile Ser Gly Arg Phe Phe Asp Gln Thr Asp Phe Pro Val Arg Val
290 295 300

Leu Val Gly Gly Glu Pro Cys Asp Ile Leu Asn Val Thr Glu Asn Ser
305 310 315 320

Ile Cys Cys Lys Thr Pro Pro Lys Pro His Ile Leu Lys Thr Val Tyr
325 330 335

Pro Gly Gly Arg Gly Leu Lys Leu Glu Val Trp Asn Asn Ser Arg Pro
340 345 350

Ile Arg Leu Glu Glu Ile Leu Glu Tyr Asn Glu Lys Thr Pro Gly Tyr

355 360 365

Met Gly Ala Ser Trp Val Asp Ser Ala Ser Tyr Ile Trp Leu Met Glu
370 375 380

Gln Asp Thr Phe Val Ala Arg Phe Ser Gly Phe Leu Val Ala Pro Asp
385 390 395 400

Ser Asp Val Tyr Arg Phe Tyr Ile Lys Gly Asp Asp Arg Tyr Ala Ile
405 410 415

Tyr Phe Ser Gln Thr Gly Leu Pro Glu Asp Lys Val Arg Ile Ala Tyr
420 425 430

His Ser Ala Asn Ala Asn Ser Tyr Phe Ser Ser Pro Thr Gln Arg Ser
435 440 445

Asp Asp Ile His Leu Gln Lys Gly Lys Glu Tyr Tyr Ile Glu Ile Leu
450 455 460

Leu Gln Glu Tyr Arg Leu Ser Ala Phe Val Asp Val Gly Leu Tyr Gln
465 470 475 480

Tyr Arg Asn Val Tyr Thr Glu Gln Gln Thr Gly Asp Ala Val Asn Glu
485 490 495

Glu Gln Val Ile Lys Ser Gln Ser Thr Ile Leu Gln Glu Val Gln Val
500 505 510

Ile Thr Leu Glu Asn Trp Glu Thr Thr Asn Ala Ile Asn Glu Val Gln
515 520 525

Lys Ile Lys Val Thr Ser Pro Cys Val Glu Ala Asn Ser Cys Ser Leu
530 535 540

Tyr Gln Tyr Arg Leu Ile Tyr Asn Met Glu Lys Thr Val Phe Leu Pro
545 550 555 560

Ala Asp Ala Ser Glu Phe Ile Leu Gln Ser Ala Leu Asn Asp Leu Trp
565 570 575

Ser Ile Lys Pro Asp Thr Val Gln Val Ile Arg Thr Gln Asn Pro Gln
580 585 590

Ser Tyr Val Tyr Met Val Thr Phe Ile Ser Thr Arg Gly Asp Phe Asp
595 600 605

Leu Leu Gly Tyr Glu Val Val Glu Gly Asn Asn Val Thr Leu Asp Ile
610 615 620

Thr Glu Gln Thr Lys Gly Lys Pro Asn Leu Glu Thr Phe Thr Leu Asn
625 630 635 640

Trp Asp Gly Ile Ala Ser Lys Pro Leu Thr Leu Trp Ser Ser Glu Ala
645 650 655

Glu Phe Gln Gly Ala Val Glu Glu Met Val Ser Thr Lys Cys Pro Pro
660 665 670

Gln Ile Ala Asn Phe Glu Glu Gly Phe Val Val Lys Tyr Phe Arg Asp
675 680 685

Tyr Glu Thr Asp Phe Asn Leu Glu His Ile Asn Arg Gly Gln Lys Thr

690 695 700

Ala Glu Thr Asp Ala Tyr Cys Gly Arg Tyr Ser Leu Lys Asn Pro Ala
705 710 715 720

Val Leu Phe Asp Ser Ala Asp Val Lys Pro Asn Arg Arg Pro Tyr Gly
725 730 735

Asp Ile Leu Leu Phe Pro Tyr Asn Gln Leu Cys Leu Ala Tyr Lys Gly
740 745 750

Phe Leu Ala Asn Tyr Ile Gly Leu Lys Phe Gln Tyr Gln Asp Asn Ser
755 760 765

Lys Ile Thr Arg Ser Thr Asp Thr Gln Phe Thr Tyr Asn Phe Ala Tyr
770 775 780

Gly Asn Asn Trp Thr Tyr Thr Cys Ile Asp Leu Leu Asp Leu Val Arg
785 790 795 800

Thr Lys Tyr Thr Gly Thr Asn Val Ser Leu Gln Arg Ile Ser Leu His
805 810 815

Lys Ala Ser Glu Ser Gln Ser Phe Tyr Val Asp Val Val Tyr Ile Gly
820 825 830

His Thr Ser Thr Ile Ser Thr Leu Asp Glu Met Pro Lys Arg Arg Leu
835 840 845

Pro Ala Leu Ala Asn Lys Gly Ile Phe Leu Glu His Phe Gln Val Asn
850 855 860

Gln Thr Lys Thr Asn Gly Pro Thr Met Thr Asn Gln Tyr Ser Val Thr
865 870 875 880

Met Thr Ser Tyr Asn Cys Ser Tyr Asn Ile Pro Met Met Ala Val Ser
885 890 895

Phe Gly Gln Ile Ile Thr His Glu Thr Glu Asn Glu Phe Val Tyr Arg
900 905 910

Gly Asn Asn Trp Pro Gly Glu Ser Lys Ile His Ile Gln Arg Ile Gln
915 920 925

Ala Ala Ser Pro Pro Leu Ser Gly Ser Phe Asp Ile Gln Ala Tyr Gly
930 935 940

His Ile Leu Lys Gly Leu Pro Ala Ala Val Ser Ala Ala Asp Leu Gln
945 950 955 960

Phe Ala Leu Gln Ser Leu Glu Gly Met Gly Arg Ile Ser Val Thr Arg
965 970 975

Glu Gly Thr Cys Ala Gly Tyr Ala Trp Asn Ile Lys Trp Arg Ser Thr
980 985 990

Cys Gly Lys Gln Asn Leu Leu Gln Ile Asn Asp Ser Asn Ile Ile Gly
995 1000 1005

Glu Lys Ala Asn Met Thr Val Thr Arg Ile Lys Glu Gly Gly Leu Phe
1010 1015 1020

Arg Gln His Val Leu Gly Asp Leu Leu Arg Thr Pro Ser Gln Gln Pro

1025 1030 1035 1040

Gln Val Glu Val Tyr Val Asn Gly Ile Pro Ala Lys Cys Ser Gly Asp
1045 1050 1055

Cys Gly Phe Thr Trp Asp Ser Asn Ile Thr Pro Leu Val Leu Ala Ile
1060 1065 1070

Ser Pro Ser Gln Gly Ser Tyr Glu Glu Gly Thr Ile Leu Thr Ile Val
1075 1080 1085

Gly Ser Gly Phe Ser Pro Ser Ser Ala Val Thr Val Ser Val Gly Pro
1090 1095 1100

Val Gly Cys Ser Leu Leu Ser Val Asp Glu Lys Glu Leu Lys Cys Gln
1105 1110 1115 1120

Ile Leu Asn Gly Ser Ala Gly His Ala Pro Val Ala Val Ser Met Ala
1125 1130 1135

Asp Val Gly Leu Ala Gln Asn Val Gly Gly Glu Glu Phe Tyr Phe Val
1140 1145 1150

Tyr Gln Ser Gln Ile Ser His Ile Trp Pro Asp Ser Gly Ser Ile Ala
1155 1160 1165

Gly Gly Thr Leu Leu Thr Leu Ser Gly Phe Gly Phe Asn Glu Asn Ser
1170 1175 1180

Lys Val Leu Val Gly Asn Glu Thr Cys Asn Val Ile Glu Gly Asp Leu
1185 1190 1195 1200

Asn Arg Ile Thr Cys Arg Thr Pro Lys Lys Thr Glu Gly Thr Val Asp
1205 1210 1215

Ile Ser Val Thr Thr Asn Gly Phe Gln Ala Thr Ala Arg Asp Ala Phe
1220 1225 1230

Ser Tyr Asn Cys Leu Gln Thr Pro Ile Ile Thr Asp Phe Ser Pro Lys
1235 1240 1245

Val Arg Thr Ile Leu Gly Glu Val Asn Leu Thr Ile Lys Gly Tyr Asn
1250 1255 1260

Phe Gly Asn Glu Leu Thr Gln Asn Met Ala Val Tyr Val Gly Gly Lys
1265 1270 1275 1280

Thr Cys Gln Ile Leu His Trp Asn Phe Thr Asp Ile Arg Cys Leu Leu
1285 1290 1295

Pro Lys Leu Ser Pro Gly Lys His Asp Ile Tyr Val Glu Val Arg Asn
1300 1305 1310

Trp Gly Phe Ala Ser Thr Arg Asp Lys Leu Asn Ser Ser Ile Gln Tyr
1315 1320 1325

Val Leu Glu Val Thr Ser Met Phe Pro Gln Arg Gly Ser Leu Phe Gly
1330 1335 1340

Gly Thr Glu Ile Thr Ile Arg Gly Phe Gly Phe Ser Thr Ile Pro Ala
1345 1350 1355 1360

Glu Asn Thr Val Leu Leu Gly Ser Ile Pro Cys Asn Val Thr Ser Ser

1365 1370 1375

Ser Glu Asn Val Ile Lys Cys Ile Leu His Ser Thr Gly Asn Ile Phe
1380 1385 1390

Arg Ile Thr Asn Asn Gly Lys Asp Ser Val His Gly Leu Gly Tyr Ala
1395 1400 1405

Trp Ser Pro Pro Val Leu Asn Val Ser Val Gly Asp Thr Val Ala Trp
1410 1415 1420

His Trp Gln Thr His Pro Phe Leu Arg Gly Ile Gly Tyr Arg Ile Phe
1425 1430 1435 1440

Ser Val Ser Ser Pro Gly Ser Val Ile Tyr Asp Gly Lys Gly Phe Thr
1445 1450 1455

Ser Gly Arg Gln Lys Ser Thr Ser Gly Ser Phe Ser Tyr Gln Phe Thr
1460 1465 1470

Ser Pro Gly Ile His Tyr Tyr Ser Ser Gly Tyr Val Asp Glu Ala His
1475 1480 1485

Ser Ile Phe Leu Gln Gly Val Ile Asn Val Leu Pro Ala Glu Thr Arg
1490 1495 1500

His Ile Pro Leu His Leu Phe Val Gly Arg Ser Glu Ala Thr Tyr Ala
1505 1510 1515 1520

Tyr Gly Gly Pro Glu Asn Leu His Leu Gly Ser Ser Val Ala Gly Cys
1525 1530 1535

Leu Ala Thr Glu Pro Leu Cys Ser Leu Asn Asn Thr Arg Val Lys Asn
1540 1545 1550

Ser Lys Arg Leu Leu Phe Glu Val Ser Ser Cys Phe Ser Pro Ser Ile
1555 1560 1565

Ser Asn Ile Thr Pro Ser Thr Gly Thr Val Asn Glu Leu Ile Thr Ile
1570 1575 1580

Ile Gly His Gly Phe Ser Asn Leu Pro Trp Ala Asn Lys Val Thr Ile
1585 1590 1595 1600

Gly Ser Tyr Pro Cys Val Val Glu Glu Ser Ser Glu Asp Ser Ile Thr
1605 1610 1615

Cys His Ile Asp Pro Gln Asn Ser Met Asp Val Gly Ile Arg Glu Thr
1620 1625 1630

Val Thr Leu Thr Val Tyr Asn Leu Gly Thr Ala Ile Asn Thr Leu Ser
1635 1640 1645

Asn Glu Phe Asp Arg Arg Phe Val Leu Leu Pro Asn Ile Asp Leu Val
1650 1655 1660

Leu Pro Asn Ala Gly Ser Thr Thr Gly Met Thr Ser Val Thr Ile Lys
1665 1670 1675 1680

Gly Ser Gly Phe Ala Val Ser Ser Ala Gly Val Lys Val Leu Met Gly
1685 1690 1695

His Phe Pro Cys Lys Val Leu Ser Val Asn Tyr Thr Ala Ile Glu Cys

1700 1705 1710

Glu Thr Ser Pro Ala Ala Gln Gln Leu Val Asp Val Asp Leu Leu Ile
1715 1720 1725

His Gly Val Pro Ala Gln Cys Gln Gly Asn Cys Thr Phe Ser Tyr Leu
1730 1735 1740

Glu Ser Ile Thr Pro Tyr Ile Thr Gly Val Phe Pro Asn Ser Val Ile
1745 1750 1755 1760

Gly Ser Val Lys Val Leu Ile Glu Gly Glu Gly Leu Gly Thr Val Leu
1765 1770 1775

Glu Asp Ile Ala Val Phe Ile Gly Asn Gln Gln Phe Arg Ala Ile Glu
1780 1785 1790

Val Asn Glu Asn Asn Ile Thr Ala Leu Val Thr Pro Leu Pro Val Gly
1795 1800 1805

His His Ser Val Ser Val Val Val Gly Ser Lys Gly Leu Ala Leu Gly
1810 1815 1820

Asn Leu Thr Val Ser Ser Pro Pro Val Ala Ser Leu Ser Pro Thr Ser
1825 1830 1835 1840

Gly Ser Ile Gly Gly Gly Thr Thr Leu Val Ile Thr Gly Asn Gly Phe
1845 1850 1855

Tyr Pro Gly Asn Thr Thr Val Thr Ile Gly Asp Glu Pro Cys Gln Ile
1860 1865 1870

Ile Ser Ile Asn Pro Asn Glu Val Tyr Cys Arg Thr Pro Ala Gly Thr
1875 1880 1885

Thr Gly Met Val Asp Val Lys Ile Phe Val Asn Thr Ile Ala Tyr Pro
1890 1895 1900

Pro Leu Leu Phe Thr Tyr Ala Leu Glu Asp Thr Pro Phe Leu Arg Gly
1905 1910 1915 1920

Ile Ile Pro Ser Arg Gly Pro Pro Gly Thr Glu Ile Glu Ile Thr Gly
1925 1930 1935

Ser Asn Phe Gly Phe Glu Ile Leu Glu Ile Ser Val Met Ile Asn Asn
1940 1945 1950

Ile Gln Cys Asn Val Thr Met Ala Asn Asp Ser Val Val Gln Cys Ile
1955 1960 1965

Val Gly Asp His Ala Gly Gly Thr Phe Pro Val Met Met His His Lys
1970 1975 1980

Thr Lys Gly Ser Ala Met Ser Thr Val Val Phe Glu Tyr Pro Leu Asn
1985 1990 1995 2000

Ile Gln Asn Ile Asn Pro Ser Gln Gly Ser Phe Gly Gly Gly Gln Thr
2005 2010 2015

Met Thr Val Thr Gly Thr Gly Phe Asn Pro Gln Asn Ser Ile Ile Leu
2020 2025 2030

Val Cys Gly Ser Glu Cys Ala Ile Asp Arg Leu Arg Ser Asp Tyr Thr

2035 2040 2045

Thr Leu Leu Cys Glu Ile Pro Ser Asn Asn Gly Thr Gly Ala Glu Gln
2050 2055 2060

Ala Cys Glu Val Ser Val Val Asn Gly Lys Asp Leu Ser Gln Ser Met
2065 2070 2075 2080

Thr Pro Phe Thr Tyr Ala Val Ser Leu Thr Pro Leu Ile Thr Ala Val
2085 2090 2095

Ser Pro Lys Arg Gly Ser Thr Ala Gly Gly Thr Arg Leu Thr Val Val
2100 2105 2110

Gly Ser Gly Phe Ser Glu Asn Met Glu Asp Val His Ile Thr Ile Ala
2115 2120 2125

Glu Ala Lys Cys Asp Val Glu Tyr Ser Asn Lys Thr His Ile Ile Cys
2130 2135 2140

Met Thr Asp Ala His Thr Leu Ser Gly Trp Ala Pro Val Cys Val His
2145 2150 2155 2160

Ile Arg Gly Val Gly Met Ala Lys Leu Asp Asn Ala Asp Phe Leu Tyr
2165 2170 2175

Val Asp Ala Trp Ser Ser Asn Phe Ser Trp Gly Gly Lys Ser Pro Pro
2180 2185 2190

Glu Glu Gly Ser Leu Val Val Ile Thr Lys Gly Gln Thr Ile Leu Leu
2195 2200 2205

Asp Gln Ser Thr Pro Ile Leu Lys Met Leu Leu Ile Gln Gly Gly Thr
2210 2215 2220

Leu Ile Phe Asp Glu Ala Asp Ile Glu Leu Gln Ala Glu Asn Ile Leu
2225 2230 2235 2240

Ile Thr Asp Gly Gly Val Leu Gln Ile Gly Thr Glu Thr Ser Pro Phe
2245 2250 2255

Gln His Lys Ala Val Ile Thr Leu His Gly His Leu Arg Ser Pro Glu
2260 2265 2270

Leu Pro Val Tyr Gly Ala Lys Thr Leu Ala Val Arg Glu Gly Ile Leu
2275 2280 2285

Asp Leu His Gly Val Pro Val Pro Val Thr Trp Thr Arg Leu Ala His
2290 2295 2300

Thr Ala Lys Ala Gly Glu Arg Ile Leu Ile Leu Gln Glu Ala Val Thr
2305 2310 2315 2320

Trp Lys Pro Gly Asp Asn Ile Val Ile Ala Ser Thr Gly His Arg His
2325 2330 2335

Ser Gln Gly Glu Asn Glu Lys Met Thr Ile Ala Ser Val Ser Ala Asp
2340 2345 2350

Gly Ile Asn Ile Thr Leu Ser Asn Pro Leu Asn Tyr Thr His Leu Gly
2355 2360 2365

Ile Thr Val Thr Leu Pro Asp Gly Thr Leu Phe Glu Ala Arg Ala Glu

2370 2375 2380

Val Gly Ile Leu Thr Arg Asn Ile Leu Ile Arg Gly Ser Asp Asn Val
2385 2390 2395 2400

Glu Trp Asn Asn Lys Ile Pro Ala Cys Pro Asp Gly Phe Asp Thr Gly
2405 2410 2415

Glu Phe Ala Thr Gln Thr Cys Leu Gln Gly Lys Phe Gly Glu Glu Ile
2420 2425 2430

Gly Ser Asp Gln Phe Gly Gly Cys Val Met Phe His Ala Pro Val Pro
2435 2440 2445

Gly Ala Asn Met Val Thr Gly Arg Ile Glu Tyr Val Glu Val Phe His
2450 2455 2460

Ala Gly Gln Ala Phe Arg Leu Gly Arg Tyr Pro Ile His Trp His Leu
2465 2470 2475 2480

Leu Gly Asp Leu Gln Phe Lys Ser Tyr Val Arg Gly Cys Ala Ile His
2485 2490 2495

Gln Ala Tyr Asn Arg Ala Val Thr Ile His Asn Thr His His Leu Leu
2500 2505 2510

Val Glu Arg Asn Ile Ile Tyr Asp Ile Lys Gly Gly Ala Phe Phe Ile
2515 2520 2525

Glu Asp Gly Ile Glu His Gly Asn Ile Leu Gln Tyr Asn Leu Ala Val
2530 2535 2540

Phe Val Gln Gln Ser Thr Ser Leu Leu Asn Asp Asp Val Thr Pro Ala
2545 2550 2555 2560

Ala Phe Trp Val Thr Asn Pro Asn Asn Thr Ile Arg His Asn Ala Val
2565 2570 2575

Ala Gly Gly Thr His Phe Gly Phe Trp Tyr Arg Met Asn Asn His Pro
2580 2585 2590

Asp Gly Pro Ser Tyr Asp Arg Asn Ile Cys Gln Lys Arg Val Pro Leu
2595 2600 2605

Gly Glu Phe Phe Asn Asn Thr Val His Ser Gln Gly Trp Phe Gly Met
2610 2615 2620

Trp Ile Phe Glu Glu Tyr Phe Pro Met Gln Thr Gly Ser Cys Thr Ser
2625 2630 2635 2640

Thr Val Pro Ala Pro Ala Ile Phe Asn Ser Leu Thr Thr Trp Asn Cys
2645 2650 2655

Gln Lys Gly Ala Glu Trp Val Asn Gly Gly Ala Leu Gln Phe His Asn
2660 2665 2670

Phe Val Met Val Asn Asn Tyr Glu Ala Gly Ile Glu Thr Lys Arg Ile
2675 2680 2685

Leu Ala Pro Tyr Val Gly Gly Trp Gly Glu Thr Asn Gly Ala Val Ile
2690 2695 2700

Lys Asn Ala Lys Ile Val Gly His Leu Asp Glu Leu Gly Met Gly Ser

2705 2710 2715 2720

Ala Phe Cys Thr Ala Lys Gly Leu Val Leu Pro Phe Ser Glu Gly Leu
2725 2730 2735

Thr Val Ser Ser Val His Phe Met Asn Phe Asp Arg Pro Asn Cys Val
2740 2745 2750

Ala Leu Gly Val Thr Ser Ile Ser Gly Val Cys Asn Asp Arg Cys Gly
2755 2760 2765

Gly Trp Ser Ala Lys Phe Val Asp Val Gln Tyr Ser His Thr Pro Asn
2770 2775 2780

Lys Ala Gly Phe Arg Trp Glu His Glu Met Val Met Ile Asp Val Asp
2785 2790 2795 2800

Gly Ser Leu Thr Gly His Lys Gly His Thr Val Ile Pro His Ser Ser
2805 2810 2815

Leu Leu Asp Pro Ser His Cys Thr Gln Glu Ala Glu Trp Ser Ile Gly
2820 2825 2830

Phe Pro Gly Ser Val Cys Asp Ala Ser Val Ser Phe His Arg Leu Ala
2835 2840 2845

Phe Asn Gln Pro Ser Pro Val Ser Leu Leu Glu Lys Asp Val Val Leu
2850 2855 2860

Ser Asp Ser Phe Gly Thr Ser Ile Ile Pro Phe Gln Lys Lys Arg Leu
2865 2870 2875 2880

Thr His Met Ser Gly Trp Met Ala Leu Ile Pro Asn Ala Asn His Ile
2885 2890 2895

Asn Trp Tyr Phe Lys Gly Val Asp His Ile Thr Asn Ile Ser Tyr Thr
2900 2905 2910

Ser Thr Phe Tyr Gly Phe Lys Glu Glu Asp Tyr Val Ile Ile Ser His
2915 2920 2925

Asn Phe Thr Gln Asn Pro Asp Met Phe Asn Ile Ile Asp Met Arg Asn
2930 2935 2940

Gly Ser Ser Asn Pro Leu Asn Trp Asn Thr Ser Lys Asn Gly Asp Trp
2945 2950 2955 2960

His Leu Glu Ala Asn Thr Ser Thr Leu Tyr Tyr Leu Val Ser Gly Arg
2965 2970 2975

Asn Asp Leu His Gln Ser Gln Leu Ile Ser Gly Asn Leu Asp Pro Asp
2980 2985 2990

Val Lys Asp Val Val Ile Asn Phe Gln Ala Tyr Cys Cys Ile Leu Gln
2995 3000 3005

Asp Cys Phe Pro Val His Pro Pro Ser Arg Lys Pro Ile Pro Lys Lys
3010 3015 3020

Arg Pro Ala Thr Tyr Asn Leu Trp Ser Asn Asp Ser Phe Trp Gln Ser
3025 3030 3035 3040

Ser Arg Glu Asn Asn Tyr Thr Val Pro His Pro Gly Ala Asn Val Ile

3045 3050 3055

Ile Pro Glu Gly Thr Trp Ile Val Ala Asp Ile Asp Met Pro Ser Met
3060 3065 3070

Glu Arg Leu Ile Ile Trp Gly Val Leu Glu Leu Glu Asp Lys Tyr Asn
3075 3080 3085

Val Gly Ala Ala Glu Ser Ser Tyr Arg Glu Val Val Leu Asn Ala Thr
3090 3095 3100

Tyr Ile Ser Leu Gln Gly Gly Arg Leu Ile Gly Gly Trp Glu Asp Asn
3105 3110 3115 3120

Pro Phe Lys Gly Asp Leu Lys Ile Val Leu Arg Gly Asn His Thr Thr
3125 3130 3135

Gln Asp Trp Ala Leu Pro Glu Gly Pro Asn Gln Gly Ala Lys Val Leu
3140 3145 3150

Gly Val Phe Gly Glu Leu Asp Leu His Gly Ile Pro His Ser Ile Tyr
3155 3160 3165

Lys Thr Lys Leu Ser Glu Thr Ala Phe Ala Gly Ser Lys Val Leu Ser
3170 3175 3180

Leu Met Asp Ala Val Asp Trp Gln Glu Gly Glu Glu Ile Val Ile Thr
3185 3190 3195 3200

Thr Thr Ser Tyr Asp Phe His Gln Thr Glu Thr Arg Ser Ile Val Lys
3205 3210 3215

Ile Leu His Asp His Lys Ile Leu Ile Leu Asn Asp Ser Leu Ser Tyr
3220 3225 3230

Thr His Phe Ala Glu Lys Tyr His Val Pro Gly Thr Gly Glu Ser Tyr
3235 3240 3245

Thr Leu Ala Ala Asp Val Gly Ile Leu Ser Arg Asn Ile Lys Ile Val
3250 3255 3260

Gly Glu Asp Tyr Pro Gly Trp Ser Glu Asp Ser Phe Gly Ala Arg Val
3265 3270 3275 3280

Leu Val Gly Ser Phe Thr Glu Asn Met Met Thr Phe Lys Gly Asn Ala
3285 3290 3295

Arg Ile Ser Asn Val Glu Phe Tyr His Ser Gly Gln Glu Gly Phe Arg
3300 3305 3310

Asp Ser Thr Asp Pro Arg Tyr Ala Val Thr Phe Leu Asn Leu Gly Gln
3315 3320 3325

Ile Gln Glu His Gly Ser Ser Tyr Ile Arg Gly Cys Ala Phe His His
3330 3335 3340

Gly Phe Ser Pro Ala Ile Gly Val Phe Gly Thr Asp Gly Leu Asp Ile
3345 3350 3355 3360

Asp Asp Asn Ile Ile His Phe Thr Val Gly Glu Gly Ile Arg Ile Trp
3365 3370 3375

Gly Asn Ala Asn Arg Val Arg Gly Asn Leu Ile Ala Leu Ser Val Trp

3380 3385 3390

Pro Gly Thr Tyr Gln Asn Arg Lys Asp Leu Ser Ser Thr Leu Trp His
3395 3400 3405

Ala Ala Ile Glu Ile Asn Arg Gly Thr Asn Thr Val Leu Gln Asn Asn
3410 3415 3420

Val Val Ala Gly Phe Gly Arg Ala Gly Tyr Arg Ile Asp Gly Glu Pro
3425 3430 3435 3440

Cys Pro Gly Gln Phe Asn Pro Val Glu Lys Trp Phe Asp Asn Glu Ala
3445 3450 3455

His Gly Gly Leu Tyr Gly Ile Tyr Met Asn Gln Asp Gly Leu Pro Gly
3460 3465 3470

Cys Ser Leu Ile Gln Gly Phe Thr Ile Trp Thr Cys Trp Asp Tyr Gly
3475 3480 3485

Ile Tyr Phe Gln Thr Thr Glu Ser Val His Ile Tyr Asn Val Thr Leu
3490 3495 3500

Val Asp Asn Gly Met Ala Ile Phe Pro Met Ile Tyr Met Pro Ala Ala
3505 3510 3515 3520

Ile Ser His Lys Ile Ser Ser Lys Asn Val Gln Ile Lys Ser Ser Leu
3525 3530 3535

Ile Val Gly Ser Ser Pro Gly Phe Asn Cys Ser Asp Val Leu Thr Asn
3540 3545 3550

Asp Asp Pro Asn Ile Glu Leu Thr Ala Ala His Arg Ser Pro Arg Ser
3555 3560 3565

Pro Ser Gly Gly Arg Ser Gly Ile Cys Trp Pro Thr Phe Ala Ser Ala
3570 3575 3580

His Asn Met Ala Pro Arg Lys Pro His Ala Gly Ile Met Ser Tyr Asn
3585 3590 3595 3600

Ala Ile Ser Gly Leu Leu Asp Ile Ser Gly Ser Thr Phe Val Gly Phe
3605 3610 3615

Lys Asn Val Cys Ser Gly Glu Thr Asn Val Ile Phe Ile Thr Asn Pro
3620 3625 3630

Leu Asn Glu Asp Leu Gln His Pro Ile His Val Lys Asn Ile Lys Leu
3635 3640 3645

Val Asp Thr Thr Glu Gln Ser Lys Ile Phe Ile His Arg Pro Asp Ile
3650 3655 3660

Ser Lys Val Asn Pro Ser Asp Cys Val Asp Met Val Cys Asp Ala Lys
3665 3670 3675 3680

Arg Lys Ser Phe Leu Arg Asp Ile Asp Gly Ser Phe Leu Gly Asn Ala
3685 3690 3695

Gly Ser Val Ile Pro Gln Ala Glu Tyr Glu Trp Asp Gly Asn Ser Gln
3700 3705 3710

Val Gly Ile Gly Asp Tyr Arg Ile Pro Lys Ala Met Leu Thr Phe Leu

3715 3720 3725

Asn Gly Ser Arg Ile Pro Val Thr Glu Lys Ala Pro His Lys Gly Ile
3730 3735 3740

Ile Arg Asp Ser Thr Cys Lys Tyr Leu Pro Glu Trp Gln Ser Tyr Gln
3745 3750 3755 3760

Cys Phe Gly Met Glu Tyr Ala Met Met Val Ile Glu Ser Leu Asp Pro
3765 3770 3775

Asp Thr Glu Thr Arg Arg Leu Ser Pro Val Ala Ile Met Gly Asn Gly
3780 3785 3790

Tyr Val Asp Leu Ile Asn Gly Pro Gln Asp His Gly Trp Cys Ala Gly
3795 3800 3805

Tyr Thr Cys Gln Arg Arg Leu Ser Leu Phe His Ser Ile Val Ala Leu
3810 3815 3820

Asn Lys Ser Tyr Glu Val Tyr Phe Thr Gly Thr Ser Pro Gln Asn Leu
3825 3830 3835 3840

Arg Leu Met Leu Leu Asn Val Asp His Asn Lys Ala Val Leu Val Gly
3845 3850 3855

Ile Phe Phe Ser Thr Leu Gln Arg Leu Asp Val Tyr Val Asn Asn Leu
3860 3865 3870

Leu Val Cys Pro Lys Thr Thr Ile Trp Asn Ala Gln Gln Lys His Cys
3875 3880 3885

Glu Leu Asn Asn His Leu Tyr Lys Asp Gln Phe Leu Pro Asn Leu Asp
3890 3895 3900

Ser Thr Val Leu Gly Glu Asn Tyr Phe Asp Gly Thr Tyr Gln Met Leu
3905 3910 3915 3920

Tyr Leu Leu Val Lys Gly Thr Ile Pro Val Glu Ile His Thr Ala Thr
3925 3930 3935

Val Ile Phe Val Ser Phe Gln Leu Ser Val Ala Thr Glu Asp Asp Phe
3940 3945 3950

Tyr Thr Ser His Asn Leu Val Lys Asn Leu Ala Leu Phe Leu Lys Ile
3955 3960 3965

Pro Ser Asp Lys Ile Arg Ile Ser Lys Ile Arg Gly Lys Ser Leu Arg
3970 3975 3980

Arg Lys Arg Ser Met Gly Phe Ile Ile Glu Ile Glu Ile Gly Asp Pro
3985 3990 3995 4000

Pro Ile Gln Phe Ile Ser Asn Gly Thr Thr Gly Gln Met Gln Leu Ser
4005 4010 4015

Glu Leu Gln Glu Ile Ala Gly Ser Leu Gly Gln Ala Val Ile Leu Gly
4020 4025 4030

Asn Ile Ser Ser Ile Leu Gly Phe Asn Ile Ser Ser Met Ser Ile Thr
4035 4040 4045

Asn Pro Leu Pro Ser Pro Ser Asp Ser Gly Trp Ile Lys Val Thr Ala

```
    4050                4055                4060

Gln Pro Val Glu Arg Ser Ala Phe Pro Val His His Val Ala Phe Val
4065                4070                4075                4080

Ser Ser Leu Leu Val Ile Thr Gln Pro Val Ala Ala Gln Pro Gly Gln
                4085                4090                4095

Pro Phe Pro Gln Gln Pro Ser Val Lys Ala Thr Asp Ser Asp Gly Asn
            4100                4105                4110

Cys Val Ser Val Gly Ile Thr Ala Leu Thr Leu Arg Ala Ile Leu Lys
        4115                4120                4125

Asp Ser Asn Asn Asn Gln Val Asn Gly Leu Ser Gly Asn Thr Thr Ile
    4130                4135                4140

Pro Phe Ser Ser Cys Trp Ala Asn Tyr Thr Asp Leu Thr Pro Leu Arg
4145                4150                4155                4160

Thr Gly Lys Asn Tyr Lys Ile Glu Phe Ile Leu Asp Asn Val Val Gly
                4165                4170                4175

Val Glu Ser Arg Thr Phe Ser Leu Leu Ala Glu Ser Val Ser Ser Ser
            4180                4185                4190

Gly Ser Ser Ser Ser Ser Asn Ser Lys Ala Ser Thr Val Gly Thr Tyr
        4195                4200                4205

Ala Gln Ile Met Thr Val Val Ile Ser Cys Leu Val Gly Arg Met Trp
    4210                4215                4220

Leu Leu Glu Ile Phe Met Ala Ala Val Ser Thr Leu Asn Ile Thr Leu
4225                4230                4235                4240

Arg Ser Tyr
```

## Description

[TECHNICAL FIELD]

The present invention relates to a fusion polypeptide comprising GDF15 (Growth differentiation factor 15) and a polypeptide region capable of O-glycosylation, a pharmaceutical composition comprising the fusion polypeptide, and a method for increasing in vivo duration of GDF15 comprising the step of fusing a polypeptide region capable of O-glycosylation.

[BACKGROUND ART]

Most protein or peptide drugs have a short duration of activity in the body, and their absorption rate is low when administered by methods other than intravenous administration, and therefore, there is an inconvenience of having to continuously inject these drugs repeatedly at short administration intervals when treatment of long-term drug administration is required. In order to solve such inconvenience, it is required to develop a technology for continuously releasing a drug with single administration. As a part to meet these needs, a sustained-release formulation for sustained release is being developed.

For examples, research on a sustained-release formulation in which the drug is slowly released while the matrix substance is slowly decomposed in vivo when it is administered, by preparing a microparticle in the form of a protein or peptide drug surrounded by a biodegradable polymer matrix is actively progressed.

For example, U.S. Patent NO. 5,416,017 discloses a sustained-release injection of erythropoietin using a gel having a hyaluronic acid concentration of 0.01 to 3 %, and Japanese Patent Publication No. 1-287041 discloses a sustained-release injection in which insulin is contained in a gel having a hyaluronic acid concentration of 1%, and Japanese Patent Publication No. 2-213 discloses a sustained-release formulation in which calcitonin, elcatonin or human GDF15 is contained in hyaluronic acid having a concentration of 5%. In such a formulation, the protein drug dissolved in the gel of hyaluronic acid passes through the gel matrix with high viscosity at a slow speed, so it can exhibit a sustained release effect, but there are disadvantages in that it is not easy to administer by injection due to high viscosity, and it is difficult to release the drug for more than 1 day as the gel is easily diluted or decomposed by body fluids after injection.

On the other hand, there are examples of preparing solid microparticles by emulsion solvent extraction using a hyaluronic acid derivative having hydrophobicity (for example, hyaluronic acid-benzyl ester) (N.S. Nightlinger, et al., Proceed. Intern. Symp. Control. Rel. Bioact. Mater., 22nd, Paper No. 3205 (1995); L. Ilum, et al., J. Controlled Rel., 29, 133(1994)). Since it is necessary to use an organic solvent in preparation of the drug release formulation particles using a hydrophobic hyaluronic acid derivative, there is a risk of denaturation of the protein drug by contact with the organic solvent, and the possibility of denaturation of the protein due to the hydrophobicity of the hyaluronic acid derivative is high.

Therefore, in order to improve in vivo persistence of a protein or peptide drug, an approach different from the conventional studies is required.

On the other hand, GDF15 (Growth differentiation factor 15) is a member of the TGF-beta family, and is a 25kDa homodimer, and is a secretory protein circulating in plasma. The plasma level of GDF15 is related to BMI (body mass index) and GDF15 plays a role as a long-term regulator of energy homeostasis. GDF15 also has protective actions in pathological conditions such as cardiovascular disease, myocardial hypertrophy and ischemic injury. In addition, GDF15 plays a protective role against renal tubular and renal interstitial damage in models of type 1 diabetes and type 2 diabetes. Furthermore, GDF15 has a protective effect against age-related sensory and motor nerve loss, and can contribute to peripheral nerve damage recovery. Moreover, GDF15 has effects of weight loss and body fat reduction and glucose tolerance, and has an effect of increasing systemic energy consumption and oxidative metabolism. GDF15 exhibits an effect of glycemic control through body weight-dependent and non-dependent mechanisms.

The development of a technology for improving in vivo persistence of GDF15 protein exhibiting such various pharmacological effects is required.

[DISCLOSURE]

[TECHNICAL PROBLEM]

In the present description, provided is a technology of increasing an in vivo half-life of GDF15 to enhance the in vivo duration and thereby, increasing the administration interval, by linking a polypeptide capable of O-glycosylation (for example, immunoglobulin hinge region, etc.) to GDF15 (Growth differentiation factor 15) to form a fusion polypeptide, compared to the case where it is not fused with a polypeptide region capable of O-glycosylation.

One embodiment provides a fusion polypeptide comprising GDF15 and a polypeptide region capable of O-glycosylation.

In the fusion polypeptide, the polypeptide region capable of O-glycosylation may be comprised in the N-terminus of the GDF15.

The total number of the polypeptide region capable of O-glycosylation comprised in the fusion polypeptide may be 1 or more, for example, 1 to 10, 1 to 8, 1 to 6, 1 to 4, 2 to 10, 2 to 8, 2 to 6, 2 to 4 (for example, 1, 2, 3, 4, 5, 6, 7, 8, 9 or 10).

In one embodiment, the fusion polypeptide may be represented by the following general formula:

N'-(Z)n-Y -C'    [general formula]

in the formula,

N' is the N-terminus of the fusion polypeptide, and C' is the C-terminus of the fusion polypeptide, and
Y is GDF15, and
Z is a polypeptide region capable of O-glycosylation, and
n is the number of the polypeptide region capable of O-glycosylation positioned at the N-terminus of the fusion polypeptide (bound to the N-terminus of GDF15) and an integer of 1 to 10 (i.e., 1, 2, 3, 4, 5, 6, 7, 8, 9, 10), 1 to 7, 1 to 5, or 1 to 3.

The n polypeptide regions capable of O-glycosylation comprised in the fusion polypeptide may be each independently selected among polypeptide regions comprising amino acid residues capable of O-glycosylation. For example, the polypeptide regions comprising amino acid residues capable of O-glycosylation may be immunoglobulin hinge regions. In one embodiment, the polypeptide regions capable of O-glycosylation may be selected from the group consisting of immunoglobulin D (IgD) hinge regions and immunoglobulin A (IgA, for example, IgA1) hinge regions (i.e., n immunoglobulin hinge regions may be same or different each other).

In the fusion polypeptide, the GDF15 fused with the polypeptide region capable of O-glycosylation, is characterized by having increased in vivo (or in blood) stability (duration), compared to the GDF15 not fused with the polypeptide region capable of O-glycosylation (for example, in vivo or blood half-life increase).

Another embodiment provides a nucleic acid molecule encoding the fusion polypeptide.

Another embodiment provides a recombinant vector comprising the nucleic acid molecule.

Another embodiment provides a recombinant cell comprising the recombinant vector.

Another embodiment provides a method for preparation of GDF15 with an increased in vivo (or in blood) half-life, or a method for preparation of a fusion polypeptide comprising the GDF15 with an increased in vivo (or in blood) half-life, comprising expressing the recombinant vector in a cell.

Another embodiment provides a method for increasing in vivo duration of GDF15, or a method for enhancing in vivo (or in blood) stability of a GDF15 (protein or peptide) drug and/or increasing an in vivo (or in blood) half-life, comprising fusing (or linking or binding) GDF15 and a polypeptide region capable of O-glycosylation. In one specific example, the fusing may comprise fusing (or linking or binding) one or more polypeptide regions capable of O-glycosylation at the N-terminus of GDF15 through or not through a linker. The fusing (or linking or binding) may be performed in vitro.

Another embodiment provides a fusion polypeptide dimer, comprising two of the fusion polypeptides. The fusion polypeptide dimer may be formed by being linked by a bond (for example, disulfide bond) between GDF15 comprised in each fusion polypeptide. The fusion polypeptide dimer may be a homodimer.

Another embodiment provides a pharmaceutical composition comprising one or more selected from the group consisting of the fusion polypeptide, a fusion polypeptide dimer comprising the fusion polypeptide, a nucleic acid molecule encoding the fusion polypeptide, a recombinant vector comprising the nucleic acid molecule and a recombinant cell comprising the recombinant vector.

Another embodiment provides a method for preparing a pharmaceutical composition using one or more selected from the group consisting of the fusion polypeptide, a fusion polypeptide dimer comprising the fusion polypeptide, a nucleic acid molecule encoding the fusion polypeptide, a recombinant vector comprising the nucleic acid molecule and a recombinant cell comprising the recombinant vector.

Another embodiment provides a use of one or more selected from the group consisting of the fusion polypeptide, a fusion polypeptide dimer comprising the fusion polypeptide, a nucleic acid molecule encoding the fusion polypeptide, a recombinant vector comprising the nucleic acid molecule and a recombinant cell comprising the recombinant vector, for preparing a pharmaceutical composition.

Another embodiment provides a use of a polypeptide region capable of O-glycosylation for enhancing in vivo (or in blood) stability and/or increasing an in vivo (or in blood) half-life of a GDF15 (protein or peptide) drug. Specifically, an embodiment provides a composition for enhancing in vivo (or in blood) stability and/or increasing an in vivo (or in blood) half-life of a GDF15 (protein or peptide) drug, the composition comprising a polypeptide region capable of O-glycosylation.

[TECHNICAL SOLUTION]

The present description provides a technology capable of enhancing in vivo (or in blood) stability and/or in vivo (or in blood) duration in case of in vivo application of GDF15, by providing a fusion polypeptide form in which a polypeptide region capable of O-glycosylation such as an immunoglobulin hinge region is fused to GDF15.
One embodiment provides a fusion polypeptide comprising GDF15 and a polypeptide region capable of O-glycosylation.
In the fusion polypeptide, the polypeptide region capable of O-glycosylation may be comprised at the N-terminus of the GDF15.
The total number of the polypeptide region capable of O-glycosylation comprised in the fusion polypeptide may be 1 or more, for example, 1 to 10, 1 to 8, 1 to 6, 1 to 4, 2 to 10, 2 to 8, 2 to 6, 2 to 4 (for example, 1, 2, 3, 4, 5, 6, 7, 8, 9 or 10).
In one embodiment, the fusion polypeptide may be represented by the following general formula:

N'-(Z)n-Y -C' [general formula]

in the formula,

N' is the N-terminus of the fusion polypeptide, and C' is the C-terminus of the fusion polypeptide, and
Y is GDF15, and
Z is a polypeptide region capable of O-glycosylation, and
n is the number of the polypeptide region capable of O-glycosylation positioned at the N-terminus of the fusion polypeptide (bound to the N-terminus of GDF15) and an integer of 1 to 10 (i.e., 1, 2, 3, 4, 5, 6, 7, 8, 9, 10), 1 to 7, 1 to 5, or 1 to 3.

In one embodiment, in the fusion polypeptide, when the active site of GDF15 is positioned at the C-terminus, the polypeptide region capable of O-glycosylation may be fused to the N-terminus.
The n polypeptide regions capable of O-glycosylation comprised in the fusion polypeptide may be each independently selected among polypeptide regions comprising amino acid residues capable of O-glycosylation. For example, the polypeptide regions comprising amino acid residues capable of O-glycosylation may be immunoglobulin hinge regions. In one embodiment, the polypeptide regions capable of O-glycosylation may be selected from the group consisting of immunoglobulin D (IgD) hinge regions and immunoglobulin A (IgA, for example, IgA1) hinge regions (i.e., n immunoglobulin hinge regions may be same or different each other).
In one specific embodiment, the polypeptide region capable of O-glycosylation positioned (comprised) at the N-terminus of the fusion polypeptide may be 1 or 2, and in case of 2 or more, each of the polypeptide regions capable of O-glycosylation may be same or different each other. In one specific embodiment, one or more (for example, 1, 2, 3, 4, 5, 6, 7, 8, 9, or 10) polypeptide regions capable of O-glycosylation positioned at the N-terminus may be all IgD hinge regions or IgA (for example, IgA1) hinge regions, or comprise one or more (for example, 1, 2, 3, 4, 5, 6, 7, 8, 9, or 10) IgD hinge regions and one or more (for example, 1, 2, 3, 4, 5, 6, 7, 8, 9, or 10) IgA (for example, IgA1) hinge regions in various orders.
In other specific embodiment, when all the n polypeptide regions capable of O-glycosylation comprised in the fusion polypeptide are positioned only at the N-terminus of the fusion polypeptide (in other words, when one or more polypeptide regions capable of O-glycosylation are present only at the N-terminus of the fusion polypeptide), the one or more (for example, 1, 2, 3, 4, 5, 6, 7, 8, 9, or 10) polypeptide regions capable of O-glycosylation may be all IgD hinge regions or IgA hinge regions, or comprise one or more (for example, 1, 2, 3, 4, 5, 6, 7, 8, 9, or 10) IgD hinge regions and one or more (for example, 1, 2, 3, 4, 5, 6, 7, 8, 9, or 10) IgA hinge regions in various orders.
The polypeptide region capable of O-glycosylation (each region when the polypeptide region capable of O-glycosylation is 2 or more) may comprise 1 or more, 2 or more, 3 or more, 4 or more, 5 or more, 6 or more, or 7 or more O-glycosylation residues (the upper limit is 100, 50, 25, 20, 19, 18, 17, 16, 15, 14, 13, 12, 11, 10, 9, or 8) (for example, 1, 2, 3, 4, 5, 6, 7 or 8). For example, the polypeptide region capable of O-glycosylation (each region when the polypeptide region capable of O-glycosylation is 2 or more) may comprise 1 to 10 or 3 to 10 O-glycosylation residues (amino acid residues capable of O-glycosylation).
In one embodiment, the polypeptide region capable of O-glycosylation may be one or more selected from immunoglobulin (for example, human immunoglobulin) hinge regions, and for example, may be IgD hinge regions, IgA hinge regions or a combination thereof.
Since hinge regions such as IgD hinge regions (for example, human IgD hinge regions) and/or IgA hinge regions (for example, human hinge regions) among the regions of immunoglobulin (for example, human immunoglobulin) comprise a residue capable of O-glycosylation, the polypeptide region capable of O-glycosylation may necessarily comprise one or more (human) IgD hinge regions and/or one or more (human) IgA hinge regions, or necessarily consist of the hinge regions. In one specific embodiment, the polypeptide region capable of O-glycosylation may not comprise one or more (e.g., 1, 2, or all 3) selected from the group consisting of CH1, CH2, and CH3 of immunoglobulin regions not comprising a residue capable of O-glycosylation (for example, IgD and/or IgA).

In addition, considering the number of appropriate residues capable of O-glycosylation in the fusion polypeptide provided in the present description, the polypeptide capable of O-glycosylation may comprise one or more, more specifically, 2 or more (for example, 2, 3, 4, 5, 6, 7, 8, 9, or 10) IgD hinge regions (for example, human IgD hinge regions) and/or IgA hinge regions (for example, human IgA hinge regions).

More specifically, the IgD may be human IgD (for example, UniProKB P01880 (invariant domain; SEQ ID NO: 7), etc.), and the hinge region of IgD may be one or more selected from the group consisting of

a polypeptide comprising an amino caid sequence of "N'-ESPKAQA**SS**VP**T**AQPQAEGSLAKA**TT**APA**TT**RNT-C' (SEQ ID NO: 1); amino acid residues in bold are residues capable of O-glycosylation (7 in total)" or essentially consisting of the amino acid sequence ("IgD hinge"),
a polypeptide comprising 5 or more, 7 or more, 10 or more, 15 or more, 20 or more, 22 or more, or 24 or more (the upper limit is 34 or 33) consecutive amino acids comprising one or more, 2 or more, 3 or more, 4 or more, 5 or more, 6 or more, or 7 or more O-glycosylation residues in the amino acid sequence of SEQ ID NO: 1, or essentially consisting of the amino acids ("a part of IgD hinge"; for example, a polypeptide comprising 5 or more continuous amino acids comprising "SSVPT" (SEQ ID NO: 9) in SEQ ID NO: 1 or a polypeptide comprising 7 or more continuous amino acids comprising "TTAPATT" (SEQ ID NO: 10)), and
a polypeptide comprising 34 or more or 35 or more continuous amino acids comprising the amino acid sequence (IgD hinge) of SEQ ID NO: 1, in the IgD (for example, SEQ ID NO: 7) or 7 or more, 10 or more, 15 or more, 20 or more, 22 or more or 24 or more continuous amino acids comprising a part of the IgD hinge, or essentially consisting of the amino acids ("extension of IgD hinge"; for example, a polypeptide comprising 34 or more or 35 or more continuous amino acids comprising SEQ ID NO: 1 in "ESPKAQASS VPTAQPQAEG SLAKATTAPA TTRNTGRGGE EKKKEKEKEE QEERETKTP" (SEQ ID NO: 11) in the IgD (SEQ ID NO: 7) or a part of the IgD hinge).

The IgA may be human IgA (for example, IgA1 (UniProKB P01876, invariant domain; SEQ ID NO: 8), etc.), and the hinge region of the IgA may be one or more selected from the group consisting of

a polypeptide comprising an amino acid sequence of "N'-VP**ST**PP**T**P**S**P**S**TPP**T**P**S**P**S**-C' (SEQ ID NO: 2); amino acid residues in bold are residues capable of O-glycosylation (8 in total)" or essentially consisting of the amino acid sequence ("IgA hinge"),
a polypeptide comprising 5 or more, 6 or more, 7 or more, 8 or more, 9 or more, 10 or more, 12 or more, 15 or more, 17 or more or 18 continuous amino acids comprising 1 or more, 2 or more, 3 or more, 4 or more, 5 or more, 6 or more, 7 or more or 8 O-glycosylation residues in the amino acid sequence of SEQ ID NO: 2, or essentially consisting of the amino acid sequence ("a part of IgA hinge"; for example, a polypeptide comprising 8 or more or 9 or more amino acids comprising "STPPTPSP" (SEQ ID NO: 12) in SEQ ID NO: 2), and
19 or more or 20 or more continuous amino acids comprising the amino acid sequence (IgA (for example, IgA1) hinge), in IgA (for example, IgA1 (SEQ ID NO: 8)), or a polypeptide comprising 7 or more, 10 or more, 12 or more, 15 or more, 17 or more, or 18 continuous amino acids comprising a part of the IgA (for example, IgA1) hinge, or essentially consisting of the amino acid sequence ("extension of IgA hinge").

In other embodiment, the polypeptide region capable of O-glycosylation may be a polypeptide region comprising 5 or more, 7 or more, 10 or more, 12 or more, 15 or more, 17 or more, 20 or more, 22 or more, 25 or more, 27 or more, 30 or more, 32 or more or 35 or more (the upper limit is 40, 50, 60, 70, 80, 90, 100, 150, 200, 250, 300 or the total amino acid number of each protein) continuous amino acids comprising 1 or more, 2 or more, 5 or more, 7 or more, 10 or more, 12 or more, 15 or more, 17 or more, 20 or more, or 22 or more (for example, 1 to 10, 3 to 10; or 1, 2, 3, 4, 5, 6, 7, 8, 9, 10, 11, 12, 13, 14, 15, 16, 17, 18, 15, 16, 17, 18, 19, 20, 21, 22, 23, 24 or 25) amino acid residues capable of O-glycosylation in the proteins indicated in the following Table 1 (for example, proteins comprising an amino acid sequence selected from the group consisting of SEQ ID NOs: 23 to 113) or essentially consisting of the amino acids. In the present description, it is preferred that the polypeptide region capable of O-glycosylation does not affect the function of GDF15. The polypeptide region capable of O-glycosylation of the proteins indicated in Table below may be selected from among regions not involved in the original function of a full-length protein, and thereby, the polypeptide region capable of O-glycosylation may only serve to increase the half-life without affecting the function of GDF15:

[Table 1]

| UniProtKB Entry No. | UniProtKB Entry name | Protein names | Gene names | Length | O-Glycosylation (site) | SEQ ID NO: |
|---|---|---|---|---|---|---|
| Q96DR8 | MUCL1_HUMAN | Mucin-like protein 1 | MUCL1 SBEM UNQ590/PRO1160 | 90 | 23T, 24T, 30T, 34T, 46T, 47T, 51T, 52T, 54T, 55T, 59T, 60T, 62T, 63T, 66S, 67T, 68T | 23 |
| Q0VAQ4 | SMAGP_HUMAN | Small cell adhesion glycoprotein | SMAGP | 97 | 2T, 3S, 6T, 7T, 9S, 16T, 17T, 23T | 24 |
| P04921 | GLPC_HUMAN | Glycophorin-C | GYPC GLPC GPC | 128 | 3S, 4T, 6S, 9S, 10T, 15S, 24S, 26S, 27T, 28T, 31T, 32T, 33T, 42S | 25 |
| P16860 | ANFB_HUMAN | Natriuretic peptides B | NPPB | 134 | 62T, 63S, 70S, 74T, 79S, 84T, 97T | 26 |
| P04141 | CSF2_HUMAN | Granulocyte-macrophage colony-stimulating factor | CSF2 GMCSF | 144 | 22S, 24S, 26S, 27T | 27 |
| P02724 | GLPA_HUMAN | Glycophorin-A | GYPA GPA | 150 | 21S, 22T, 23T, 29T, 30S, 31T, 32S, 36T, 38S, 41S, 44T, 52T, 56T, 63S, 66S, 69T | 28 |
| P10124 | SRGN_HUMAN | Serglycin | SRGN PRG PRG1 | 158 | 94S, 96S, 100S, 102S, 104S, 106S, 108S, 110S | 29 |
| Q86YL7 | PDPN_HUMAN | Podoplanin | PDPN GP36 PSEC0003 PSEC0025 | 162 | 25T, 32T, 34T, 35T, 52T, 55T, 65T, 66T, 76T, 85T, 86S, 88S, 89T, 96S, 98S, 100T, 102S, 106T, 107S, 109S, 110T, 117T, 119T, 120T | 30 |
| P0DN87 | CGB7_HUMAN | Choriogonadotropin subunit beta 7 | CGB7 | 165 | 139S, 141S, 147S, 150S, 152S, 158S | 31 |
| P0DN86 | CGB3_HUMAN | Choriogonadotropin subunit beta 3 | CGB3 CGB; CGB5; CGB8 | 165 | 139S, 141S, 147S, 150S, 152S, 158S | 32 |
| P01344 | IGF2_HUMAN | Insulin-like growth factor II | IGF2 PP1446 | 180 | 96T, 99T, 163T | 33 |
| P07498 | CASK_HUMAN | Kappa-casein | CSN3 CASK CSN10 CSNK | 182 | 133T, 143T, 148T, 151T, 157T, 167T, 169T, 178T | 34 |
| P31431 | SDC4_HUMAN | Syndecan-4 | SDC4 | 198 | 39S, 61S, 63S | 35 |
| P34741 | SDC2_HUMAN | Syndecan-2 | SDC2 HSPG1 | 201 | 41S, 55S, 57S, 101T | 36 |

(continued)

| UniProtKB Entry No. | UniProtKB Entry name | Protein names | Gene names | Length | O-Glycosylation (site) | SEQ ID NO: |
|---|---|---|---|---|---|---|
| Q99075 | HBEGF_HUMAN | Proheparin-binding EGF-like growth factor | HBEGF DTR DTS HEGF L | 208 | 37T, 38S, 44T, 47T, 75T, 85T | 37 |
| P13727 | PRG2_HUMAN | Bone marrow proteoglycan (BMPG) | PRG2 MBP | 222 | 23T, 24S, 25T, 34T, 62S | 38 |
| P24592 | IBP6_HUMAN | Insulin-like growth factor-binding protein 6 (IBP-6) | IGFBP 6 IBP6 | 240 | 126T, 144S, 145T, 146T, 152S | 39 |
| Q9UHG2 | PCSK1_HUMAN | ProSAAS (Proprotein convertase subtilisin/kexin type 1 inhibitor) | PCSK 1N | 260 | 53T, 228S, 247T | 40 |
| P01589 | IL2RA_HUMAN | Interleukin-2 receptor subunit alpha (IL-2 receptor subunit alpha) | IL2RA | 272 | 218T, 224T, 229T, 237T | 41 |
| P21583 | SCF_HUMAN | Kit ligand (Mast cell growth factor) (MGF) | KITLG MGF SCF | 273 | 167S, 168T, 180T | 42 |
| A1E959 | ODAM_HUMAN | Odontogenic ameloblast-associated protein (Apin) | ODAM APIN | 279 | 115T, 119T, 244T, 249S, 250T, 251T, 255T, 256S, 261T, 263T, 273T, 275S | 43 |
| P10451 | OSTP_HUMAN | Osteopontin | SPP1 BNSP OPN PSEC 0156 | 314 | 134T, 138T, 143T, 147T, 152T | 44 |
| P21815 | SIAL_HUMAN | Bone sialoprotein 2 (Bone sialoprotein II) (BSP II) | IBSP BNSP | 317 | 119T, 122T, 227T, 228T, 229T, 238T, 239T | 45 |
| P02649 | APOE_HUMAN | Apolipoprotein E (Apo-E) | APOE | 317 | 26T, 36T, 212T, 307T, 308S, 314S | 46 |
| Q99645 | EPYC_HUMAN | Epiphycan (Dermatan sulfate proteoglycan 3) | EPYC DSPG3 PGLB SLRR3 B | 322 | 60T, 64S, 96S | 47 |

(continued)

| UniProtKB Entry No. | UniProtKB Entry name | Protein names | Gene names | Length | O-Glycosylation (site) | SEQ ID NO: |
|---|---|---|---|---|---|---|
| Q6UXG3 | CLM9_HUMAN | CMRF35-like molecule 9 (CLM-9) | CD300LG CLM9 TREM4 UNQ422/PRO846 | 332 | 137T, 143T, 144T, 155T, 161T, 170T, 171T, 177T, 187T, 195T, 196S, 199T, 201T, 202S, 207T, 208S, 213S, 214S, 222S, 223T, 224S, 228T, 229S, 237S | 48 |
| Q9GZM5 | YIPF3_HUMAN | Protein YIPF3 (Killer lineage protein 1) | YIPF3 C6orf109 KLIP1 | 350 | 333T, 334T, 339T, 346T | 49 |
| P51681 | CCR5_HUMAN | C-C chemokine receptor type 5 (C-C CKR-5) | CCR5 CMKBR5 | 352 | 6S, 7S, 16T, 17S | 50 |
| P40225 | TPO_HUMAN | Thrombopoietin (C-mpl ligand) (ML) | THPO MGDF | 353 | 22S, 58T, 131T, 179T, 180T, 184S, 213T, 265S | 51 |
| P01876 | IGHA1_HUMAN | Immunoglobulin heavy constant alpha 1 (lg alpha-1 chain C region) | IGHA1 | 353 | 105S, 106T, 109T, 111S, 113S, 117T, 119S, 121S | 8 |
| P02765 | FETUA_HUMAN | Alpha-2-HS-glycoprotein (Alpha-2-Z-globulin) | AHSG FETUA PRO2743 | 367 | 270T, 280S, 293S, 339T, 341T, 346S | 52 |
| P21810 | PGS1_HUMAN | Biglycan | BGN SLRR1A | 368 | 42S, 47S, 180S, 198S | 53 |
| P01860 | IGHG3_HUMAN | Immunoglobulin heavy constant gamma 3 (HDC) | IGHG3 | 377 | 122T, 137T, 152T | 54 |
| P80370 | DLK1_HUMAN | Protein delta homolog 1 (DLK-1) | DLK1 DLK | 383 | 94S, 143T, 163S, 214S, 222T 251S 256T, 260S | 55 |
| P01880 | IGHD_HUMAN | Immunoglobulin heavy constant delta (lg delta chain C region) | IGHD | 384 | 109S,110S, 113T, 126T, 127T, 131T, 132T | 7 |
| P15529 | MCP_HUMAN | Membrane cofactor protein (TLX) | CD46 MCP MIC10 | 392 | 290S, 291S, 292T, 298S, 300S, 302S, 303T, 304S, 305S, 306T, 307T, 309S, 312S, 313S, 315S, 320T, 326S | 56 |
| P04280 | PRP1_HUMAN | Basic salivary proline-rich protein 1 | PRB1 | 392 | 40S, 87S, 150S, 330S | 57 |

(continued)

| UniProtKB Entry No. | UniProtKB Entry name | Protein names | Gene names | Length | O-Glycosylation (site) | SEQ ID NO: |
|---|---|---|---|---|---|---|
| P78423 | X3CL1_HUMAN | Fractalkine (C-X3-C motif chemokine 1) | CX3CL1 FKN NTT SCYD1 A-152E5.2 | 397 | 183T, 253S, 329T | 58 |
| P16150 | LEUK_HUMAN | Leukosialin (GPL115) | SPN CD43 | 400 | 21T, 22T, 26T, 28T, 29S, 35S, 36T, 37S, 41S, 42S, 46T, 47T, 48S, 50T, 58T, 69T, 99S, 103S, 109T, 113T, 114S, 136T, 137T, 173T, 178T | 59 |
| P13473 | LAMP2_HUMAN | Lysosome-associated membrane glycoprotein 2 (LAMP-2) | LAMP 2 | 410 | 195S, 196T, 200T, 203T, 204T, 207S, 209T, 210T, 211T,213T | 60 |
| P11279 | LAMP1_HUMAN | Lysosome-associated membrane glycoprotein 1 (LAMP-1) | LAMP 1 | 417 | 197S, 199T, 200T, 207S, 209S, 211S, | 61 |
| P21754 | ZP3_HUMAN | Zona pellucida sperm-binding protein 3 (Sperm receptor) | ZP3 ZP3A ZP3B ZPC | 424 | 156T, 162T, 163T | 62 |
| P05783 | K1C18_HUMAN | Keratin, type I cytoskeletal 18 | KRT18 CYK18 PIG46 | 430 | 30S, 31S, 49S | 63 |
| Q08629 | TICN1_HUMAN | Testican-1 (Protein SPOCK) | SPOCK1 SPOCK TIC1 TICN1 | 439 | 228T, 383S, 388S | 64 |
| O75056 | SDC3_HUMAN | Syndecan-3 (SYND3) | SDC3 KIAA0468 | 442 | 80S, 82S, 84S, 91S, 314S, 367S | 65 |
| P10645 | CMGA_HUMAN | Chromogranin-A (CgA) | CHGA | 457 | 181T, 183T, 251T | 66 |
| P15169 | CBPN_HUMAN | Carboxypeptidase N catalytic chain (CPN) | CPN1 ACBP | 458 | 400T, 402T, 409T | 67 |
| P00740 | FA9_HUMAN | Coagulation factor IX (EC 3.4.21.22) | F9 | 461 | 85T, 99S, 107S | 68 |

(continued)

| UniProtKB Entry No. | UniProtKB Entry name | Protein names | Gene names | Length | O-Glycosylation (site) | SEQ ID NO: |
|---|---|---|---|---|---|---|
| P20333 | TNR1B_HUMAN | Tumor necrosis factor receptor superfamily member 1B | TNFRSF1B TNFBR TNFR2 | 461 | 30T, 206T, 221S, 222T, 224S, 230T, 234S, 235T, 239T, 240S, 248S | 69 |
| P08670 | VIME_HUMAN | Vimentin | VIM | 466 | 7S, 33T, 34S | 70 |
| Q8WXD2 | SCG3_HUMAN | Secretogranin-3 (Secretogranin III) (SgIII) | SCG3 UNQ2502/PRO5990 | 468 | 216T, 231T, 359S | 71 |
| Q16566 | KCC4_HUMAN | Calcium/calmodulin-dependent protein kinase type IV (CaMK IV) (EC 2.7.11.17) | CAMK4 CAMK CAMK-GR CAMKIV | 473 | 57T, 58S, 137S, 189S, 344S, 345S,356S | 72 |
| P31749 | AKT1_HUMAN | RAC-alpha serine/threonine-protein kinase (EC 2.7.11.1) | AKT1 PKB RAC | 480 | 126S, 129S, 305T, 312T, 473S | 73 |
| P31751 | AKT2_HUMAN | RAC-beta serine/threonine-protein kinase (EC 2.7.11.1) | AKT2 | 481 | 128S, 131S, 306T, 313T | 74 |
| O60883 | G37L1_HUMAN | G-protein coupled receptor 37-like 1 | GPR37L1 ETBRLP2 | 481 | 79T, 85T, 86S, 95T, 107T | 75 |
| Q9BXF9 | TEKT3_HUMAN | Tektin-3 | TEKT3 | 490 | 7T, 9T, 10T | 76 |
| P05155 | IC1_HUMAN | Plasma protease C1 inhibitor (C1 Inh) | SERPING1 C1IN C1NH | 500 | 47T, 48T, 64S, 71T, 83T, 88T, 92T, 96T | 77 |
| P11831 | SRF_HUMAN | Serum response factor (SRF) | SRF | 508 | 277S, 307S, 309S, 316S, 383S | 78 |
| P0DOX3 | IGD_HUMAN | Immunoglobulin delta heavy chain | | 512 | 238S, 255T, 256T, 260T, 261T, | 79 |
| O75487 | GPC4_HUMAN | Glypican-4 (K-glypican) | GPC4 UNQ474/PRO937 | 556 | 494S, 498S, 500S | 80 |

(continued)

| UniProtKB Entry No. | UniProtKB Entry name | Protein names | Gene names | Length | O-Glycosylation (site) | SEQ ID NO: |
|---|---|---|---|---|---|---|
| P35052 | GPC1_HUMAN | Glypican-1 | GPC1 | 558 | 486S, 488S, 490S | 81 |
| P78333 | GPC5_HUMAN | Glypican-5 | GPC5 | 572 | 441S, 486S, 495S, 507S, 509S | 82 |
| Q8N158 | GPC2_HUMAN | Glypican-2 | GPC2 | 579 | 55S, 92S, 155S, 500S, 502S | 83 |
| P00748 | FA12_HUMAN | Coagulation factor XII (EC 3.4.21.38) | F12 | 615 | 109T, 299T, 305T, 308S, 328T, 329T, 337T | 84 |
| P01042 | KNG1_HUMAN | Kininogen-1 (Alpha-2-thiol proteinase inhibitor) | KNG1 BDK KNG | 644 | 401T, 533T, 542T, 546T, 557T, 571T, 577S, 628T | 85 |
| P51693 | APLP1_HUMAN | Amyloid-like protein 1 (APLP) (APLP-1) | APLP1 | 650 | 215T, 227S, 228T | 86 |
| Q9NQ79 | CRAC1_HUMAN | Cartilage acidic protein 1 (68 kDa chondrocyte-expressed protein) (CEP-68) (ASPIC) | CRTAC1 ASPIC 1 CEP68 | 661 | 608T, 618T, 619T, 621T, 626T | 87 |
| Q14515 | SPRL1_HUMAN | SPARC-like protein 1 (High endothelial venule protein) (Hevin) (MAST 9) | SPARCL1 | 664 | 31T, 40T, 44S, 116T | 88 |
| Q16820 | MEP1B_HUMAN | Meprin A subunit beta (EC 3.4.24.63) | MEP1 B | 701 | 593S, 594T, 599T, 603S | 89 |
| P17600 | SYN1_HUMAN | Synapsin-1 (Brain protein 4.1) (Synapsin I) | SYN1 | 705 | 55S, 87T, 96S, 103S, 261S, 432S, 526T, 564T, 578S | 90 |
| P19835 | CEL_HUMAN | Bile salt-activated lipase (BAL) (EC 3.1.1.13) (EC 3.1.1.3) | CEL BAL | 753 | 558T, 569T, 579T, 607T, 618T, 629T, 640T, 651T, 662T, 673T | 91 |
| Q9HCU0 | CD248_HUMAN | Endosialin (Tumor endothelial marker 1) (CD antigen CD248) | CD248 CD164 L1 TEM1 | 757 | 60T, 401T, 428T, 448T, 456T, 459T, 472T, 519T, 541T, 543T, 544T, 545T, 587T, 593T, 594T, 595T, 598S, 601S, 612T, 619T, 623S, 625S, 627T, 630T, 631S, 636T, 640S, | 92 |

(continued)

| UniProtKB Entry No. | UniProtKB Entry name | Protein names | Gene names | Length | O-Glycosylation (site) | SEQ ID NO: |
|---|---|---|---|---|---|---|
| P05067 | A4_HUMAN | Amyloid-beta precursor protein (APP) | APP A4 AD1 | 770 | 633T, 651T, 652T, 656S, 659T, 663T, 667S, | 93 |
| Q9NR71 | ASAH2_HUMAN | Neutral ceramidase (N-CDase) (NCDase) (EC 3.5.1.-) (EC 3.5.1.23) | ASAH2 HNAC1 | 780 | 62T, 67S, 68T, 70T, 73S, 74T, 76T, 78S, 79S, 80T, 82T, 84T | 94 |
| P08047 | SP1_HUMAN | Transcription factor Sp1 | SP1 TSFP1 | 785 | 491S, 612S, 640T, 641S, 698S, 702S | 95 |
| Q17R60 | IMPG1_HUMAN | Interphotoreceptor matrix proteoglycan 1 | IMPG1 IPM150 SPACR | 797 | 403T, 421T, 432T, 442T | 96 |
| P19634 | SL9A1_HUMAN | Sodium/hydrogen exchanger 1 (APNH) | SLC9A1 APNH1 NHE1 | 815 | 42T, 56S, 61 T, 62T, 68T | 97 |
| P12830 | CADH1_HUMAN | Cadherin-1 (CAM 120/80) | CDH1 CDHE UVO | 882 | 280S, 285T, 358T, 470T, 472T, 509T, 576T, 578T, 580T | 98 |
| Q14118 | DAG1_HUMAN | Dystroglycan (Dystrophin-associated glycoprotein 1) | DAG1 | 895 | 63T, 317T, 319T, 367T, 369T, 372T, 379T, 388T, 455T | 99 |
| Q14624 | ITIH4_HUMAN | Inter-alpha-trypsin inhibitor heavy chain H4 (ITI heavy chain H4) (ITI-HC4) | ITIH4 IHRP ITIHL1 PK120 PRO1851 | 930 | 719T, 720T, 722T | 100 |
| P19823 | ITIH2_HUMAN | Inter-alpha-trypsin inhibitor heavy chain H2 (ITI heavy chain H2) (ITI-HC2) | ITIH2 IGHEP2 | 946 | 666T, 673S, 675T, 691T | 101 |
| Q9UPV9 | TRAK1_HUMAN | Trafficking kinesin-binding protein 1 | TRAK1 KIAA1042 OIP106 | 953 | 447S, 680S, 719S, 935T | 102 |
| P15941 | MUC1_HUMAN | Mucin-1 (MUC-1) | MUC1 PUM | 1255 | 131T, 139T, 140S, 144T | 103 |
| Q7Z589 | EMSY_HUMAN | BRCA2-interacting transcriptional repressor EMSY | EMSY C11orf30 GL002 | 1322 | 228S, 236S, 271T, 501T, 506T, 557S, 1120T | 104 |

(continued)

| UniProtKB Entry No. | UniProtKB Entry name | Protein names | Gene names | Length | O-Glycosylation (site) | SEQ ID NO: |
|---|---|---|---|---|---|---|
| Q92954 | PRG4_HUMAN | Proteoglycan 4 (Lubricin) | PRG4 MSF SZP | 1404 | 123S, 136S, 240T, 253T, 277T, 291T, 305T, 306S, 310T, 317S, 324T, 332T, 338T, 367T, 373S, 376T, 384T, 385T, 388S, 391T, 399T, 400T, 407T, 408T, 415T, 423T, 427S, 430T, 438T, 439T, 446T, 447T, 454T, 455T, 477T, 478T, 485T, 493T, 494T, 501T, 502T, 509T, 525T, 529S, 532T, 540T, 541T, 553S, 555T, 563T, 564T, 571T, 572T, 579T, 580T, 587T, 588T, 595T,603T, 604T, 611T, 612T, 616T, 619T, 627T, 676T, 683T, 684T, 691T, 692T, 699T, 700T, 704T, 707T, 723T, 724T, 736T, 768T, 769T, 776T, 777T, 792T, 793T, 805T, 812S, 829T, 837T, 838T, 892S, 900T, 930T, 931T, 962S, 963T, 968T, 975T, 978T, 979T, 980T, 1039T, 1161T | 105 |
| Q76LX8 | ATS13_HUMAN | A disintegrin and metalloprotease with thrombospondin motifs 13 (ADAM-TS 13) | ADAMTS13 C9orf8 UNQ6102/PRO20085 | 1427 | 399S, 698S, 757S,907S, 965S, 1027S, 1087S | 106 |
| P49790 | NU153_HUMAN | Nuclear pore complex protein Nup153 (153 kDa nucleoporin) (Nucleoporin Nup153) | NUP153 | 1475 | 534S, 544S, 908S, 909S, 1113S, 1156T | 107 |
| P31327 | CPSM_HUMAN | Carbamoyl-phosphate synthase [ammonia], mitochondrial (EC 6.3.4.16) | CPS1 | 1500 | 537S, 1331S, 1332T | 108 |
| Q8N6G6 | ATL1_HUMAN | ADAMTS-like protein 1 (ADAMTSL-1) (Punctin-1) | ADAMTSL1 ADAMTSR1 C9orf94 UNQ528/PRO1071 | 1762 | 48T, 312T, 391S, 451T | 109 |

(continued)

| UniProtKB Entry No. | UniProtKB Entry name | Protein names | Gene names | Length | O-Glycosylation (site) | SEQ ID NO: |
|---|---|---|---|---|---|---|
| P46531 | NOTC1_HUMAN | Neurogenic locus notch homolog protein 1 (Notch 1) (hN1) | NOTCH1 TAN1 | 2555 | 65S, 73T, 116T, 146S, 194T, 232T, 311T, 341S, 349T, 378S, 435S, 458S, 466T, 496S, 534S, 609S, 617T, 647S, 692T, 722S, 759S, 767T, 784S, 797S, 805T, 921S, 951S, 997T, 1027S, 1035T, 1065S, 1159T, 1189S, 1197T, 1273S, 1362T, 1379T, 1402T, | 110 |
| P04275 | VWF_HUMAN | von Willebrand factor (vWF) | VWF F8VWF | 2813 | 1248T, 1255T, 1256T, 1263S, 1468T, 1477T, 1486S, 1487T, | 111 |
| Q9UPA5 | BSN_HUMAN | Protein bassoon (Zinc finger protein 231) | BSN KIAA0434 ZNF231 | 3926 | 1343T, 1384T, 2314T, 2691T, 2936T | 112 |
| Q86WI1 | PKHL1_HUMAN | Fibrocystin-L (Polycystic kidney and hepatic disease 1-like protein 1) (PKHD1-like protein 1) | PKHD1L1 | 4243 | 122T, 445T, 1803T, 1839T, 2320T, 3736T | 113 |

The fusion polypeptide may have the total number of actually comprised O-glycan of 13 or more, 14 or more, 15 or more, 16 or more, 17 or more, 18 or more, 19 or more, 20 or more, or 21 or more (the maximum value is determined by the number of the disclosed polypeptide region capable of O-glycosylation and the number of O-glycosylation residues comprised in each of the polypeptide region capable of O-glycosylation), or have the total number of theoretically comprised O-glycan of 20 or more, 21 or more, 23 or 24 or more (the maximum value is determined by the number of the disclosed polypeptide region capable of O-glycosylation and the number of O-glycosylation residues comprised in each of the polypeptide region capable of O-glycosylation). In addition, in the fusion polypeptide, the total number of actually comprised O-glycan may be related to stability upon administration in the body (for example, in blood), and specifically, in the fusion polypeptide, as the total number of the actually comprised O-glycan increases, the in vivo stability of the fusion polypeptide or GDF15 comprised in the fusion polypeptide may increases (in other words, in vivo (in blood) half-life increase and/or in vivo (in blood) concentration increase and/or in vivo (in blood) rate of degradation decrease, etc.).

The fusion polypeptide may further comprise a peptide linker between GDF15 and a polypeptide region capable of O-glycosylation and/or between polypeptide regions capable of O-glycosylation when 2 or more of the polypeptide regions capable of O-glycosylation are comprised. In one embodiment, the peptide linker may be a GS linker repeatedly comprising one or more Gly(G) and one or more Ser(S), and for example, it may be (GGGGS)n (n is a repetition time of GGGGS (SEQ ID NO: 13) and is an integer of 1 to 10 or 1 to 5 (for example, 1, 2, 3, 4, or 5)), but not limited thereto.

Other embodiment provides a fusion polypeptide dimer, comprising 2 of the fusion polypeptides. The fusion polypeptide dimer may be formed by being linked by a bond (for example, disulfide bond) between GDF15 comprised in each of the fusion polypeptides. The fusion polypeptide dimer may be a homodimer.

In the fusion polypeptide and/or fusion polypeptide dimer, the GDF15 fused with the polypeptide region capable of O-glycosylation is characterized by increased in vivo (or in blood) stability, compared to GDF15 in which the polypeptide region capable of O-glycosylation is not fused (for example, in vivo or in blood half-life increase).

Other embodiment provides a nucleic acid molecule encoding the fusion polypeptide.

Other embodiment provides a recombinant vector comprising the nucleic acid molecule.

Other embodiment provides a recombinant cell comprising the recombinant vector.

Other embodiment provides a method for preparation of GGF15 with increased in vivo (or in blood) half-life, or a method for preparation of a fusion polypeptide comprising the GDF15 with increased in vivo (or in blood) half-life, comprising expressing the recombinant vector in a cell.

Other embodiment provides a method for increasing in vivo duration of GDF15 comprising fusing (or linking or binding) GDF15 and a polypeptide region capable of O-glycosylation. In one specific embodiment, the fusing may comprise fusing (or linking or binding) one or more polypeptide regions capable of O-glycosylation at the N-terminus, C-terminus or both terminuses of GDF15 through or not through a linker. The fusing (or linking or binding) may be progressed in vitro.

Other embodiment provides a pharmaceutical composition comprising one or more selected from the group consisting of the fusion polypeptide, a fusion polypeptide dimer comprising the fusion polypeptide, a nucleic acid molecule encoding the fusion polypeptide, a recombinant vector comprising the nucleic acid molecule and a recombinant cell comprising the recombinant vector.

Other embodiment provides a use of a polypeptide region capable of O-glycosylation for enhancing in vivo (or in blood) stability and/or increasing in vivo (or in blood) half-life of a polypeptide (protein or peptide) drug. Specifically, one embodiment provides a composition for enhancing in vivo (or in blood) stability and/or increasing in vivo (or in blood) half-life of a polypeptide (protein or peptide) drug comprising a polypeptide region capable of O-glycosylation. As used in the present description, enhancing stability and/or increasing half-life mean that the stability is enhanced and/or the half-life is increased, compared to a polypeptide (protein or peptide) not comprising a polypeptide region capable of O-glycosylation.

Hereinafter, the present invention will be described in more detail:

In the present description, GDF15 (Growth differentiation factor 15) (corresponding to Y in the general formula) is a soluble polypeptide, and consists of amino acids from the 197th (A) to 308th (I) except for a signal peptide and a propeptide in total 308 amino acids (UniProt Q99988) (SEQ ID NO: 3; See FIG. 1; mature form):

In the present description, GDF15 means, unless otherwise mentioned,

(1) the amino acid sequence from 197th (A) to 308th (I) of the full-length protein (UniProt Q99988) (SEQ ID NO: 3, See FIG. 1; ARNG DHCPLGPGRC CRLHTVRASL EDLGWADWVL SPREVQVTMC IGACPSQFRA ANMHAQIKTS LHRLKPDTVP APCCVPASYN PMVLIQKTDT GVSLQTYDDL LAKDCHCI);
(2) a functional variant of GDF15; and/or
(3) a polypeptide essentially comprising the amino acid sequence having the sequence homology of 80% or more, 85% or more, 90% or more, 95% or more, 96% or more, 97% or more, 98% or more, or 99% or more to the amino acid sequence of the (1) and/or (2) in a range of maintaining the intrinsic activity and structure.

In the present description, the functional variant of GDF15 may be a variant mutated to be advantageous for dimer structure formation, while maintaining the intrinsic activity and structure. In one embodiment, the functional variant of GDF15 may be a N-terminal deletion variant in which one or more (1, 2, 3, 4, 5, 6, 7, 8, 9, 10, 11, 12, 13, or 14) (for example, one or more from the N-terminus in order) at the N-terminus of the amino acid sequence of GDF15 of SEQ ID NO: 3 (in other words, 14 amino acid residues in total from the 1st to 14th) in SEQ ID NO: 1), for example, all the 14 amino acid residues are deleted. In one specific embodiment, the functional variant of GDF15 may be a polypeptide essentially comprising the amino acid sequence of SEQ ID NO: 4 (CRLHTVRASL EDLGWADWVL SPREVQVTMC IGACPSQFRA ANMHAQIKTS LHRLKPDTVP APCCVPASYN PMVLIQKTDT GVSLQTYDDL LAKDCHCI) or the amino acid sequence having the sequence homology of 80% or more, 85% or more, 90% or more, 95% or more, 96% or more, 97% or more, 98% or more, or 99% or more to the amino acid sequence in a range of maintaining the intrinsic activity and structure of GDF15.

In the fusion polypeptide comprising GDF15 and a polypeptide region capable of O-glycosylation provided in the present description, the GDF15 and polypeptide region capable of O-glycosylation and/or 2 or more of polypeptide regions capable of O-glycosylation may be linked directly (for example, without a linker) or linked through an appropriate linker (for example, peptide linker) covalently or non-covalently. The peptide linker may be a polypeptide consisting of any amino acids of 1 to 20, 1 to 15, 1 to 10, 2 to 20, 2 to 15 or 2 to 10, and the kind of the comprised amino acids is not limited. The peptide linker may comprise, for example, Gly, Asn and/or Ser residues, and may also comprise neutral amino acids such as Thr and/or Ala, but not limited thereto, and the amino acid sequence suitable for a peptide linker is known in the art. In one embodiment, the peptide linker may be a GS linker repeatedly comprising one or more Gly(G) and one or more Ser(S), and for example, may be (GGGGS)n (n is a repetition time of GGGGS (SEQ ID NO: 13) and is an integer of 1 to 10 or 1 to 5 (for example, 1, 2, 3, 4, or 5)), but not limited thereto.

In addition, the fusion polypeptide may comprise total 1 or more or total 2 or more (for example, 2 to 10, 2 to 8, 2 to 6, 2 to 5, 2 to 4, 2 or 3) polypeptide regions capable of O-glycosylation. When 2 or more of the polypeptide regions capable of O-glycosylation are comprised, in the fusion polypeptide, 2 or more of the polypeptide regions capable of O-glycosylation are linked to the N-terminus of GDF15 and each of the polypeptide regions capable of O-glycosylation may be same or different each other. Then, between the polypeptide regions capable of O-glycosylation and/or between the polypeptide region capable of O-glycosylation and human GDF15, the aforementioned peptide linker may be further comprised.

The fusion polypeptide provided in the present description may be recombinantly or synthetically produced, and it may not be naturally occurring.

The in vivo (in blood) half-life in a mammal of GDF15 comprised in the fusion polypeptide provided in the present description may be increased about 1.1 time or more, about 1.15 times or more, about 1.2 times or more, about 1.5 times or more, about 2 times or more, about 2.5 time or more, about 3 times or more, about 3.5 times or more, about 4 times or more, about 5 times or more, about 6 times or more, about 7 times or more, about 8 times or more, about 9 times or more, or about 10 times or more, compared to the GDF15 in which the polypeptide region capable of O-glycosylation is not fused. Otherwise, the highest blood concentration in case of administration in a mammal body of GDF15 comprised in the fusion polypeptide provided in the present description may be higher about 1.2 times or more, about 1.5 times or more, about 2 times or more, about 2.5 times or more, about 3 times or more, about 3.5 times or more, or about 4 times or more, compared to the not fused GDF15. Otherwise, the time of reaching the highest blood concentration in case of administration in a mammal body of the GDF15 comprised in the fusion polypeptide provided in the present description may be extended about 2 times or more, about 3 times or more, about 4 times or more, about 5 times or more, about 6 times or more, about 7 times or more, about 8 times or more, about 9 times or more, about 10 times or more, about 11 times or more, about 12 times or more, about 13 times or more, about 14 times or more, about 15 times or more, about 18 times or more, about 20 times or more, or about 22 times or more, compared to the not fused GDF15. Otherwise, the area under the blood concentration-time curve up to the measurable last blood gathering time ($AUC_{last}$) and/or the area under the blood concentration-time curve calculated by extrapolating from the measurable last blood gathering time to the infinite time ($AUC_{inf}$), in case of administration in a mammal body of the GDF15 comprised in the fusion polypeptide provided in the present description may be increased about 2 times or more, about 2.5 times or more, about 3 times or more, about 3.5 times or more, about 4 times or more, about 4.5 times or more, about 5 times or more, about 6 times or more, about 7 times or more, about 8 times or more, about 9 times or more, about 10 times or more, about 11 times or more, about 12 times or more, about 13 times or more, about 14 times or more, or about 15 times or more, compared to the GDF15 not fused with the polypeptide region capable of O-glycosylation.

As such, due to the increased GDF15 half-life, the GDF15 in a fusion polypeptide form to which a polypeptide region capable of O-glycosylation is linked, has an advantage of having a longer administration interval, compared to the GDF15 in a form to which a polypeptide region capable of O-glycosylation is not linked.

The fusion polypeptide comprising GDF15 and a polypeptide region capable of O-glycosylation may be prepared by a common chemical synthesis method or recombinant method.

In the present description, the term "vector" means an expression means to express a target gene in a host cell, and for example, may be selected from the group consisting of a plasmid vector, a cosmid vector and a virus vector such as a bacteriophage vector, an adenovirus vector, a retrovirus vector, and an adeno-related virus vector, and the like. In one embodiment, the vector which can be used for the recombinant vector may be produced on the basis of a plasmid (for example, pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, etc.), phage (for example, λgt4λB, λ-Charon, λΔz1, M13, etc.) or virus (for example, SV40, etc.), but not limited thereto.

The nucleic acid molecule encoding the fusion polypeptide in the recombinant vector may be operatively linked to a promoter. The term "operatively linked" means functional binding between a nucleic acid expression regulatory sequence (for example, promoter sequence) and other nucleic acid sequence. The regulatory sequence may regulate transcription and/or translation of other nucleic acid sequence by being "operatively linked".

The recombinant vector may be typically constructed as a vector for cloning or an expression vector for expression. As the expression vector, common ones used for expressing a foreign protein in a plant, animal or microorganism may be used. The recombinant vector may be constructed by various methods known in the art.

The recombinant vector may be expressed using a eukaryote as a host. When an eukaryote is to be expressed as a host, the recombinant vector may comprise a replication origin such as f1 replication origin, SV40 replication origin, pMB1 replication origin, adeno replication origin, AAV replication origin and/or BBV replication origin, and the like, but not limited thereto, in addition to a nucleic acid molecule to be expressed and the aforementioned promoter, a ribosome binding site, a secretory signal sequence (See Patent Publication No. 2015-0125402) and/or a transcription/translation termination sequence. In addition, a promoter derived from genome of a mammal cell (for example, metallothionein promoter) or a promoter derived from a mammal virus (for example, adenovirus late promoter, vaccinia virus 7.5K promoter, SV40 promoter, cytomegalovirus and *tk* promoter of HSV) may be used and all secretory signal sequences commonly available as a secretory signal sequence may be used, and for example, the secretory signal sequence disclosed in Patent Publication No. 2015-0125402 may be used, but not limited thereto, and as a transcription termination sequence, a polyadenylation sequence may be comprised.

The recombinant cell may be obtained by introducing (transforming or transfecting) the recombinant vector into an appropriate host cell. The host cell may be selected from all eukaryotes which can stably and continuously clone or express the recombinant vector. The eukaryote available as a host includes a yeast (*Saccharomyces cerevisiae*), an insect cell, a plant cell and an animal cell, and the like, and for example, includes mice (for example, COP, L, C127, Sp2/0, NS-0, NS-1, At20, or NIH3T3), rats (for example, PC12, PC12h, GH3, or MtT), hamsters (for example, BHK, CHO, GS genetic defect CHO, or DHFR genetic defect CHO), monkeys (for example, COS (COS1, COS3, COS7, etc.), CV1 orVero), humans (for example, HeLa, HEK-293, retina-derived PER-C6, cell derived from diploid fibroblast, myeloma cell or HepG2), other animal cells (for example, MDCK, etc.), insect cells (for example, Sf9 cell, Sf21 cell, Tn-368 cell, BTI-TN-5B1-4 cell, etc.), hybridoma, and the like, but not limited thereto.

By expressing a nucleic acid molecule encoding the fusion polypeptide provided in the present description in the aforementioned appropriate host cell, GDF15 with enhanced in vivo stability compared to the not fused form and a fusion polypeptide comprising thereof may be prepared. The method for preparation of the fusion polypeptide may comprise culturing a recombinant vector comprising the nucleic acid molecule. The culturing may be performed under a common culturing condition. In addition, the method for preparation may further comprise separating and/or purifying the fusion polypeptide from the culture, after the culturing.

For delivery (introduction) of the nucleic acid molecule or recombinant vector comprising the same, a delivery method widely known in the art may be used. As the delivery method, for example, when the host cell is a eukaryote, microinjection, calcium phosphate precipitation, electroporation, liposome-mediated transfection and gene bombardment, and the like may be used, but not limited thereto.

The method for selecting the transformed (recombinant vector-introduced) host cell may be easily conducted according to a method widely known in the art, using a phenotype expressed by a selection marker. For example, when the selection marker is a specific antibiotic resistant gene, a recombinant cell in which a recombinant vector is introduced may be easily selected by culturing in a medium containing the antibiotic.

The fusion polypeptide may be used in prevention and/or treatment of all diseases which are related to GDF15 deficiency and/or dysfunction or can be treated, alleviated or improved by GDF15 activity.

Accordingly, in one embodiment, a pharmaceutical composition comprising one or more selected from the group consisting of the fusion polypeptide, a nucleic acid molecule encoding the fusion polypeptide, a recombinant vector comprising the nucleic acid molecule and a recombinant cell comprising the recombinant vector is provided. The pharmaceutical composition may be a pharmaceutical composition for prevention and/or treatment of diseases related to deficiency and/or dysfunction of GDF15 comprised in the fusion protein or diseases having a therapeutic and/or preventive effect of the GDF15.

Other embodiment provides a method for prevention and/or treatment of diseases related to deficiency and/or dysfunction of GDF15 comprised in the fusion protein or diseases having a therapeutic and/or preventive effect of the GDF15, comprising administering one or more selected from the group consisting of the fusion polypeptide, a nucleic acid molecule encoding the fusion polypeptide, a recombinant vector comprising the nucleic acid molecule and a recombinant cell comprising the recombinant vector, into a patient in need of prevention and/or treatment of diseases related to deficiency and/or dysfunction of GDF15 comprised in the fusion protein or diseases having a therapeutic and/or preventive effect of the GDF15. The method may further comprise confirming a patient in need of prevention and/or treatment of diseases related to deficiency and/or dysfunction of GDF15 comprised in the fusion protein or diseases having a therapeutic and/or preventive effect of the GDF15, before the administering.

The example of the diseases related to deficiency and/or dysfunction of GDF15 comprised in the fusion protein or diseases (or symptoms) having a therapeutic and/or preventive effect of the GDF15 may include obesity, diabetes (type 1 diabetes, type 2 diabetes), cardiovascular disease, myocardial hypertrophy, liver disease (e.g., nonalcoholic steatohepatitis (NASH), etc.), ischemic injury (ischemic brain damage, ischemic retina injury), peripheral nerve injury, age-related sensory and/or motor nerves loss, renal tubular and/or renal epileptic injury, but not limited thereto.

In other embodiment, the pharmaceutical composition or method comprising administering the same provided in the present description may have one or more effects selected from the group consisting of body weight loss, diet control (intake reduction), body fat reduction, and giving and/or enhancing glucose tolerance, and in this case, the pharmaceutical composition or method may be applied as a use for reducing body weight, reducing body fat and/or giving and/or enhancing glucose tolerance.

Therefore, in one embodiment, it may be a pharmaceutical composition or food composition (health functional food) for reducing a body weight, regulating a diet (reducing an amount of food), reducing body fat, or giving and/or enhancing glucose tolerance, as a composition comprising one or more selected from the group consisting of the fusion polypeptide, a nucleic acid molecule encoding the fusion polypeptide, a recombinant vector comprising the nucleic acid molecule, and a recombinant cell comprising the recombinant vector.

Other embodiment provides a method for reducing a body weight, regulating a diet (reducing an amount of food), reducing body fat, or giving and/or enhancing glucose tolerance, comprising administering one or more selected from the group consisting of the fusion polypeptide, a nucleic acid molecule encoding the fusion polypeptide, a recombinant vector comprising the nucleic acid molecule and a recombinant cell comprising the recombinant vector, into a patient in need of reducing a body weight, regulating a diet (reducing an amount of food), reducing body fat, or giving and/or enhancing glucose tolerance. The method may further comprise confirming the patient in need of reducing a body weight, regulating a diet (reducing an amount of food), reducing body fat, or giving and/or enhancing glucose tolerance, before the administering.

The pharmaceutical composition may comprise one or more of active ingredients selected from the group consisting of the fusion polypeptide, a fusion polypeptide dimer, a nucleic acid molecule, a recombinant vector and a recombinant cell comprising the fusion polypeptide in a pharmaceutically effective dose. The pharmaceutically effective dose means a contained amount or a dosage of the active ingredient capable of obtaining a desired effect. The contained amount or dosage of the active ingredient may be variously prescribed by factors such as preparation method, administration method, patient's age, body weight, gender, morbid condition, food, administration time, administration interval, administration route, excretion rate and reaction sensitivity. For example, the single dosage of the active ingredient may be in a range of 0.001 to 1000 mg/kg, 0.01 to 100 mg/kg, 0.01 to 50 mg/kg, 0.01 to 20 mg/kg, or 0.01 to 1mg/kg, but not limited thereto.

Furthermore, the pharmaceutical composition may further comprise a pharmaceutically acceptable carrier, in addition the active ingredients. The carrier is one commonly used in preparation of a drug comprising a protein, nucleic acid or cell, and may be one or more selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil and the like, but not limited thereto. The pharmaceutical composition may also comprise one or more selected from the group consisting of diluents, excipients, lubricants, wetting agents, sweeteners, flavoring agents, emulsifiers, suspending agents, preservatives, and the like, commonly used in preparation of pharmaceutical compositions additionally.

The administration subject of the pharmaceutical composition may be a mammal including primates such as humans and monkeys, rodents such as mice and rats, and the like, or a cell, tissue, cell culture or tissue culture derived therefrom.

The pharmaceutical composition may be administered by oral administration or parenteral administration, or may be administered by contacting it to a cell, tissue or body fluid. Specifically, in case of parenteral administration, it may be administered by intravenous injection, subcutaneous injection, intramuscular injection, intraperitoneal injection, endothelial administration, local administration, intranasal administration, intrapulmonary administration and intrarectal administration, and the like. In case of oral administration, since proteins or peptides are digested, an oral composition should be formulated to coat an active agent or to protect it from degradation in the stomach.

In addition, the pharmaceutical composition may be formulated in a form of solution, suspension, syrup or emulsion in an oil or aqueous medium, or in a form of extract, powder, granule, tablet or capsule, or the like, and for formulation, it may further comprise a dispersing agent or stabilizing agent.

[ADVANTAGEOUS EFFECTS]

The GDF15 fused with a polypeptide region capable of O-glycosylation provided in the present description has a long duration when administered in vivo, so it is possible to increase the administration interval and thereby reduce the administration dose, and therefore, it has an advantageous effect in terms of administration convenience and/or economics and can be usefully applied to fields requiring GDF15 treatment.

[BRIEF DESCRIPTION OF THE DRAWINGS]

FIG. 1 schematically shows the amino acid sequence of GDF15.
FIG. 2a schematically shows the fusion polypeptide comprising His Tag according to one example.
FIG. 2b schematically shows the structure of the fusion polypeptide according to one example.
FIG. 3 schematically shows the structures of the various types of fusion polypeptides.
FIG. 4 shows the result of analyzing fusion polypeptides HT-ID1-GDF15, HT-ID2-GDF15 and HT-ID3-GDF15 synthesized in one example by SDS-PAGE.
FIG. 5 shows the result of analyzing fusion polypeptides HT-ID1-GDF15, HT-ID2-GDF15 and HT-ID3-GDF15 synthesized in one example by Q-TOF Mass Spectrometry.
FIG. 6 is a graph showing the body weight change when each of the fusion polypeptides HT-ID1-GDF15, HT-ID2-GDF15 and HT-ID3-GDF15 is administered to mice once.
FIG. 7 is a graph extracting and showing the result at Day 4 among the result of FIG. 6.
FIG. 8 is a graph showing the feed intake change of mice administered with the fusion polypeptides HT-ID1-GDF15, HT-ID2-GDF15 and HT-ID3-GDF15.
FIG. 9 is a graph showing the body weight change upon repeated administration of each of the fusion polypeptides HT-ID2-GDF15 and HT-ID3-GDF15 to mice.
FIG. 10a is a graph extracting and showing the result at Day 7 and Day 14 among the result of FIG. 9.

FIG. 10b is a graph extracting and showing the result at Day 21 and Day 28 among the result of FIG. 9.
FIG. 11 is a graph showing the cumulative feed intake up to Day 7, Day 14, Day 21 and Day 28 when the fusion polypeptides HT-ID2-GDF15 and HT-ID3-GDF15 are repeatedly administered to mice, respectively.
FIG. 12 is a graph showing the change in the blood fusion polypeptide concentration with time when the fusion polypeptides HT-ID1-GDF15, HT-ID2-GDF15 and HT-ID3-GDF15 are administered to SD Rat.

[MODE FOR INVENTION]

Hereinafter, the present invention will be described in more detail by the following examples. However, they are intended to illustrate the present invention only, but the scope of the present invention is not limited by these examples.

**Example 1: Preparation of fusion polypeptide**

**1.1. Preparation of fusion polypeptide comprising GDF15**

Fusion polypeptides IgD-GDF15 (ID1-GDF15), IgD-IgD-GDF15 (ID2-GDF15), IgD-IgD-IgD-GDF15 (ID3-GDF15) (See FIGs. 2a and 2b) in which a combination of IgD hinge (ESPKAQA**SS**VP**T**AQPQAEGSLAKA**TT**APA**TT**RNT; SEQ ID NO: 1; the underlined parts were sites capable of O-glycosylation) or several (1, 2 or 3) IgD hinges were fused with GDF15 (SEQ ID NO: 3, FIG. 1) were prepared. For convenience of purification, a fusion polypeptide comprising His-tag (SEQ ID NO: 15) and TEV cleavage Site (SEQ ID NO: 16) was also prepared. The amino acid sequences of each part comprised in the fusion polypeptide were summarized in Table 2 below.

[Table 2]

| | Amino acid sequence (N terminus→C terminus) | SEQ ID NO: |
|---|---|---|
| Signal Peptide (SP7.2) | MHRPEAMLLL LTLALLGGPT WA | 14 |
| Target polypeptide (GDF15) | ARNGDHCPLG PGRCCRLHTV RASLEDLGWA DWVLSPREVQ VTMCIGACPS QFRAANMHAQ IKTSLHRLKP DTVPAPCCVP ASYNPMVLIQ KTDTGVSLQT YDDLLAKDCH CI | 3 |
| Hinge region of immunoglobulin IgD (ID) | ESPKAQASSV PTAQPQAEGS LAKATTAPAT TRNT | 1 |
| His-Tag | HHHHHHHH | 15 |
| TEV Cleavage Site | ENLYFQG | 16 |
| GS Linker | GGGGSGGGGS GGGGSGGGGS | 17 |

**1.1.1. Preparation of recombinant expression vector**

**1.1.1.1. Mature GDF15**

In order to obtain a gene encoding mature GDF15, referring to the amino acid sequence information of UniprotKB Q99968, a gene encoding mature GDF15 (SEQ ID NO: 5) was synthesized (Bioneer).

SEQ ID NO: 5 (339bp)

1 GCCCGGAACG GCGACCACTG CCCCCTGGGG CCCGGACGGT GCTGCCGGCT
51 GCACACCGTG CGGGCCTCCC TGGAGGACCT GGGCTGGGCC GACTGGGTGC
101 TGTCCCCAAG GGAGGTGCAA GTGACCATGT GCATCGGCGC CTGCCCATCT
151 CAGTTCCGGG CCGCCAACAT GCACGCTCAG ATCAAGACCA GCCTGCACCG
201 GCTGAAGCCC GACACCGTGC CCGCCCCCTG CTGCGTGCCC GCCTCCTACA
251 ACCCCATGGT GCTGATTCAG AAGACCGACA CCGGCGTGAG CCTGCAGACC
301 TACGACGACC TGCTGGCCAA GGACTGCCAC TGCATCTAA

1.1.1.2. **IgD Hinge (ID)**

In order to obtain a gene encoding Human IgD Hinge, referring to the amino acid sequence information of UniprotKB P01880, a gene encoding 3 Human IgD Hinges (hereinafter, referred to as 'ID3') (SEQ ID NO: 6) was synthesized in Bioneer.

SEQ ID NO: 6 (306bp)

1 GAGAGCCCTA AGGCTCAGGC CTCTAGCGTG CCAACAGCTC AGCCACAAGC
51 TGAAGGAAGC CTGGCCAAGG CTACAACCGC CCCTGCCACA ACACGGAATA

101 CA**GAGTCCCC CAAGGCCCAG GCTAGCAGCG TGCCTACCGC CCAGCCTCAG**
151 **GCCGAGGGCT CCCTGGCTAA GGCCACAACC GCTCCCGCTA CAACCAGGAA**
201 **CACCGAGTCT CCAAAGGCAC AGGCCTCCTC CGTGCCCACT GCACAACCCC**
251 **AAGCAGAGGG CAGCCTCGCC AAGGCAACCA CAGCCCCAGC CACCACCCGG**
301 **AACACA**

(1-102 polynucleotide (underlined), 103-204 polynucleotide (bold), and 205-306 polynucleotide (bold + underlined) encode IgD Hinge, respectively)

**1.1.1.3. Preparation of expression vector**

A variant of pcDNA3.1(+) (Invitrogen, Cat. No. V790-20), pDHDD-D1G1 (comprising the promoter of KR10-1868139B1) was cut with BamHI and NotI, and a gene designed to encode a fusion protein having the structure below (See FIG. 3) by combining the above genes (mature GDF15 encoding gene and ID3 encoding gene) was inserted thereto to prepare each recombinant vector.

pGDF15
'(N-terminus)-[BamHI restriction site (GGATCC)-signal peptide (SEQ ID NO: 14)-Mature GDF15 (SEQ ID NO: 3)-NotI restriction site (GCGGCCGC)]-(C-terminus)'

pHT-GDF15
'(N-terminus)-[BamHI restriction site-signal peptide (SEQ ID NO: 14)-His-Taq (SEQ ID NO: 15)-TEV Cleavage Site (SEQ ID NO: 16)-Mature GDF15 (SEQ ID NO: 3)- NotI restriction site]-(C-terminus)'

pID1-GDF15
'(N-terminus)-[BamHI restriction site-signal peptide (SEQ ID NO: 14)-IgD Hinge (SEQ ID NO: 1)-GS Linker (SEQ ID NO: 17)-Mature GDF15 (SEQ ID NO: 3)- NotI restriction site]-(C-terminus)'

pHT-ID1-GDF15
'(N-terminus)-[BamHI restriction site-signal peptide (SEQ ID NO: 14)-His-Taq (SEQ ID NO: 15)-TEV Cleavage Site (SEQ ID NO: 16)- IgD Hinge (SEQ ID NO: 1)-GS Linker (SEQ ID NO: 17)-GDF15 (SEQ ID NO: 3)- NotI restriction site]-(C-terminus)'

pID2-GDF15

'(N-terminus)-[BamHI restriction site-signal peptide (SEQ ID NO: 14)-IgD Hinge (SEQ ID NO: 1)- IgD Hinge (SEQ ID NO: 1)-GS Linker (SEQ ID NO: 17)-GDF15 (SEQ ID NO: 3)- NotI restriction peptide]-(C-terminus)'

pHT-ID2-GDF15

'(N-terminus)-[BamHI restriction site-signal peptide (SEQ ID NO: 14)- His-Taq (SEQ ID NO: 15)-TEV Cleavage Site (SEQ ID NO: 16)-IgD Hinge (SEQ ID NO: 1)-IgD Hinge (SEQ ID NO: 1)-GS Linker (SEQ ID NO: 17)-GDF15 (SEQ ID NO: 3)- NotI restriction site]-(C-terminus)'

pID3-GDF15

'(N-terminus)-[BamHI restriction site-signal peptide (SEQ ID NO: 14)-IgD Hinge (SEQ ID NO: 1)-IgD Hinge (SEQ ID NO: 1)-IgD Hinge (SEQ ID NO: 1)-GS Linker (SEQ ID NO: 17)-GDF15 (SEQ ID NO: 3)- NotI restriction site]-(C-terminus)'

pHT-ID3-GDF15

'(N-terminus)-[BamHI restriction site-signal peptide (SEQ ID NO: 14)- His-Taq (SEQ ID NO: 15)-TEV Cleavage Site (SEQ ID NO: 16)-IgD Hinge (SEQ ID NO: 1)-IgD Hinge (SEQ ID NO: 1)-IgD Hinge (SEQ ID NO: 1)-GS Linker (SEQ ID NO: 17)-GDF15 (SEQ ID NO: 3)- NotI restriction site]-(C-terminus)'

### 1.1.2. Expression of fusion polypeptide

The prepared recombinant expression vectors, pGDF15, pHT-GDF15, pID1-GDF15, pHT-ID1-GDF15, pID2-GDF15, pHT-ID2-GDF15, pID3-GDF15, and pHT-ID3-GDF15 were introduced into ExpiCHO-S™ cell (Thermo Fisher Scientific) and cultured (Fed-Batch Culture; Day 1 & Day 5 Feeding) in ExpiCHO Expression Medium (Thermo Fisher Scientific; 400 mL) for 12 days, to express the fusion polypeptides GDF15, HT-GDF15, ID1-GDF15, HT-ID1-GDF15, ID2-GDF15, HT-ID2-GDF15, ID3-GDF15, and HT-ID3-GDF15.

### 1.1.3. Purification of fusion polypeptide

The fusion polypeptides HT-ID1-GDF15, HT-ID2-GDF15 and HT-ID3-GDF15 produced through the recombinant expression vector were purified and O-Glycan site Occupancy was analyzed using Sialic Acid content analysis and Q-TOF Mass Spectrometry.

Specifically, the fusion polypeptides were purified by continuously performing ultrafiltration/diafiltration, Immobilized Metal Affinity Chromatography (IMAC), and Anion Exchange Chromatography (AEX). At first, the culture solution of the fusion protein in which cells were removed was filtered with a 0.22 μm filter. For the filtered solution, concentration was performed using TFF System and then buffer exchange was conducted with a tromethamine buffer solution. A column in which HiTrap™ Chelating HP (GE Healthcare Life Sciences) resin was packed was equipped and an equilibrium buffer (20 mM Tris pH 8.0, 0.5 M NaCl, 5 mM Imidazole) was flowed to equilibrate the column. The process solution in which the ultrafiltration/diafiltration was completed previously was injected into the column, and then the equilibrium buffer was flowed again to wash the column. After completing the washing operation of the column, the elution buffer (20 mM Tris pH 8.0, 0.5 M NaCl, 0.5 M Imidazole) was flowed into the column to elute a target protein.

For the obtained eluted solution, concentration was performed using Amicon Ultra Filter Device (MWCO 10K, Merck) and a centrifuge, and then buffer exchange was conducted with a tromethamine buffer solution. The process solution prepared as such was injected into the equilibrated anion exchange column, and the equilibrium buffer (20 mM Tris pH 8.0) was flowed and the column was washed. After completing the washing operation of the column, the elution buffer (20 mM Tris pH 8.0, 0.5 M NaCl) was flowed into the column under a concentration gradient condition to elute the target protein. Among eluted fractions, fractions with high concentration and high purity of the fusion polypeptide were collected and kept frozen.

For an animal experiment, concentration and buffer exchange for samples were performed with Phosphate Buffered Saline (PBS, 10 mM Sodium Phosphate, 150 mM NaCl pH 7.4) using Amicon Ultra Filter Device (MWCO 10K, Merck) and a centrifuge.

The quantitative analysis of the fusion polypeptide was conducted by measuring the absorbance at 280 nm and 340 nm in UV Spectrophotometer (G113A, Agilent Technologies) by the following equation. As the extinction coefficient, a value theoretically calculated using the amino acid sequence was used.

$$\text{Protein concentration (mg/mL)} = \frac{\text{Absorbance } (A_{280\text{ nm}} - A_{340\text{ nm}})}{\text{*Extinction Coefficient}} \times \text{Dilution Factor}$$

*Extinction coefficient (0.1%): theoretical absorbance at 280 nm, assuming that the protein concentration is 0.1% (1g/L), and all cysteines in Primary Sequence are oxidized to form disulfide bonds. Calculated via ProtParam tool (https://web.expasy.org/protparam/).

[Table 3]

| Extinction coefficient of fusion polypeptide | |
|---|---|
| Sample name | Extinction coefficient (0.1%, 1 mg/mL) |
| HT-ID1-GDF15 | 0.833 |
| HT-ID2-GDF15 | 0.706 |
| HT-ID3-GDF15 | 0.612 |

For the purified fusion polypeptides HT-ID1-GDF15, HT-ID2-GDF15 and HT-ID3-GDF15, after Sialic Acid content analysis and reducing, O-Glycan site Occupancy was analyzed using Q-TOF Mass Spectrometry.

The result of analyzing the fusion polypeptides HT-ID1-GDF15, HT-ID2-GDF15 and HT-ID3-GDF15 by SDS-PAGE was shown in FIG. 4, and the result of analyzing them by Q-TOF Mass Spectrometry was shown in FIG. 5 and Table 5. In addition, the sialic acid content was also shown in Table 4.

[Table 4]

| Average O-Glycan number and Sialic Acid content | | | | |
|---|---|---|---|---|
| Sample | Theoretical O-Glycan number (One Chain) | Average O-Glycan number | O-Glycan distribution (main) | Sialic Acid content (mol/mol) |
| HT-ID1-GDF15 | 7 | 5.4 | 2-7 (6) | 14.4 |
| HT-ID2-GDF15 | 14 | 9.7 | 3-15* (10) | 16.3 |
| HT-ID3-GDF15 | 21 | 13.5 | 5-21 (13) | 18.9 |
| * More than the theoretical O-glycan number is presumed that O-glycan is attached to the GS Linker (Spahr et al., 2014, mAbs, 6: 904) | | | | |

## Example 2. Pharmacological effect of fusion polypeptide (in vivo)

### 2.1. Single administration

#### 2.1.1 Test process

The pharmacological effect of the fusion polypeptides produced and purified in Example 1 above was tested in mice (C57BL/6J, 6-week-old, male, 100 mice; Raonbio).

In the present example, DIO mouse model (Mouse, C57BL/6J -DIO, male, 100 mice, 14-week-old (obesity feed feeding for 8 weeks)) in which obesity was induced by feeding a high-fat diet into the C57BL/6J mice for 8 weeks was used. The DIO mouse model is an animal model widely used for evaluation of diabetes and insulin improvement efficacy, as it exhibits clinical characteristics of type 2 diabetes such as hyperlipidemia, insulin resistance, and hyperglycemia, and a lot of comparable basic data have been accumulated for the study of metabolic diseases such as obesity, diabetes and hyperlipidemia, and therefore, it was suitable for the pharmacological effect test of the present example, and thus this model was selected.

The mouse model fed with the obesity feed for 8 weeks was subjected to a quarantine and acclimatization period of 2 weeks, and during this period, general symptoms were observed once a day, and healthy animals were selected by confirming whether they were healthy and suitable for conducting the experiment. During the acclimatization period, the animal's tail was marked with a red oil pen at the time of acquisition (tail marking), and temporary individual identification cards (test name, individual number, stocking time) were attached to the breeding box during the quarantine acclimatization period. At the time of group separation, individuals were marked on the tails of animals using a black oil pen and individual identification cards (test name, group information, individual number, gender, stocking time, administration period) were attached to each cage.

In order to minimize stress experienced by the experimental animals due to subcutaneous administration of the test substance (fusion polypeptide), 200 uL/head of sterile distilled physiological saline was administered subcutaneously to all animals using a 1 mL syringe from 3 days before the administration of the test substance. Pre-adaptation training for subcutaneous administration was conducted.

For healthy animals with no abnormalities found during the quarantine and acclimatization period, the body weight and feed intake were measured for all individuals after the acclimatization period.

The body weight and feed intake were measured, and group separation was performed so that the averages of the two measured values were similar between groups based on body weight. Test substance administration was started from the day after group separation. Remaining animals that were not selected were excluded from the test system after group separation was terminated.

The information of the high fat diet (obesity feed; HFD) fed to the C57BL/6J - DIO was as follows:
5.24 kcal/g, fat 60 % by weight, protein 20 % by weight, and carbohydrate-derived calories 20 % by weight; Research Diet Inc., U.S.A.; Product No. High fat diet (Fat 60 kcal%, D12492).

The feed was fed by a free feeding (feeding during the acclimatization and test period) method.

The drinking water method was that tap water was filtered with a filter oil-water sterilizer and then ultraviolet rays were irradiated and it was freely ingested using a polycarbonate drinking water bottle (250 mL).

The administration of the test substances HT-ID1-GDF15, HT-ID2-GDF15, and HT-ID3-GDF15 and the control substance Semaglutide (Bachem) was conducted from the next day after group separation, and the administration time was performed at 9 AM every day. Subcutaneous administration was conducted for all the control substance and test substances. For the administration route of the control substance and test substances, subcutaneous administration was selected depending on the clinically scheduled administration route.

For all the control substance and test substances, the amount of the administration solution was set to 5 mL/kg and the administration liquid by individual was calculated on the basis of the recently measured body weight and it was administered by subcutaneous injection once on the start day of the test using a disposable syringe (1 mL). The test substances were administered only once. For comparison, the control group in which the control substance Semaglutide was administered was prepared, and the comparison group in which Semaglutide was administered was administered once a day, and all the administration was progressed from 9 AM.

The composition of the test groups and administration dose were summarized in Table 5 below:

[Table 5]

| Fusion polypeptide administration group composition | | | | | |
|---|---|---|---|---|---|
| High Fat Diet | Test substance | Administr ation route | Administrati on dose (nmol/kg) | Administrat ion volume (mL/kg) | Animal number |
| ○ | Vehicle, qw | Subcutan eous | - | 5 | 5 |
| ○ | Semaglutide, qd | Subcutan eous | 3 | 5 | 5 |
| ○ | HT-ID1-GDF15 | Subcutan eous | 10 | 5 | 5 |
| ○ | HT-ID2-GDF15 | Subcutan eous | 10 | 5 | 5 |
| ○ | HT-ID3-GDF15 | Subcutan eous | 10 | 5 | 5 |

As for the observation, measurement and test schedule for the test groups, the administration start date was set to Day 0, and 7 days from the administration start date were set to one week of administration.

The test schedule was summarized in Table 6:

【Table 6】

Test schedule

| Observation item | Acclimatization period (week) | | Period (day) | | | | | | | | | |
|---|---|---|---|---|---|---|---|---|---|---|---|---|
| | 1 | 2 | 0 | 1 | 2 | 3 | 4 | 5 | 6 | 7 | 8 | 9 |
| High fat feed feeding | ● | ● | ● | ● | ● | ● | ● | ● | ● | ● | ● | ● |
| Adaptation to oral and subcutaneous administration | | ● | | | | | | | | | | |
| Administration | | | ● | | | | | | | | | |
| Body weight measurement | | ● | ● | ● | ● | ● | ● | ● | ● | ● | ● | ● |
| Feed intake measurement | | | ● | ● | ● | ● | ● | ● | ● | ● | ● | ● |

General clinical symptoms were observed once a day for all animals, and the presence or absence of moribund and dead animals was checked twice a day, and these observations were conducted from the 1st day of administration to the end of administration. Only when there were abnormal symptoms during observation, it was recorded on the recording sheet.

The body weight of each mouse was measured on the day of the start of administration of the test substances (before administration), and thereafter, the body weight was measured every day (measured up to 9 days), and the amount of the administration solution of the test substances was determined on the basis of the most recently measured body weight.

In addition, after administering the test substances into mice, the daily feed intake was measured, and the feeding amount was measured using an electronic scale for each breeding box, and the remaining amount was measured to calculate the daily feed intake. In case of an individual that gnawed heavily on heed, it was excluded from the measurement.

All the experimental results obtained in the present example were expressed as mean ± standard error and tested using Prism5 (version 5.01). One-way analysis of variance (ANOVA) was performed on all data, and when significance was observed, Dunnett's test was performed to find out the test groups with a significant difference from the control group (significance level: two-sided 5% and 1%, 0.1%).

**2.1.2. Body weight loss test result**

The change in the body weight measured in Example 2.1.1 above was shown in FIG. 6 and FIG. 7, and Table 7 (Body Weight (Group, % of initial).

[Table 7]

| **Group** | **Day** | **0** | **1** | **2** | **3** | **4** | **5** | **6** | **7** | **8** | **9** |
|---|---|---|---|---|---|---|---|---|---|---|---|
| DIO Vehicle | Mean | 100 | 100 | 100 | 99 | 100 | 100 | 100 | 100 | 101 | 101 |
| Control | S.E. | 0 | 0 | 1 | 0 | 0 | 0 | 0 | 1 | 0 | 1 |
| (Daily Inj.) | | | | | | | | | | | |

(continued)

| Group | Day | 0 | 1 | 2 | 3 | 4 | 5 | 6 | 7 | 8 | 9 |
|---|---|---|---|---|---|---|---|---|---|---|---|
| DIO Semaglutide 3 nmol/kg (Daily Inj.) | Mean | 100 | 94 | 91 | 90 | 87 | 87 | 85 | 86 | 83 | 85 |
| | S.E. | 0 | 0 | 1 | 2 | 2 | 3 | 3 | 3 | 3 | 3 |
| HT-ID1-GDF15 10 nmol/kg (Single Inj.) | Mean | 100 | 99 | 98 | 97 | 96 | **95** | 95 | 96 | 96 | 97 |
| | S.E. | 0 | 0 | 0 | 1 | 1 | 1 | 1 | 1 | 1 | 1 |
| HT-ID2-GDF15 10 nmol/kg (Single Inj.) | Mean | 100 | 98 | 97 | 96 | **94** | 94 | 94 | 93 | 93 | 94 |
| | S.E. | 0 | 0 | 1 | 1 | 1 | 2 | 2 | 2 | 2 | 2 |
| HT-ID3-GDF15 10 nmol/kg (Single Inj.) | Mean | 100 | 98 | 97 | 96 | 95 | **94** | 95 | 95 | 95 | 96 |
| | S.E. | 0 | 0 | 0 | 1 | 0 | 1 | 1 | 1 | 1 | 1 |

FIG. 6 and Table 7 shows the change in the body weight when the fusion polypeptides HT-ID1-GDF15, HT-ID2-GDF15 and HT-ID3-GDF15 were administered once, respectively, compared to the negative control group (vehicle administration group) and positive control group (Semaglutide daily administration group). In addition, FIG. 7 is a graph showing the result at Day 4 by extracting it among the result of FIG. 6.

As shown in the above result, it could be confirmed that there was little change in the body weight in case of the negative control group (vehicle administration group), while the body weight loss effect was continuously shown from Day 1 after administration in case of the positive control group (Semaglutide daily administration group). In addition, it could be confirmed that the body weight loss effect was shown immediately after single administration at Day 0 in case of the fusion polypeptide in which GDF15 was fused with IgD Hinge, and the body weight loss effect was not reduced and appeared continuously until 3-4 days.

### 2.1.3. Diet intake test result

The change in the feed intake measured in Example 2.1.1 above was shown in Table 8 and FIG. 8 (cumulative intake up to Day 6), respectively.

[Table 8]

| Group | Day | 0 | 1 | 2 | 3 | 4 | 5 | 6 | 7 | 8 | 9 |
|---|---|---|---|---|---|---|---|---|---|---|---|
| DIO Vehicle Control (Daily Inj.) | Mean | 3.3 | 2.7 | 3.0 | 3.0 | 3.1 | 3.5 | 3.0 | 3.5 | 3.3 | 3.4 |
| | S.E. | 0.1 | 0.1 | 0.2 | 0.1 | 0.1 | 0.2 | 0.2 | 0.1 | 0.1 | 0.2 |
| DIO Semaglutide 3nmol/kg(Daily Inj.) | Mean | 3.7 | 2.1 | 3.3 | 2.8 | 2.3 | 2.9 | 2.7 | 3.2 | 2.4 | 3.7 |
| | S.E. | 0.3 | 0.7 | 1.4 | 0.6 | 0.4 | 0.9 | 0.6 | 0.3 | 0.4 | 0.1 |
| HT-ID1-GDF15 10nmol/kg(Single Inj.) | Mean | 3.3 | 2.2 | 2.9 | 2.3 | 2.8 | 3.1 | 3.1 | 3.7 | 3.4 | 3.5 |
| | S.E. | 0.2 | 0.3 | 0.2 | 0.5 | 0.2 | 0.3 | 0.1 | 0.3 | 0.3 | 0.5 |
| HT-ID2-GDF15 10nmol/kg(Single Inj.) | Mean | 2.7 | 1.7 | 2.3 | 2.2 | 2.6 | 2.4 | 2.8 | 3.1 | 2.7 | 3.3 |
| | S.E. | 0.2 | 0.2 | 0.1 | 0.1 | 0.3 | 0.2 | 0.1 | 0.1 | 0.2 | 0.2 |
| HT-ID3-GDF15 10nmol/kg(Single Inj.) | Mean | 3.2 | 2.0 | 2.5 | 2.3 | 2.3 | 2.7 | 2.8 | 3.3 | 3.3 | 3.6 |
| | S.E. | 0.2 | 0.2 | 0.3 | 0.2 | 0.2 | 0.3 | 0.2 | 0.3 | 0.2 | 0.2 |

As shown in the above result, in case of the fusion polypeptide administration group in which GDF15 was fused with IgD Hinge, compared to the negative control group (vehicle administration group) administration group, the feed intake reduction effect was shown up to Day 6 at maximum depending on the fusion polypeptide, and this feed intake reduction effect of the fusion polypeptide can be said to be comparable to the case of administering Semaglutide, the positive control group, once a day throughout the test period.

### 2.2. Repeated administration

### 2.2.1 Test process

Except for the administration dose, animal number and administration cycle, most of the test processes were the same as in Example 2.1.1 above.

The test substances were administered twice a week (Days 0, 4, 7, 11, 14, 18, 21, 25) for a total of 8 times. For comparison, the control group in which the control substance Semaglutide was administered every day was prepared, and the comparison group in which Semaglutide was administered every day was administered once a day daily, and all the administration was progressed from 9 AM.

The composition of the test groups and administration dose, and the like were summarized in Table 9 below:

[Table 9]

| Fusion polypeptide administration group composition | | | | | | |
|---|---|---|---|---|---|---|
| High Fat Diet | Test substance | Administr ation route | Administr ation cycle | Administr ation dose (nmol/kg) | Administr ation volume (mL/kg) | Animal numbe r |
| ○ | Lean Vehicle Control | Subcutan eous | Once a week | - | 5 | 4 |
| ○ | DIO Vehicle Control | Subcutan eous | Once a week | - | 5 | 8 |
| ○ | Semaglutide | Subcutan eous | Once a day | 3 | 5 | 8 |
| ○ | HT-ID2-GDF15 | Subcutan eous | Twice a week | 3 | 5 | 8 |
| ○ | HT-ID2-GDF15 | Subcutan eous | Twice a week | 10 | 5 | 8 |
| ○ | HT-ID2-GDF15 | Subcutan eous | Twice a week | 30 | 5 | 8 |
| ○ | HT-ID3-GDF15 | Subcutan eous | Twice a week | 3 | 5 | 8 |
| 0 | HT-ID3-GDF15 | Subcutan eous | Twice a week | 10 | 5 | 8 |
| 0 | HT-ID3-GDF15 | Subcutan eous | Twice a week | 30 | 5 | 8 |

### 2.2.2. Body weight loss test result

The change in the body weight measured in Example 2.2.1 above was shown in FIG. 9, FIG. 10a, FIG. 10b and Table 10 (Body Weight (Group, % of initial).

【Table 10】

| Group | Day | 0 | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|---|
| Lean Vehicle Control | Mean | 100 | 100 | 100 | 100 | 100 | 100 | 100 | 99 | 100 | 102 | 102 | 101 | 101 | 102 | 101 |
| | S.E. | 0 | 0 | 0 | 0 | 1 | 1 | 1 | 1 | 1 | 1 | 1 | 0 | 1 | 1 | 1 |
| DIO Vehicle Control | Mean | 100.0 | 99.4 | 99.1 | 98.6 | 98.6 | 98.3 | 98.8 | 97.7 | 95.8 | 96.2 | 97.3 | 99.0 | 99.8 | 100.2 | 100.5 |
| | S.E. | 0.0 | 0.4 | 0.4 | 0.5 | 0.5 | 0.6 | 0.7 | 1.1 | 1.5 | 1.3 | 1.1 | 1.1 | 1.0 | 1.0 | 1.0 |
| DIO Semaglutide 3 nmol/kg | Mean | 100.0 | 93.9 | 92.5 | 90.4 | 88.4 | 86.2 | 84.1 | 82.6 | 81.9 | 80.7 | 80.3 | 80.3 | 79.1 | 78.8 | 78.7 |
| | S.E. | 0.0 | 0.3 | 0.6 | 0.8 | 1.1 | 1.5 | 1.5 | 1.6 | 1.6 | 1.8 | 2.2 | 1.9 | 2.0 | 2.0 | 2.0 |
| HT-ID2-GDF15 3 nmol/kg | Mean | 100.0 | 98.0 | 96.5 | 95.0 | 93.9 | 91.8 | 90.8 | 90.3 | 89.2 | 89.2 | 90.1 | 92.1 | 92.5 | 92.7 | 93.3 |
| | S.E. | 0.0 | 0.3 | 0.4 | 0.4 | 0.4 | 0.4 | 0.4 | 0.5 | 0.6 | 0.6 | 0.7 | 0.6 | 0.6 | 0.7 | 0.7 |
| HT-ID2-GDF15 10 nmol/kg | Mean | 100.0 | 98.3 | 96.7 | 95.4 | 94.6 | 93.0 | 91.7 | 90.8 | 90.1 | 90.3 | 90.6 | 92.1 | 91.2 | 92.0 | 92.2 |
| | S.E. | 0.0 | 0.3 | 0.5 | 0.5 | 0.4 | 0.5 | 0.6 | 0.7 | 0.7 | 0.7 | 0.7 | 0.8 | 0.8 | 0.9 | 0.8 |
| HT-ID2-GDF15 30 nmol/kg | Mean | 100.0 | 98.4 | 97.3 | 96.2 | 95.2 | 94.0 | 93.1 | 92.3 | 91.5 | 91.2 | 91.3 | 93.2 | 91.9 | 92.1 | 93.0 |
| | S.E. | 0.0 | 0.3 | 0.5 | 0.6 | 0.6 | 0.8 | 0.9 | 1.1 | 1.2 | 1.2 | 1.6 | 1.6 | 1.7 | 1.9 | 2.1 |
| HT-ID3-GDF15 3 nmol/kg | Mean | 100.0 | 98.2 | 96.9 | 95.6 | 94.4 | 92.8 | 91.9 | 91.0 | 90.0 | 89.6 | 89.1 | 91.5 | 91.5 | 92.3 | 92.5 |
| | S.E. | 0.0 | 0.3 | 0.3 | 0.3 | 0.5 | 0.6 | 0.6 | 0.7 | 0.8 | 1.0 | 1.4 | 1.2 | 1.6 | 1.4 | 1.4 |
| HT-ID3-GDF15 10 nmol/kg | Mean | 100.0 | 98.5 | 97.3 | 95.7 | 94.6 | 92.8 | 91.5 | 90.7 | 89.8 | 89.3 | 88.7 | 90.9 | 89.8 | 90.1 | 91.0 |
| | S.E. | 0.0 | 0.3 | 0.4 | 0.5 | 0.5 | 0.6 | 0.6 | 0.7 | 0.9 | 0.9 | 1.0 | 1.0 | 1.3 | 1.4 | 1.3 |
| HT-ID3-GDF15 30 nmol/kg | Mean | 100.0 | 98.2 | 97.1 | 95.8 | 94.8 | 92.8 | 91.7 | 90.8 | 89.8 | 89.8 | 89.7 | 92.0 | 90.0 | 90.4 | 90.9 |
| | S.E. | 0.0 | 0.3 | 0.3 | 0.4 | 0.5 | 0.4 | 0.4 | 0.5 | 0.6 | 0.8 | 1.1 | 1.3 | 1.3 | 1.5 | 1.5 |

| Group | Day | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|
| Lean Vehicle Control | Mean | 101 | 102 | 101 | 102 | 102 | 102 | 101 | 101 | 102 | 102 | 101 | 101 | 102 | 101 |
| | S.E. | 2 | 1 | 1 | 1 | 2 | 1 | 1 | 1 | 1 | 1 | 1 | 1 | 1 | 1 |
| DIO Vehicle Control | Mean | 100.8 | 101.3 | 102.0 | 102.1 | 102.4 | 101.9 | 101.5 | 101.8 | 102.2 | 103.3 | 103.3 | 104.1 | 104.3 | 103.9 |
| | S.E. | 1.0 | 1.0 | 1.1 | 1.0 | 1.1 | 1.1 | 1.7 | 1.6 | 1.7 | 1.6 | 1.5 | 1.5 | 1.7 | 1.5 |
| DIO Semaglutide 3 nmol/kg | Mean | 78.1 | 78.3 | 77.2 | 76.7 | 77.1 | 76.1 | 76.8 | 75.9 | 75.6 | 76.0 | 76.3 | 76.5 | 76.2 | 75.7 |
| | S.E. | 1.8 | 2.0 | 2.3 | 2.2 | 2.1 | 2.3 | 2.3 | 2.2 | 2.3 | 2.4 | 2.8 | 2.7 | 2.4 | 2.7 |
| HT-ID2-GDF15 3 nmol/kg | Mean | 93.8 | 94.3 | 94.7 | 95.1 | 95.9 | 95.7 | 96.2 | 96.6 | 96.8 | 97.8 | 98.5 | 99.3 | 99.5 | 99.8 |
| | S.E. | 0.9 | 0.7 | 0.8 | 0.9 | 0.8 | 0.8 | 1.0 | 1.1 | 1.2 | 1.2 | 1.2 | 1.3 | 1.3 | 1.4 |
| HT-ID2-GDF15 10 nmol/kg | Mean | 92.1 | 92.7 | 93.6 | 94.2 | 94.0 | 93.7 | 94.0 | 93.8 | 94.3 | 95.4 | 95.8 | 95.8 | 96.3 | 96.6 |
| | S.E. | 0.9 | 0.9 | 0.9 | 0.9 | 0.9 | 1.0 | 0.9 | 1.0 | 0.8 | 0.9 | 0.9 | 0.8 | 0.8 | 0.7 |
| HT-ID2-GDF15 30 nmol/kg | Mean | 92.1 | 92.8 | 93.4 | 93.6 | 92.9 | 92.5 | 93.2 | 92.4 | 92.6 | 93.1 | 93.9 | 93.6 | 93.2 | 93.4 |
| | S.E. | 2.2 | 2.4 | 2.4 | 2.4 | 2.5 | 2.6 | 2.6 | 2.8 | 2.7 | 2.7 | 2.6 | 2.8 | 2.7 | 2.6 |
| HT-ID3-GDF15 3 nmol/kg | Mean | 92.6 | 93.3 | 94.0 | 94.4 | 95.0 | 95.2 | 95.8 | 96.4 | 97.1 | 97.4 | 97.6 | 98.5 | 98.0 | 98.6 |
| | S.E. | 1.3 | 1.3 | 1.2 | 1.2 | 1.2 | 1.2 | 1.3 | 1.3 | 1.5 | 1.4 | 1.3 | 1.5 | 1.7 | 1.7 |
| HT-ID3-GDF15 10 nmol/kg | Mean | 90.0 | 91.2 | 91.8 | 92.3 | 91.9 | 91.2 | 92.1 | 92.1 | 92.5 | 93.2 | 93.7 | 94.2 | 94.4 | 94.1 |
| | S.E. | 1.5 | 1.5 | 1.5 | 1.5 | 1.7 | 1.7 | 1.7 | 2.1 | 2.0 | 2.0 | 1.9 | 2.3 | 2.3 | 2.2 |
| HT-ID3-GDF15 30 nmol/kg | Mean | 89.3 | 89.6 | 90.4 | 91.0 | 90.0 | 89.6 | 90.4 | 90.0 | 90.0 | 91.0 | 91.5 | 91.5 | 91.2 | 91.4 |
| | S.E. | 1.4 | 1.6 | 1.5 | 1.7 | 1.5 | 1.5 | 1.4 | 1.3 | 1.4 | 1.5 | 1.5 | 1.5 | 1.7 | 1.6 |

FIG. 9 and Table 10 shows the change in the body weight in case of repeated administration of the fusion proteins HT-ID2-GDF15 and HT-ID3-GDF15, respectively (Table 9), compared to the negative control group (vehicle administration group) and positive control group (Semaglutide daily administration group). In addition, FIG. 10a is a graph showing the results at Day 7 and Day 14 by extracting them from the result of FIG. 9 above and FIG. 10b is a graph showing the results at Day 21 and Day 28 by extracting them from the result of FIG. 9.

As shown in the above result, it could be confirmed that there was little change in the body weight in case of the negative control group (vehicle administration group), while the body weight loss effect was continuously shown from Day 1 after administration in case of the positive control group (Semaglutide daily administration group). In addition, it could be confirmed that the body weight loss effect was shown immediately after single administration at Day 0 in case of the fusion polypeptide in which GDF15 was fused with IgD Hinge, and the body weight loss effect was continuously shown without being reduced, and the body weight loss effect was concentration-dependent.

### 2.2.3. Diet intake test result

The change in the feed intake measured in Example 2.2.1 above was shown in Table 11 and FIG. 11 (cumulative intake up to Day 7, Day 14, Day 21 and Day 28), respectively.

【Table 11】

| Group | Day | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| Lean Vehicle Control | Mean | 4.4 | 3.9 | 4.1 | 4.5 | 4.4 | 3.9 | 4.3 | 4.1 | 4.1 | 4.6 | 4.5 | 4.4 | 4.2 | 4.3 |
| | S.E. | 0.2 | 0.1 | 0.1 | 0.1 | 0.3 | 0.1 | 0.2 | 0.1 | 0.3 | 0.2 | 0.1 | 0.2 | 0.2 | 0.2 |
| DIO Vehicle Control | Mean | 2.9 | 2.6 | 2.7 | 3.6 | 3.3 | 2.5 | 3.0 | 2.7 | 1.7 | 2.2 | 3.0 | 4.0 | 3.1 | 3.4 |
| | S.E. | 0.2 | 0.5 | 0.3 | 0.8 | 0.4 | 0.2 | 0.1 | 0.1 | 0.4 | 0.2 | 0.1 | 0.2 | 0.1 | 0.2 |
| DIO Semaglutide 3 nmol/kg | Mean | 2.9 | 0.7 | 1.3 | 1.4 | 1.7 | 1.5 | 1.6 | 1.9 | 2.0 | 2.3 | 2.2 | 3.2 | 2.2 | 2.5 |
| | S.E. | 0.1 | 0.1 | 0.2 | 0.1 | 0.1 | 0.2 | 0.2 | 0.4 | 0.5 | 0.3 | 0.3 | 0.3 | 0.2 | 0.2 |
| HT-ID2-GDF15 3 nmol/kg | Mean | 3.0 | 1.5 | 2.1 | 2.1 | 2.4 | 1.7 | 2.3 | 2.3 | 2.0 | 2.8 | 3.3 | 4.2 | 3.7 | 3.4 |
| | S.E. | 0.1 | 0.1 | 0.1 | 0.1 | 0.4 | 0.1 | 0.2 | 0.2 | 0.1 | 0.2 | 0.1 | 0.2 | 0.7 | 0.1 |
| HT-ID2-GDF15 10 nmol/kg | Mean | 2.6 | 1.5 | 1.8 | 2.0 | 2.0 | 1.8 | 2.1 | 2.1 | 1.9 | 2.5 | 3.1 | 3.7 | 2.4 | 3.0 |
| | S.E. | 0.2 | 0.1 | 0.2 | 0.1 | 0.1 | 0.1 | 0.1 | 0.1 | 0.1 | 0.1 | 0.1 | 0.1 | 0.1 | 0.1 |
| HT-ID2-GDF15 30 nmol/kg | Mean | 3.0 | 1.7 | 2.3 | 2.3 | 2.3 | 2.1 | 2.4 | 2.3 | 2.2 | 2.6 | 3.0 | 3.9 | 2.3 | 3.2 |
| | S.E. | 0.2 | 0.2 | 0.2 | 0.2 | 0.2 | 0.2 | 0.2 | 0.2 | 0.2 | 0.3 | 0.2 | 0.3 | 0.2 | 0.2 |
| HT-ID3-GDF15 3 nmol/kg | Mean | 2.9 | 1.5 | 1.9 | 1.9 | 2.0 | 1.6 | 2.1 | 2.1 | 1.9 | 2.4 | 2.5 | 3.9 | 2.9 | 3.2 |
| | S.E. | 0.1 | 0.1 | 0.1 | 0.1 | 0.2 | 0.1 | 0.1 | 0.1 | 0.1 | 0.2 | 0.3 | 0.2 | 0.1 | 0.1 |
| HT-ID3-GDF15 10 nmol/kg | Mean | 2.9 | 1.7 | 2.3 | 2.2 | 2.1 | 1.9 | 2.2 | 2.1 | 2.1 | 2.4 | 3.0 | 4.1 | 2.5 | 3.3 |
| | S.E. | 0.2 | 0.2 | 0.2 | 0.1 | 0.2 | 0.1 | 0.2 | 0.2 | 0.1 | 0.2 | 0.2 | 0.2 | 0.2 | 0.2 |
| HT-ID3-GDF15 30 nmol/kg | Mean | 2.5 | 1.6 | 2.1 | 2.2 | 2.3 | 1.8 | 2.2 | 2.1 | 2.0 | 2.5 | 2.9 | 4.0 | 2.1 | 3.0 |
| | S.E. | 0.2 | 0.1 | 0.1 | 0.2 | 0.1 | 0.1 | 0.1 | 0.1 | 0.1 | 0.2 | 0.2 | 0.1 | 0.1 | 0.2 |

| Group | Day | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|
| Lean Vehicle Control | Mean | 4.3 | 4.1 | 4.2 | 4.1 | 4.9 | 3.8 | 4.8 | 4.2 | 3.9 | 4.2 | 4.2 | 3.8 | 3.7 | 4.3 |
| | S.E. | 0.2 | 0.2 | 0.1 | 0.3 | 0.2 | 0.2 | 0.1 | 0.3 | 0.2 | 0.2 | 0.2 | 0.2 | 0.2 | 0.3 |
| DIO Vehicle Control | Mean | 3.2 | 3.2 | 3.1 | 3.1 | 3.1 | 2.7 | 3.2 | 2.7 | 2.8 | 3.1 | 3.4 | 3.5 | 2.9 | 3.2 |
| | S.E. | 0.1 | 0.1 | 0.1 | 0.1 | 0.1 | 0.1 | 0.1 | 0.4 | 0.1 | 0.2 | 0.2 | 0.5 | 0.1 | 0.1 |
| DIO Semaglutide 3 nmol/kg | Mean | 2.5 | 2.2 | 2.4 | 2.2 | 2.6 | 2.6 | 2.5 | 2.9 | 2.0 | 2.4 | 2.9 | 2.4 | 2.4 | 2.7 |
| | S.E. | 0.3 | 0.2 | 0.1 | 0.2 | 0.1 | 0.3 | 0.1 | 0.4 | 0.2 | 0.1 | 0.3 | 0.2 | 0.2 | 0.3 |
| HT-ID2-GDF15 3 nmol/kg | Mean | 3.2 | 3.4 | 3.4 | 3.4 | 3.7 | 3.3 | 3.2 | 3.4 | 3.1 | 3.3 | 3.4 | 3.3 | 3.1 | 3.4 |
| | S.E. | 0.2 | 0.5 | 0.4 | 0.2 | 0.4 | 0.5 | 0.1 | 0.3 | 0.1 | 0.2 | 0.1 | 0.2 | 0.1 | 0.1 |
| HT-ID2-GDF15 10 nmol/kg | Mean | 3.0 | 2.4 | 3.0 | 3.2 | 3.3 | 2.4 | 2.9 | 3.1 | 2.3 | 3.1 | 3.3 | 3.1 | 2.5 | 3.0 |
| | S.E. | 0.1 | 0.1 | 0.1 | 0.1 | 0.1 | 0.1 | 0.1 | 0.1 | 0.1 | 0.1 | 0.1 | 0.1 | 0.1 | 0.1 |
| HT-ID2-GDF15 30 nmol/kg | Mean | 3.3 | 2.4 | 3.1 | 3.2 | 3.1 | 2.1 | 3.2 | 3.1 | 2.1 | 3.0 | 3.1 | 3.3 | 2.1 | 2.9 |
| | S.E. | 0.3 | 0.2 | 0.2 | 0.2 | 0.1 | 0.1 | 0.2 | 0.1 | 0.2 | 0.2 | 0.1 | 0.1 | 0.2 | 0.2 |
| HT-ID3-GDF15 3 nmol/kg | Mean | 3.1 | 2.7 | 3.0 | 3.1 | 3.3 | 2.7 | 3.0 | 3.1 | 2.9 | 3.1 | 2.9 | 3.0 | 2.8 | 2.7 |
| | S.E. | 0.1 | 0.1 | 0.1 | 0.1 | 0.1 | 0.1 | 0.1 | 0.1 | 0.1 | 0.1 | 0.1 | 0.1 | 0.1 | 0.1 |
| HT-ID3-GDF15 10 nmol/kg | Mean | 3.2 | 2.2 | 3.3 | 3.3 | 3.4 | 2.4 | 3.1 | 3.2 | 2.7 | 3.1 | 3.3 | 3.3 | 2.7 | 3.2 |
| | S.E. | 0.2 | 0.3 | 0.2 | 0.1 | 0.1 | 0.1 | 0.1 | 0.1 | 0.2 | 0.1 | 0.1 | 0.1 | 0.2 | 0.2 |
| HT-ID3-GDF15 30 nmol/kg | Mean | 3.1 | 1.9 | 3.0 | 3.2 | 3.3 | 1.9 | 3.1 | 3.4 | 2.3 | 3.3 | 3.2 | 3.2 | 2.2 | 3.2 |
| | S.E. | 0.1 | 0.0 | 0.1 | 0.1 | 0.1 | 0.1 | 0.1 | 0.2 | 0.2 | 0.2 | 0.1 | 0.1 | 0.1 | 0.2 |

As shown in the above result, the administration group of the fusion polypeptide in which GDF15 was fused with IgD Hinge showed the feed intake reduction effect throughout the test period depending on the fusion polypeptide, compared to the negative control group (vehicle administration group) administration group, and showed a concentration-dependent tendency.

## Example 3. Pharmacokinetic test of fusion polypeptide

### 3.1. Test group and control group serum preparation

For evaluation of pharmacokinetic characteristics when each fusion polypeptide was subcutaneously administered to rats, the fusion polypeptides HT-ID1-GDF15, HT-ID2-GDF15 and HT-ID3-GDF15 were subcutaneously administered into SD rats (Koatech, male, 7-week-old, about 250g; n=3 each; test group) in an amount of 2 mg/kg, respectively, and about 200 μl of blood was collected through the caudal vein at a predetermined time. The blood collection time was performed before administration, and 1, 2, 4, 8, 24, 48, 72, 96, 168, 240 and 336 hours after administration. As the control group for comparison of pharmacokinetic characteristics, GDF15 (R&D Systems) was subcutaneously administered in an amount of 2 mg/kg by the same method to prepare a GDF15 administration group.

After administering into SD Rats as above, blood collected by time-point was centrifuged to obtain serum, and ELISA was performed using Human GDF15 Immunoassay (SGD150, R&D Systems), and the concentration in the serum was measured depending on the time of each polypeptide. Using this data, values of parameters including AUC (area under the curve) were obtained using a software for PK analysis (WinNonlin (Certara L.P.), etc.).

### 3.2 Pharmacokinetic test result

The obtained pharmacokinetic parameters of the fusion polypeptide were shown in Table 12, and the change in the concentration of the fusion polypeptide with time was shown in FIG. 12.

[Table 12]

| PK parameter | Group 1 | Group 2 | Group 3 | Group 4 |
|---|---|---|---|---|
| | rhGDF15 | HT-ID1-GDF15 | HT-ID2-GDF15 | HT-ID3-GDF15 |
| $C_{max}$ (ug/mL) | 0.443 | 2.09 | 0.945 | 1.11 |
| $T_{max}$ (hr) | 1 | 8 | 24 | 24 |
| $AUC_{last}$(ug*hr/mL) | 4.86 | 81.2 | 53.5 | 76.2 |
| $AUC_{inf}$(ug*hr/mL) | 4.88 | 81.3 | 53.5 | 76.4 |
| $t_{1/2}$ (hr) | 19.0 | 24.7 | 22.3 | 26.2 |
| $AUC_{extp}$ (%) | 0.463 | 0.0700 | 0.100 | 0.287 |

($C_{max}$: maximum concentration in blood, $T_{max}$: reaching time to maximum concentration in blood, $AUC_{inf}$: area under a blood concentration-time curve by extrapolating from the last measurable blood collecting time to infinity, $AUC_{last}$: area under a blood concentration-time curve up to the last measurable blood collecting time, $T_{1/2}$: loss half-life, $AUC_{Extp}$(%): [($AUC_{inf}$-$AUC_{last}$)/$AUC_{inf}$]*100)

As shown in the above result, it could be confirmed that the half-life was increased in case of the fusion polypeptide fused with IgD Hinge, compared to GDF15 (half-life: 19 hours), and in particular, in case of $AUC_{last}$, it was increased 16.7 times at maximum compared to GDF15.

From the above description, those skilled in the art to which the present invention pertains will understand that the present invention may be embodied in other specific forms without changing the technical spirit or essential characteristics thereof. In this regard, it should be understood that the embodiments described above are illustrative and not restrictive in all respects. The scope of the present invention should be construed that all changes or modifications derived from the meaning and scope of the claims to be described later and their equivalent concepts are included in the scope of the present invention, rather than the above detailed description.

## Claims

1. A fusion polypeptide, comprising,

   GDF15 (Growth differentiation factor 15), and
   a total of 1 to 10 polypeptide regions capable of O-glycosylation, which is bound to the N-terminus of the GDF15, wherein each of the 1 to 10 polypeptide regions capable of O-glycosylation is a polypeptide comprising 3 to 10 amino acid residues capable of O-glycosylation.

2. The fusion polypeptide according to claim 1, represented by the following formula:

   N'-(Z)n-Y -C'

   in the formula,

   N' is the N-terminus of the fusion polypeptide, and C' is the C-terminus of the fusion polypeptide, and
   Y is the GDF15, and
   Z is a polypeptide region capable of O-glycosylation, and
   n is the number of the polypeptide regions capable of O-glycosylation bound to the N-terminus of GDF15 and is an integer of 0 to 10.

3. The fusion polypeptide according to claim 1, wherein the 1 to 10 polypeptide regions capable of O-glycosylation are polypeptide regions comprising 1 to 10 immunoglobulin hinge regions or 10 or more continuous amino acids comprising 3 to 10 amino acid residues capable of O-glycosylation selected from proteins of SEQ ID NOs: 23 to 113.

**4.** The fusion polypeptide according to claim 3, wherein the 1 to 10 immunoglobulin hinge regions are immunoglobulin D (IgD) hinge regions.

**5.** The fusion polypeptide according to claim 4, wherein the 1 to 10 immunoglobulin hinge regions are each independently selected from the group consisting of the following:

(1) a polypeptide comprising the amino acid sequence of SEQ ID NO: 1,
(2) a polypeptide comprising 5 or more continuous amino acids comprising 3 to 7 O-glycosylation residues in the amino acid sequence of SEQ ID NO: 1, and
(3) a polypeptide comprising 34 or more continuous amino acids comprising the polypeptide of (1) or (2) in the IgD.

**6.** The fusion polypeptide according to claim 4, wherein the 1 to 10 immunoglobulin hinge regions are each independently selected from the group consisting of the following:

(1) a polypeptide comprising the amino acid sequence of SEQ ID NO: 1,
(2) a polypeptide comprising 5 or more continuous amino acids comprising SEQ ID NO: 9 or 7 or more continuous amino acids comprising SEQ ID NO: 10 in the amino acid sequence of SEQ ID NO: 1, and
(3) a polypeptide comprising 34 or more continuous amino acids comprising the polypeptide of (1) or (2) in the IgD.

**7.** The fusion polypeptide according to any one claim of claim 1 to claim 6, wherein the area under the blood concentration-time curve ($AUC_{last}$) up to the last blood sampling point measurable upon in vivo administration of the GDF15 bound to the polypeptide region capable of O-glycosylation in the fusion polypeptide is, increased at least 2-fold, compared to GDF15 not bound to the polypeptide region capable of O-glycosylation.

**8.** A nucleic acid molecule encoding the fusion polypeptide of any one claim of claim 1 to claim 6.

**9.** A recombinant vector comprising the nucleic acid molecule of claim 8.

**10.** A recombinant cell comprising the recombinant vector of claim 9.

**11.** A method of preparation of the fusion polypeptide of any one claim of claim 1 to claim 6, wherein the fusion polypeptide comprises GDF15 and a polypeptide region capable of O-glycosylation, and the method comprises culturing the recombinant cell of claim 10.

**12.** A method for enhancing in vivo stability of GDF15,

comprising linking a total of 1 to 10 polypeptide regions capable of O-glycosylation to the N-terminus of the GDF15,
wherein the 1 to 10 polypeptide regions capable of O-glycosylation are each a polypeptide comprising 3 to 10 amino acid residues capable of O-glycosylation.

**13.** The method for enhancing in vivo stability of GDF15 according to claim 12, wherein the 1 to 10 polypeptide regions capable of O-glycosylation are polypeptide regions comprising 1 to 10 immunoglobulin hinge regions or 10 or more continuous amino acids comprising 3 to 10 O-glycosylation residues selected from proteins of SEQ ID NOs: 23 to 113.

**14.** The method for enhancing in vivo stability of GDF15 according to claim 13, wherein the 1 to 10 immunoglobulin hinge regions are immunoglobulin D (IgD) hinge regions.

**15.** The method for enhancing in vivo stability of GDF15 according to claim 14, wherein the 1 to 10 immunoglobulin hinge regions are each independently selected from the group consisting of the following:

(1) a polypeptide comprising the amino acid sequence of SEQ ID NO: 1,
(2) a polypeptide comprising 5 or more continuous amino acids comprising 3 to 7 O-glycosylation residues in the amino acid sequence of SEQ ID NO: 1, and
(3) a polypeptide comprising 34 or more continuous amino acids comprising the polypeptide of (1) or (2) in the IgD.

**16.** The method for enhancing in vivo stability of GDF15 according to claim 14, wherein the 1 to 10 immunoglobulin hinge regions are each independently selected from the group consisting of the following:

(1) a polypeptide comprising the amino acid sequence of SEQ ID NO: 1,
(2) a polypeptide comprising 5 or more continuous amino acids comprising SEQ ID NO: 9 or 7 or more continuous amino acids comprising SEQ ID NO: 10 in the amino acid sequence of SEQ ID NO: 1, and
(3) a polypeptide comprising 34 or more continuous amino acids comprising the polypeptide of (1) or (2) in the IgD.

**17.** A fusion polypeptide dimer, comprising 2 of the fusion polypeptides of any one claim of claim 1 to claim 6.

**18.** The fusion polypeptide dimer according to claim 17, wherein the GDF15 of each fusion polypeptide binds to each other to form a dimer.

**19.** The fusion polypeptide dimer according to claim 17, wherein the dimer is a homodimer.

**20.** A pharmaceutical composition for preventing or treating diseases related to GDF15 deficiency or dysfunction, comprising the fusion polypeptide of any one claim of claim 1 to claim 6, or a fusion polypeptide dimer in which two of the fusion polypeptides are linked to each other at the GDF15.

【FIG. 1】

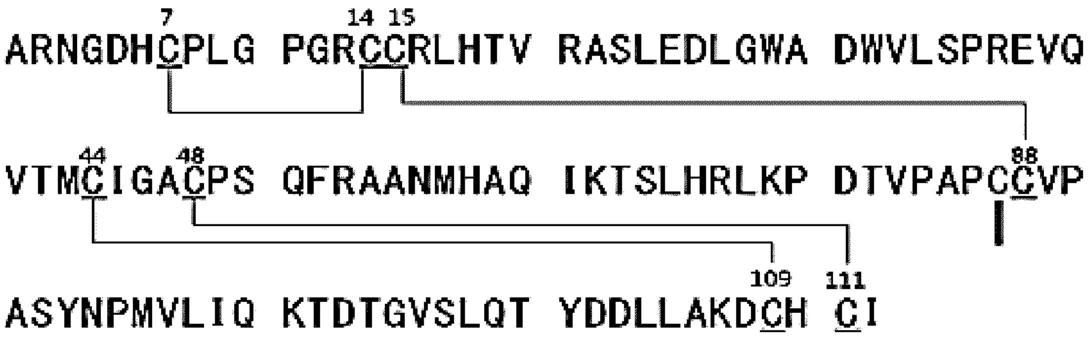


(SEQ ID NO: 3)

【FIG. 2a】

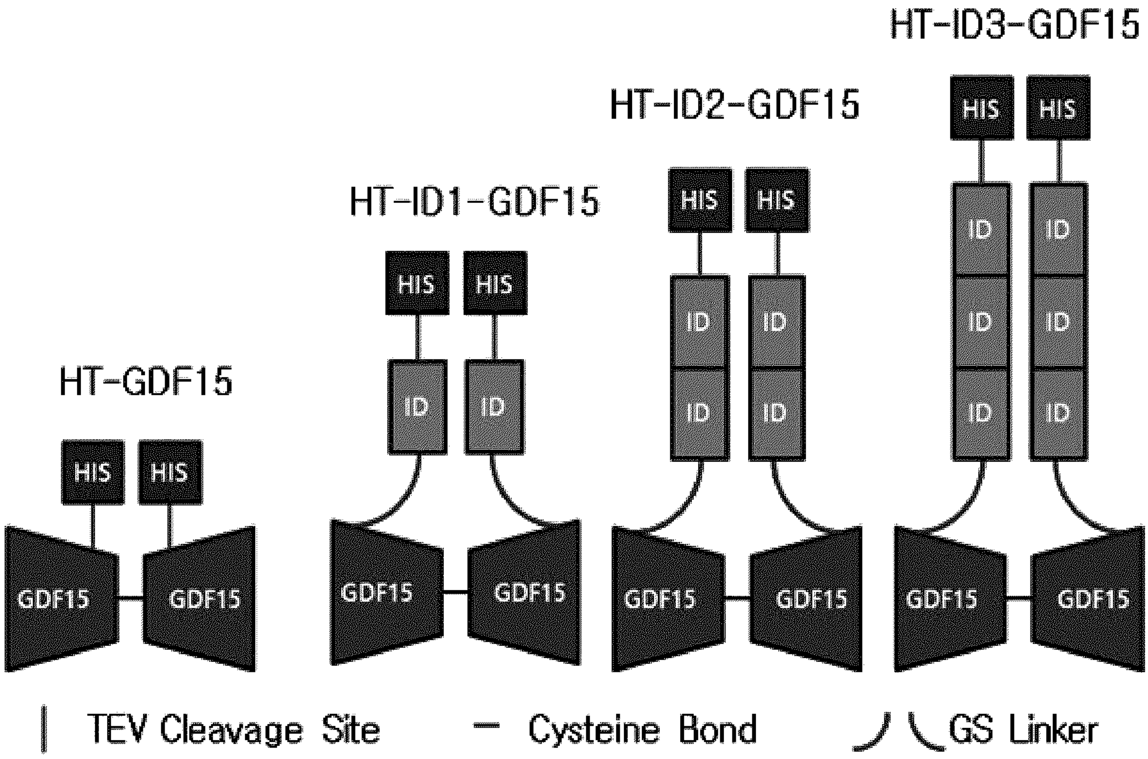


ID: ESPKAQASSVPTAQPQAEGSLAKATTAPATT RNT (SEQ ID NO: 1)
HIS: HHHHHH (SEQ ID NO: 15)
TEV Cleavage Site: ENLYFQG (SEQ ID NO: 16)
GS Linker: GGGGSGGGGS GGGGSGGGGS (SEQ ID NO: 17)
GDF15: SEQ ID NO: 3

【FIG. 2b】

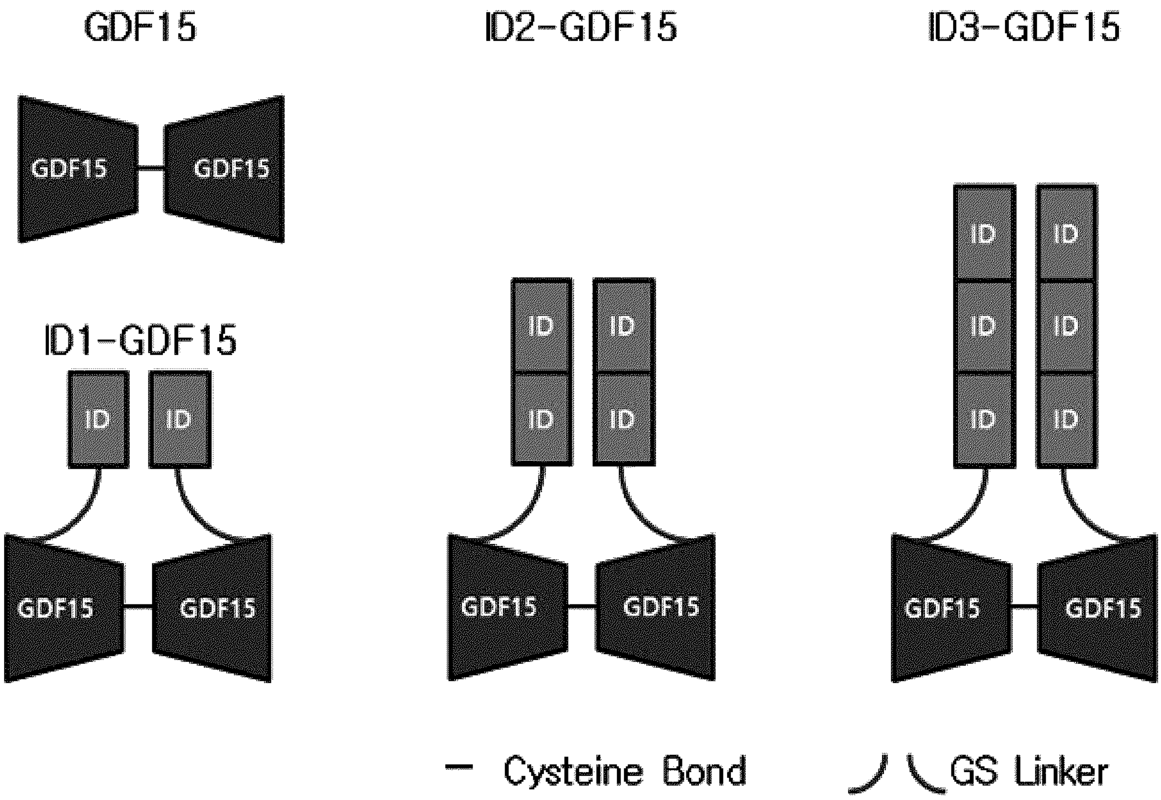


ID: ESPKAQA<u>SS</u>VP<u>T</u>AQPQAEGSLAKA<u>TT</u>APA<u>TT</u> RNT (SEQ ID NO: 1)
GS Linker: GGGGSGGGGS GGGGSGGGGS (SEQ ID NO: 17)
GDF15: SEQ ID NO: 3

【FIG. 3】

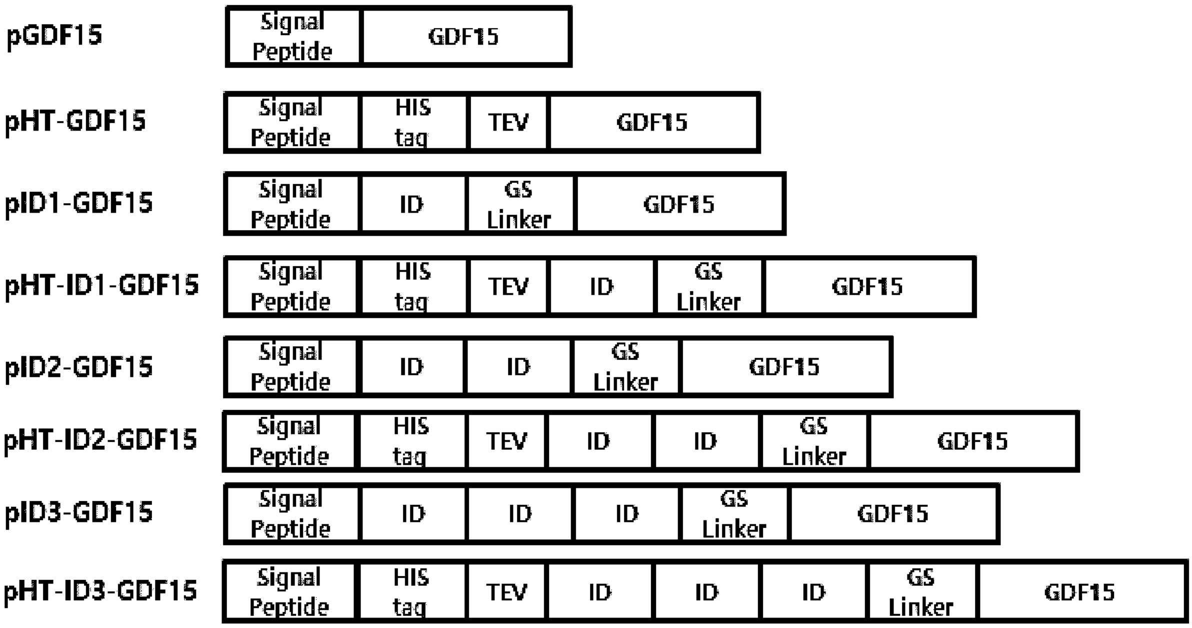
pGDF15
Signal Peptide
GDF15
pHT-GDF15
Signal Peptide
HIS tag
TEV
GDF15
pID1-GDF15
Signal Peptide
ID
GS Linker
GDF15
pHT-ID1-GDF15
Signal Peptide
HIS tag
TEV
ID
GS Linker
GDF15
pID2-GDF15
Signal Peptide
ID
ID
GS Linker
GDF15
pHT-ID2-GDF15
Signal Peptide
HIS tag
TEV
ID
ID
GS Linker
GDF15
pID3-GDF15
Signal Peptide
ID
ID
ID
GS Linker
GDF15
pHT-ID3-GDF15
Signal Peptide
HIS tag
TEV
ID
ID
ID
GS Linker
GDF15

【FIG. 4】

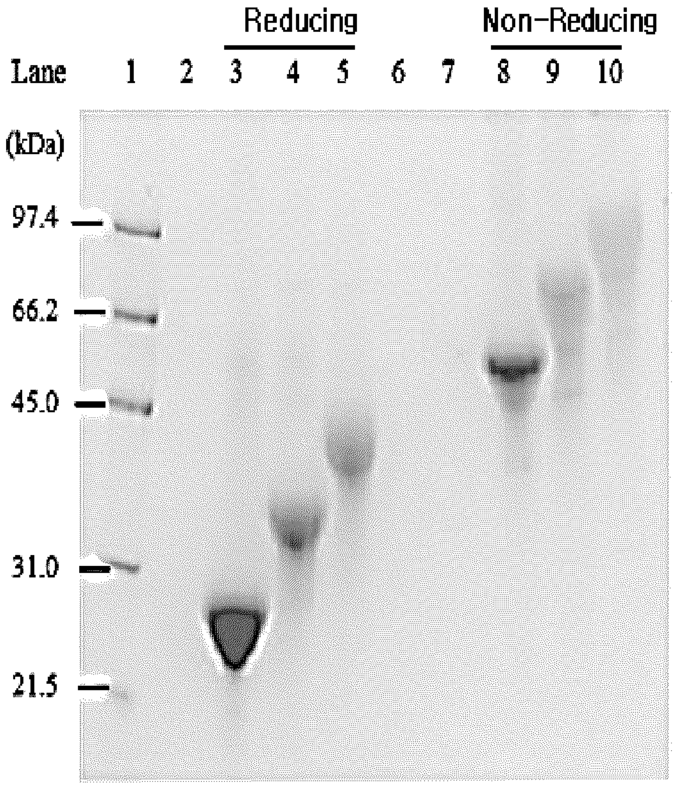


| Lane | Sample |
|---|---|
| 1 | Low Range MW Standard |
| 2, 6, 7 | Empty |
| 3 | HT-ID1-GDF15 (R) |
| 4 | HT-ID2-GDF15 (R) |
| 5 | HT-ID3-GDF15 (R) |
| 8 | HT-ID1-GDF15 (NR) |
| 9 | HT-ID2-GDF15 NR) |
| 10 | HT-ID3-GDF15 NR) |

【FIG. 5】

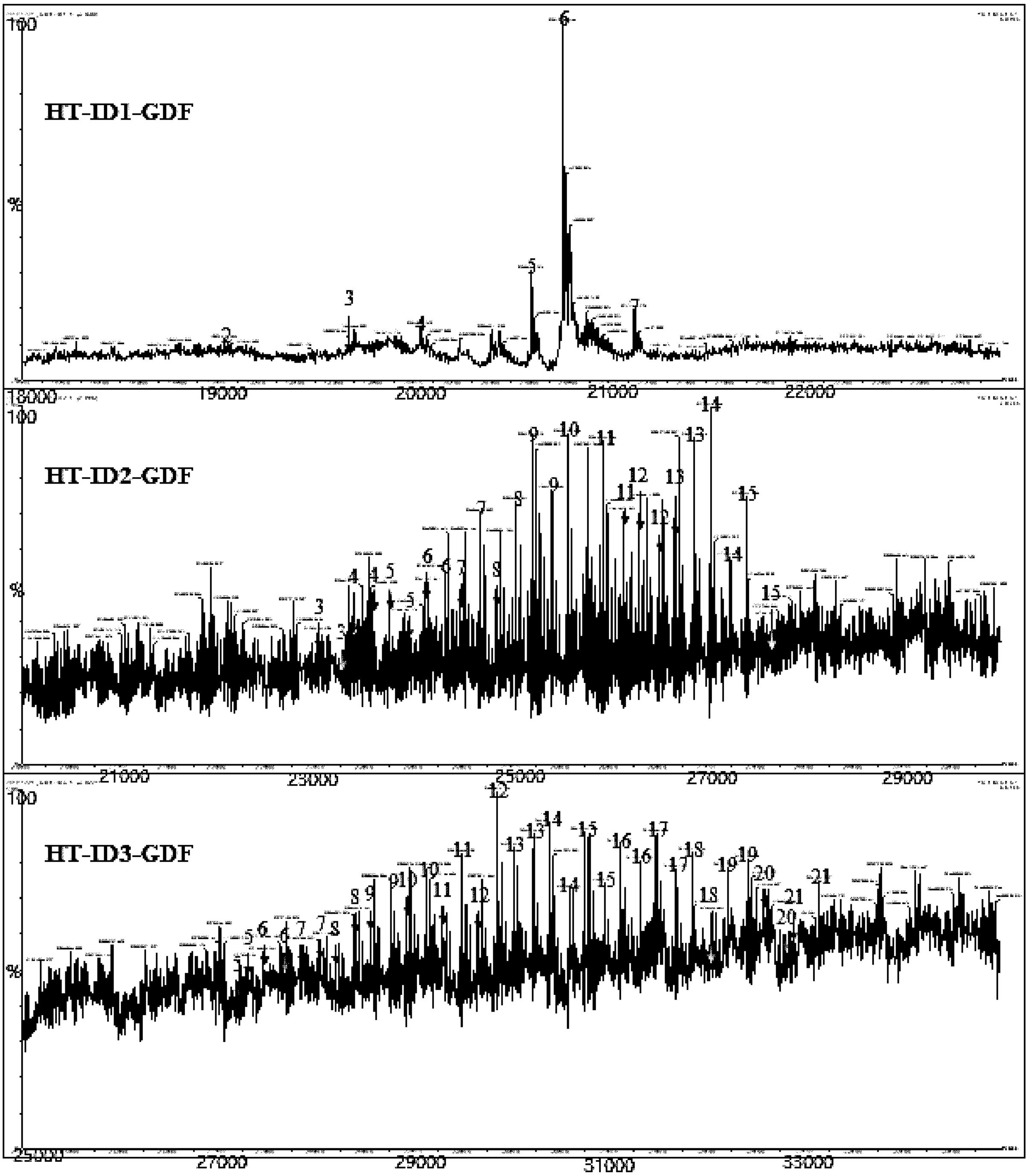
HT-ID1-GDF
HT-ID2-GDF
HT-ID3-GDF
100
%
18000
19000
20000
21000
22000
23000
25000
27000
29000
31000
33000

【FIG. 6】

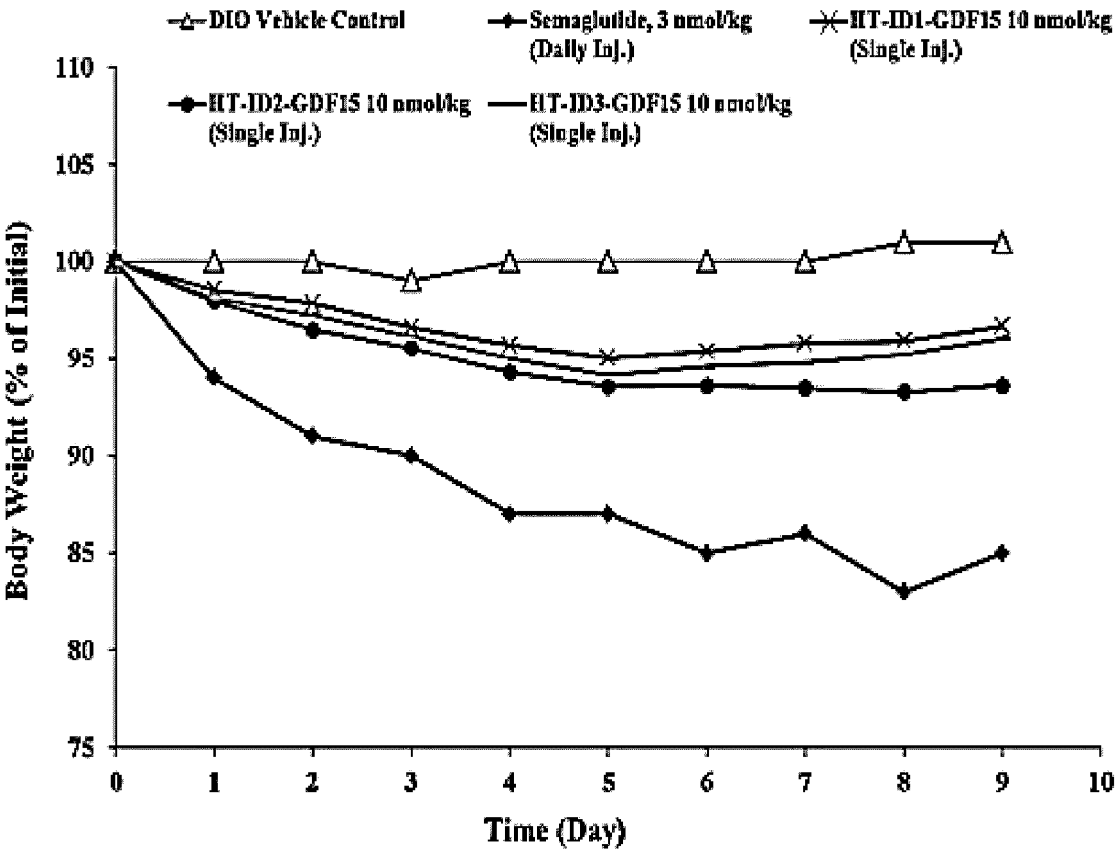
DIO Vehicle Control
Semaglutide, 3 nmol/kg (Daily Inj.)
HT-ID1-GDF15 10 nmol/kg (Single Inj.)
HT-ID2-GDF15 10 nmol/kg (Single Inj.)
HT-ID3-GDF15 10 nmol/kg (Single Inj.)
Body Weight (% of Initial)
110
105
100
95
90
85
80
75
0
1
2
3
4
5
6
7
8
9
10
Time (Day)

【FIG. 7】

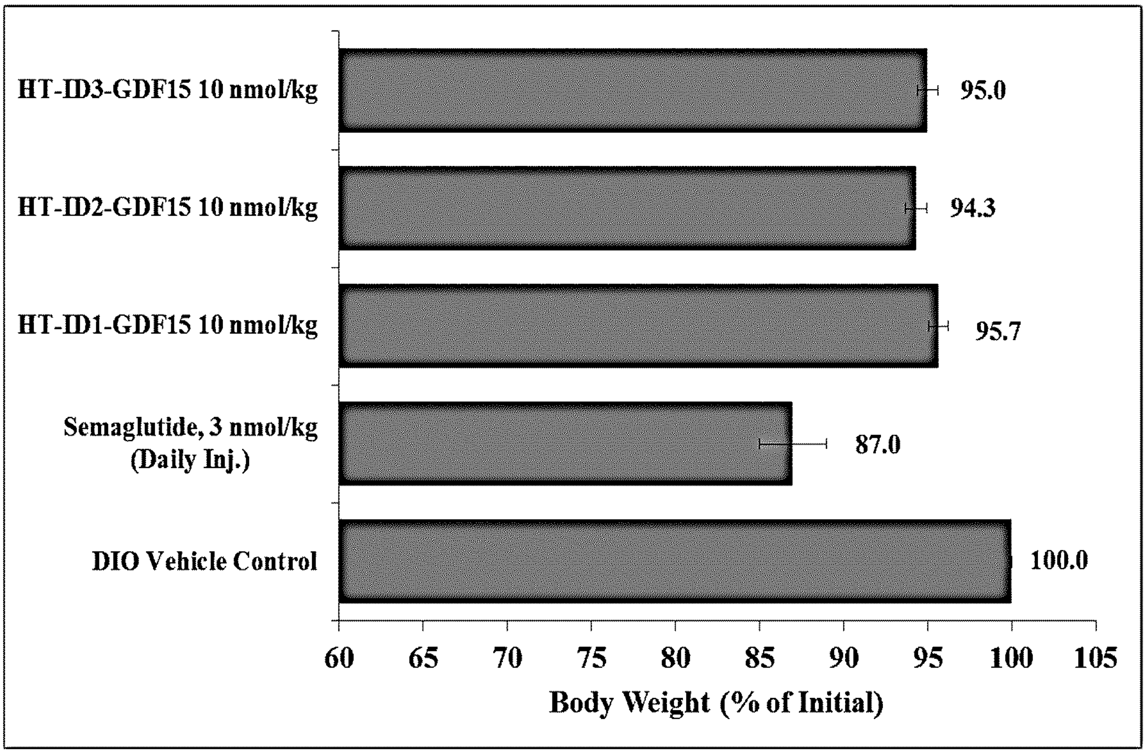
HT-ID3-GDF15 10 nmol/kg
HT-ID2-GDF15 10 nmol/kg
HT-ID1-GDF15 10 nmol/kg
Semaglutide, 3 nmol/kg (Daily Inj.)
DIO Vehicle Control
95.0
94.3
95.7
87.0
100.0
60
65
70
75
80
85
90
95
100
105
Body Weight (% of Initial)

【FIG. 8】

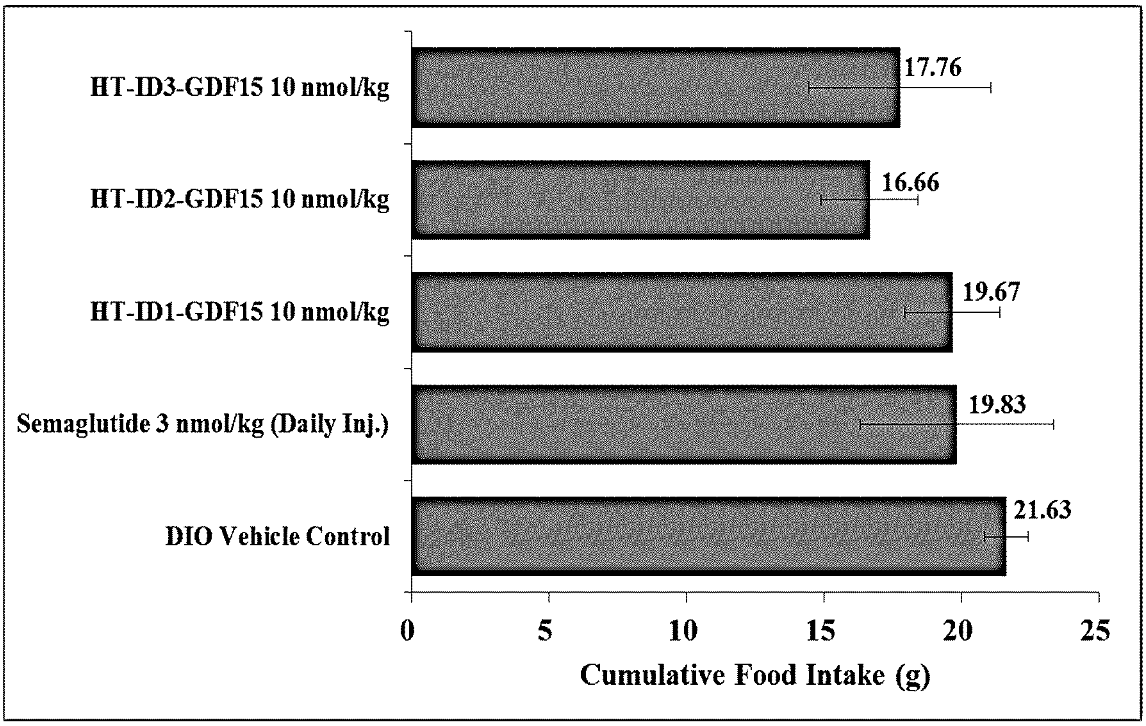
HT-ID3-GDF15 10 nmol/kg
HT-ID2-GDF15 10 nmol/kg
HT-ID1-GDF15 10 nmol/kg
Semaglutide 3 nmol/kg (Daily Inj.)
DIO Vehicle Control
17.76
16.66
19.67
19.83
21.63
0
5
10
15
20
25
Cumulative Food Intake (g)

【FIG. 9】

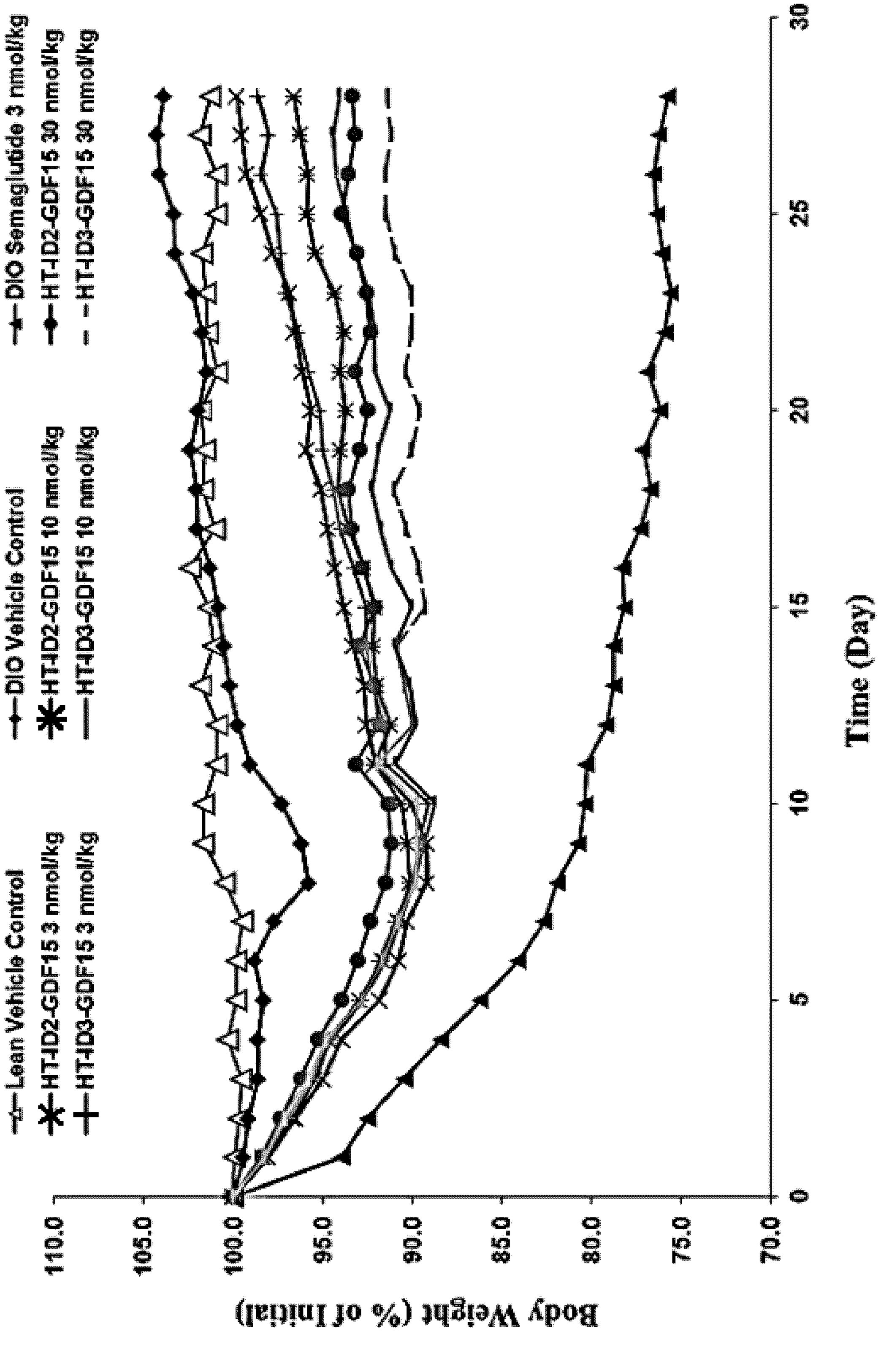
Lean Vehicle Control
HT-ID2-GDF15 3 nmol/kg
HT-ID3-GDF15 3 nmol/kg
DIO Vehicle Control
HT-ID2-GDF15 10 nmol/kg
HT-ID3-GDF15 10 nmol/kg
DIO Semaglutide 3 nmol/kg
HT-ID2-GDF15 30 nmol/kg
HT-ID3-GDF15 30 nmol/kg
Body Weight (% of Initial)
110.0
105.0
100.0
95.0
90.0
85.0
80.0
75.0
70.0
Time (Day)
0
5
10
15
20
25
30

【FIG. 10a】

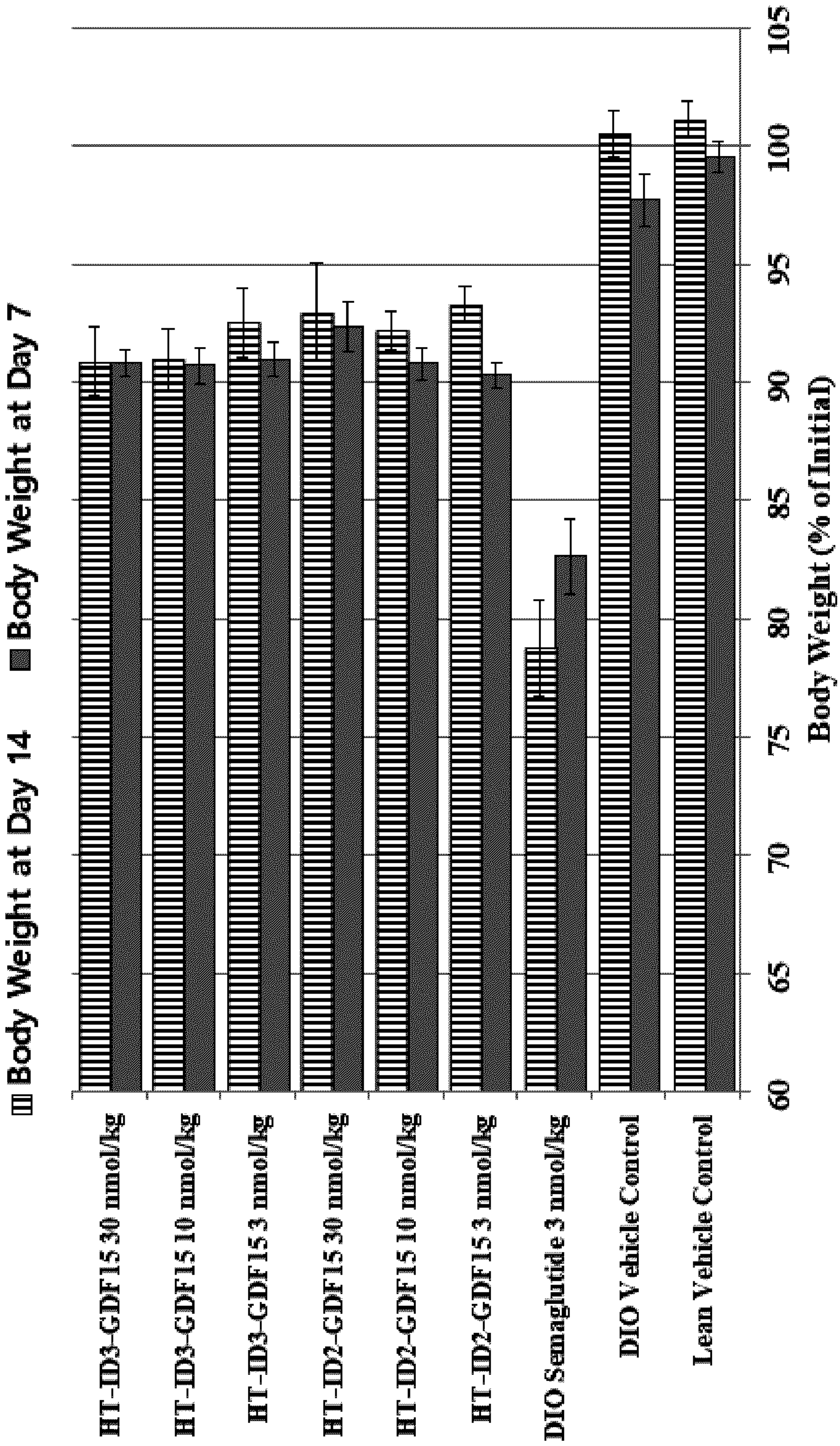
Body Weight at Day 14
Body Weight at Day 7
HT-ID3-GDF15 30 nmol/kg
HT-ID3-GDF15 10 nmol/kg
HT-ID3-GDF15 3 nmol/kg
HT-ID2-GDF15 30 nmol/kg
HT-ID2-GDF15 10 nmol/kg
HT-ID2-GDF15 3 nmol/kg
DIO Semaglutide 3 nmol/kg
DIO Vehicle Control
Lean Vehicle Control
60
65
70
75
80
85
90
95
100
105
Body Weight (% of Initial)

【FIG. 10b】

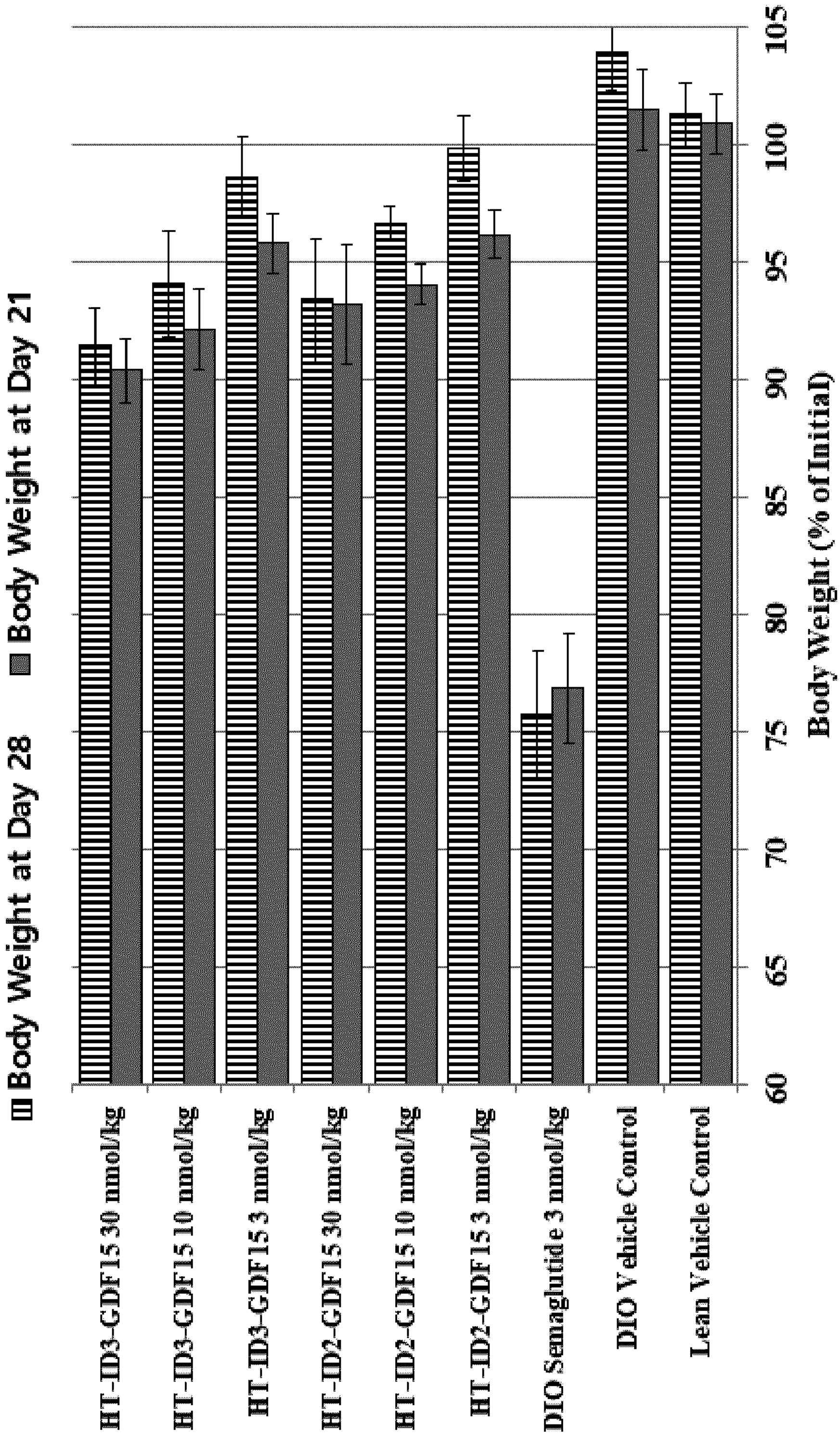

Body Weight at Day 28
Body Weight at Day 21
HT-ID3-GDF15 30 nmol/kg
HT-ID3-GDF15 10 nmol/kg
HT-ID3-GDF15 3 nmol/kg
HT-ID2-GDF15 30 nmol/kg
HT-ID2-GDF15 10 nmol/kg
HT-ID2-GDF15 3 nmol/kg
DIO Semaglutid e 3 nmol/kg
DIO Vehicle Control
Lean Vehicle Control
60
65
70
75
80
85
90
95
100
105
Body Weight (% of Initial)

【FIG. 11】

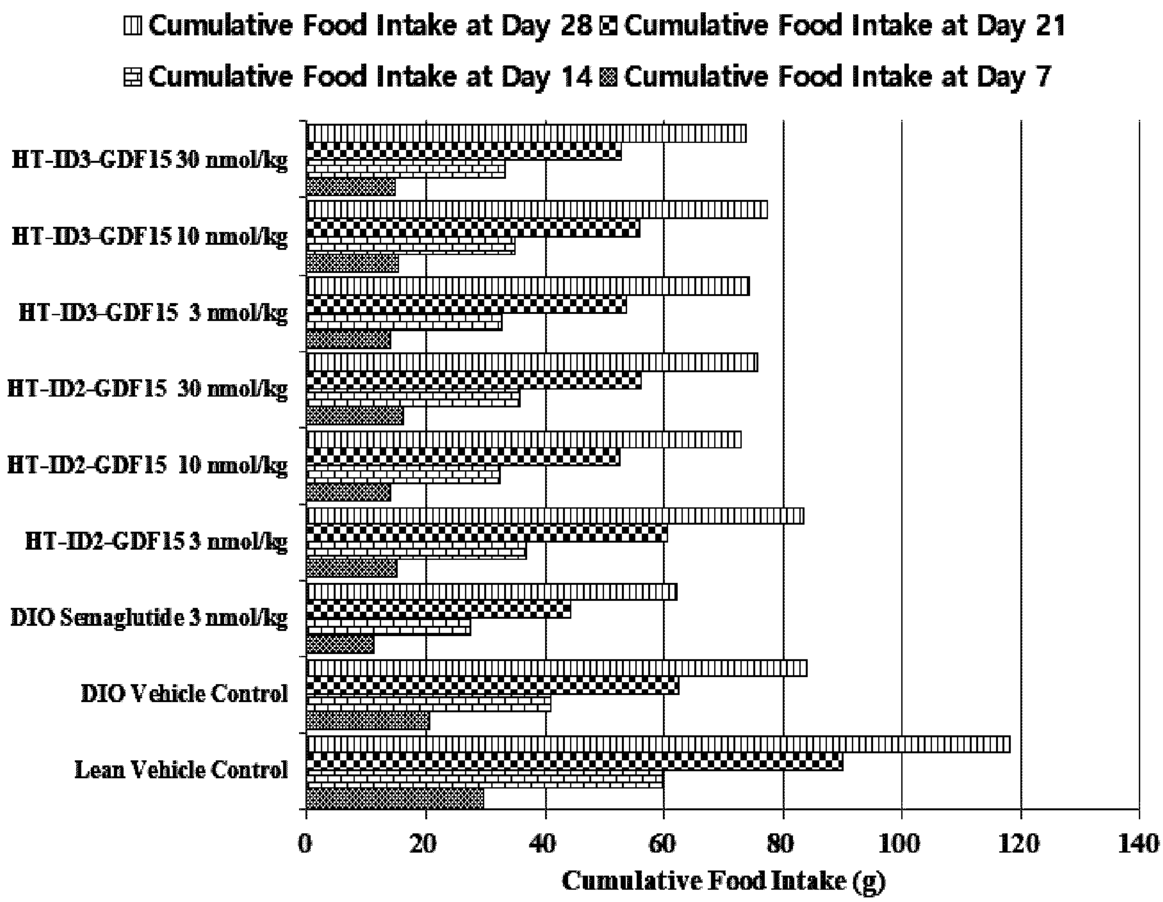
Cumulative Food Intake at Day 28
Cumulative Food Intake at Day 21
Cumulative Food Intake at Day 14
Cumulative Food Intake at Day 7
HT-ID3-GDF15 30 nmol/kg
HT-ID3-GDF15 10 nmol/kg
HT-ID3-GDF15 3 nmol/kg
HT-ID2-GDF15 30 nmol/kg
HT-ID2-GDF15 10 nmol/kg
HT-ID2-GDF15 3 nmol/kg
DIO Semaglutide 3 nmol/kg
DIO Vehicle Control
Lean Vehicle Control
0
20
40
60
80
100
120
140
Cumulative Food Intake (g)

【FIG. 12】

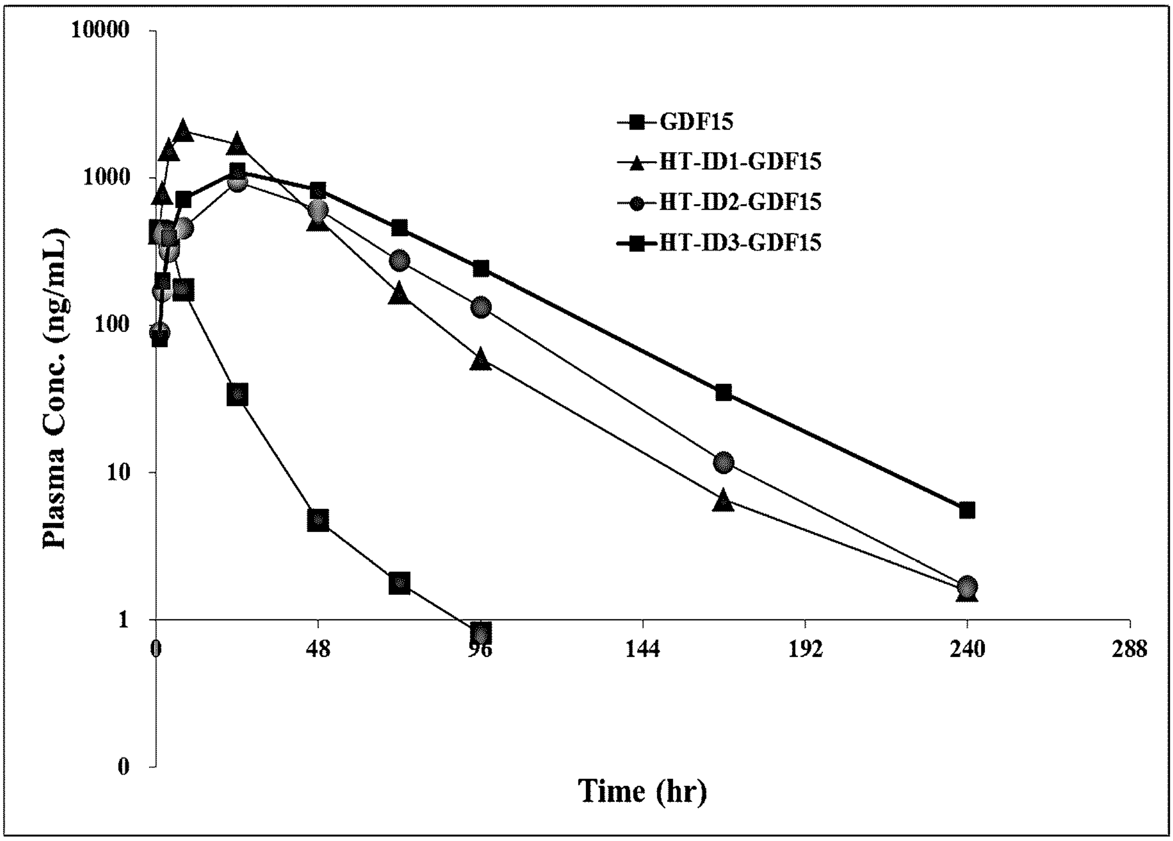

10000
1000
100
10
1
0
Plasma Conc. (ng/mL)
0
48
96
144
192
240
288
Time (hr)
GDF15
HT-ID1-GDF15
HT-ID2-GDF15
HT-ID3-GDF15

## INTERNATIONAL SEARCH REPORT

International application No.
**PCT/KR2020/018053**

**A. CLASSIFICATION OF SUBJECT MATTER**

**C07K 14/475**(2006.01)i; **A61K 38/18**(2006.01)i; **A61K 47/68**(2017.01)i

According to International Patent Classification (IPC) or to both national classification and IPC

**B. FIELDS SEARCHED**

Minimum documentation searched (classification system followed by classification symbols)

C07K 14/475(2006.01); A61K 38/00(2006.01); A61K 38/36(2006.01); A61K 47/50(2017.01); C07K 14/47(2006.01); C07K 19/00(2006.01); C12N 15/09(2006.01); C12N 5/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: GDF15, O-글리코실화(O-glycosylation), 융합 폴리펩타이드(fusion polypeptide), 반감기(half-life)

**C. DOCUMENTS CONSIDERED TO BE RELEVANT**

| Category* | Citation of document, with indication, where appropriate, of the relevant passages | Relevant to claim No. |
|---|---|---|
| Y | KR 10-1380729 B1 (GENEXINE, INC. et al.) 10 April 2014 (2014-04-10) See abstract; claims 1-17; and paragraphs [0009], [0019], [0039], [0051], [0064], [0068], [0097] and [0123]. | 1-10,12-20 |
| Y | KR 10-2017-0074852 A (NGM BIOPHARMACEUTICALS, INC.) 30 June 2017 (2017-06-30) See paragraphs [0013], [0015], [0091], [0262] and [0264]; and example 1. | 1-10,12-20 |
| A | JP 2010-531135 A (NOVO NORDISK AKTIE SELSXAB) 24 September 2010 (2010-09-24) See entire document. | 1-10,12-20 |
| A | WO 2017-198435 A1 (OCTAPHARMA AG) 23 November 2017 (2017-11-23) See entire document. | 1-10,12-20 |
| A | KR 10-2016-0038896 A (AMGEN INC.) 07 April 2016 (2016-04-07) See entire document. | 1-10,12-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 April 2021** | **06 April 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2020/018053**

**C. DOCUMENTS CONSIDERED TO BE RELEVANT**

| Category* | Citation of document, with indication, where appropriate, of the relevant passages | Relevant to claim No. |
|---|---|---|
| A | US 2007-0041967 A1 (JUNG, S. Y. et al.) 22 February 2007 (2007-02-22)<br>See entire document. | 1-10,12-20 |

Form PCT/ISA/210 (second sheet) (July 2019)

**INTERNATIONAL SEARCH REPORT**

International application No.
**PCT/KR2020/018053**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**

International application No.
**PCT/KR2020/018053**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☑ Claims Nos.: **11**
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2020/018053**

| Patent document cited in search report | Publication date (day/month/year) | Patent family member(s) | Publication date (day/month/year) |
|---|---|---|---|
| KR 10-1380729 B1 | 10 April 2014 | AU 2008-257923 A1 | 04 December 2008 |
| | | AU 2008-257923 B2 | 21 March 2013 |
| | | BR PI0811637 A2 | 18 November 2014 |
| | | BR PI0811637 B1 | 24 November 2020 |
| | | CA 2687377 A1 | 04 December 2008 |
| | | CA 2687377 C | 14 May 2013 |
| | | CN 101687933 A | 31 March 2010 |
| | | CN 101687933 B | 25 November 2015 |
| | | CN 103626875 A | 12 March 2014 |
| | | CN 103626875 B | 13 January 2016 |
| | | CN 103641919 A | 19 March 2014 |
| | | CN 103641919 B | 05 April 2017 |
| | | CN 105131127 A | 09 December 2015 |
| | | CN 105131127 B | 07 September 2018 |
| | | CN 105175553 A | 23 December 2015 |
| | | CN 105175553 B | 22 November 2019 |
| | | CN 105198998 A | 30 December 2015 |
| | | DK 2662449 T3 | 15 May 2017 |
| | | EP 2162472 A2 | 17 March 2010 |
| | | EP 2162472 B1 | 27 February 2013 |
| | | EP 2662449 A2 | 13 November 2013 |
| | | EP 2662449 A3 | 18 November 2015 |
| | | EP 2662449 B1 | 15 February 2017 |
| | | EP 3173484 A1 | 31 May 2017 |
| | | EP 3173484 B1 | 26 September 2018 |
| | | EP 3176264 A1 | 07 June 2017 |
| | | EP 3176264 B1 | 26 September 2018 |
| | | ES 2415604 T3 | 26 July 2013 |
| | | ES 2623925 T3 | 12 July 2017 |
| | | HK 1141303 A1 | 05 November 2010 |
| | | HK 1191959 A1 | 08 August 2014 |
| | | HK 1218127 A1 | 03 February 2017 |
| | | HK 1219284 A1 | 31 March 2017 |
| | | HK 1219490 A1 | 07 April 2017 |
| | | IL 202128 B | 29 November 2012 |
| | | JP 2010-531134 A | 24 September 2010 |
| | | JP 5577243 B2 | 20 August 2014 |
| | | KR 10-0897938 B1 | 18 May 2009 |
| | | KR 10-1380732 B1 | 10 April 2014 |
| | | KR 10-2008-0094781 A | 24 October 2008 |
| | | KR 10-2009-0045953 A | 08 May 2009 |
| | | KR 10-2014-0037961 A | 27 March 2014 |
| | | RU 2009141975 A | 20 May 2011 |
| | | RU 2530168 C2 | 10 October 2014 |
| | | US 2008-0300188 A1 | 04 December 2008 |
| | | US 2011-0091416 A1 | 21 April 2011 |
| | | US 2012-0276043 A1 | 01 November 2012 |
| | | US 2012-0276096 A1 | 01 November 2012 |
| | | US 2012-0276097 A1 | 01 November 2012 |
| | | US 7867491 B2 | 11 January 2011 |

Form PCT/ISA/210 (patent family annex) (July 2019)

**INTERNATIONAL SEARCH REPORT**
**Information on patent family members**

International application No.
**PCT/KR2020/018053**

| Patent document cited in search report | Publication date (day/month/year) | Patent family member(s) | Publication date (day/month/year) |
|---|---|---|---|
| | | US 8529899 B2 | 10 September 2013 |
| | | US 8586038 B2 | 19 November 2013 |
| | | US 8586048 B2 | 19 November 2013 |
| | | US 8586531 B2 | 19 November 2013 |
| | | WO 2008-147143 A2 | 04 December 2008 |
| | | WO 2008-147143 A3 | 26 February 2009 |
| KR 10-2017-0074852 A | 30 June 2017 | AR 102471 A1 | 01 March 2017 |
| | | AU 2015-339130 A1 | 06 April 2017 |
| | | AU 2015-339130 B2 | 25 June 2020 |
| | | AU 2015-339130 C1 | 26 November 2020 |
| | | BR 112017005986 A2 | 19 December 2017 |
| | | CA 2961587 A1 | 06 May 2016 |
| | | CL 2017000864 A1 | 11 May 2018 |
| | | CN 106687128 A | 17 May 2017 |
| | | DK 3212226 T3 | 15 June 2020 |
| | | DO P2017000097 A | 15 May 2017 |
| | | EA 036985 B1 | 25 January 2021 |
| | | EA 201790405 A1 | 29 September 2017 |
| | | EC SP17021754 A | 31 May 2017 |
| | | EP 3212226 A1 | 06 September 2017 |
| | | EP 3212226 B1 | 18 March 2020 |
| | | EP 3701969 A1 | 02 September 2020 |
| | | ES 2799503 T3 | 18 December 2020 |
| | | GT 201700072 A | 27 November 2018 |
| | | IL 251066 A | 30 April 2017 |
| | | JP 2017-538395 A | 28 December 2017 |
| | | JP 6722175 B2 | 15 July 2020 |
| | | MD 20170035 A2 | 30 September 2017 |
| | | MX 2017005237 A | 30 June 2017 |
| | | MX 357994 B | 01 August 2018 |
| | | NI 201700042 A | 04 May 2017 |
| | | PE 10582017 A1 | 21 July 2017 |
| | | PE 20171058 A1 | 21 July 2017 |
| | | PH 12017500680 A1 | 09 October 2017 |
| | | PL 3212226 T3 | 02 November 2020 |
| | | PT 3212226 T | 22 June 2020 |
| | | SG 11201702580 A | 30 May 2017 |
| | | SV 2017005420 A | 17 October 2017 |
| | | TN 2017000113 A1 | 04 July 2018 |
| | | TW 201625698 A | 16 July 2016 |
| | | TW I703161 B | 01 September 2020 |
| | | UA 121973 C2 | 25 August 2020 |
| | | US 10562965 B2 | 18 February 2020 |
| | | US 2016-0120999 A1 | 05 May 2016 |
| | | US 2018-0237514 A1 | 23 August 2018 |
| | | US 2020-0140536 A1 | 07 May 2020 |
| | | US 9920118 B2 | 20 March 2018 |
| | | WO 2016-069921 A1 | 06 May 2016 |
| JP 2010-531135 A | 24 September 2010 | CN 101778937 A | 14 July 2010 |
| | | EP 2162535 A2 | 17 March 2010 |

Form PCT/ISA/210 (patent family annex) (July 2019)

**INTERNATIONAL SEARCH REPORT**
**Information on patent family members**

International application No.
**PCT/KR2020/018053**

| Patent document cited in search report | Publication date (day/month/year) | Patent family member(s) | Publication date (day/month/year) |
|---|---|---|---|
| | | US 2011-0177029 A1 | 21 July 2011 |
| | | WO 2008-151258 A2 | 11 December 2008 |
| | | WO 2008-151258 A3 | 12 February 2009 |
| WO 2017-198435 A1 | 23 November 2017 | AU 2017-266758 A1 | 29 November 2018 |
| | | AU 2017-267047 A1 | 29 November 2018 |
| | | BR 112018073669 A2 | 26 February 2019 |
| | | BR 112018073674 A2 | 26 February 2019 |
| | | CA 3024349 A1 | 23 November 2017 |
| | | CA 3024378 A1 | 23 November 2017 |
| | | CN 109152809 A | 04 January 2019 |
| | | CN 109152817 A | 04 January 2019 |
| | | EP 3458080 A1 | 27 March 2019 |
| | | EP 3458085 A1 | 27 March 2019 |
| | | JP 2019-519220 A | 11 July 2019 |
| | | JP 2019-522962 A | 22 August 2019 |
| | | KR 10-2019-0007500 A | 22 January 2019 |
| | | KR 10-2019-0012189 A | 08 February 2019 |
| | | RU 2018144112 A | 22 June 2020 |
| | | RU 2018145062 A | 23 June 2020 |
| | | RU 2018145062 A3 | 08 October 2020 |
| | | US 2019-0169268 A1 | 06 June 2019 |
| | | US 2019-0201495 A1 | 04 July 2019 |
| | | WO 2017-198877 A1 | 23 November 2017 |
| KR 10-2016-0038896 A | 07 April 2016 | AR 097181 A1 | 24 February 2016 |
| | | AU 2014-296107 A1 | 04 February 2016 |
| | | AU 2014-296107 B2 | 26 July 2018 |
| | | CA 2918624 A1 | 05 February 2015 |
| | | CL 2016000249 A1 | 22 July 2016 |
| | | CN 105980400 A | 28 September 2016 |
| | | CR 20160097 A | 29 July 2016 |
| | | DK 3027642 T3 | 02 November 2020 |
| | | EA 201690297 A1 | 30 June 2016 |
| | | EP 3027642 A1 | 08 June 2016 |
| | | EP 3027642 B1 | 19 August 2020 |
| | | EP 3763734 A1 | 13 January 2021 |
| | | HK 1225738 A1 | 15 September 2017 |
| | | IL 243749 A | 21 April 2016 |
| | | IL 243749 B | 28 June 2018 |
| | | JP 2016-532690 A | 20 October 2016 |
| | | JP 2019-178132 A | 17 October 2019 |
| | | JP 6509852 B2 | 08 May 2019 |
| | | MA 38873 A1 | 29 September 2017 |
| | | MA 38873 B1 | 30 November 2018 |
| | | MX 2016001164 A | 26 July 2016 |
| | | MX 2019012848 A | 28 November 2019 |
| | | MX 369152 B | 30 October 2019 |
| | | PE 01892016 A1 | 06 May 2016 |
| | | PE 20160189 A1 | 06 May 2016 |
| | | PH 12016500208 A1 | 25 April 2016 |
| | | PH 12016500208 B1 | 25 April 2016 |

Form PCT/ISA/210 (patent family annex) (July 2019)

**INTERNATIONAL SEARCH REPORT**
**Information on patent family members**

International application No.
**PCT/KR2020/018053**

| Patent document cited in search report | Publication date (day/month/year) | Patent family member(s) | Publication date (day/month/year) |
|---|---|---|---|
| | | PL 3027642 T3 | 11 January 2021 |
| | | SG 11201600713 A | 26 February 2016 |
| | | SI 3027642 T1 | 30 November 2020 |
| | | TN 2016000035 A1 | 05 July 2017 |
| | | TW 201602345 A | 16 January 2016 |
| | | TW I648401 B | 21 January 2019 |
| | | UA 116665 C2 | 25 April 2018 |
| | | US 10894814 B2 | 19 January 2021 |
| | | US 2016-0168213 A1 | 16 June 2016 |
| | | US 2018-0079790 A1 | 22 March 2018 |
| | | US 9862752 B2 | 09 January 2018 |
| | | UY 35686 A | 30 January 2015 |
| | | WO 2015-017710 A1 | 05 February 2015 |
| US 2007-0041967 A1 | 22 February 2007 | AR 069458 A1 | 20 January 2010 |
| | | AU 2004-282984 A1 | 14 July 2005 |
| | | AU 2004-282984 B2 | 14 July 2011 |
| | | AU 2004-282985 A1 | 30 June 2005 |
| | | AU 2004-282985 B2 | 14 August 2008 |
| | | AU 2008-330315 A1 | 04 June 2009 |
| | | AU 2008-330315 B2 | 21 June 2012 |
| | | BR PI0406605 A | 06 December 2005 |
| | | BR PI0406605 B1 | 29 October 2019 |
| | | BR PI0406606 A | 06 December 2005 |
| | | BR PI0819864 A2 | 23 May 2017 |
| | | CA 2512657 A1 | 26 May 2005 |
| | | CA 2512657 C | 07 January 2014 |
| | | CA 2512933 A1 | 26 May 2005 |
| | | CA 2512933 C | 06 December 2011 |
| | | CA 2706627 A1 | 04 June 2009 |
| | | CA 2706627 C | 18 November 2014 |
| | | CN 101878036 A | 03 November 2010 |
| | | CN 101878036 B | 14 May 2014 |
| | | CN 103212084 A | 24 July 2013 |
| | | CN 103212084 B | 13 July 2018 |
| | | CN 108743967 A | 06 November 2018 |
| | | CN 1723219 A | 18 January 2006 |
| | | CN 1723219 B | 26 May 2010 |
| | | CN 1723219 C | 18 January 2006 |
| | | CN 1723220 A | 18 January 2006 |
| | | CN 1723220 C | 18 January 2006 |
| | | DK 1682583 T3 | 07 May 2012 |
| | | DK 2227243 T3 | 06 October 2014 |
| | | DK 2239273 T3 | 09 December 2013 |
| | | DK 2256134 T3 | 24 February 2014 |
| | | EP 1682581 A1 | 26 July 2006 |
| | | EP 1682581 B1 | 25 April 2012 |
| | | EP 1682582 A1 | 26 July 2006 |
| | | EP 1682582 B1 | 31 August 2011 |
| | | EP 1682583 A1 | 26 July 2006 |
| | | EP 1682583 B1 | 11 January 2012 |

Form PCT/ISA/210 (patent family annex) (July 2019)

**INTERNATIONAL SEARCH REPORT**
**Information on patent family members**

International application No.
**PCT/KR2020/018053**

| Patent document cited in search report | Publication date (day/month/year) | Patent family member(s) | Publication date (day/month/year) |
|---|---|---|---|
| | | EP 1682584 A1 | 26 July 2006 |
| | | EP 1682584 B1 | 17 April 2013 |
| | | EP 2227243 A2 | 15 September 2010 |
| | | EP 2227243 B1 | 13 August 2014 |
| | | EP 2239273 A1 | 13 October 2010 |
| | | EP 2239273 B1 | 09 October 2013 |
| | | EP 2256134 A1 | 01 December 2010 |
| | | EP 2256134 B1 | 08 January 2014 |
| | | ES 2372495 T3 | 20 January 2012 |
| | | ES 2378167 T3 | 09 April 2012 |
| | | ES 2383300 T3 | 20 June 2012 |
| | | ES 2426169 T3 | 21 October 2013 |
| | | ES 2438098 T3 | 15 January 2014 |
| | | ES 2454666 T3 | 11 April 2014 |
| | | ES 2521497 T3 | 12 November 2014 |
| | | HK 1149570 A1 | 07 October 2011 |
| | | HK 1150029 A1 | 28 October 2011 |
| | | HK 1150612 A1 | 06 January 2012 |
| | | HR P20140997 T1 | 19 December 2014 |
| | | IL 205667 A | 30 November 2010 |
| | | IL 205667 B | 30 November 2016 |
| | | JP 2007-531513 A | 08 November 2007 |
| | | JP 2007-532098 A | 15 November 2007 |
| | | JP 2007-536211 A | 13 December 2007 |
| | | JP 2007-537992 A | 27 December 2007 |
| | | JP 2011-256211 A | 22 December 2011 |
| | | JP 2011-505355 A | 24 February 2011 |
| | | JP 2012-001560 A | 05 January 2012 |
| | | JP 2012-224635 A | 15 November 2012 |
| | | JP 4762904 B2 | 31 August 2011 |
| | | JP 4870569 B2 | 08 February 2012 |
| | | JP 5216216 B2 | 19 June 2013 |
| | | JP 5226080 B2 | 03 July 2013 |
| | | JP 5425150 B2 | 26 February 2014 |
| | | KR 10-0725314 B1 | 07 June 2007 |
| | | KR 10-0725315 B1 | 07 June 2007 |
| | | KR 10-0775343 B1 | 08 November 2007 |
| | | KR 10-1135244 B1 | 24 April 2012 |
| | | KR 10-2005-0047030 A | 19 May 2005 |
| | | KR 10-2005-0047031 A | 19 May 2005 |
| | | KR 10-2005-0047032 A | 19 May 2005 |
| | | KR 10-2005-0047033 A | 19 May 2005 |
| | | KR 10-2006-0054252 A | 22 May 2006 |
| | | KR 10-2008-0098216 A | 07 November 2008 |
| | | KR 10-2009-0056796 A | 03 June 2009 |
| | | MX 2010005866 A | 23 June 2010 |
| | | MX PA05007210 A | 10 February 2006 |
| | | MX PA05007211 A | 10 February 2006 |
| | | MY 155454 A | 15 October 2015 |
| | | NZ 585314 A | 26 October 2012 |

Form PCT/ISA/210 (patent family annex) (July 2019)

**INTERNATIONAL SEARCH REPORT**
**Information on patent family members**

International application No.
**PCT/KR2020/018053**

| Patent document cited in search report | Publication date (day/month/year) | Patent family member(s) | Publication date (day/month/year) |
|---|---|---|---|
| | | PL 2227243 T3 | 31 March 2015 |
| | | PL 2239273 T3 | 30 April 2014 |
| | | PL 2256134 T3 | 30 June 2014 |
| | | PT 1682583 E | 13 April 2012 |
| | | PT 2227243 E | 22 October 2014 |
| | | PT 2239273 E | 10 December 2013 |
| | | PT 2256134 E | 05 March 2014 |
| | | RU 2005120239 A | 20 April 2006 |
| | | RU 2005120240 A | 20 April 2006 |
| | | RU 2010126478 A | 10 January 2012 |
| | | RU 2352583 C2 | 20 April 2009 |
| | | RU 2356909 C2 | 27 May 2009 |
| | | RU 2446816 C2 | 10 April 2012 |
| | | TW 200936154 A | 01 September 2009 |
| | | TW I403331 B | 01 August 2013 |
| | | US 10071166 B2 | 11 September 2018 |
| | | US 10272159 B2 | 30 April 2019 |
| | | US 2006-0269553 A1 | 30 November 2006 |
| | | US 2006-0275254 A1 | 07 December 2006 |
| | | US 2006-0276633 A1 | 07 December 2006 |
| | | US 2008-0085862 A1 | 10 April 2008 |
| | | US 2008-0124347 A1 | 29 May 2008 |
| | | US 2009-0238838 A1 | 24 September 2009 |
| | | US 2010-0255014 A1 | 07 October 2010 |
| | | US 2010-0261248 A1 | 14 October 2010 |
| | | US 2010-0330108 A1 | 30 December 2010 |
| | | US 2011-0245472 A1 | 06 October 2011 |
| | | US 2013-0288333 A1 | 31 October 2013 |
| | | US 2015-0025227 A1 | 22 January 2015 |
| | | US 2015-0025228 A1 | 22 January 2015 |
| | | US 2018-0326083 A1 | 15 November 2018 |
| | | US 2019-0269787 A1 | 05 September 2019 |
| | | US 7736653 B2 | 15 June 2010 |
| | | US 7737260 B2 | 15 June 2010 |
| | | US 8029789 B2 | 04 October 2011 |
| | | US 8110665 B2 | 07 February 2012 |
| | | US 8263084 B2 | 11 September 2012 |
| | | US 8822650 B2 | 02 September 2014 |
| | | US 8846874 B2 | 30 September 2014 |
| | | US 9750820 B2 | 05 September 2017 |
| | | WO 2005-047334 A1 | 26 May 2005 |
| | | WO 2005-047335 A1 | 26 May 2005 |
| | | WO 2005-047336 A1 | 26 May 2005 |
| | | WO 2005-047337 A1 | 26 May 2005 |
| | | WO 2008-136611 A1 | 13 November 2008 |
| | | WO 2009-069983 A2 | 04 June 2009 |
| | | WO 2009-069983 A3 | 20 August 2009 |
| | | ZA 201003796 B | 23 February 2011 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5416017 A
- JP 1287041 A
- JP 2000213 A

### Non-patent literature cited in the description


- **N.S. NIGHTLINGER et al.** *Proceed. Intern. Symp. Control. Rel. Bioact. Mater.,* 1995
- **L. ILUM et al.** *J. Controlled Rel.,* 1994, vol. 29, 133